(19) Europäisches Patentamt
European Patent Office
Office européen des brevets

(11) EP 3 967 695 B1

(12) EUROPEAN PATENT SPECIFICATION

(45) Date of publication and mention of the grant of the patent: **17.01.2024 Bulletin 2024/03**

(21) Application number: **20796227.5**

(22) Date of filing: **20.04.2020**

(51) International Patent Classification (IPC):
***C07D 471/10*** (2006.01) ***C07D 487/10*** (2006.01)
***C07D 519/00*** (2006.01) ***C07D 451/04*** (2006.01)
***C07D 401/14*** (2006.01) ***A61K 31/517*** (2006.01)
***A61P 35/00*** (2006.01)

(52) Cooperative Patent Classification (CPC):
**A61P 35/00; C07D 401/14; C07D 451/04; C07D 471/10; C07D 487/10; C07D 519/00**

(86) International application number:
**PCT/CN2020/085704**

(87) International publication number:
**WO 2020/216190 (29.10.2020 Gazette 2020/44)**

(54) **1-(7-(QUINAZOLIN-4-YL)-2,7-DIAZASPIRO[3.5]NONAN-2-YL)PROP-2-EN-1-ONE DERIVATIVES AS KRAS INHIBITORS FOR THE TREATMENT OF CANCER**

1-(7-(QUINAZOLIN-4-YL)-2,7-DIAZASPIRO[3.5]NONAN-2-YL)PROP-2-EN-1-ON-DERIVATE ALS KRAS-INHIBITOREN ZUR BEHANDLUNG VON KREBS

DÉRIVÉS DE 1-(7-(QUINAZOLIN-4-YL)-2,7-DIAZASPIRO[3.5]NONAN-2-YL)PROP-2-EN-1-ONE EN TANT QU'INHIBITEURS KRAS POUR LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2019 PCT/CN2019/083677**
**31.05.2019 PCT/CN2019/089397**

(43) Date of publication of application:
**16.03.2022 Bulletin 2022/11**

(73) Proprietor: **Betta Pharmaceuticals Co., Ltd**
**Yuhang**
**Hangzhou**
**Zhejiang 311100 (CN)**

(72) Inventors:
- **WU, Hao**
**Hangzhou, Zhejiang 311100 (CN)**
- **LU, Yuan**
**Hangzhou, Zhejiang 311100 (CN)**
- **YU, Jun**
**Hangzhou, Zhejiang 311100 (CN)**
- **ZHOU, Xiao**
**Hangzhou, Zhejiang 311100 (CN)**
- **LI, Boyan**
**Hangzhou, Zhejiang 311100 (CN)**
- **HE, Jiangqi**
**Hangzhou, Zhejiang 311100 (CN)**
- **FU, Shuibiao**
**Hangzhou, Zhejiang 311100 (CN)**
- **YANG, Rongwen**
**Beijing 100176 (CN)**
- **LI, Yabin**
**Beijing 100176 (CN)**
- **WANG, Chao**
**Beijing 100176 (CN)**
- **WANG, Jiabing**
**Beijing 100176 (CN)**
- **LAN, Hong**
**Beijing 100176 (CN)**
- **DING, Lieming**
**Hangzhou, Zhejiang 311100 (CN)**

(74) Representative: **Pfenning, Meinig & Partner mbB**
**Patent- und Rechtsanwälte**
**Theresienhöhe 11a**
**80339 München (DE)**

(56) References cited:
**WO-A1-2018/143315 CN-A- 106 488 910**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

CN-A- 107 849 022
CN-A- 108 779 097

## Description

### Technical field

The present invention relates to a novel compound, which has cancer therapeutic activity. The present invention also relates to preparation methods for these compounds and pharmaceutical compositions containing them.

### Background technique

RAS protein is a low-molecular-weight guanosine triphosphate (GTP) binding protein with only one polypeptide chain. It includes two conformations: an active GTP binding conformation and an inactive GDP binding conformation. Under certain conditions, they can be transformed into each other to form the RAS cycle and regulate the activation of multiple downstream signaling pathways. The most important ones include the RAF-MEK-ERK and PI3K-AKT-mTOR signaling pathways. RAS is known as "molecular switch" of the transmission of cellular signaling network. Under normal circumstances, RAS is in an inactive state combined with GDP, and RAS is activated after receiving upstream signal stimulation, and the signal chain is only temporarily active. However, when RAS is mutated, the frequency of exchange between RAS and GDP/GTP is accelerated. RAS can bind to GTP for a long time, so that RAS and downstream signals are activated for a long time, and cell proliferation is out of control, leading to malignant transformation of cells. Clinical data shows that RAS is the gene with the highest mutation rate in human tumors. About 20-30% of all tumors have RAS mutations, about 98% of pancreatic cancer, 52% of colon cancer, 43% of multiple myeloma, and 32% of lung adenocarcinomas have RAS gene mutations. The most common mode of RAS mutation is point mutation, which often occurs in codons 12, 13, and 61, among which codon 12 is the most common mutation. KRAS-G12C mutations account for approximately 10-20% of KRAS mutations and 14% of non-small cell lung cancers.

It is very difficult to find drugs that target RAS. Due to the strong binding ability of GTP and RAS, it is difficult to find small molecules that can competitively inhibit their binding, and the surface of the RAS protein is very smooth, and the structure lacks structural space for small molecules or drugs to bind. For more than 30 years, there has been no breakthrough in finding drugs that specifically target KRAS. Therefore, KRAS is generally regarded as an "Undruggable Target" protein target.

In recent years, after the druggability of KRAS-G12C was discovered, KRAS-G12C inhibitors have become one of the current hot areas of drug research and development. In 2013, the University of California found that a common mutant KRAS-G12C protein of KRAS, after binding to GDP, forms a new pocket on the molecular surface through protein crystallography research. Small molecule inhibitors can cooperate with KRAS-G12C protein with valence binds at this site to lock the protein in an inactive state. KRAS-G12C inhibitors that are currently undergoing rapid progress mainly include ARS-1620 from Araxes, AMG-510 from Amgen and MRTX-849 from Mirati. Among them, AMG-510 has made the fastest progress. It started phase I clinical trials in 2018 and was the first KRAS-G12C inhibitor to enter clinical trials.

WO2018/143315A1 relates to a pharmaceutical composition, a G12C mutant KRAS inhibitor, and a quinazoline compound expected to be useful as an active ingredient of a pharmaceutical composition for treating lung cancer. CN108779097A, CN107849022A and CN106488910A relate to compounds having activity as inhibitors of G12C mutant KRAS protein, methods associated with preparation and use of such compounds, pharmaceutical compositions comprising such compounds and methods to modulate the activity of G12C mutant KRAS protein for treatment of disorders, such as cancer.

The present invention provides a KRAS inhibitor with a novel structure, which can regulate the activity of G12C mutant KRAS protein and has good anti-tumor activity.

### Summary

The invention is set out in the appended set of Claims.

The present invention provides a compound represented by general formula (I), or a tautomer, pharmaceutically acceptable salt, solvate, chelate, non-covalent complex thereof,

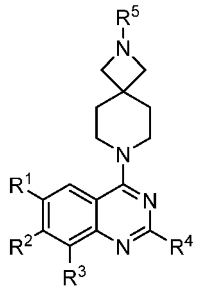

(I)

wherein,

$R^1$ is selected from the group consisting of $C_{1-6}$ alkyl, substituted $C_{1-6}$ alkyl, halogen, $C_{2-6}$ alkenyl, substituted $C_{2-6}$ alkenyl, $C_{3-6}$ cycloalkyl and substituted $C_{3-6}$ cycloalkyl;
$R^2$ is 6-10 membered heteroaryl or substituted 6-10 membered heteroaryl, wherein the 6-10 membered heteroaryl independently contains 1, 2 and 3 heteroatom selected from the group consisting of N, O and S;
$R^3$ is selected from the group consisting of H, amino, cyano, halogen, hydroxy, $C_{1-6}$ alkyl, substituted $C_{1-6}$ alkyl, $C_{3-8}$ cycloalkyl, substituted $C_{3-8}$ cycloalkyl, $C_{1-6}$ alkoxy, substituted $C_{1-6}$ alkoxy, $C_{3-8}$ cycloalkyloxy, substituted $C_{3-8}$ cycloalkyloxy and cyano substituted cyclopropyl $C_{1-6}$ alkyleneoxy;
$R^4$ is

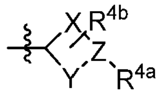
;

wherein X and Y are independently $C_{1-2}$ alkylene;
Z is selected from the group consisting of CH, N and O;
$R^{4a}$ and $R^{4b}$ are independently absent or are selected from the group consisting of H, $C_{1-6}$ alkyl, substituted $C_{1-6}$ alkyl, acryloyl, $C_{3-6}$ cycloalkyl, substituted $C_{3-6}$ cycloalkyl, 3-8 membered heterocyclyl, substituted 3-8 membered heterocyclyl and -(C=O) $C_{1-6}$ alkyl, wherein the 3-8 membered heterocyclyl independently contains 1, 2 and 3 heteroatoms independently selected from the group consisting of N, O and S;
$R^5$ is unsubstituted or substituted acryloyl.

In one aspect, $R^1$ in formula (I) is selected from the group consisting of $C_{1-3}$ alkyl, $C_{3-6}$ cycloalkyl, halogen and $C_{2-4}$ alkenyl.
In one aspect, $R^1$ in formula (I) is selected from the group consisting of ethyl, cyclopropyl, Cl and vinyl.
In one aspect, $R^2$ in formula (I) is indazolyl or $C_{1-3}$ alkyl substituted indazolyl.
In one aspect, $R^2$ in formula (I) is methyl substituted indazolyl.
In one aspect, $R^2$ in formula (I) is

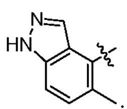
.

In one aspect, $R^3$ in formula (I) is selected from the group consisting of H, $C_{1-3}$ alkyl, halogen, cyano-substituted cyclopropyl $C_{1-3}$ alkyleneoxy and $-OR^{3a}$, wherein $R^{3a}$ is $C_{1-6}$ alkyl or $C_{3-8}$ cycloalkyl, $R^{3a}$ is independently unsubstituted or further substituted with one or more substituents selected from the group consisting of hydroxyl, halogen, $C_{1-3}$ alkyl, $C_{1-3}$ alkoxy and cyclopropyl.
In one aspect, $R^3$ in formula (I) is selected from the group consisting of H, F, methyl,

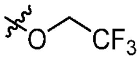
,

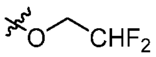, 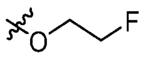, 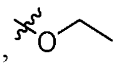, 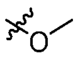, 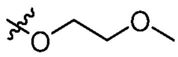, 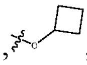, 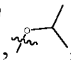, 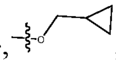, 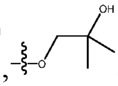 and 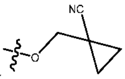.

In one aspect, $R^{4a}$ in formula (I) is absent or is selected from the group consisting of H, acryloyl, $C_{1-3}$ alkyl, $C_{4-6}$ cycloalkyl,

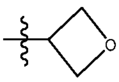, 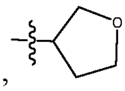, 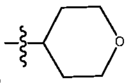, 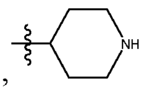 and 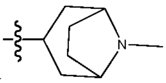,

wherein any of the $C_{1-3}$ alkyl, $C_{4-6}$ cycloalkyl ,

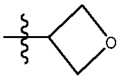, 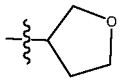, 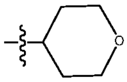, 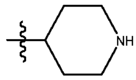 or 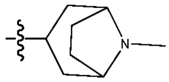

is unsubstituted or substituted with one or more substituents selected from the group consisting of halogen, hydroxyl, cyano, =O, $C_{1-3}$ alkyl, $-S(O_2)C_{1-3}$ alkyl, $C_{1-3}$ alkoxy, $C_{1-3}$ halo-alkyl, cyclopropyl, cyclobutyl, halocyclopropyl, halocyclobutyl, phenyl, $C_{1-3}$ alkyl substituted pyrazolyl, $C_{1-6}$ acyl and

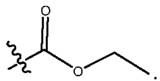.

In one aspect, $R^{4a}$ in formula (I) is independently absent or is selected from the group consisting of H, methyl, ethyl, propyl, acetyl,

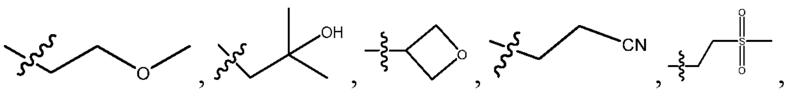

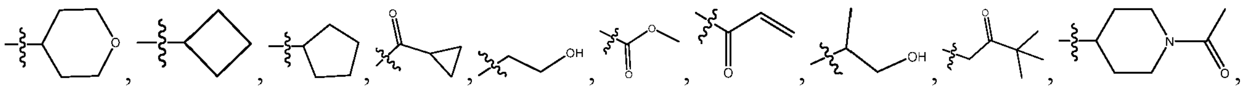

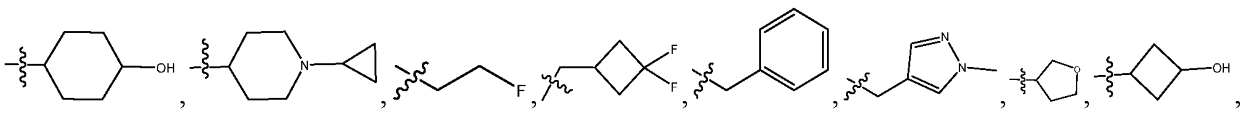

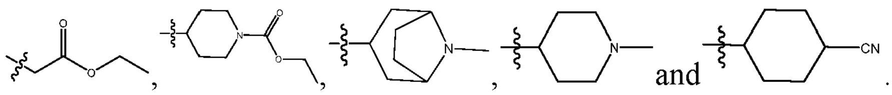

In one aspect, $R^{4b}$ in formula (I) is independently absent, H or $C_{1-3}$ alkyl.

In one aspect, $R^4$ in formula (I) is

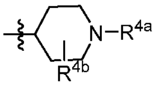, 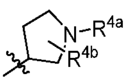, 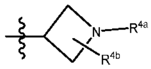, 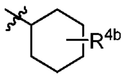, 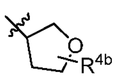

or

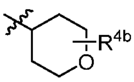.

In one aspect, $R^4$ in formula (I) is

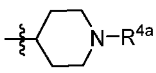, 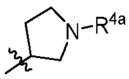, 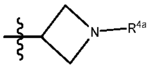, 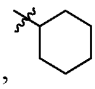, 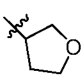 or 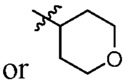.

In one aspect, $R^5$ in formula (I) is unsubstituted or halo substituted

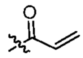.

In one aspect, $R^5$ in formula (I) is

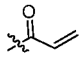 or 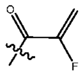.

In one aspect, a compound of formula (I), a stereoisomer or a pharmaceutically acceptable salt, which is independently selected from compounds of formula (IA) to formula (ID),

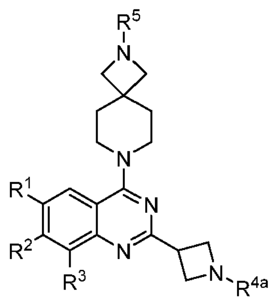

(IA)

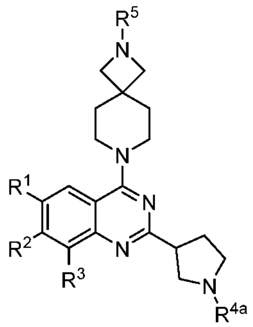

(IB)

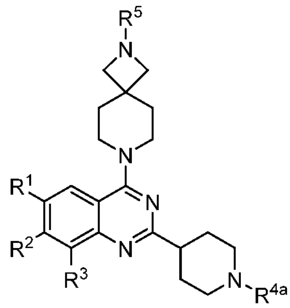

(IC)

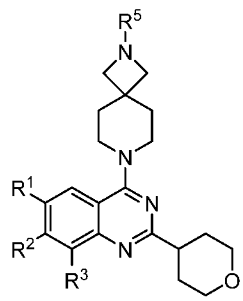

(ID)

wherein the substituents are as defined in formula (I).

The present invention further provides a compound, a tautomer or a pharmaceutically acceptable salt, wherein any of the compound is selected from the group consisting of:

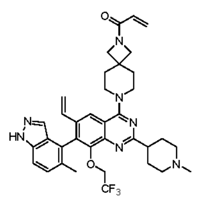

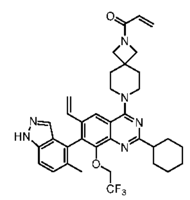

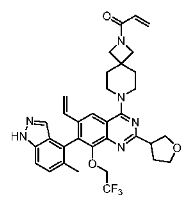

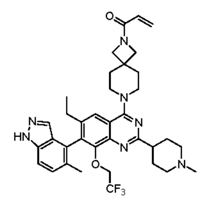

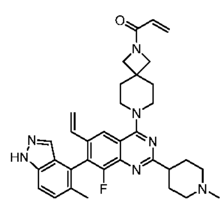

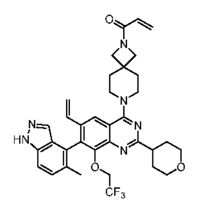

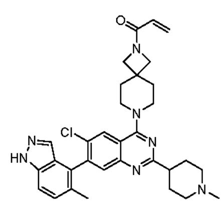

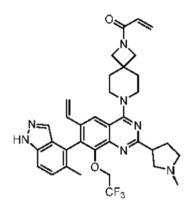

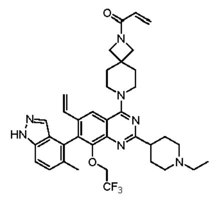
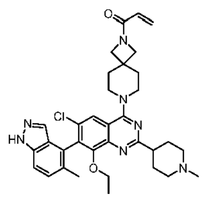
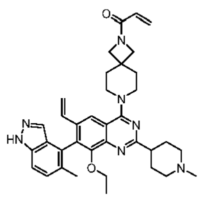
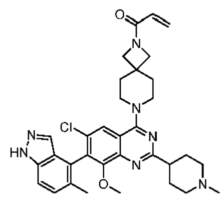

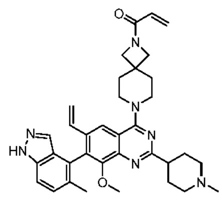
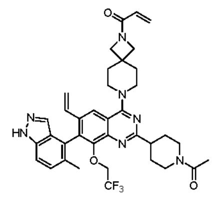
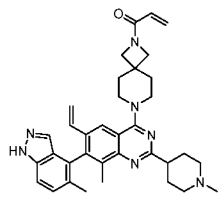
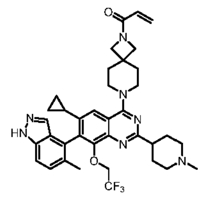

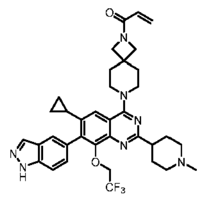
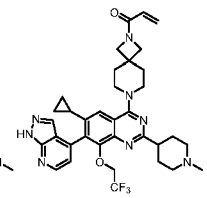
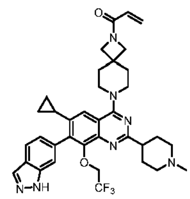
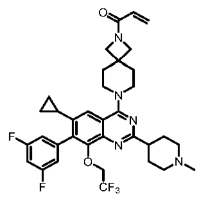

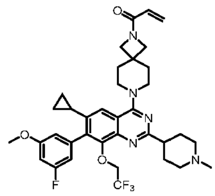
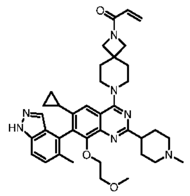
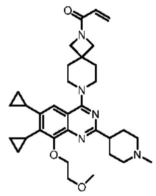
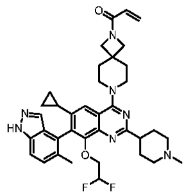

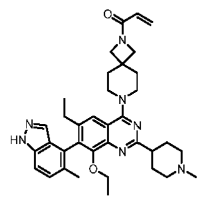
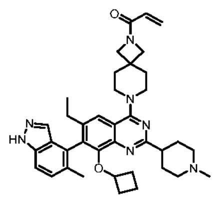
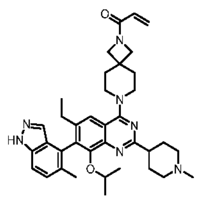
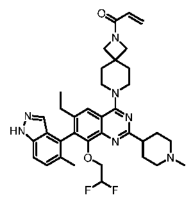

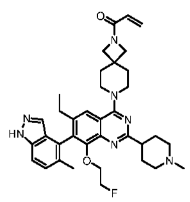
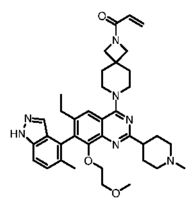
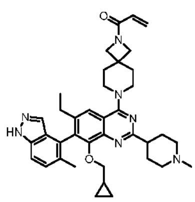
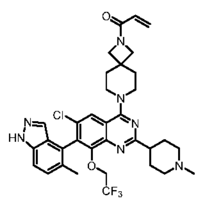

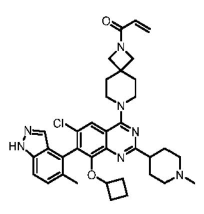
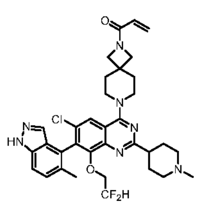
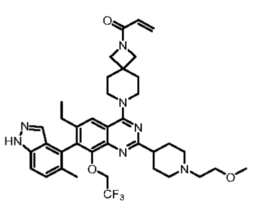
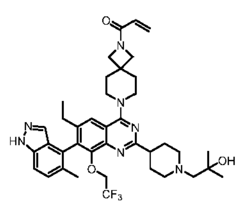

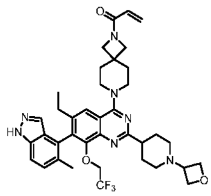 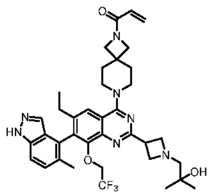 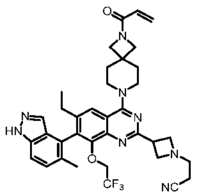 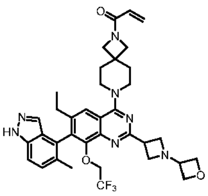

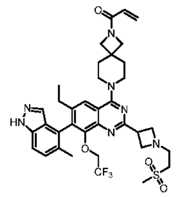 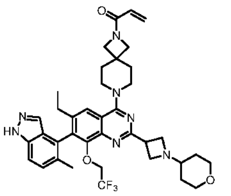 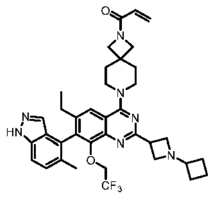 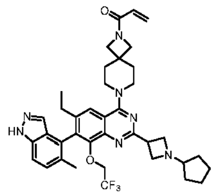

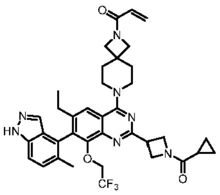 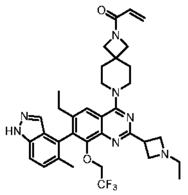 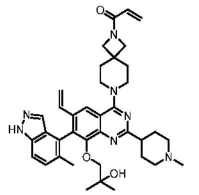 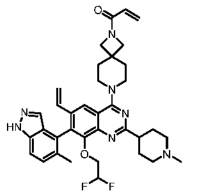

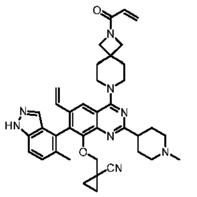 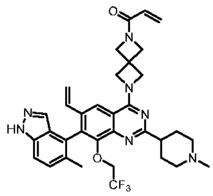 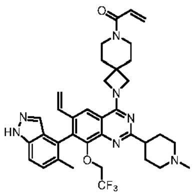 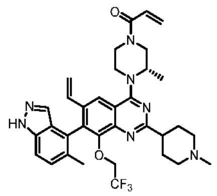

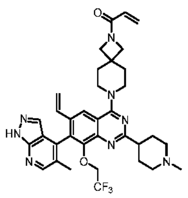 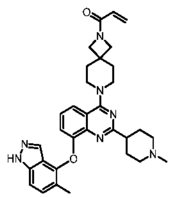 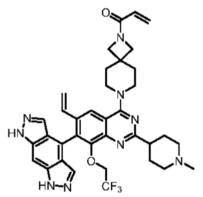 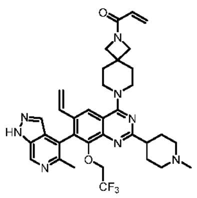

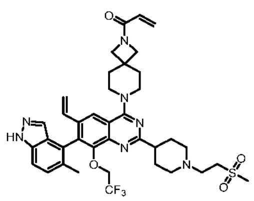 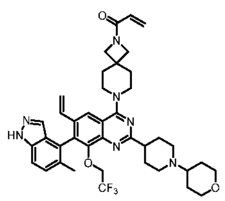 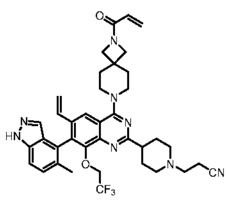 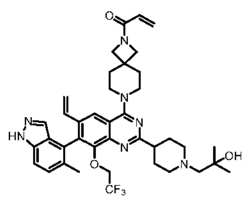

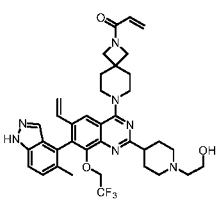 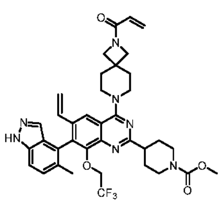 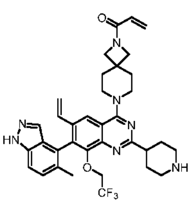 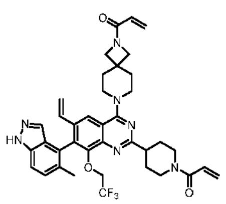

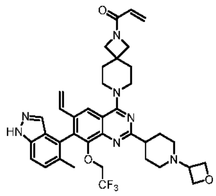 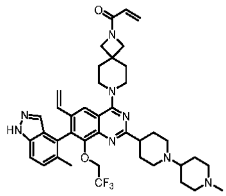 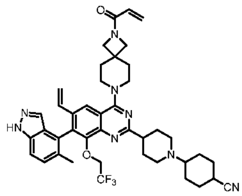 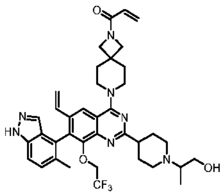

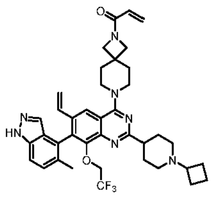 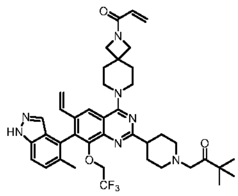 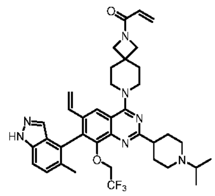 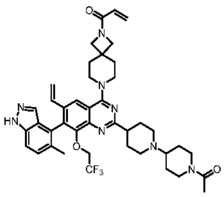

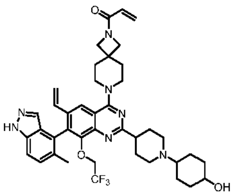 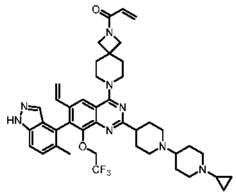 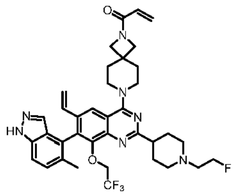 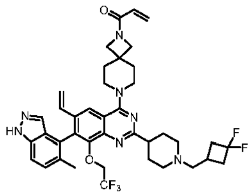

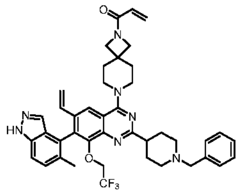 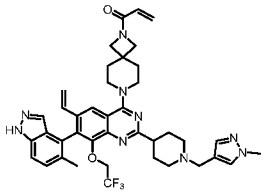 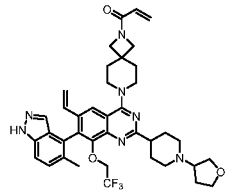 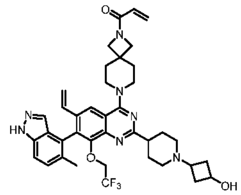

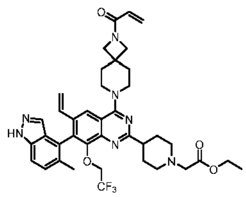 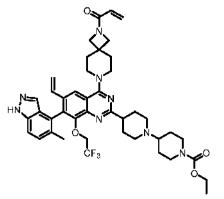 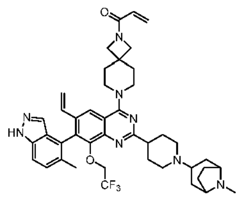 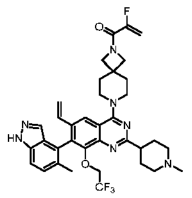

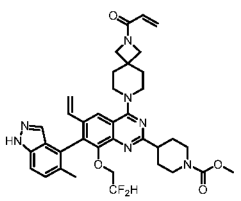 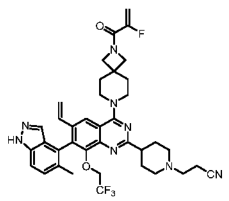 and 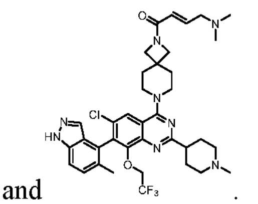 .

The present invention also provides a pharmaceutical composition, comprising a therapeutically effective amount of at least one compound represented by formula (I) and at least one pharmaceutically acceptable carrier.

The present invention further provides a pharmaceutical composition, wherein the weight ratio of the compound represented by formula (I) to the pharmaceutically acceptable carrier is 0.0001:1-10.

The present invention provides the compound or pharmaceutical composition represented by the structural formula (I) for use as a medicament.

The present invention further provides a preferred technical solution:

In a further embodiment, the compound or pharmaceutical composition represented by the structural formula (I) is for use as a medicament for treating and/or preventing cancer.

In a further embodiment, the compound or pharmaceutical composition represented by the structural formula

(I) is for use in treating diseases mediated by KRAS G12C.

In a further embodiment, the disease is cancer.

In a further embodiment, the cancer is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophagus cancer, melanoma, colorectal cancer, hepatocellular carcinoma, head and neck tumor, hepatobiliary cell carcinoma, myelodysplastic syndrome, malignant glioma, prostate cancer, thyroid cancer, xuwang's cell tumor, lung squamous cell carcinoma, lichenoid keratosis, synovial sarcoma, skin cancer, pancreatic cancer, testicular cancer and liposarcoma.

Unless otherwise specified, the general chemical terms used in the general structural formula have their usual meanings.

For example, unless otherwise specified, the term "halogen" used in the present invention refers to fluorine, chlorine, bromine or iodine.

In the present invention, unless otherwise specified, "alkyl" will be understood to mean a linear or branched monovalent saturated hydrocarbon group. For example, alkyl includes methyl, ethyl, propyl, isopropyl, n-butyl, isobutyl, sec-butyl, tert-butyl, n-pentyl, 3-(2-methyl)butyl, 2-pentyl, 2-methylbutyl, neopentyl, n-hexyl, 2-hexyl, 2-methylpentyl, etc. Similarly, the "$_{1-8}$" in "$C_{1-8}$ alkyl" refers to a straight-chain or branched group containing 1, 2, 3, 4, 5, 6, 7 or 8 carbon atoms.

"$C_{1-2}$ alkylene" refers to methylene or 1,2-ethylene.

"Alkoxy" refers to the oxyether form of the aforementioned linear or branched alkyl group, which is -O-alkyl.

$C_{3-8}$ cycloalkyloxy" refers to -O-($C_{3-8}$ cycloalkyl).

"Cyclopropyl $C_{1-6}$ alkyleneoxy" refers to -O-$C_{1-6}$ alkylene-cyclopropyl.

In the present invention, "a", "an", "the", "at least one" and "one or more" can be used interchangeably. Thus, for example, a composition comprising "a" pharmaceutically acceptable excipient can be interpreted to mean that the composition includes "one or more" pharmaceutically acceptable excipients.

The term "aryl" in the present invention, unless otherwise specified, will be understood to mean an monocyclic or condensed ring aromatic group including carbon ring atoms. In a further embodiment, the aryl group is a 6 to 10 membered monocyclic or bicyclic aromatic ring group. In a further embodiment, it is phenyl and naphthyl. Most preferred is phenyl.

The term "heterocyclyl", as used herein, unless otherwise specified, will be understood to mean an 3-8 membered stable monocyclic ring consisting of carbon atoms and 1-3 heteroatoms selected from N, O or S. The system in which nitrogen or sulfur heteroatoms can be selectively oxidized, and nitrogen heteroatoms can be selectively quaternized. The heterocyclyl can be attached to any heteroatom or carbon atom to form a stable structure. Examples of these heterocyclyls include, but are not limited to azetidinyl, pyrrolidinyl, piperidinyl, piperazinyl, oxopiperazinyl, oxopiperidinyl, tetrahydrofuranyl, dioxolane, tetrahydroimidazolyl, tetrahydrothiazolyl, tetrahydrooxazolyl, tetrahydropyranyl, morpholinyl, thiomorpholinyl, thiomorpholinyl sulfoxide, thiomorpholinylsulfone and tetrahydro oxadiazolyl.

The term "heteroaryl", as used herein, unless otherwise specified, will be understood to mean an stable 5- or 6-membered monocyclic aromatic ring system or an 9- or 10-membered benzo-fused heteroaromatic ring system or bicyclic heteroaromatic ring system, which consists of carbon atoms and 1-4 heteroatoms selected from N, O or S, and wherein the nitrogen or sulfur heteroatoms can be selectively oxidized. The nitrogen heteroatoms can be selectively quaternized. The heteroaryl group can be attached to any heteroatom or carbon atom to form a stable structure. Examples of heteroaryl groups include, but are not limited to thienyl, furyl, imidazolyl, isoxazolyl, oxazolyl, pyrazolyl, pyrrolyl, thiazolyl, thiadiazolyl, triazolyl, pyridyl, pyridyl azinyl, indolyl, azaindolyl, indazolyl, benzimidazolyl, benzofuranyl, benzothienyl, benzisoxazolyl, benzothiazolyl, benzothiazolyl, benzene and thiadiazolyl, benzotriazolyl adenine, quinolinyl or isoquinolinyl.

The term "cycloalkyl" will be understood to mean a cyclic saturated alkyl chain having 3-10 carbon atoms, for example, cyclopropyl, cyclobutyl, cyclopentyl or cyclohexyl.

The term "substituted" will be understood to mean that one or more hydrogen atoms in the group are replaced by the same or different substituents. Typical substituents include, but are not limited to halogen (F, Cl, Br or I), Ci-s alkyl, $C_{3-12}$ cycloalkyl, -ORi, -$SR_1$, =O, =S, -C(O)$R_1$ , -C(S)$R_1$, =$NR_1$, - C(O)$OR_1$, -C(S)ORi, -$NR_1R_2$, -C(O)$NR_1R_2$, cyano, nitro, -$S(O)_2R_1$, -OS ($O_2$) $OR_1$, -$OS(O)_2R_1$, - OP(O)($OR_1$)($OR_2$). Wherein $R_1$ and $R_2$ are independently selected from -H, $C_{1-6}$ alkyl, and $C_{1-6}$ haloalkyl. In a further aspect, the substituents are independently selected from the group comprising -F, -Cl, -Br, -I, -OH, trifluoromethoxy, ethoxy, propoxy, isopropoxy, n-butoxy group, isobutoxy, tert-butoxy, -$SCH_3$, -$SC_2H_5$, formaldehyde, -C($OCH_3$), cyano, nitro, -$CF_3$, -$OCF_3$, amino, dimethylamino, methylthio, sulfonyl and acetyl groups.

Examples of substituted alkyl groups include, but are not limited to, 2,3-dihydroxypropyl, 2-aminoethyl, 2-hydroxyethyl, pentachloroethyl, trifluoromethyl, methoxymethyl, pentafluoroethyl , phenylmethyl, dioxenylmethyl and piperazinylmethyl.

Examples of substituted alkoxy groups include, but are not limited to, 2-hydroxyethoxy, 2-fluoroethoxy, 2,2-difluoroethoxy, 2-methoxyethoxy, 2-aminoethoxy, 2,3-dihydroxypropoxy, cyclopropylmethoxy, aminomethoxy, trifluoromethoxy, 2-diethylaminoethoxy, 2-ethoxycarbonylethoxy, 3- hydroxypropoxy.

The term "pharmaceutically acceptable salt" will be understood to mean a salt prepared from a pharmaceutically acceptable non-toxic base or acid. When the compound provided by the present invention is an acid, its corresponding salt can be conveniently prepared from pharmaceutically acceptable non-toxic bases, including inorganic bases and organic bases. Salts derived from inorganic bases include aluminum, ammonium, calcium, copper (high and low prices), ferric, ferrous, lithium, magnesium, manganese (high and low prices), potassium, sodium, zinc and the like. Particularly preferred are ammonium, calcium, magnesium, potassium and sodium salts. Pharmaceutically acceptable non-toxic organic bases that can be derivatized into salts include primary, secondary and tertiary amines, as well as cyclic amines and amines containing substituents, such as naturally occurring and synthetic amines containing substituents. Other pharmaceutically acceptable non-toxic organic bases capable of forming salts, including ion exchange resins and arginine, betaine, caffeine, choline, *N*',*N*'-dibenzylethylenediamine, diethylamine, 2-diethylaminoethanol, 2-dimethylaminoethanol, ethanolamine, ethylenediamine, *N*-ethylmorpholine, *N*-ethylpiperidine, reduced glucosamine, glucosamine, histidine, haamine, isopropylamine , lysine, methyl glucamine, morpholine, piperazine, piperidine, polyamine resin, procaine, purine, theobromine, triethylamine, trimethylamine, tripropylamine, tromethamine, etc.

When the compound provided by the present invention is a base, the corresponding salt can be conveniently prepared from pharmaceutically acceptable non-toxic acids, including inorganic acids and organic acids. Such acids include, for example, acetic acid, benzenesulfonic acid, benzoic acid, camphorsulfonic acid, citric acid, ethanesulfonic acid, isethionic acid, formic acid, fumaric acid, gluconic acid, glutamic acid, hydrobromic acid, Hydroiodic acid, perchloric acid, hydrochloric acid, isethionic acid, propionic acid, glycolic acid, lactic acid, maleic acid, malic acid, mandelic acid, methanesulfonic acid, mucic acid, nitric acid, oxalic acid, hexanoic acid, pantothenic acid, phosphoric acid , succinic acid, sulfuric acid, 2-naphthalenesulfonic acid, cyclohexylamine sulfonic acid, salicylic acid, saccharic acid, trifluoroacetic acid, tartaric acid and *p*-toluenesulfonic acid. Preferably, citric acid, hydrobromic acid, formic acid, hydrochloric acid, maleic acid, phosphoric acid, sulfuric acid and tartaric acid. More preferably, formic acid and hydrochloric acid.

Since the compound represented by formula (I) will be used as a medicine, it is preferable to use a certain purity, for example, at least 60% purity, more suitable purity is at least 75%, and particularly suitable purity is at least 98% (% is weight ratio).

Obviously, the definition of any substituent or variable at a specific position in a molecule is independent of other positions in the molecule. It is easy to understand that those skilled in the art can select the substituents or substituted forms of the compounds of the present invention through the existing technical means and the methods described in the present invention to obtain chemically stable and easy-to-synthesize compounds.

The compound of the present invention may contain one or more asymmetric centers, and may produce diastereomers and optical isomers from this. The present invention includes all possible diastereomers and their racemic mixtures, their substantially pure resolved enantiomers, all possible geometric isomers and their pharmaceutically acceptable salts.

The above formula (I) does not exactly define the three-dimensional structure of a certain position of the compound. The present invention includes all stereoisomers of the compound represented by formula (I) and pharmaceutically acceptable salts thereof. Further, mixtures of stereoisomers and specific isolated stereoisomers are also included in the present invention. In the synthetic process of preparing such compounds, or in the process of racemization or epimerization known to those skilled in the art, the product obtained may be a mixture of stereoisomers.

When the compound represented by formula (I) has tautomers, unless otherwise stated, the present invention includes any possible tautomers, pharmaceutically acceptable salts thereof, and mixtures thereof.

When the compound represented by formula (I) and its pharmaceutically acceptable salt have solvates or polymorphs, the present invention includes any possible solvates and polymorphs. The type of solvent that forms the solvate is not particularly limited, as long as the solvent is pharmaceutically acceptable. For example, water, ethanol, propanol, acetone and similar solvents can be used.

The term "composition", as used herein, will be understood to mean a product comprising a specified amount of each specified ingredient, and any product produced directly or indirectly from a combination of specified amounts of each specified ingredient. Therefore, pharmaceutical compositions containing the compounds of the present invention as active ingredients and methods for preparing the compounds of the present invention are also part of the present invention. In addition, some crystalline forms of the compound may exist in polymorphs, and this polymorph is included in the present invention. In addition, some compounds can form solvates with water (ie, hydrates) or common organic solvents, and such solvates also fall within the scope of the present invention.

The pharmaceutical composition provided by the present invention includes as an active component a compound represented by formula (I) (or a pharmaceutically acceptable salt thereof), a pharmaceutically acceptable excipient and other optional therapeutic components or accessories. Although in any given case, the most suitable way of administering the active ingredient depends on the particular subject to be administered, the nature of the subject and the severity of the disease, the pharmaceutical composition of the present invention includes oral, rectal, topical and a pharmaceutical composition for parenteral administration (including subcutaneous administration, intramuscular injection, and intravenous administration). The pharmaceutical composition of the present invention can be conveniently prepared in a unit dosage form known in the art and prepared by any preparation method known in the pharmaceutical field.

In fact, according to conventional drug mixing technology, the compound represented by formula (I) of the present invention, or pharmaceutically acceptable salt, can be used as an active component and mixed with a drug carrier to form a drug combination Things. The pharmaceutical carrier can take various forms, depending on the desired mode of administration, for example, oral or injection (including intravenous injection). Therefore, the pharmaceutical composition of the present invention may adopt a separate unit suitable for oral administration, such as a capsule, cachet or tablet containing a predetermined dose of the active ingredient. Further, the pharmaceutical composition of the present invention may take the form of powder, granule, solution, aqueous suspension, non-aqueous liquid, oil-in-water emulsion, or water-in-oil emulsion. In addition, in addition to the common dosage forms mentioned above, the compound represented by formula (I) or a pharmaceutically acceptable salt thereof can also be administered by a controlled release method and/or a delivery device. The pharmaceutical composition of the present invention can be prepared by any pharmaceutical method. Generally, this method includes the step of associating the active ingredient with the carrier which constitutes one or more necessary ingredients. In general, the pharmaceutical composition is prepared by uniformly and intimately mixing the active ingredient with a liquid carrier or a finely divided solid carrier or a mixture of both. In addition, the product can be easily prepared into the desired appearance.

Therefore, the pharmaceutical composition of the present invention includes a pharmaceutically acceptable carrier and a compound represented by formula (I) or its stereoisomers, tautomers, polymorphs, solvates, and pharmaceutically acceptable salt. The combination of the compound represented by formula (I) or its pharmaceutically acceptable salt, and one or more other compounds with therapeutic activity is also included in the pharmaceutical composition of the present invention.

The drug carrier used in the present invention can be, for example, a solid carrier, a liquid carrier or a gas carrier. Solid carriers include, but are not limited to lactose, gypsum powder, sucrose, talc, gelatin, agar, pectin, acacia, magnesium stearate, and stearic acid. Liquid carriers include but are not limited to syrup, peanut oil, olive oil and water. Gas carriers include but are not limited to carbon dioxide and nitrogen. When preparing oral pharmaceutical preparations, any pharmacologically convenient medium can be used. For example, water, ethylene glycol, oils, alcohols, flavoring agents, preservatives, coloring agents, etc. can be used for oral liquid preparations such as suspensions, elixirs and solutions; and carriers such as starches, sugars, microcrystalline cellulose, diluents, granulating agents, lubricants, binders, disintegrating agents, etc. can be used in oral solid preparations such as powders, capsules and tablets. In view of ease of administration, tablets and capsules are preferred for oral preparations, and solid pharmaceutical carriers are used here. Alternatively, standard aqueous or non-aqueous formulation techniques can be used for tablet coating.

The tablet containing the compound or pharmaceutical composition of the present invention can be compressed or molded, and independently, can be made into a tablet together with one or more auxiliary components or adjuvants. The active ingredient is in a free-flowing form such as powder or granules, mixed with a binder, lubricant, inert diluent, surfactant or dispersant, and compressed in a suitable machine to prepare compressed tablets. The powdered compound or pharmaceutical composition is soaked with an inert liquid diluent, and then molded in a suitable machine to make a molded tablet. Preferably, each tablet contains about 0.05 mg to 5 g of active ingredient, and each cachet or capsule contains about 0.05 mg to 5 g of active ingredient. For example, a formulation intended for oral administration to humans contains about 0.5 mg to about 5 g of active ingredient, compounded with a suitable and convenient metering auxiliary material, which accounts for about 5% to 95% of the total pharmaceutical composition. The unit dosage form generally contains about 1 mg to about 2 g of the active ingredient, typically 25 mg, 50 mg, 100 mg, 200 mg, 300 mg, 400 mg, 500 mg, 600 mg, 800 mg or 1000 mg.

The pharmaceutical composition suitable for parenteral administration provided by the present invention can be prepared as an aqueous solution or suspension by adding active components into water. A suitable surfactant such as hydroxypropyl cellulose may be included. In glycerol, liquid polyethylene glycol, and their mixture in oil, dispersion systems can also be prepared. Further, a preservative may also be included in the pharmaceutical composition of the present invention to prevent the growth of harmful microorganisms.

The present invention provides pharmaceutical compositions suitable for injection, including sterile aqueous solutions or dispersion systems. Further, the above-mentioned pharmaceutical composition can be prepared into a sterile powder form for immediate preparation of sterile injection or dispersion. In any case, the final injection form must be sterile, and for easy injection, it must be easy to flow. In addition, the pharmaceutical composition must be stable during preparation and storage. Therefore, it is preferable that the pharmaceutical composition be stored under conditions of anti-microbial contamination such as bacteria and fungi. The carrier can be a solvent or dispersion medium, for example, water, ethanol, polyol (such as glycerol, propylene glycol, liquid polyethylene glycol), vegetable oil, and suitable mixtures thereof.

The pharmaceutical composition provided by the present invention may be in a form suitable for topical administration, for example, aerosol, emulsion, ointment, lotion, dusting powder or other similar dosage forms. Further, the pharmaceutical composition provided by the present invention can be in a form suitable for use in a transdermal drug

delivery device. These preparations can be prepared by using the compound represented by formula (I) of the present invention, or a pharmaceutically acceptable salt thereof, through conventional processing methods. As an example, a cream or ointment is prepared by adding about 5 wt% to 10 wt% of a hydrophilic material and water to produce a cream or ointment with the desired consistency.

The pharmaceutical composition provided by the present invention may use a solid as a carrier and is suitable for rectal administration. A unit dose suppository is the most typical dosage form. Suitable auxiliary materials include cocoa butter and other materials commonly used in the art. Suppositories can be conveniently prepared by mixing the pharmaceutical composition with softened or melted auxiliary materials, then cooling and molding.

In addition to the above-mentioned adjuvant components, the above formulations may also include, as appropriate, one or more additional adjuvant components, such as diluents, buffers, flavoring agents, binders, surfactants, thickeners, lubricants and preservatives (including antioxidants), etc. Further, other adjuvants may also include penetration enhancers that regulate the isotonic pressure of the drug and blood. The pharmaceutical composition containing the compound represented by formula (I), or a pharmaceutically acceptable salt thereof, can be prepared in the form of a powder or a concentrated solution.

In general, to treat the conditions or discomforts shown above, the dose level of the drug is about 0.01 mg/kg body weight to 150 mg/kg body weight per day, or 0.5 mg to 7 g per patient per day. For example, inflammation, cancer, psoriasis, allergies/asthma, diseases and discomforts of the immune system, diseases and discomforts of the central nervous system (CNS), the effective treatment drug dosage level is 0.01 mg/kg body weight to 50 mg/kg body weight per day, or 0.5mg to 3.5g per patient per day.

However, it is understood that lower or higher dosages than those mentioned above may be required. The specific dosage level and treatment plan for any particular patient will depend on many factors, including the activity of the specific compound used, age, weight, overall health, gender, diet, administration time, administration route, excretion rate, and the condition of drug combination and the severity of the specific disease being treated.

## Examples

In order to make the above content clearer and clearer, the present invention will use the following embodiments to further illustrate the technical solution of the present invention. The following examples are only used to illustrate specific implementations of the present invention, so that those skilled in the art can understand the present invention.

Unless otherwise specified, all parts and percentages in the present invention are calculated by weight, and all temperatures refer to °C.

The following abbreviations have been used:

BOP: carter condensing agent;
CDI: carbonyl diimidazole;
DBU: 1,8-diazabicyclo[5.4.0]undec-7-ene;
DIEA: N,N-diisopropylethylamine;
DMF: N,N-dimethylformamide;
DCM: dichloromethane;
Dioxane: dioxane;
ESI-MS: electrospray ionization mass spectrometry;
EtOH: ethanol;
HOAc: glacial acetic acid;
MeOH: methanol;
NIS: N-iodosuccinimide;
NCS: N-chlorosuccinimide;
PE:EA: the ratio of petroleum ether to ethyl acetate;
$POCl_3$: Phosphorus oxychloride;
$SOCl_2$: Thionyl chloride;
THF: Tetrahydrofuran;
TFA: trifluoroacetic acid;
TEA: Triethylamine;
Toluene: toluene;
Sphos Pd G2: Chlorine (2-dicyclohexylphosphino-2,6-dimethoxy-1,1-biphenyl)(2-amino-1,1-biphenyl-2-yl)palladium(II);
$Pd(dppf)Cl_2.CH_2Cl_2$: [1,1'-bis(diphenylphosphine)ferrocene] palladium dichloride dichloro methane complex;
$Pd(PPh_3)_4$: tetrakis(triphenylphosphine)palladium;
Pre-TLC: Thin layer chromatography silica gel plate.

## Intermediate M1:

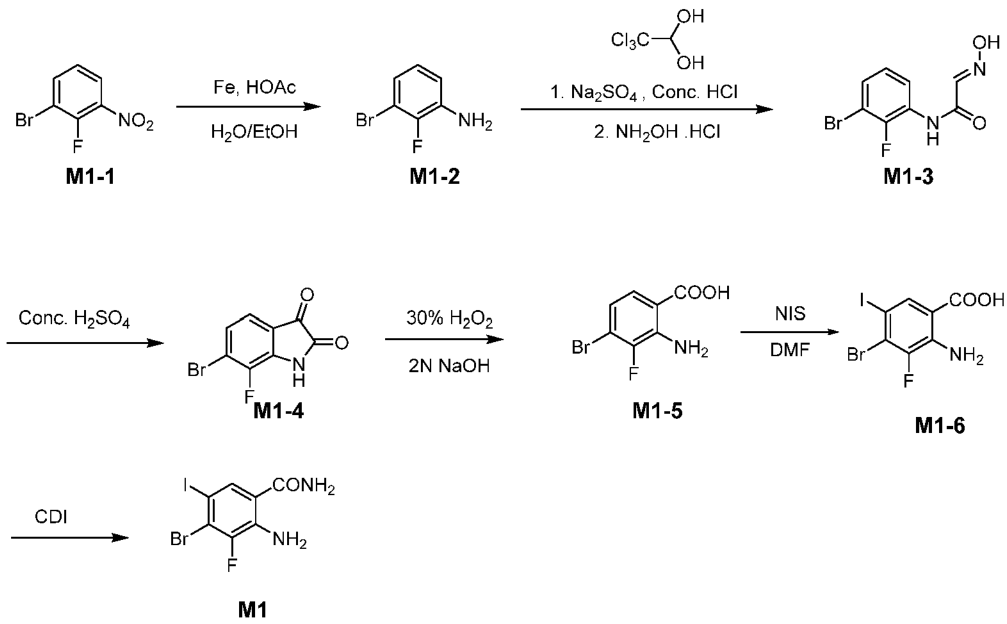

M1-1 M1-2 M1-3 M1-4 M1-5 M1-6 M1

Step 1: Synthesis of compound M1-2

At room temperature, to a mixture of compound M1-1 (40 g, 182.7 mmol), HOAc (76.8 g, 1278.94 mmol), EtOH (400 mL) and $H_2O$ (160 mL) was added iron powder (26.52 g, 475.02 mmol) in portions. The resulting mixture was stirred at room temperature for 2 hours, and then neutralized with NaOH (5 N) solution. The mixture was then extracted with ethyl acetate, the organic layer was washed with brine, dried over $Na_2SO_4$ and concentrated in vacuo to obtain the desired crude product (34 g, 98% yield) as a brown oil, namely compound M1-2. ESI-MS m/z: 190.02$[M+H]^+$.

Step 2: Synthesis of compound M1-3

2,2,2-Trichloroethane-1,1-diol (66.4 g, 401.94 mmol) and $Na_2SO_4$ (503.4 g, 3544.77 mmol) were dissolved in water (560 mL), and then heated to 55°C. Water (240 mL) and 35% HCl (72 mL) containing compound M1-2 (34 g, 182.7 mmol) were added, and an aqueous solution (100 mL) of hydroxylamine hydrochloride (81.4 g, 1171.1 mmol) was added. The resulting mixture was stirred at 90°C for 3 hours and a yellow precipitate formed. The mixture was cooled to room temperature. The solid was collected by filtration, washed with water, and air-dried to obtain a yellow-brown solid product (47 g, 99% yield), namely compound M1-3. ESI-MS m/z: 261.03 $[M+H]^+$.

Step 3: Synthesis of compound M1-4

At 60°C, compound M1-3 (47 g, 180.8 mmol) was added to concentrated sulfuric acid (300 mL), the temperature was increased to 90°C and maintained for 3 hours. The reaction was complete, the reaction mixture was cooled to room temperature and poured into ice water. The yellow precipitate was collected by filtration and dried to obtain a black solid product (43 g, 99% yield), namely compound M1-4.

Step 4: Synthesis of compound M1-5

To a solution of compound M1-4 (43 g, 180.8 mmol) in NaOH (2 N, 500 mL) at 0°C was added $H_2O_2$ solution (30%, 80 mL) and the resulting mixture was stirred at 0°C for 30 minutes. Move to room temperature and stir for 2 hours. The reaction was complete. The mixture was poured into ice water and then acidified with concentrated HCl solution. The precipitate was collected by filtration and air-dried to obtain a white solid product (20 g, 48.9% yield), which was the compound M1-5. ESI-MS m/z: 233.97 $[M+H]^+$.

Step 5: Synthesis of compound M1-6

At room temperature, to a solution of compound M1-5 (20 g, 85.86 mmol) in DMF (200 mL) was added NIS

(29 g, 128.78 mmol) and the resulting mixture was stirred at 70°C overnight. After the reaction was complete, the mixture was poured into ice water, and extracted with ethyl acetate, the organic layer was washed with brine, dried over $Na_2SO_4$ and concentrated in vacuo to obtain the desired crude product (30 g, 98% yield) as a brown solid, namely compound M1-6. ESI-MS m/z: 359.87 $[M+H]^+$. $^1H$ NMR (500MHz, DMSO) δ 13.34 (s, 1H), 7.99 (s, 1H), 6.87 (s, 2H).

Step 6: Synthesis of compound M1

At room temperature, bis (imidazol-1-yl)methanone (2.70 g, 16.67 mmol) was added to the crude compound M1-6 (4.0 g, 11.11 mmol) in THF (20 mL), and the N-ethyl-N-isopropylpropan-2-amine (1.44 g, 11.11 mmol, 1.94 mL) was added to it, and the mixture was moved to 50°C for reaction. After about 2 hours, compound M1-6 was almost completely converted into an intermediate product, and then the mixture was dropped dropwise to iced ammonia (35 mL), stirring for 5 min, and the reaction was complete. The mixture was poured into ice water, and extracted with ethyl acetate, the organic layer was washed with brine, dried over $Na_2SO_4$ and concentrated in vacuo. The residue was purified by flash silica gel column chromatography (petroleum ether/ethyl acetate=70:30) to give a brown target product compound M1 (1.64 g) as a solid.

**Synthesis of intermediate compound M2:**

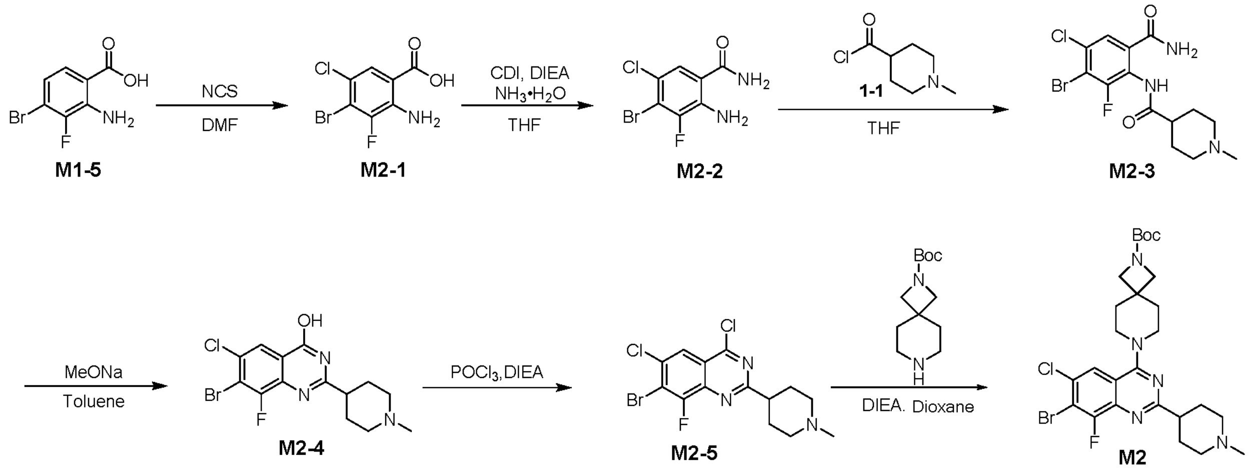


M1-5 M2-1 M2-2 1-1 M2-3 M2-4 M2-5 M2

Step 1: Synthesis of compound M2-1

At room temperature, the compound M1-5 (3.6 g, 13.93 mmo1) was dissolved in DMF (20 mL), NCS (2.05 g, 15.32 mmo1) was added, and the reaction was carried out at 70°C for 1.5 h. After the reaction was complete, it was cooled to room temperature, diluted with water, and extracted three times with EA. The organic phases were combined and dried over anhydrous $Na_2SO_4$, filtered, and spin-dried to obtain a brown solid M2-1 (3.86 g, crude product). ESI-MS m/z: 267.91$[M+H]^+$.

Step 2: Synthesis of compound M2-2

At room temperature, dissolve compound M2-1 (3.7 g, 13.78 mmo1) in THF (20 mL), CDI (2.98 g, 20.67 mmol) and DIEA (5.34 g, 41.35 mmol) were added, and the mixture was reacted at 50°C for 1.5 h. The reaction solution was dropped into ammonia (40 mL) under ice bath conditions, and the reaction was complete after stirring for 5 min. The mixture was diluted with water and extracted three times with EA, the organic phases were combined and dried with anhydrous $Na_2SO_4$, filtered and spin-dried. Separation and purification by silica gel column (PE:EA=2:1) gave a yellow solid M2-2 (2.32 g, 62.93% yield). ESI-MS m/z: 266.93$[M+H]^+$.

Step 3: Synthesis of compound M2-3

At room temperature, compound 1-1 (4.17 g, 25.80 mmol) was dissolved in THF (10 mL), and M2-2 (2.3 g, 8.60 mmol) was added, and the reaction was carried out at 40°C for 4 h. It was cooled to room temperature, quenched with water, extracted three times with EA, the organic phases were combined and dried over anhydrous sodium sulfate,

filtered, and spin-dried. M2-3 (3.4 g, crude product) was obtained as a brown solid. ESI-MS m/z: 392.01[M+H]+.

Step 4: Synthesis of compound M2-4

At room temperature, sodium methoxide (2.34 g, 43.30 mmol) was added to toluene (50 mL) of compound M2-3 (3.4 g, 8.66 mmol), and the mixture was moved to 110°C and stirred under reflux for 5 hours. Cool to room temperature, dilute with water, the reaction mixture was adjusted pH to 6 with 3N HCl and extracted three times with EA, the organic phases were combined and dried with anhydrous $Na_2SO_4$, filtered and spin-dried to obtain brown solid M2-4 (2.63 g, crude product). ESI-MS m/z: 374.01[M+H]+.

Step 5: Synthesis of compound M2-5

At room temperature, under the protection of nitrogen, DIEA (5 mL) was added to $POCl_3$ (50 mL) of compound M2-4 (2.6 g, 6.94 mmol), and the mixture was moved to 110°C, and stirred for 2 hours. After the reaction was complete and directed concentration to remove $POCl_3$ to obtain a brown solid M2-5 (2.8 g, crude product). ESI-MS m/z: 391.97[M+H]+.

Step 6: Synthesis of compound M2

At room temperature, DIEA (4.60 g, 35.62 mmol) was added to compound M2-5 (2.8 g, 7.12 mmol), 2,7-diazaspiro[3.5]nonane-2-carboxylic acid tert-butyl ester (1.93 g, 18.55 mmol) of dioxane (30 mL), reacting at room temperature for 5min. The reaction mixture was quenched with water, extracted with EA for three times, the organic phases were combined, dried with anhydrous $Na_2SO_4$, filtered, spin-dried, separated and purified on silica gel column (DCM:MeOH=10:1) to obtain brown solid M2 (3.4 g, 81.9% yield). ESI-MS m/z: 582.16[M+H]+.

**Example 1: (1-(7-(7-(5-methyl-1H-indazol-4-yl)-2-(1 methylpiperidin-4-yl)-8-(2,2,2-trifluoroethoxy)-6-vinylquinazolin-4-yl)-2,7-diazaspiro [3.5] nonan-2-yl)prop-2-en-1-one)**

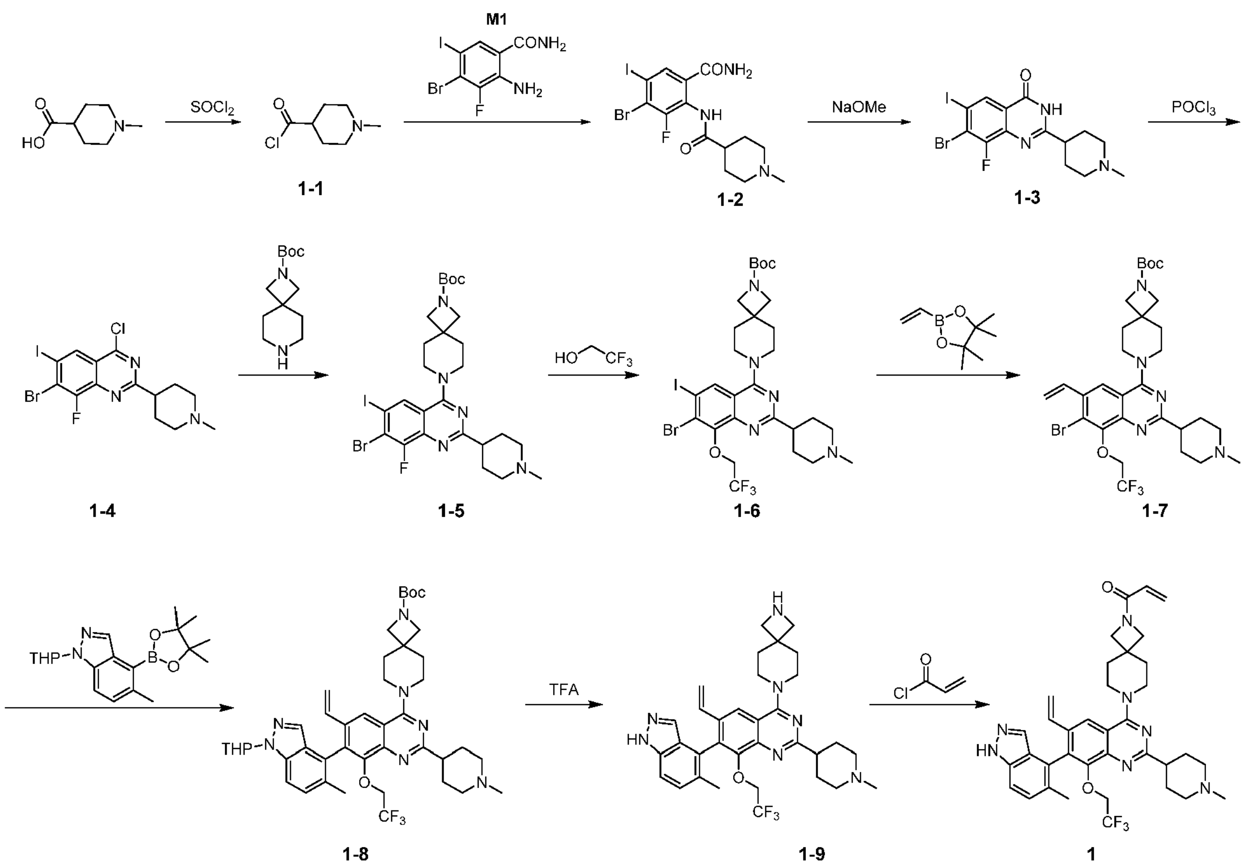


1-1 1-2 1-3 1-4 1-5 1-6 1-7 1-8 1-9 1

Step 1: Synthesis of compound 1-1

At room temperature, under the protection of nitrogen, 1-methylpiperidine-4-carboxylic acid (500 mg, 3.49 mmol) was dissolved in $SOCl_2$ (2 mL), the reaction mixture was moved to 70°C, and stirred for 1 h. It was concentrated in vacuo to remove the thionyl chloride to obtain the target product as a white solid, namely compound 1-1 (520 mg, crude product).

Step 2: Synthesis of compound 1-2

At room temperature, compound 1-1 (405.27 mg, 2.51 mmol) was added to compound M1 (450 mg, 1.25 mmol) in THF (10 mL), and moved to 40°C and stirred for 4 hours. After the reaction was complete, the THF was directly concentrated to obtain a white solid crude product, namely compound 1-2 (600 mg, crude product). ESI-MS m/z: 483.94$[M+H]^+$.

Step 3: Synthesis of compound 1-3

At room temperature, sodium methoxide (200.87 mg, 3.72 mmol) was added to compound 1-2 (600 mg, 1.24 mmol) in toluene (15 mL), and the mixture was moved to 110°C and stirred under reflux for 5 hours. After the reaction was completed, the mixture was poured into ice water, and the mixture was extracted with ethyl acetate. The organic phase was dried over $Na_2SO_4$ and concentrated in vacuo to obtain a white solid crude product, namely compound 1-3 (600 mg, crude product). ESI-MS m/z: 465.96$[M+H]^+$.

Step 4: Synthesis of compound 1-4

Under the protection of nitrogen at room temperature, DIEA (0.5 mL) was added to $POCl_3$ (5 mL) of compound 1-3 (600 mg, 1.29 mmol), and the mixture was moved to 110°C and stirred for 3 hours. After the reaction was complete, it was directly concentrated to remove $POCl_3$ to obtain the crude target product as a tan solid, namely compound 1-4 (600 mg, crude product). ESI-MS m/z: 483.96$[M+H]^+$.

Step 5: Synthesis of compound 1-5

At room temperature, DIEA (480.12 mg, 3.71 mmol, 647.06 μL) was added to compound 1-4 (600 mg, 1.24 mmol), 2,7-diazaspiro[3.5]nonane-2-carboxylic acid tert-butyl ester (280.25 mg, 1.24 mmol) in 1,4-dioxane (10 mL), stirred at room temperature for 3 hours. The mixture was poured into ice water, the mixture was extracted with ethyl acetate/methanol=10:1, the organic layer was washed with brine, dried over $Na_2SO_4$ and concentrated in vacuo, and the residue was purified by flash silica gel column chromatography (DCM:MeOH=10:1). The desired target product, compound 1-5 (750 mg, 89.81% yield), was obtained as a yellow solid. ESI-MS m/z: 674.10$[M+H]^+$.

Step 6: Synthesis of compound 1-6

Under nitrogen protection, trifluoroethanol (244.76 mg, 2.45 mmol) was added to compound 1-5 (550 mg, 815.56 μmol) and $Cs_2CO_3$ (531.45 mg, 1.63 mmol) in 1,4-dioxane (5 mL), the reaction mixture was moved to 100°C and reacted for 30 min. The mixture was poured into ice water, the mixture was extracted with ethyl acetate/methanol=10:1, the organic layer was washed with brine, dried over $Na_2SO_4$ and concentrated in vacuo to obtain the desired target product as a yellow solid, namely compound 1-6 (590 mg, Crude). ESI-MS m/z: 755.30$[M+H]^+$.

Step 7: Synthesis of compounds 1-7

Under the protection of nitrogen, $Pd(dppf)Cl_2.CH_2Cl_2$ (63.82 mg, 78.21 μmol) was added to compound 1-6 (590 mg, 782.06 μmol), $K_2CO_3$ (216.17 mg, 1.56 mmol) and 4,4,5,5-tetramethyl-2-vinyl-1,3,2-dioxaborolane (120.45 mg, 782.06 μmol) in 1,4-dioxane (5.0 mL) and $H_2O$ (0.5 mL), stirring at 70°C for 1h. The reaction mixture was cooled to room temperature, water was added to the reaction mixture, extracted with ethyl acetate: methanol = 10:1, the organic layer was washed with brine, dried over $Na_2SO_4$ and concentrated in vacuo. The concentrate was passed through pre-TLC (DCM/MeOH= 10:1) to obtain the desired product as a yellow solid, namely compound 1-7 (329 mg, 64.27% yield). ESI-MS m/z: 655.63$[M+H]^+$.

Step 8: Synthesis of compounds 1-8

Under the protection of nitrogen, $Pd(PPh_3)_4$ (116.16 mg, 100.53 umol) was added to compound 1-7 (329mg, 502.63μmol), 5-methyl-1-tetrahydropyran-2-yl-4-(4,4,5,5-tetramethyl-1,3,2-dioxaborolan-2-yl)indazole (344.04 mg, 1.01 mmol), $K_3PO_4$ (213.38 mg, 1.01 mmol) of 1,4- dioxane (3 mL) and $H_2O$ (0.75 mL) solution, the mixture was moved to 85°C and reacted for about 3 hours. The reaction mixture was cooled to room temperature, water was added to the reaction mixture, extracted with ethyl acetate: methanol = 10:1, the organic layer was washed with brine, dried over $Na_2SO_4$ and concentrated in vacuo. The concentrate was passed through pre-TLC (DCM/MeOH= 10:1) to obtain the desired product as a yellow solid, namely compound 1-8 (244 mg, 61.45% yield). ESI-MS m/z: 874.10 $[M+H]^+$.

Step 9: Synthesis of compound 1-9

At room temperature, compound 1-8 (244 mg, 308.89 μmol) was dissolved in a mixed solvent of DCM (4 mL) and TFA (2 mL), the mixture was moved to 40°C and reacted for about 1 h. After the reaction was complete, it was directly concentrated to remove the solvent to obtain the crude target product as a yellow solid, namely compound 1-9 (190 mg, crude product). ESI-MS m/z: 606.38$[M+H]^+$.

Step 10: Synthesis of compound 1

Under the protection of nitrogen, in an ice water bath, the THF solution of acryloyl chloride (17.03 mg, 188.21 μmol) was added to the compound 1-9 (95 mg, 156.84 μmol), THF (4 mL), saturated $Na_2CO_3$ (1 mL) solution, the reaction was complete. The reaction mixture was poured into water, extracted with ethyl acetate: methanol = 10:1, the organic layer was washed with brine, dried over $Na_2SO_4$ and concentrated in vacuo. The concentrate was purified by pre-TLC (DCM/MeOH=10:1) to obtain the desired product was compound 1 (24.1 mg, 22.96% yield, 98.59% purity) as a yellow solid. ESI-MS m/z: 660.37$[M+H]^+$. $^1H$ NMR (500 MHz, Methanol-$d_4$) δ 8.08 (s, 1H), 7.52 (d, *J* = 8.6 Hz, 1H), 7.43-7.32 (m, 2H), 6.40 (d, *J* = 17.0, 10.3 Hz, 1H), 6.32-6.17(m, 2H), 5.78-5.69(m, 2H), 5.09 (d, *J* = 11.1 Hz, 1H), 4.86-4.75(m, 1H), 4.51(m, 1H), 4.15 (s, 2H), 3.90 (s, 2H), 3.72 (td, *J* = 6.4, 5.2, 3.0 Hz, 4H), 3.02 (d, *J* = 11.4 Hz, 2H), 2.85 (t, *J* = 11.5 Hz, 1H), 2.34 (s, 3H), 2.25 (m, 2H), 2.13 (m, 5H), 2.06 (m, 3H), 1.90-1.83 (m, 4H).

**Example 2: (1-(7-(2-cyclohexyl-7-(5-methyl-1H-indazol-4-yl)-8-(2,2,2-trifluoroethoxy)-6-vinyl quinazolin-4-yl)-2,7-diazaspiro[3.5]nonan-2-yl)prop-2-en-1-one)**

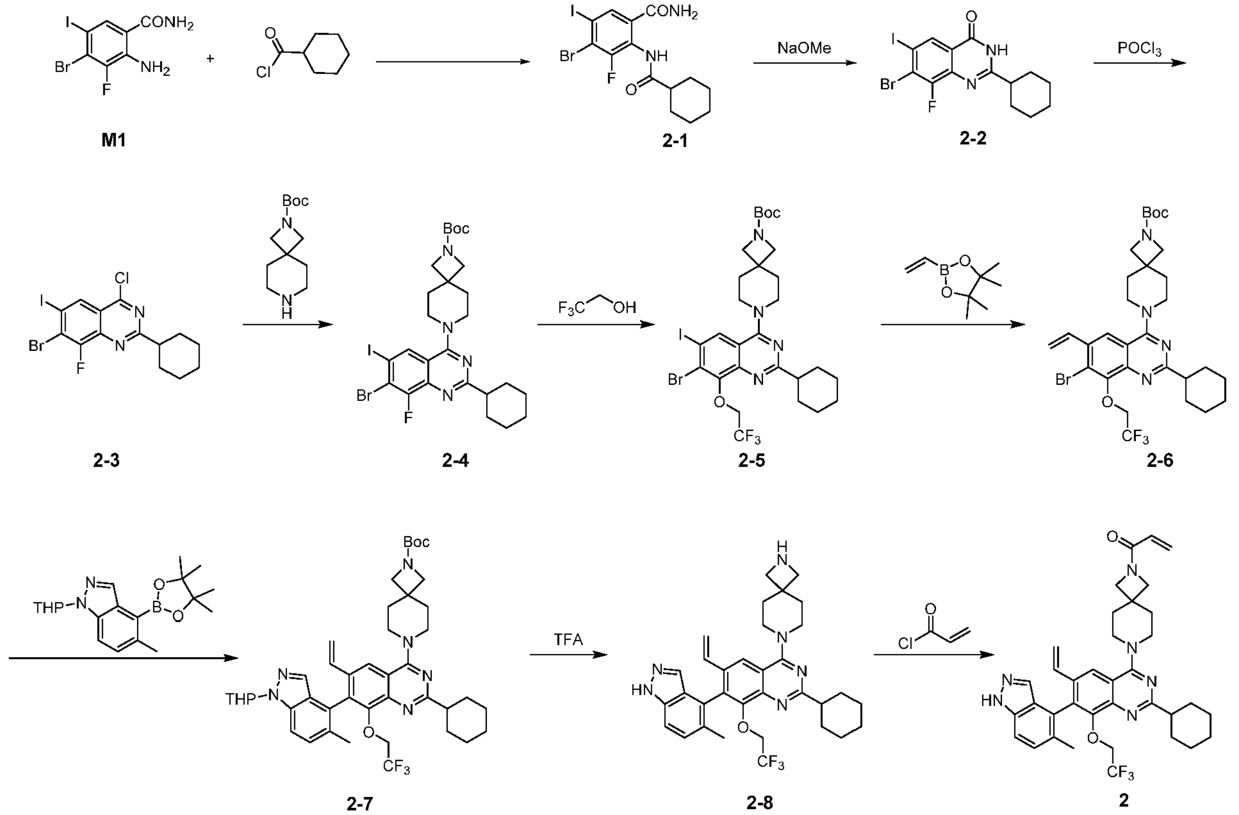


M1 2-1 2-2 2-3 2-4 2-5 2-6 2-7 2-8 2

Step 1: Synthesis of compound 2-1

At room temperature, cyclohexanoyl chloride (367.63 mg, 2.51 mmol) was added to compound M1 (300 mg, 0.84 mmol) in THF (10 mL), and the mixture was moved to 40°C, and stirred for 4 hours. After the reaction was complete, the THF was directly concentrated to obtain a white solid crude product, namely compound 2-1 (390 mg, crude product). ESI-MS m/z: 469.00 $[M+H]^+$.

Step 2: Synthesis of compound 2-2

At room temperature, sodium methoxide (224.56 mg, 4.16 mmol) was added to toluene (15 mL) of 2-1 (390 mg, 0.83 mmol), and the mixture was moved to 110°C under reflux and stirred for 5 hours. After the reaction was completed, the mixture was poured into ice water, and the mixture was extracted with ethyl acetate. The organic phase was dried over $Na_2SO_4$ and concentrated in vacuo to obtain a white solid crude product, namely compound 2-2 (350 mg, crude product). ESI-MS m/z: 450.94$[M+H]^+$.

Step 3: Synthesis of compound 2-3

At room temperature, under the protection of nitrogen, DIEA (1.0 mL) was added to the $POCl_3$ (10.0 mL) of compound 2-2 (350 mg, 0.78 mmol), and the mixture was moved to 110°C and stirred for 12 hours. After the reaction was complete, it was directly concentrated to remove $POCl_3$ to obtain the crude target product as a tan solid, namely compound 2-3 (340 mg, crude product). ESI-MS m/z: 468.87$[M+H]^+$.

Step 4: Synthesis of compound 2-4

At room temperature, DIEA (279 mg, 2.16 mmol) was added to compound 2-3 (340 mg, 0.72 mmol), 2,7-diazaspiro[3.5]nonane-2-carboxylic acid tert-butyl ester (163 mg, 0.72 mmol) in 1,4-dioxane (10 mL), the mixture was stirred at room temperature for 5 minutes. The mixture was poured into ice water, and extracted with ethyl acetate, the organic layer was washed with brine, dried over $Na_2SO_4$ and concentrated in vacuo. The residue was purified by flash silica gel column chromatography (PE:EA=10:1-15:1) to obtain the desired target product which was compound 2-4 (348 mg, 42.62% yield) as a yellow solid. ESI-MS m/z: 659.06$[M+H]^+$.

Step 5: Synthesis of compound 2-5

Under the protection of nitrogen, trifluoroethanol (158.4 mg, 1.58 mmol) was added to compound 2-4 (348 mg, 0.53 mmol), $Cs_2CO_3$ (343.92 mg, 1.06 mmol) in 1,4-dioxane (5 mL), the mixture was moved to 100°C and reacted for 30min. The reaction mixture was poured into ice water, the mixture was extracted with ethyl acetate, the organic layer was washed with brine, dried over $Na_2SO_4$ and concentrated in vacuo to obtain the desired target product, compound 2-5 (400 mg, crude product) as a yellow solid. ESI-MS m/z: 739.01$[M+H]^+$.

Step 6: Synthesis of compound 2-6

Under the protection of nitrogen, $Pd(dppf)Cl_2.CH_2Cl_2$ (63.82 mg, 78.21 μmol) was added to compound 2-5 (400 mg, 0.54 mmol), $K_2CO_3$ (149.53 mg, 1.08 mmol), 4,4,5,5-tetramethyl-2-vinyl-1,3,2-dioxaborolane (83.32 mg, 0.54 mmol) in 1,4-dioxane (4.0 mL) and $H_2O$ (0.4 mL), the mixture was stirred at 70°C for 1 h. The reaction mixture was cooled to room temperature, water was added to the reaction mixture, extracted with ethyl acetate: methanol = 10: 1, the organic layer was washed with brine, dried over $Na_2SO_4$ and concentrated in vacuo. The residue was passed through flash silica gel column chromatography (PE:EA =10:1-15:1) to obtain the desired target product as a yellow solid, namely compound 2-6 (213 mg, 61.56% yield). ESI-MS m/z: 639.01$[M+H]^+$.

Step 7: Synthesis of compound 2-7

Under the protection of nitrogen, $Pd(PPh_3)_4$ (116.16 mg, 100.53 μmol) was added to compound 2-6 (213 mg, 33.05 μmol), 5-methyl-1-tetrahydropyran-2-yl-4-(4,4,5,5-tetramethyl-1,3,2-dioxaborolan-2-yl)indazole (227.96 mg, 666.10 mmol), $K_3PO_4$ (141.39 mg, 666.10 mmol) of 1,4-dioxane (2 mL), $H_2O$ (0.5 mL) solution, the mixture was moved to 85°C and reacted for about 3 hours. The reaction mixture was cooled to room temperature, water was added to the reaction mixture, extracted with ethyl acetate, the organic layer was washed with brine, dried over $Na_2SO_4$ and concentrated in vacuo. The concentrate was purified by pre-TLC (PE:EA=2:1), the desired product, compound 2-7 (180 mg, 69.74% yield) was obtained as a yellow solid. ESI-MS m/z: 775.42 $[M+H]^+$.

Step 8: Synthesis of compound 2-8

At room temperature, compound 2-7 (180 mg, 232.28 μmol) was dissolved in a mixed solvent of DCM (6 mL) and TFA (3 mL), and moved to 40°C reacting for about 1 h. After the reaction was complete, it was directly concentrated to remove the solvent to obtain the crude target product as a yellow solid, namely compound 2-8 (135 mg, crude product). ESI-MS m/z: 591.40$[M+H]^+$.

Step 9: Synthesis of compound 2-9

Under the protection of nitrogen, in an ice water bath, the THF solution of acryloyl chloride (17.03 mg, 188.21 μmol) was added to the compound 2-8 (135 mg, 228.55 μmol), THF (4 mL), and saturated $Na_2CO_3$ (1 mL) solution, and add it the reaction was complete. The reaction mixture was poured into water, extracted with ethyl acetate: methanol = 10:1, the organic layer was washed with brine, dried over $Na_2SO_4$ and concentrated in vacuo. The concentrate was purified by pre-TLC (DCM/MeOH=10:1) to obtain. The desired product was compound 2 (66.71 mg, 44.36% yield, 97.99% purity) as a yellow solid. ESI-MS m/z: 645.44$[M+H]^+$. $^1$HNMR(500MHz,$CDCl_3$)δ: 7.94 (s, 1H), 7.45-7.48 (m, 2H), 7.36-7.36 (m, 1H), 6.37-6.41 (m, 1H), 6.22-6.28 (m, 2H), 5.70-5.73 (m, 1H), 5.62-5.66 (m, 1H), 5.05-5.07 (m, 1H), 4.83-4.91 (m, 1H), 4.47-4.54 (m, 1H), 4.04 (s, 2H), 3.94 (s, 2H), 3.70-3.79 (m, 4H), 2.81-2.87 (m, 1H), 2.15 (s, 3H), 2.07-2.09 (m, 2H), 1.85-1.87 (m, 2H), 1.74-1.77 (m, 1H), 1.61-1.71 (m, 3H), 1.41-1.48 (m, 3H), 1.25-1.37 (m, 3H).

**Example 3: (1-(7-(2-tetrahydrofuran-3-yl-7-(5-methyl-1H-indazol-4-yl)-8-(2,2,2-trifluoro ethoxy)-6-vinylquinazolin-4-yl)-2,7-diazaspiro[3.5]nonan-2-yl)prop-2-en-1-one)**

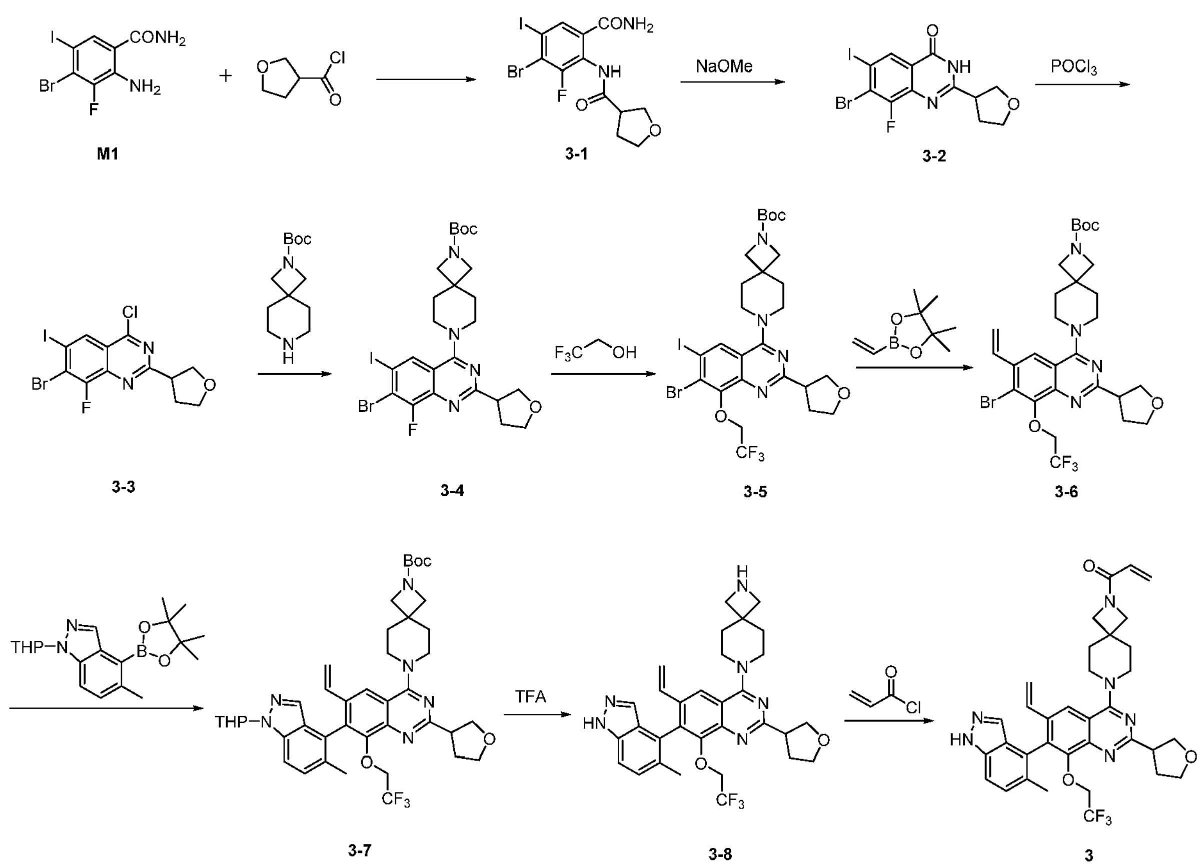


M1 3-1 3-2

3-3 3-4 3-5 3-6

3-7 3-8 3

Step 1: Synthesis of compound 3-1

At room temperature, tetrahydrofuran-3-carbonyl chloride (402 mg, 3.0 mmol) was added to compound M1 (300 mg, 0.84 mmol) in THF (10 mL), and the mixture was moved to 40°C and stirred for 4 hours. After the reaction was complete, the THF was directly concentrated to obtain the crude target product as a yellow solid, namely compound 3-1 (460 mg, crude product). ESI-MS m/z: 456.94 $[M+H]^+$.

Step 2: Synthesis of compound 3-2

At room temperature, sodium methoxide (224.56 mg, 4.16 mmol) was added to compound 3-1 (460 mg, 0.83 mmol) in toluene (10 mL), and the mixture was moved to 110°C under reflux and stirred for 8 hours. After the reaction was completed, the mixture was poured into ice water, and the mixture was extracted with ethyl acetate. The organic phase was dried over $Na_2SO_4$ and concentrated in vacuo to obtain a white solid crude product, namely compound 3-2 (200 mg, crude product). ESI-MS m/z: 438.96$[M+H]^+$.

Step 3: Synthesis of compound 3-3

At room temperature, under nitrogen protection, DIEA (1.0 mL) was added to compound 3-2 (200 mg, 0.46 mmol) in $POCl_3$ (10.0 mL), and the mixture was moved to 110°C and stirred for 12 hours. After the reaction was complete, it was directly concentrated to remove $POCl_3$ to obtain the crude target product as a tan solid, namely compound 3-3 (230 mg, crude product). ESI-MS m/z: 456.87$[M+H]^+$.

Step 4: Synthesis of compound 3-4

At room temperature, DIEA (279 mg, 2.16 mmol) was added to compound 3-3 (200 mg, 0.4 mmol), 2,7-diazaspiro[3.5]nonane-2-carboxylic acid tert-butyl ester (90 mg, 0.4 mmol) in 1,4-dioxane (2 mL), the reaction mixture was stirred at room temperature for 5 minutes. The mixture was poured into ice water, and extracted with ethyl acetate, the organic layer was washed with brine, dried over $Na_2SO_4$ and concentrated in vacuo. The residue was purified by climbing a large plate to obtain the desired target product as a yellow solid, namely compound 3-4 (88 mg, 34.1% yield).

ESI-MS m/z: 647.14 $[M+H]^+$.

Step 5: Synthesis of compound 3-5

Under the protection of nitrogen, trifluoroethanol (68 mg, 0.681 mmol) was added to compound 3-4 (88 mg, 0.136 mmol), $Cs_2CO_3$ (88 mg, 0.272 mmol) in 1,4-dioxane (2 mL), and transferred reaction at 100°C for 30 min. The mixture was poured into ice water, the mixture was extracted with ethyl acetate, the organic layer was washed with brine, dried over $Na_2SO_4$ and concentrated in vacuo to obtain the desired target product, compound 3-5 (97 mg, crude product) as a yellow solid. ESI-MS m/z: 727.08 $[M+H]^+$.

Step 6: Synthesis of compound 3-6

Under the protection of nitrogen, $Pd(dppf)Cl_2.CH_2Cl_2$ (10 mg, 12.4 μmol) was added to compound 3-5 (90 mg, 0.124 mmol), $K_2CO_3$ (34 mg, 0.248 mmol), 4,4,5,5-tetramethyl-2-vinyl-1,3,2-dioxaborolane (19 mg, 0.124 mmol) in 1,4-dioxane (2.5 mL), $H_2O$ (0.5 mL) mixed solution, the reaction mixture was stirred at 70°C for 1 h. The reaction mixture was cooled to room temperature, water was added to the reaction mixture, extracted with ethyl acetate: methanol = 10:1, the organic layer was washed with brine, dried over $Na_2SO_4$ and concentrated in vacuo. The residue was passed through flash silica gel column chromatography (PE:EA =10:1-3:1) to obtain the desired target product as a yellow solid, namely compound 3-6 (213 mg, 64.4% yield). ESI-MS m/z: 627.21 $[M+H]^+$.

Step 7: Synthesis of compound 3-7

Under the protection of nitrogen, $Pd(PPh_3)_4$ (18.5 mg, 16 μmol) was added to compound 3-6 (50 mg, 79.9 μmol), 5-methyl-1-tetrahydropyran-2-yl-4-(4,4,5,5-tetramethyl-1,3,2-dioxaborolan-2-yl)indazole (54.6 mg, 0.16 mmol), $K_3PO_4$ (34 mg, 0.16 mmol) of 1,4-dioxane (2 mL), $H_2O$ (0.5 mL) solution, the reaction mixture was moved to 85°C and react for about 4 hours. The reaction mixture was cooled to room temperature, water was added to the reaction mixture, extracted with ethyl acetate, the organic layer was washed with brine, dried over $Na_2SO_4$ and concentrated in vacuo. The concentrate was purified by pre-TLC (PE:EA=2:1), and the desired product, compound 3-7 (45 mg, 73.8% yield) was obtained as a yellow solid. ESI-MS m/z: 763.39$[M+H]^+$.

Step 8: Synthesis of compound 3-8

At room temperature, compound 3-7 (45 mg, 59.1 μmol) was dissolved in a mixed solvent of DCM (1 mL) and TFA (0.5 mL), and the reaction mixture was moved to 40°C and reacted for about 1 h. After the reaction was complete, the solvent was directly concentrated to remove the solvent to obtain the crude target product as a yellow solid, namely compound 3-8 (56 mg, crude product). ESI-MS m/z: 579.32$[M+H]^+$.

Step 9: Synthesis of compound 3-9

Under the protection of nitrogen, in an ice water bath, the THF solution of acryloyl chloride (5.85 mg, 65 μmol) was added to compound 3-8 (56 mg, 59.1 μmol), THF (2 mL) and saturated $Na_2CO_3$ (0.5 mL) solution, and the reaction was complete. The reaction mixture was poured into water, extracted with ethyl acetate: methanol=10:1, the organic layer was washed with brine, dried over $Na_2SO_4$ and concentrated in vacuo, the concentrate was purified by pre-TLC (DCM/MeOH=10:1) to obtain The desired product as a yellow solid, compound 3 (12.05 mg, 32.2% yield, 93.4% purity). ESI-MS m/z: 633.41 $[M+H]^+$. $^1$HNMR (500MHz, Chloroformd) δ 7.88 (s, 1H), 7.40 (d, *J*=7.5Hz, 2H), 7.19 (s, 1H), 6.32 (dd, *J*=17.0, 1.9Hz, 1H), 6.18 (m, *J* = 17.0, 10.6, 4.9 Hz, 2H), 5.65 (dd, *J* = 10.3, 1.9 Hz, 1H), 5.58 (dd, *J* = 17.4, 1.0 Hz, 1H), 5.01 (dd, *J* = 10.9, 1.0 Hz, 1H), 4.78-4.64 (m, 1H), 4.34 (m, 1H), 4.20 (td, *J* = 8.1, 3.7 Hz, 1H), 4.05 (dd, *J* = 8.3, 6.8 Hz, 1H), 4.02-3.95(m, 3H), 3.94-3.89(m, 1H), 3.87(s, 2H), 3.75(td, *J*=10.0, 9.0, 5.5Hz, 2H), 3.71-3.62(m, 3H), 2.41 (m, 1H), 2.29 (m, 1H), 2.08 (s, 3H), 1.18 (d, *J*=7.2 Hz, 4H).

**Example 4: (1-(7-(6-ethyl-7-(5-methyl-1H-indazol-4-yl)-2-(1-methylpiperidin-4-yl) -8-(2,2,2-trifluoroethoxy)quinazolin-4-yl)-2,7-diazaspiro [3.5] nonan-2-yl)prop-2-en-1-one)**

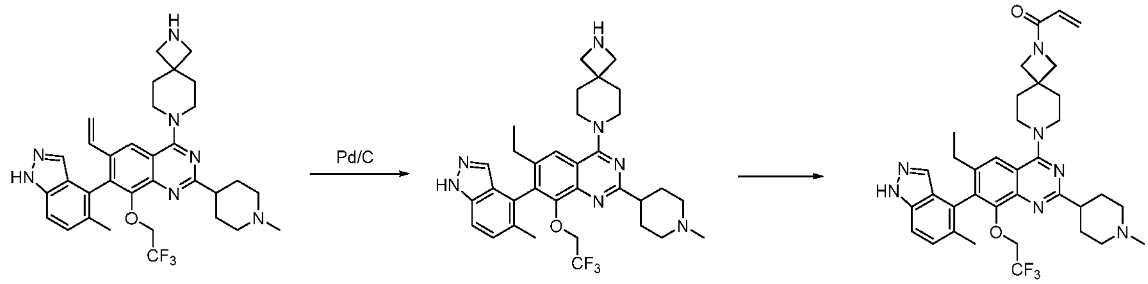


1-9 4-1 4

Step 1: Synthesis of compound 4-1

Pd/C (80 mg, 751.74 μmol) was added to a MeOH (10 mL) solution of compound 1-9 (95 mg, 156.84 μmol) in a hydrogen atmosphere at room temperature, and stirred at room temperature for 2 hours. After the reaction was complete, filtered to remove Pd/C, washed Pd/C with MeOH, and concentrated the filtrate to obtain a crude yellow solid, compound 4-1 (70 mg, crude product). ESI-MS m/z: 608.43 $[M+H]^+$.

Step 2: Synthesis of compound 4

Under the protection of nitrogen, in an ice-water bath, the THF solution of acryloyl chloride (12.51 mg, 138.22 μmol) was added to compound 4-1 (70 mg, 115.19 μmol), THF (4 mL), saturated $Na_2CO_3$ (1 mL) solution, and the reaction was complete. The reaction mixture was poured into water, extracted with ethyl acetate: methanol = 10:1, the organic layer was washed with brine, dried over $Na_2SO_4$ and concentrated in vacuo. The concentrate was purified by pre-TLC (DCM/MeOH=10:1) to obtain the desired product was compound 4 (18.3 mg, 23.79% yield, 99.10% purity) as a yellow solid. ESI-MS m/z: 662.37$[M+H]^+$.

**Example 5: (1-(7-(8-fluoro-7-(5-methyl-1H-indazol-4-yl)-2-(1-methylpiperidin-4-yl)-synthesis of 6-vinylquinazolin-4-yl)-2,7-diazaspiro[3.5]nonan-2-yl)prop-2-en-1-one)**

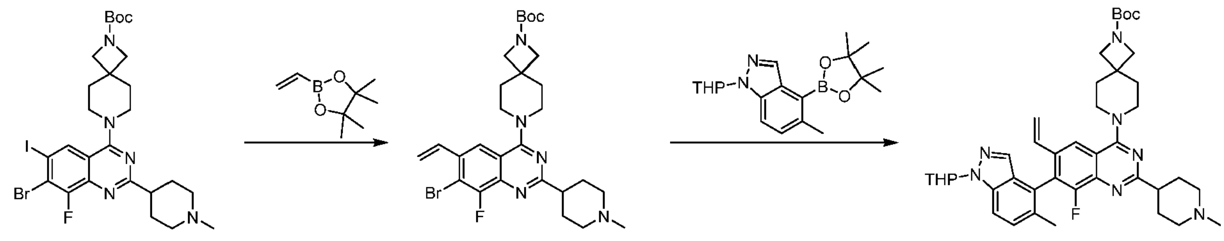

1-5 5-1 5-2

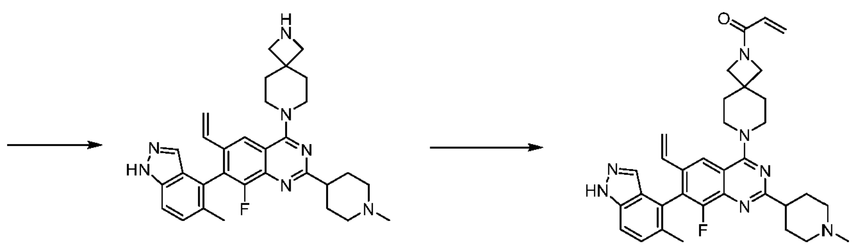

5-3 5

Step 1: Synthesis of compound 5-1

Under the protection of nitrogen, $Pd(dppf)Cl_2.CH_2Cl_2$ (24.20 mg, 29.66 μmol) was added to compound 1-5 (200 mg, 296.57 μmol), $K_2CO_3$ (81.97 mg, 593.13 μmol), 4,4,5,5-tetramethyl-2-vinyl-1,3,2-dioxaborolane (45.68 mg, 2963.57 μmol) in 1,4-dioxane (2.0 mL) and $H_2O$ (0.2 mL) mixed solution, stirred at 70°C for 1 h. The reaction mixture

was cooled to room temperature, water was added to the reaction mixture, extracted with ethyl acetate: methanol = 10:1, the organic layer was washed with brine, dried over $Na_2SO_4$ and concentrated in vacuo. The concentrate was passed through pre-TLC (DCM/MeOH= 10:1) to obtain the desired product as a yellow solid, namely compound 5-1 (145 mg, 85.10% yield). ESI-MS m/z: 574.24$[M+H]^+$.

Step 2: Synthesis of compound 5-2

Under the protection of nitrogen, $Pd(PPh_3)_4$ (58.33 mg, 50.48 μmol) was added to compound 5-1 (145 mg, 252.38 μmol), 5-methyl-1-tetrahydropyran-2-yl-4-(4,4,5,5-tetramethyl-1,3,2-dioxaborolan-2-yl)indazole (172.75 mg, 504.76 μmol), $K_3PO_4$ (107.14 mg, 504.76 μmol) 1,4-dioxane (2 mL), $H_2O$ (0.5 mL) solution, the reaction mixture was moved to 85°C and reacted for about 3 hours. The reaction mixture was cooled to room temperature, water was added to the reaction mixture, extracted with ethyl acetate: methanol = 10:1, the organic layer was washed with brine, dried over $Na_2SO_4$ and concentrated in vacuo. The concentrate was passed through pre-TLC (DCM/MeOH= 10:1) to obtain the desired product as a yellow solid, namely compound 5-2 (88 mg, 49.12% yield). ESI-MS m/z: 710.85$[M+H]^+$.

Step 3: Synthesis of compound 5-3

At room temperature, compound 5-2 (88 mg, 123.96 μmol) was dissolved in a mixed solvent of DCM (2 mL) and TFA (1 mL), and moved to 40°C reacting for about 1 h. After the reaction was completed, the solvent was directly concentrated to remove the solvent to obtain the crude target product as a yellow solid, namely compound 5-3 (70 mg, crude product). ESI-MS m/z: 526.34$[M+H]^+$.

Step 4: Synthesis of compound 5

Under the protection of nitrogen, a THF solution of acryloyl chloride (14.46 mg, 159.80 μmol) was added to compound 5-3 (70 mg, 133.17 μmol), THF (4.0 mL), saturated $Na_2CO_3$ (1.0 mL) solution in an ice water bath. The reaction was complete when added. The reaction mixture was poured into water, extracted with ethyl acetate: methanol = 10:1, the organic layer was washed with brine, dried over $Na_2SO_4$ and concentrated in vacuo. The concentrate was purified by pre-TLC (DCM/MeOH=10:1) to obtain the desired product as a yellow solid, namely compound 5 (23.3 mg, 29.62% yield, 98.15% purity). ESI-MS m/z: 580.34$[M+H]^+$. $^1H$ NMR (500MHz, Chloroform-*d*) δ7.95(s, 1H), 7.51(d, *J* =9.7Hz,2H), 7.38(d, *J* =8.4Hz, 1H), 6.39(dd, *J* =17.0, 1.8Hz, 1H), 6.25 (m, 2H), 5.76-5.61 (m, 2H), 5.10 (d, *J* = 11.1Hz, 1H), 4.03 (s, 2H), 3.93 (s, 2H), 3.89- 3.75 (m, 4H), 3.02(d, *J*=7.8Hz, 3H), 2.35(s, 3H), 2.19(s, 3H), 2.13(d, *J*=9.1Hz, 3H), 2.02(m, 6H), 1.68-1.50 (m, 2H).

**Example 6: (1-(7-(7-(5-methyl-1H-indazol-4-yl)-2-(tetrahydro-2H-pyran-4-yl)-8-(2,2,2-triflu oroethoxy)-6-vinylquinazolin-4-yl)-2,7-diazaspiro[3.5]nonan-2-yl)prop-2-en-1-one)**

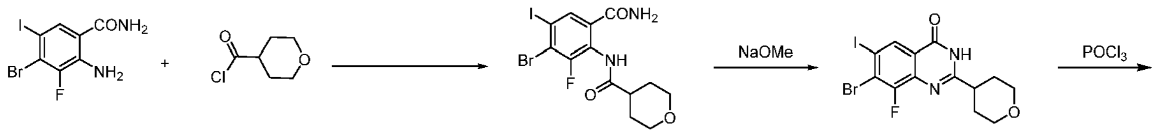


M1 6-1 6-2

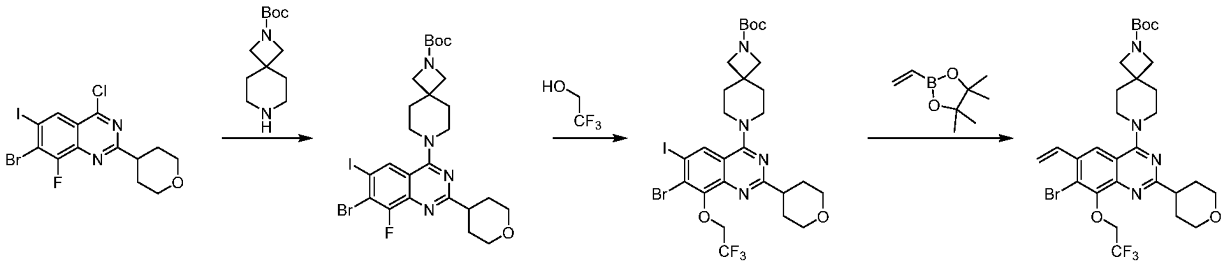

6-3 6-4 6-5 6-6

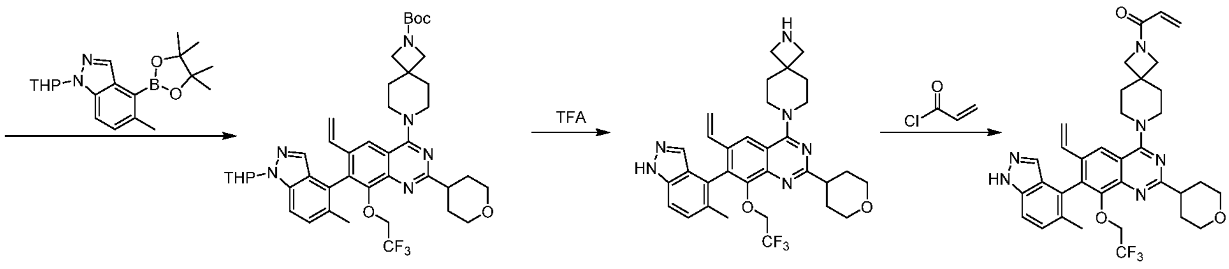


6-7 6-8 6

Step 1: Synthesis of compound 6-1

At room temperature, tetrahydropyran-4-carbonyl chloride (248.38 mg, 1.67 mmol) was added to compound M1 (300 mg, 0.84 mmol) in THF (3 mL), and the mixture was moved to 40°C, and stirred for 4 hours. After the reaction was complete, the THF was directly concentrated to obtain a white solid crude product, namely compound 6-1 (390 mg, crude product). ESI-MS m/z: 470.92 $[M+H]^+$.

Step 2: Synthesis of compound 6-2

At room temperature, sodium methoxide (224.56 mg, 4.16 mmol) was added to compound 6-1 (390 mg, 0.83 mmol) in toluene (4 mL), and the mixture was moved to 110°C under reflux and stirred for 5 hours. After the reaction was complete, the mixture was poured into ice water, and the mixture was extracted with ethyl acetate. The organic phase was dried over $Na_2SO_4$ and concentrated in vacuo to obtain a white solid crude product, namely compound 6-2 (350 mg, crude product). ESI-MS m/z: 452.96 $[M+H]^+$.

Step 3: Synthesis of compound 6-3

At room temperature and under nitrogen protection, DIEA (0.4 mL) was added to compound 6-2 (350 mg, 0.77 mmol) in $POCl_3$ (4 mL), and the mixture was moved to 110°C and stirred for 12 hours. After the reaction was complete, it was directly concentrated to remove $POCl_3$ to obtain the crude target product as a tan solid, namely compound 6-3 (210 mg, crude product). ESI-MS m/z: 470.95$[M+H]^+$.

Step 4: Synthesis of compound 6-4

At room temperature, DIEA (115.13 mg, 0.89 mmol) was added to compound 6-3 (210 mg, 0.45 mmol), 2,7-diazaspiro[3.5]nonane-2-carboxylic acid tert-butyl ester (120.96 mg, 0.53 mmol) of 1,4-dioxane (3 mL), and the mixture was stirred at room temperature for 30 minutes. The mixture was poured into ice water, and extracted with ethyl acetate, the organic layer was washed with brine, dried over $Na_2SO_4$ and concentrated in vacuo. The residue was purified by flash silica gel column chromatography (PE:EA=10:1-15:1) to obtain the desired target product was compound 6-4 (110 mg, 37.34% yield) as a yellow solid. ESI-MS m/z: 661.17$[M+H]^+$.

Step 5: Synthesis of compound 6-5

Under the protection of nitrogen, trifluoroethanol (49.92 mg, 0.50 mmol) was added to compound 6-4 (110 mg, 0.17 mmol), $Cs_2CO_3$ (108.39 mg, 0.33 mmol) in 1,4-dioxane (2 mL), the mixture was moved to 100°C and reacted for 30 min. The mixture was poured into ice water and extracted with ethyl acetate, the organic layer was washed with brine, dried over $Na_2SO_4$ and concentrated in vacuo to obtain the desired target product, compound 6-5 (105 mg, crude product) as a yellow solid. ESI-MS m/z: 741.14$[M+H]^+$.

Step 6: Synthesis of compound 6-6

Under the protection of nitrogen, $Pd(dppf)Cl_2.CH_2Cl_2$ (11.56 mg, 14.16 μmol) was added to compound 6-5 (105 mg, 0.14 mmol), $K_2CO_3$ (39.15 mg, 0.28 mmol), 4,4,5,5-tetramethyl-2-vinyl-1,3,2-dioxaborolane (21.81 mg, 0.14 mmol) in 1,4-dioxane (2 mL) and $H_2O$ (0.4 mL), the mixture was stirred at 70°C for 1 h. The reaction mixture was cooled to room temperature, water was added to the reaction mixture, extracted with ethyl acetate: methanol = 10:1, the organic layer was washed with brine, dried over $Na_2SO_4$ and concentrated in vacuo. The residue was passed through flash silica gel column chromatography (PE:EA =10:1-15:1) to obtain the desired target product as a yellow solid, namely compound 6-6 (60 mg, 66.04% yield). ESI-MS m/z: 641.25$[M+H]^+$.

Step 7: Synthesis of compound 6-7

Under the protection of nitrogen, $Pd(PPh_3)_4$ (21.60 mg, 18.71 μmol) was added to compound 6-6 (60 mg, 95.53 μmol), 5-methyl-1-tetrahydropyran-2-yl-4-(4,4,5,5-tetramethyl-1,3,2-dioxaborolan-2-yl)indazole (64.02 mg, 0.19 mmol), $K_3PO_4$ (39.71 mg, 0.19 mmol) of 1,4-dioxane (2 mL), $H_2O$ (0.2 mL) solution, the reaction mixture was moved to 85°C and reacted for about 3 hours. The reaction mixture was cooled to room temperature, water was added to the reaction mixture, extracted with ethyl acetate, the organic layer was washed with brine, dried over $Na_2SO_4$ and concentrated in vacuo. The concentrate was purified by pre-TLC (PE:EA=2:1), the desired product, compound 6-7 (25 mg, 34.41% yield) was obtained as a yellow solid. ESI-MS m/z: 777.42 $[M+H]^+$.

Step 8: Synthesis of compound 6-8

At room temperature, compound 6-7 (25 mg, 32.18 μmol) was dissolved in a mixed solvent of DCM (1 mL) and TFA (0.5 mL), and moved to 40°C reacting for about 1 h. After the reaction was complete, it was directly concentrated to remove the solvent to obtain a crude yellow oily target product, namely compound 6-8 (20 mg, crude product). ESI-MS m/z: 593.33$[M+H]^+$.

Step 9: Synthesis of compound 6

Under the protection of nitrogen, in an ice water bath, add the THF solution of acryloyl chloride (3.20 mg, 35.40 μmol) to compound 6-8 (20 mg, 32.18 μmol), THF (2 mL), and saturated $Na_2CO_3$ (0.5 mL) solution, the reaction was complete. The reaction mixture was poured into water, extracted with ethyl acetate: methanol=10:1, the organic layer was washed with brine, dried over $Na_2SO_4$ and concentrated in vacuo, the concentrate was purified by pre-TLC (DCM/MeOH=10:1) to obtain the desired product was compound 6 (5.2 mg, 33.48% yield, 98.25% purity) as a yellow solid. ESI-MS m/z: 647.33 $[M+H]^+$.

**Example 7: (1-(7-(6-chloro-7-(5-methyl-1H-indazol-4-yl)-2-(1methylpiperidin-4-yl) quinazoline -4-yl)-2,7-diazaspiro[3.5]nonan-2-yl)prop-2-en-1-one)**

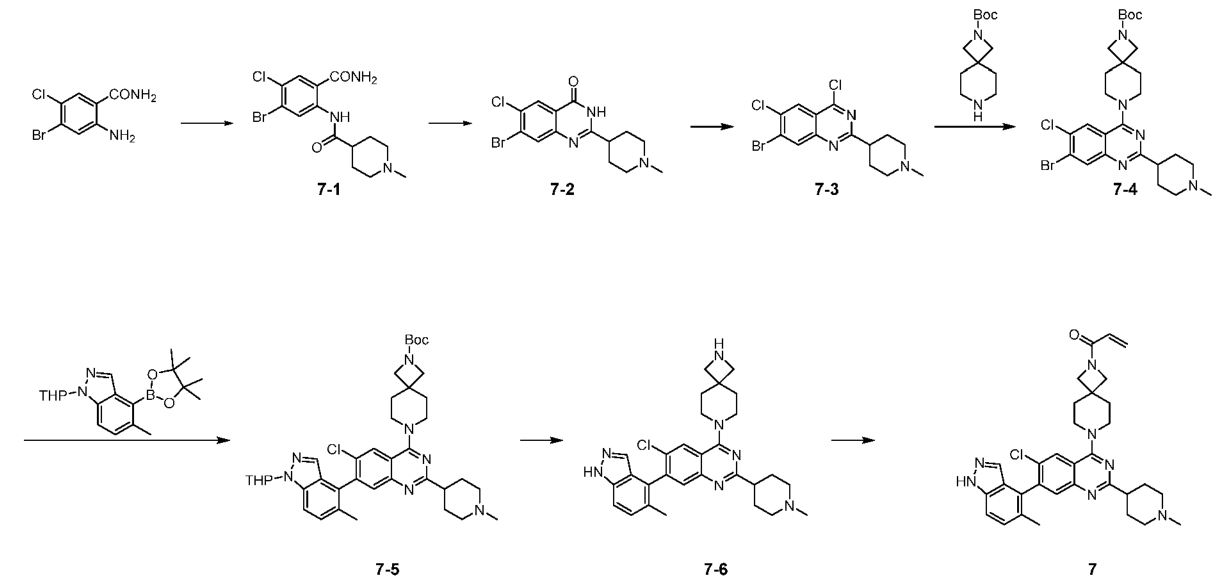

7-1 7-2 7-3 7-4

7-5 7-6 7

Step 1: Synthesis of compound 7-1

At room temperature, compound 1-1 (388.7 mg, 2.4 mmol) was added to 2-amino-4-bromo-5-chlorobenzamide (300 mg, 1.2 mmol) in THF (10 mL), moved to 40°C and stirred 4 hours. After the reaction was complete, the THF was directly concentrated to obtain the target product as a white solid crude product, namely compound 7-1 (400 mg, crude product). ESI-MS m/z: 374.13 $[M+H]^+$.

Step 2: Synthesis of compound 7-2

At room temperature, sodium methoxide (172.96 mg, 3.20 mmol) was added to compound 7-1 (400 mg, 1.07 mmol) in toluene (15 mL), and the mixture was moved to 110°C under reflux and stirred for 5 hours. After the reaction was complete, the mixture was poured into ice water, and the mixture was extracted with ethyl acetate. The organic phase was dried over $Na_2SO_4$ and concentrated in vacuo to obtain a white solid crude product, namely compound 7-2 (350 mg, crude product). ESI-MS m/z: 356.1$[M+H]^+$.

Step 3: Synthesis of compound 7-3

At room temperature and under the protection of nitrogen, DIEA (0.5 mL) was added to compound 7-2 (350 mg, 0.98 mmol) in $POCl_3$ (5 mL), and the mixture was moved to 110°C and stirred for 3 hours. After the reaction was complete, it was directly concentrated to remove $POCl_3$ to obtain the crude target product as a tan solid, namely compound 7-3 (360 mg, crude product). ESI-MS m/z: 374.0$[M+H]^+$.

Step 4: Synthesis of compound 7-4

At room temperature, DIEA (248.09 mg, 1.92 mmol) was added to compound 7-3 (360 mg, 0.96 mmol), 2,7-diazaspiro[3.5]nonane-2-carboxylic acid tert-butyl ester (239 mg, 1.06 mmol) of 1,4-dioxane (10 mL), the reaction mixture was stirred at room temperature for 3 hours. The mixture was poured into ice water, the mixture was extracted with ethyl acetate/methanol=10:1, the organic layer was washed with brine, dried over $Na_2SO_4$ and concentrated in vacuo, and the residue was purified by flash silica gel column chromatography (DCM:MeOH=10:1), the desired target product was obtained as a yellow solid, namely compound 7-4 (500 mg, 92.21% yield). ESI-MS m/z: 564.2$[M+H]^+$.

Step 5: Synthesis of compound 7-5

Under the protection of nitrogen, $Pd(PPh_3)_4$ (116.16 mg, 100.53 μmol) was added to compound 7-4 (200 mg, 354.02 μmol), 5-methyl-1-tetrahydropyran-2-yl-4-(4,4,5,5-tetramethyl-1,3,2-dioxaborolan-2-yl)indazole (242.32 mg, 0.708 mmol), $K_2CO_3$ (97.71 mg, 0.708 mmol) of 1,4 - dioxane (3 mL), $H_2O$ (0.75 mL) solution, the reaction mixture was moved to 85°C and reacted for about 3 hours. The reaction mixture was cooled to room temperature, water was added to the reaction mixture, extracted with ethyl acetate: methanol = 10:1, the organic layer was washed with brine, dried

over $Na_2SO_4$ and concentrated in vacuo. The concentrate was passed through pre-TLC (DCM/MeOH= 10:1) to obtain the desired product as a yellow solid, namely compound 7-5 (95 mg, 38.32% yield). ESI-MS m/z: 700.4 $[M+H]^+$.

Step 6: Synthesis of compound 7-6

At room temperature, compound 7-5 (95 mg, 135.65 μmol) was dissolved in a mixed solvent of DCM (2 mL) and TFA (1 mL), and the mixture was moved to 40°C for about 1 h. After the reaction was complete, it was directly concentrated to remove the solvent to obtain the crude target product as a yellow solid, namely compound 7-6 (70 mg, crude product). ESI-MS m/z: 516.32 $[M+H]^+$.

Step 7: Synthesis of compound 7

Under the protection of nitrogen, in an ice water bath, add the THF solution of acryloyl chloride (12.28.03 mg, 135.64 μmol) to compound 7-6 (70 mg, 135.64 μmol), THF (4 mL) and saturated $Na_2CO_3$ (1 mL) solution, and the reaction was complete. The reaction mixture was poured into water, extracted with ethyl acetate: methanol = 10:1, the organic layer was washed with brine, dried over $Na_2SO_4$ and concentrated in vacuo. The concentrate was purified by pre-TLC (DCM/MeOH=10:1) to obtain the desired product was compound 7 (3.2 mg, 3.88% yield, 93.68% purity) as a yellow solid. ESI-MS m/z: 570.24$[M+H]^+$.

**Example 8: (1-(7-(7-(5-methyl-1H-indazol-4-yl)-2-(N-methylpyrrol-3-yl)-8-(2,2,2-trifluoroetho xy)-6-vinylquinazolin-4-yl)-2,7-diazaspiro[3.5]nonan-2-yl)prop-2-en-1-one)**

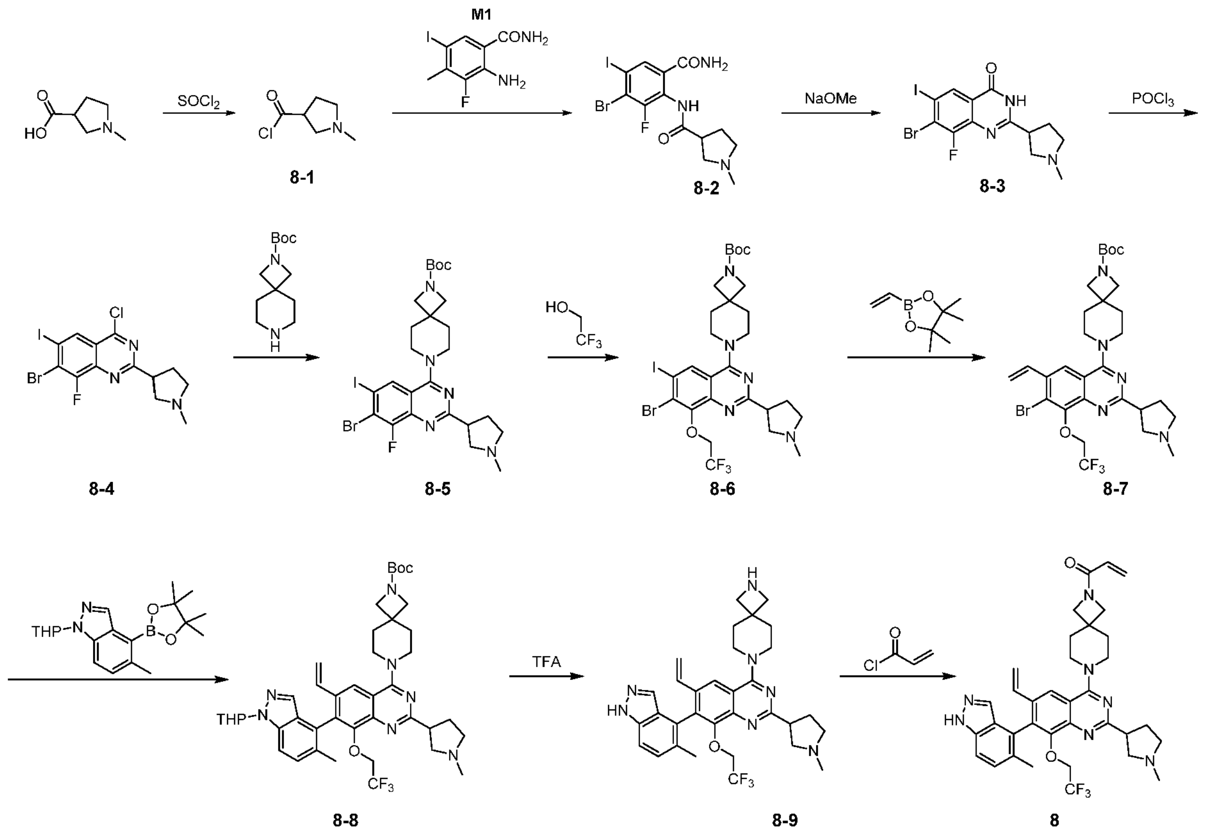


8-1 8-2 8-3 8-4 8-5 8-6 8-7 8-8 8-9 8

Step 1: Synthesis of compound 8-1

Under room temperature and nitrogen protection, N-methylpyrrole-3-carboxylic acid (600 mg, 4.65 mmol) was dissolved in $SOCl_2$ (5.5 mL), the temperature was raised to 70°C, and the reaction was stirred for 1 h. It was concentrated in vacuo to remove the thionyl chloride to obtain the target product as a white solid, namely compound 8-1 (68 mg, crude product).

Step 2: Synthesis of compound 8-2

At room temperature, compound 8-1 (369 mg, 2.5 mmol) was added to compound M1 (300 mg, 0.835 mmol) in THF (5 mL), and the mixture was stirred for 16 hours. After the reaction was complete, THF was removed by rotary evaporation. Using dichloromethane: methanol=90:10 as a developing solvent, column chromatography was used to separate the target product as a pale yellow solid, namely compound 8-2 (262 mg, LC-MS purity 93.9%). ESI-MS m/z: 469.93, 471.94 $[M+H]^+$.

Step 3: Synthesis of compound 8-3

At room temperature, sodium methoxide (151 mg, 2.79 mmol) was added to compound 8-2 (262 mg, 0.557 mmol) in toluene (10 mL), and the temperature was raised to 110°C under reflux stirring for 16 hours. After the reaction was completed, the mixture was poured into 40 mL of ice water, the mixture was extracted with ethyl acetate, the organic phase was dried over $Na_2SO_4$ and concentrated in vacuo to obtain the target product as a pale yellow solid, namely compound 8-3 (240 mg, crude). ESI-MS m/z: 451.96, 453.91$[M+H]^+$.

Step 4: Synthesis of compound 8-4

Under room temperature and nitrogen protection, DIEA (0.5 mL) was added to the $POCl_3$ (5 mL) solution of compound 8-3 (226 mg, 0.499 mmol), and the temperature was raised to 110°C stirring for 3 hours. After the reaction was complete, the $POCl_3$ was removed by rotary evaporation, the mixture was poured into ice water, the mixture was extracted with ethyl acetate, the organic phase was collected, washed with saturated brine, dried over $Na_2SO_4$ and concentrated in vacuo to obtain the target product as a brownish yellow solid, namely compound 8-4 (116 mg, crude product). ESI-MS m/z: 469.96, 471.94$[M+H]^+$.

Step 5: Synthesis of compound 8-5

At room temperature, DIEA (96 mg, 0.74 mmol) was added to compound 8-4 (116 mg, 0.246 mmol), 2,7-diazaspiro[3.5]nonane-2-carboxylic acid tert-butyl ester (56 mg, 0.246 mmol) in dioxane (5 mL) and the mixture was stirred at room temperature for 2 hours. After the reaction was complete, the dioxane was spin-dried, ice water was added, the mixture was extracted with ethyl acetate/methanol=10:1, and the organic phase was collected and spin-dried. The residue was purified by silica gel column chromatography (developing solvent: DCM:MeOH=10:1) to obtain the target yellow solid product, namely compound 8-5 (58 mg, LC-MS purity 96.8%). ESI-MS m/z: 660.08, 662.07 $[M+H]^+$.

Step 6: Synthesis of compound 8-6

Add compound 8-5 (56 mg, 84.8 μmol) to a fully dried reaction flask, dissolve it with dioxane (3 mL), then trifluoroethanol (42 mg, 424 μmol) and $Cs_2CO_3$ (55 mg, 170 μmol) were added, and the reaction was heated to 100°C for 1 h. After the reaction was complete, the reaction solution was diluted with THF, and $Cs_2CO_3$ was removed by filtration. After the filtrate was spin-dried, the sample was dried using a diaphragm pump to obtain a yellow solid target product, namely compound 8-6 (60 mg, LC-MS purity 91.7%). ESI-MS m/z: 740.03, 742.07$[M+H]^+$.

Step 7: Synthesis of compound 8-7

Under the protection of $N_2$, add compound 8-6 (60 mg, 81 μmol), 4,4,5,5-tetramethyl-2-vinyl-1,3,2-dioxaborolane alkane (13 mg, 81 μmol) to the dry reaction flask, dissolve with dioxane (5 mL), then $K_2CO_3$ (22.40 mg, 162 μmol) and $H_2O$ (0.5 mL) were added, after the dispersion was uniform, 1,1'-bisdiphenylphosphine iron palladium dichloride (5.93 mg, 8.1 μmol) was added, $N_2$ was replaced three times. The temperature was raised to 70°C and stirred for 1 hour. After the reaction was complete, the reaction solution was cooled to room temperature, water was added and extracted with ethyl acetate: methanol = 10:1, saturated brine was back extracted, the organic phase was collected, dried over $Na_2SO_4$, filtered, and spin-dried. The concentrate was passed through pre-TLC (developing solvent: DCM/MeOH=10:1) was purified to obtain a yellow solid product, namely compound 8-7 (44 mg, LC-MS purity 95.4%). ESI-MS m/z: 640.11, 642.13 $[M+H]^+$.

Step 8: Synthesis of compound 8-8

Compound 8-7 (44 mg, 68.7 μmol) was added to a nitrogen-protected reaction flask, dissolved with dioxane (5 mL), and 5-methyl-1-tetrahydropyran-2-yl-4-(4,4,5,5-tetramethyl-1,3,2-dioxaborolan-2-yl)indazole (47 mg, 137 μmol),

$K_3PO_4$ (29 mg, 137 μmol), deionized water (0.5 mL) and $Pd(PPh_3)_4$ (8 mg, 9 μmol) were added. The temperature was raised to 85°C and reacted for 3 hours. The reaction mixture was cooled to room temperature, water was added and extracted with ethyl acetate: methanol = 10:1, saturated brine was back extracted, the organic phase was collected, dried over $Na_2SO_4$, filtered, and spin-dried. The concentrate was passed through pre-TLC (DCM/MeOH=10:1) to obtain a yellow solid product, namely compound 8-8 (18 mg, LC-MS purity 97.0%). ESI-MS m/z: 776.87[M+H]$^+$.

Step 9: Synthesis of compound 8-9

At room temperature, compound 8-8 (18 mg, 22.3 μmol) was dissolved in a mixed solvent of DCM (2 mL) and TFA (1 mL), and the temperature was raised to 40°C reacting for 1 h. After the reaction was completed, the solvent was removed by concentration to obtain a yellow solid crude product, namely compound 8-9 (21 mg, crude product), which was directly used for the next step. ESI-MS m/z: 592.94[M+H]$^+$.

Step 10: Synthesis of compound 8

Under nitrogen protection and ice water bath, acryloyl chloride (2.0 mg, 22.0 μmol) in THF (1 mL) solution was add dropwise to compound 1-9 (13 mg, 22.0 μmol) in THF (4 mL) and saturated $NaHCO_3$ (1 mL) mixed solution, stirring for 10 minutes. The reaction solution was poured into water, extracted with ethyl acetate: methanol = 10:1, and back-extracted with saturated brine. The organic phase was collected, dried over $Na_2SO_4$, filtered, and spin-dried. The concentrate was passed through pre-TLC (developing solvent: DCM/ MeOH=10:1) to obtain a light yellow solid product, namely compound 8 (7 mg, LC-MS purity 96.2%). ESI-MS m/z: 646.34[M+H]$^+$.

**Example 9: (1-(7-(2-(1-ethylpiperidin-4-yl)-7-(5-methyl-1H-indazol-4-yl)-8-(2,2,2-trifluoroeth oxy)-6-vinylquinazolin-4-yl)-2,7-diazaspiro[3.5]nonan-2-yl)prop-2-en-1-one)**

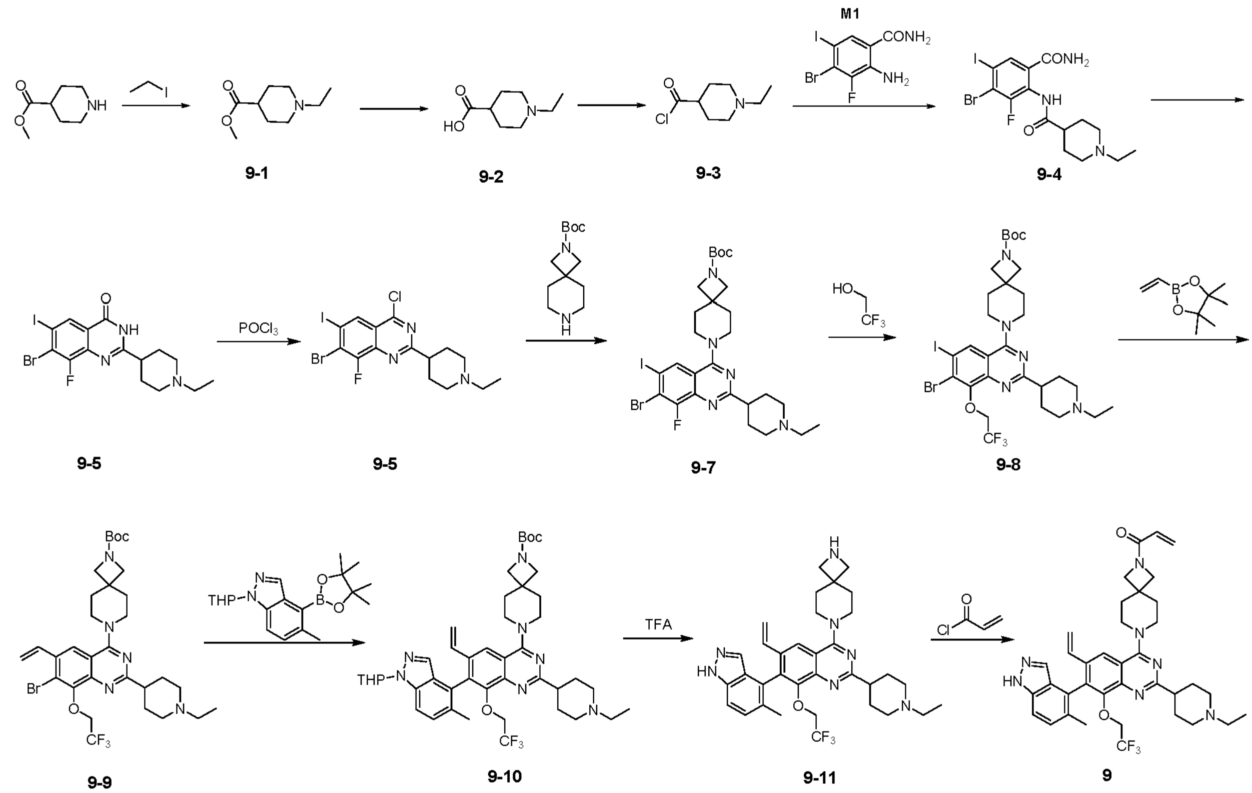


9-1 9-2 9-3 9-4
9-5 9-5 9-7 9-8
9-9 9-10 9-11 9

Step 1: Synthesis of compound 9-1

At room temperature, iodoethane (3.27 g, 20.95 mmol, 1.68 mL) and $K_2CO_3$ (2.89 g, 20.95 mmol) were added to methyl piperidine-4-carboxylate (1.5 g, 10.48 mmol) in EtOH (15 mL) solution, the mixture was moved to 85°C and

refluxed for 1h. It was concentrated in vacuo to remove EtOH to obtain the target product 9-1 (1.7 g, crude product) as a white solid. ESI-MS m/z: 172.06 $[M+H]^+$.

Step 2: Synthesis of compound 9-2

At room temperature, LiOH (447.57 mg, 18.69 mmol) was added to a MeOH (10 mL) solution of compound 9-1 (1.7 g, 9.34 mmol), and the mixture was moved to 50°C reacting for 1 hour. Add the dilute hydrochloric acid solution equivalent to LiOH to free the target product and spin to dry directly. The residue was purified by flash silica gel column chromatography (DCM:MeOH=92:8-90:10) to obtain the desired target as a yellow solid product 9-2 (700 mg, 44.85% yield). ESI-MS m/z: 158.15 $[M+H]^+$.

Step 3: Synthesis of compound 9-3

At room temperature and under the protection of nitrogen, compound 9-2 (400 mg, 3.49 mmol) was dissolved in $SOCl_2$ (3 mL), the mixture was moved to 70°C, and stirred for 1 h. It was concentrated in vacuo to remove the thionyl chloride to obtain the target product 9-3 (450 mg, crude product) as a white solid.

Step 4: Synthesis of compound 9-4

At room temperature, compound 9-3 (293.63 mg, 1.67 mmol) was added to compound M1 (300 mg, 835.81 μmol) in THF (10 mL), and the mixture was moved to 45°C and stirred for 12 hours. A large amount of solids precipitated during the reaction, and the solid was filtered with suction, and the solid was the target product 9-4 (150 mg, crude product). ESI-MS m/z: 498.00 $[M+H]^+$.

Step 5: Synthesis of compound 9-5

At room temperature, sodium methoxide (81.34 mg, 1.51 mmol) was added to toluene (5 mL) of compound 9-4 (150 mg, 301.13 μmol), and the mixture was moved to 110°C under reflux and stirred for 4 hours. After the reaction was completed, the mixture was poured into ice water, and the mixture was extracted with ethyl acetate. The organic phase was dried over $Na_2SO_4$ and concentrated in vacuo to obtain the crude target product 9-5 (150 mg, crude product) as a brown solid. ESI-MS m/z: 480.00$[M+H]^+$.

Step 6: Synthesis of compound 9-6

At room temperature and under the protection of nitrogen, DIEA (1.0 mL) was added to compound 9-5 (150 mg, 312.43 μmol) of $POCl_3$ (5 mL), and the mixture was moved to 110°C and stirred for 3 hours. After the reaction was complete, it was directly concentrated to remove $POCl_3$, diluted with EA, extracted with water three times, the organic phases are combined, dried and concentrated to obtain the target product 9-6 (150 mg, crude product) as a white solid. ESI-MS m/z: 497.99$[M+H]^+$.

Step 7: Synthesis of compound 9-7

At room temperature, DIEA (116.65 mg, 902.60 μmol, 157.21 μL) was added to compound 9-6 (150 mg, 300.87 μmol), 2,7-diazaspiro[3.5]nonane-2-carboxylic acid tert-butyl ester (68.09 mg, 300.87 μmol) of dioxane (5 mL), the mixture was moved to 45°C and stirred for 1 hour. The mixture was poured into ice water, the mixture was extracted with ethyl acetate/methanol=10:1, the organic layer was washed with brine, dried over $Na_2SO_4$ and concentrated in vacuo, and the residue was purified by flash silica gel column chromatography (DCM:MeOH=10:1), the desired target product 9-7 (50 mg, 72.63 μmol, 24.14% yield) was obtained as a yellow solid. ESI-MS m/z: 688.14$[M+H]^+$.

Step 8: Synthesis of compound 9-8

Under the protection of nitrogen, trifluoroethanol (21.80 mg, 217.89 μmol) was added to compound 9-7 (50 mg, 72.63 μmol), $Cs_2CO_3$ (47.33 mg, 145.26 μmol) in dioxane (1 mL) and moved to 100°C for 3 h. The mixture was poured into ice water, the mixture was extracted with ethyl acetate/methanol=10:1, the organic layer was washed with brine, dried over $Na_2SO_4$ and concentrated in vacuo to obtain the desired target product 9-7 (55 mg, crude) as a yellow solid. ESI-MS m/z: 768.18$[M+H]^+$.

Step 9: Synthesis of compound 9-9

Under the protection of nitrogen, Pd(dppf)$Cl_2$.$CH_2Cl_2$ (5.84 mg, 7.16 μmol) was added to compound 9-8 (55 mg, 71.57 μmol), $K_2CO_3$ (19.78 mg, 143.15 μmol), 4,4,5,5-tetramethyl-2-vinyl-1,3,2-dioxaborolane (11.02 mg, 71.57 μmol) in a mixed solution of dioxane (1.0 mL) and $H_2O$ (0.1 mL), stirring at 70°C for 1h. The reaction mixture was cooled to room temperature, water was added to the reaction mixture, extracted with ethyl acetate: methanol = 10:1, the organic layer was washed with brine, dried over $Na_2SO_4$ and concentrated in vacuo. The concentrate was passed through pre-TLC (DCM/MeOH= 10:1) to obtain the desired product 9-9 (25 mg, 37.39 μmol, 52.24% yield) as a yellow solid. ESI-MS m/z: 668.18$[M+H]^+$.

Step 10: Synthesis of compound 9-10

Under the protection of nitrogen, $Pd(PPh_3)_4$ (8.64 mg, 7.48 μmol) was added to compound 9-9 (25 mg, 37.39 μmol), 5-methyl-1-tetrahydropyran-2-yl-4-(4,4,5,5-tetramethyl-1,3,2-dioxaborolan-2-yl)indazole (25.59 mg, 74.78 μmol), $K_3PO_4$ (15.87 mg, 74.78 μmol) in the dioxane (1 mL), $H_2O$ (0.25 mL) solution, the reaction mixture was moved to 85°C and reacted for about 3 hours. The reaction mixture was cooled to room temperature, water was added to the reaction mixture, extracted with ethyl acetate: methanol = 10:1, the organic layer was washed with brine, dried over $Na_2SO_4$ and concentrated in vacuo. The concentrate was passed through pre-TLC (DCM/MeOH= 10:1) to obtain the desired product 9-10 (20 mg, 24.88 μmol, 66.53% yield) as a yellow solid. ESI-MSm/z: 804.56 $[M+H]^+$.

Step 11: Synthesis of compound 9-11

At room temperature, compound 9-10 (20 mg, 24.88 μmol) was dissolved in a mixed solvent of DCM (1 mL) and TFA (0.5 mL), and moved to 40°C reacting for about 1 h. After the reaction was completed, the solvent was directly concentrated to remove the solvent to obtain the target product 9-11 (18 mg, crude product) as a yellow solid. ESI-MS m/z: 620.28$[M+H]^+$.

Step 12: Synthesis of compound 9

Under the protection of nitrogen, in an ice water bath, add the THF solution of acryloyl chloride (3.15 mg, 34.85 μmol) to compound 9-11 (18 mg, 29.05 μmol), THF (2 mL) and saturated $Na_2CO_3$ (0.5 mL) solution, and the reaction was complete. The reaction mixture was poured into water, extracted with ethyl acetate: methanol=10:1, the organic layer was washed with brine, dried over $Na_2SO_4$ and concentrated in vacuo, the concentrate was purified by pre-TLC (DCM/MeOH=10:1) to obtain the desired product 9 (10.7 mg, 52.69% yield, 96.36% purity) as a yellow solid. ESI-MS m/z: 674.37$[M+H]^+$. $^1$H NMR(500MHz,DMSO-$d_6$) δ13.05 (s, 1H), 8.30 (s, 1H), 8.01(s, 1H), 7.50(d, *J*=8.5Hz, 1H), 7.40(s, 1H) ,7.33(d, *J*=8.5Hz, 1H), 6.35(m, 1H), 6.18-6.05 (m, 2H), 5.82-5.66 (m, 2H), 5.11 (d, *J*= 11.1Hz, 1H), 4.97(m, 1H), 4.68(m, 1H), 3.23-3.05 (m, 4H), 2.80 (m, 1H), 2.25 (m, 2H), 2.05 (d, *J*=6.7Hz, 5H), 1.95 (m, 6H), 1.62-1.55 (m, 1H), 1.32 (m, 1H), 1.07 (t, *J*=7.2Hz, 3H), 0.94 (t, *J*=7.3Hz, 2H).

**Example 10: (1-(7-(6-chloro-8-ethoxy-7-(5-methyl-1H-indazol-4-yl)-2-(1-methylpiperidine -4-yl)-quinazolin-4-yl)-2,7-diazaspiro[3.5]nonan-2-yl)prop-2-en-1-one)**

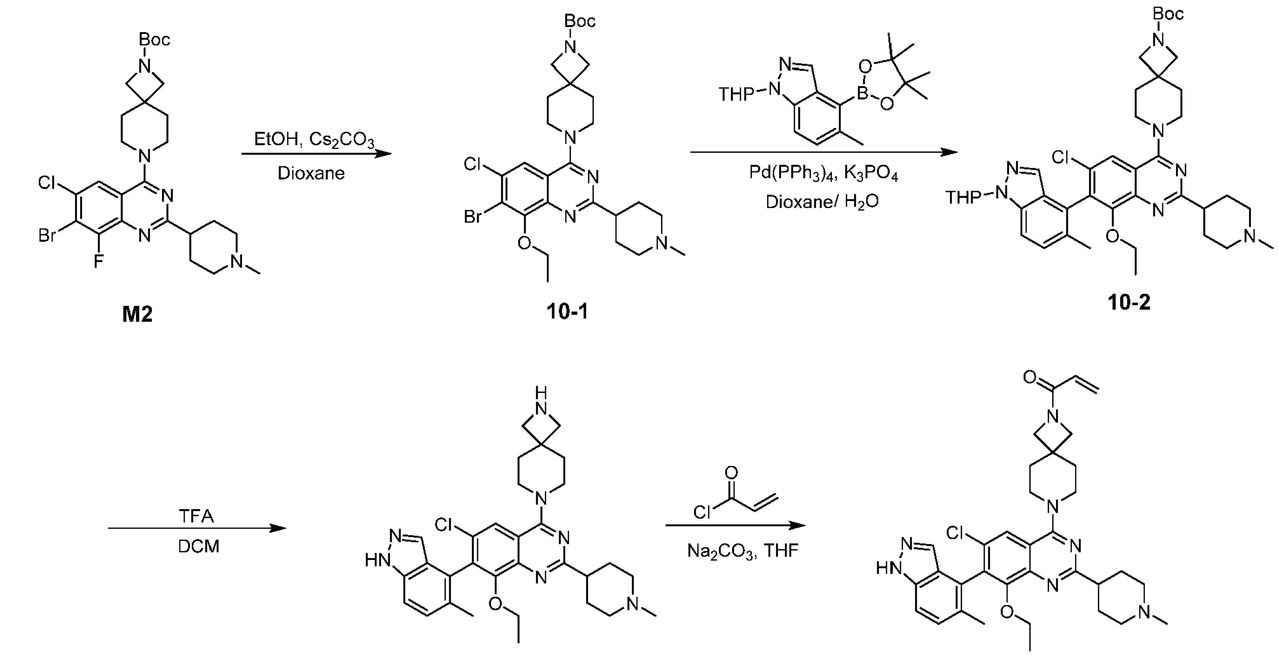


M2 10-1 10-2 10-3 10

Step 1: Synthesis of compound 10-1

Under nitrogen protection, ethanol (395 mg, 8.60 mmol) was added to compound M2 (1.0 g, 1.72 mmol), $Cs_2CO_3$ (1.68 g, 5.16 mmol) in dioxane (10 mL), and the mixture was moved to 80°C for reaction for 1 h. After cooling to room temperature, water was slowly added to precipitate a solid, which was filtered and dried to obtain a yellow solid 10-1 (0.96 g, crude product). ESI-MS m/z: 608.19[M+H]$^+$.

Step 2: Synthesis of compound 10-2

Under the protection of nitrogen, $Pd(PPh_3)_4$ (341 mg, 295 μmol) was added to compound 10-1 (900 mg, 1.48 mmol), 5-methyl-1-tetrahydropyran-2-yl-4-(4,4,5,5-tetramethyl-1,3,2-dioxaborolan-2-yl)indazole (758.6 mg, 2.22 mmol), $K_3PO_4$ (941.1 mg, 4.43 mmol) of dioxane (20 mL), $H_2O$ (5 mL) solution, the mixture was moved to 85°C to react for 4 h. It was cooled to room temperature, diluted with water, and extracted three times with EA. The organic phases were combined and dried over anhydrous $Na_2SO_4$, filtered, spin-dried, and separated and purified by silica gel column (DCM: MeOH=10: 1) to obtain a yellow solid 10-2 (665 mg, 49.9% yield). ESI-MS m/z: 744.39 [M+H]$^+$.

Step 3: Synthesis of compound 10-3

At room temperature, compound 10-2 (120 mg, 130.58 μmol) was dissolved in a mixed solvent of DCM (6 mL) and TFA (3 mL), and moved to 40°C to react for about 1 h. After the reaction was complete, it was directly concentrated to remove the solvent to obtain crude brown oil 10-3 (73 mg, crude product). ESI-MS m/z: 560.28[M+H]$^+$.

Step 4: Synthesis of compound 10

Under the protection of nitrogen, in an ice water bath, add acryloyl chloride (12.1 mg, 133.67 μmol) in THF (1 mL) solution to compound 10-3 (73 mg, 133.67 μmol), THF (4 mL) and saturated $Na_2CO_3$ (1 mL) solution, the temperature was kept and the mixture was stirred for 5 min. The reaction mixture was diluted with water, extracted three times with EA, the organic phases were combined and dried with anhydrous $Na_2SO_4$, filtered, spin dried, and separated and purified by pre-TLC (DCM:MeOH=10:1) to obtain white solid 10 (36.5 mg, 43.3% yield, 95.23% purity). ESI-MS m/z: 614.40 [M+H]$^+$.

**Example 11: (1-(7-(8-ethoxy-7-(5-methyl-1H-indazol-4-yl)-2-(1-methylpiperidin-4-yl)-6-vinyl quinazolin-4-yl)-2,7-diazaspiro[3.5]nonan-2-yl)prop-2-en-1-one)**

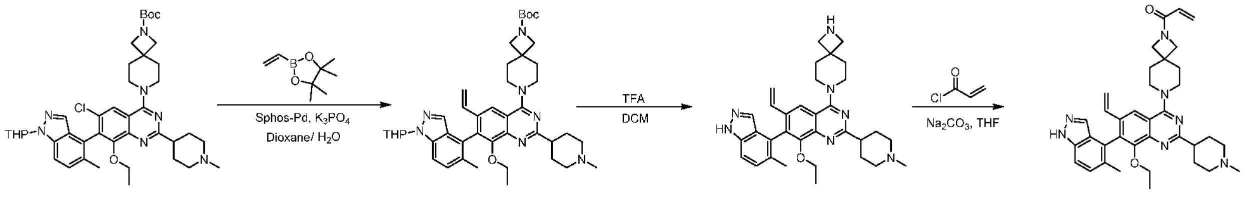


10-2 11-1 11-2 11

Step 1: Synthesis of compound 11-1

Under the protection of nitrogen, Sphos Pd G2 (51.7 mg, 71.74 μmol) was added to compound 10-2 (300 mg, 0.36 mmol), $K_3PO_4$ (228.4 mg, 1.08 mmol), vinyl borate (110.49 mg, 0.72 mmol) in a mixed solution of dioxane (4 mL) and $H_2O$ (1 mL), the mixture was stirred at 100°C for 4 h. It was cooled to room temperature, diluted with water, and extracted three times with EA. The organic phases were combined and dried over anhydrous $Na_2SO_4$, filtered, spin-dried, and separated and purified by pre-TLC (DCM:MeOH=10:1) to obtain a white solid 11-1 (185 mg, 70.08% yield). ESI-MS m/z: 736.45[M+H]$^+$.

Step 2: Synthesis of compound 11-2

At room temperature, compound 11-1 (185 mg, 251.37 μmol) was dissolved in a mixed solvent of DCM (4 mL) and TFA (2 mL), and the mixture was moved to 40°C to react for about 1 h. After the reaction was complete, it was directly concentrated to remove the solvent to obtain crude brown oil 11-2 (138 mg, crude product). ESI-MS m/z: 552.34[M+H]$^+$.

Step 3: Synthesis of compound 11

Under the protection of nitrogen, in an ice water bath, add acryloyl chloride (22.6 mg, 250.13 μmol) in THF (1 mL) solution to compound 11-2 (138 mg, 250.13 μmol), THF (4 mL) and saturated $Na_2CO_3$ (1 mL), the temperature was kept and stirred for 5 min. The mixture was diluted with water, extracted three times with EA, the organic phases were combined and dried with anhydrous $Na_2SO_4$, filtered, spin dried, and separated and purified by pre-TLC (DCM:MeOH = 10:1) to obtain a white solid 11 (62.5 mg, 38.2% yield, 92.62% purity). ESI-MS m/z: 606.45 [M+H]$^+$. $^1$H NMR(500MHz, Chloroform-d) δ7.90 (s, 1H), 7.51 (s, 1H), 7.46 (d, *J*=8.5Hz, 1H), 7.37-7.34 (m, 1H), 6.39(dd, *J* = 17.0, 1.9 Hz, 1H), 6.24 (m, 2H), 5.71 (dd, *J* = 10.2, 1.9 Hz, 1H), 5.62 (d, *J* = 17.4 Hz, 1H), 5.03 (d, *J* = 11.0 Hz, 1H), 4.22 (m, 1H), 4.03 (s, 2H), 3.93 (s, 2H), 3.91-3.84 (m, 1H), 3.75(m, 4H), 3.00 (t, *J*=6.1Hz , 2H), 2.87 (m, 1H), 2.35 (s, 3H), 2.16 (s, 3H), 2.12-2.08 (m, 5H), 2.03 (m, 4H), 1.33 (m, 4H).

**Example 12: (1-(7-(6-chloro-8-methoxy-7-(5-methyl-1H-indazol-4-yl)-2-(1-methylpiperidine - 4-yl)-quinazolin-4-yl)-2,7-diazaspiro[3.5]nonan-2-yl)prop-2-en-1-one)**

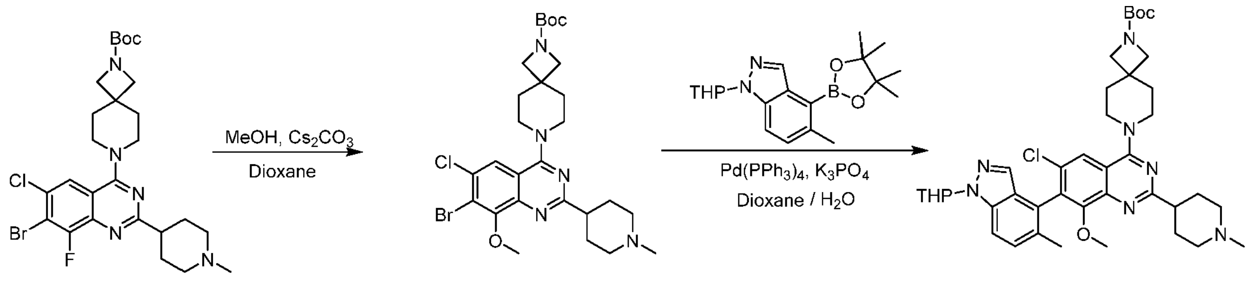


M2 12-1 12-2

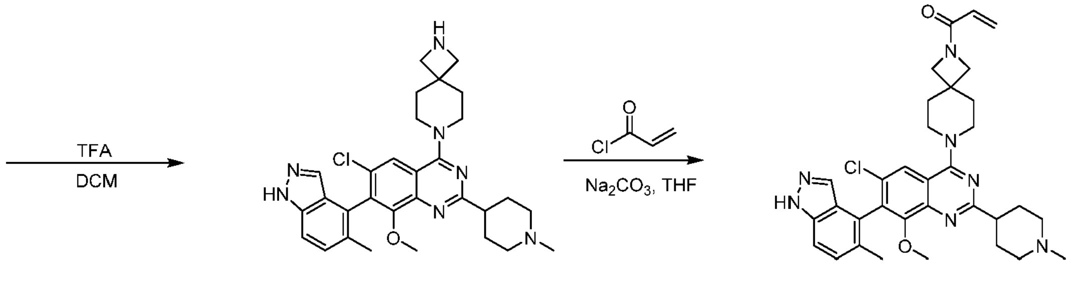


12-3 12

Step 1: Synthesis of compound 12-1

Under the protection of nitrogen, methanol (275 mg, 8.60 mmol) was added to compound M2 (1.0 g, 1.72 mol), $Cs_2CO_3$ (1.68 g, 5.16 mmol) in dioxane (10 mL), and the mixture was moved to 70°C to react for 5 h. After cooling to room temperature, water was slowly added to precipitate a solid, which was filtered and dried to obtain a yellow solid 12-1 (0.91 g, crude product). ESI-MS m/z: 594.18$[M+H]^+$.

Step 2: Synthesis of compound 12-2

Under the protection of nitrogen, $Pd(PPh_3)_4$ (77.69 mg, 67.23 μmol) was added to compound 12-1 (200 mg, 0.336 mmol), 5-methyl-1-tetrahydropyran-2-yl-4-(4,4,5,5-tetramethyl-1,3,2-dioxaborolan-2-yl)indazole (172.5 mg, 0.504 mmol), $K_3PO_4$ (214.1 mg, 1.01 mmol) of dioxane (4 mL) and $H_2O$ (1 mL) solution, the mixture was moved to 85°C and reacted for 4 h. The reaction mixture was cooled to room temperature, diluted with water, and extracted three times with EA, the organic phases were combined and dried with anhydrous $Na_2SO_4$, filtered, spin-dried, and separated and purified on silica gel column (DCM:MeOH=10:1) to obtain yellow solid 12-2 (202 mg, 79.8% yield). ESI-MS m/z: 730.38$[M+H]^+$.

Step 3: Synthesis of compound 12-3

At room temperature, compound 12-2 (200 mg, 273.85 μmol) was dissolved in a mixed solvent of DCM (6 mL) and TFA (3 mL), and moved to 40°C reacting for about 1 h. After the reaction was complete, it was directly concentrated to remove the solvent to obtain crude brown oil 12-3 (149 mg, crude product). ESI-MS m/z: 546.27 $[M+H]^+$.

Step 4: Synthesis of compound 12

Under the protection of nitrogen, in an ice water bath, acryloyl chloride (24.7 mg, 272.84 μmol) in THF (1 mL) solution was added to compound 12-3 (149 mg, 272.84 μmol), THF (4 mL), and saturated $Na_2CO_3$ (1 mL) solution, keeping the temperature and stirring for 5 min. The reaction mixture was diluted with water and extracted three times with EA, the organic phases were combined and dried with anhydrous $Na_2SO_4$, filtered, spin dried, and separated and purified by pre-TLC (DCM:MeOH=10:1) to obtain white solid 12 (80.3 mg, 47.27% yield, 96.39% purity). ESI-MS m/z: 600.35$[M+H]^+$.

**Example 13: (1-(7-(8-methoxy-7-(5-methyl-1H-indazol-4-yl)-2-(1-methylpiperidin-4-yl)-6-vinylquinazolin-4-yl)-2,7-diazaspiro[3.5]nonan-2-yl)prop-2-en-1-one)**

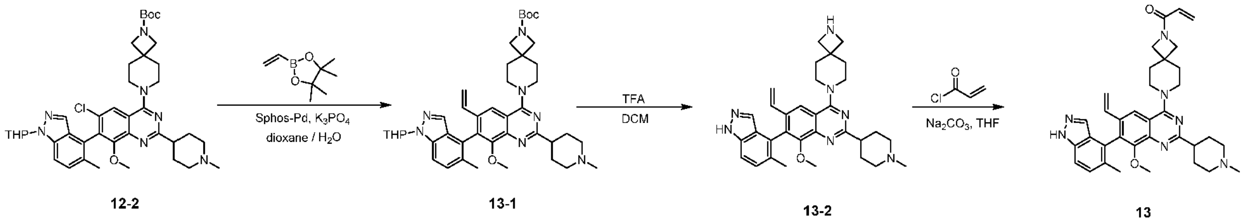


12-2 13-1 13-2 13

Step 1: Synthesis of compound 13-1

Under the protection of nitrogen, Sphos Pd G2 (59.2 mg, 82.15 μmol) was added to compound 12-2 (300 mg, 0.41 mmol), $K_3PO_4$ (261.5 mg, 1.23 mmol), vinyl borate (126.53 mg, 0.82 mmol) in a mixed solution of dioxane (4 mL) and $H_2O$ (1 mL), the mixture was stirred at 100°C for 4 h. The reaction mixture was reduced to room temperature, diluted with water, and extracted three times with EA, the organic phases were combined and dried with anhydrous $Na_2SO_4$, filtered, spin dried, and separated and purified by pre-TLC (DCM:MeOH=10:1) to obtain a white solid 13-1 (205 mg, 69.13% yield). ESI-MS m/z: 722.43 $[M+H]^+$.

Step 2: Synthesis of compound 13-2

At room temperature, compound 13-1 (205 mg, 283.93 μmol) was dissolved in a mixed solvent of DCM (6 mL) and TFA (3 mL), and moved to 40°C reacting for about 1 h. After the reaction was complete, it was directly concentrated to remove the solvent to obtain crude brown oil 13-2 (152 mg, crude product). ESI-MS m/z: 538.32$[M+H]^+$.

Step 3: Synthesis of compound 13

Under the protection of nitrogen, in an ice-water bath, acryloyl chloride (25.4 mg, 282.52 μmol) in THF (1 mL) solution was added to compound 13-2 (152 mg, 282.52 μmol), THF (4 mL), and saturated $Na_2CO_3$ (1 mL) solution, keeping the temperature and stirring for 5 min. The reaction mixture was diluted with water, extracted three times with EA, the organic phases was combined and dried with anhydrous $Na_2SO_4$, filtered, spin dried, and separated and purified by pre-TLC (DCM:MeOH=10:1) to obtain a yellow solid 13 (87.2 mg, 52.1% yield, 93.02% purity). ESI-MS m/z: 592.43 $[M+H]^+$. $^1H$ NMR (500MHz, Chloroform-d) δ 7.90 (s, 1H), 7.52 (s, 1H), 7.47 (d, *J* = 8.5 Hz, 1H), 7.36 (d, *J*=8.5Hz, 1H), 6.39 (dd, *J*=17.0, 2.0Hz, 1H), 6.22 (m, 2H), 5.72 (dd, *J*=10.2, 2.0Hz, 1H), 5.62(d, *J*=17.4Hz, 1H), 5.03(d, *J* = 11.0Hz, 1H), 4.03 (s, 2H), 3.93 (s, 2H), 3.75 (m, 8H), 3.00 (m, 3H), 2.35 (s, 3H), 2.14 (s, 3H), 2.11 (d, *J*=7.1Hz, 5H), 2.04 (m, 4H).

**Example 14: (1-(7-(2-(1-acetylpiperidin-4-yl)-7-(5-methyl-1H-indazol-4-yl)-8-(2,2,2-trifluoroe thoxy)-6-vinylquinazolin-4-yl)-2,7-diazaspiro[3.5]nonan-2-yl)prop-2-en-1-one)**

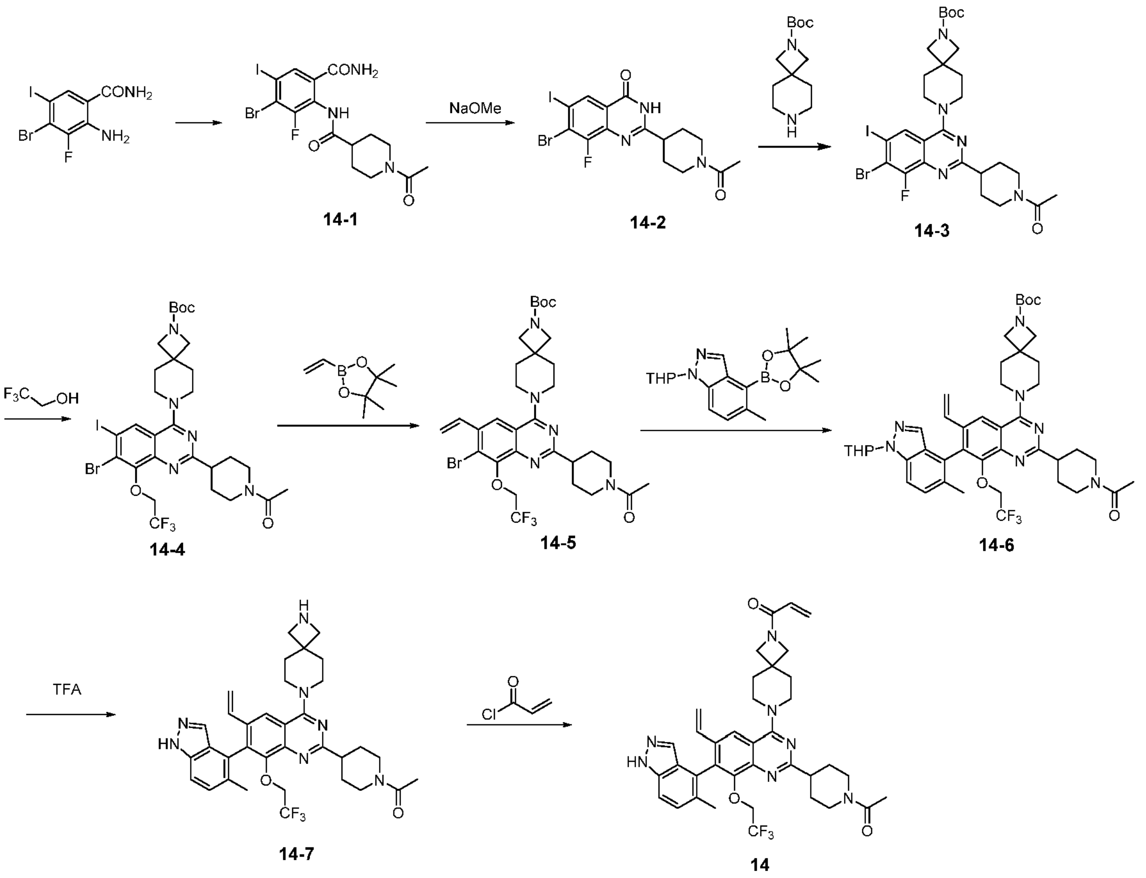


14-1 14-2 14-3 14-4 14-5 14-6 14-7 14

Step 1: Synthesis of compound 14-1

At room temperature, acetylpiperidine-4-carbonyl chloride (567 mg, 3.0 mmol) was added to compound M1 (300 mg, 0.84 mmol) in THF (10 mL), and the mixture was moved to 40°C and stirred for 4 hours. After the reaction was complete, the THF was directly concentrated to obtain the target product 14-1 (460 mg, crude product) as a yellow solid. ESI-MS m/z: 512.14 $[M+H]^+$.

Step 2: Synthesis of compound 14-2

At room temperature, sodium methoxide (224.56 mg, 4.16 mmol) was added to toluene (10 mL) of compound 14-1 (460 mg, 0.83 mmol), and the mixture was moved to 110°C under reflux and stirred for 8 hours. After the reaction was completed, the mixture was poured into ice water, and the mixture was extracted with ethyl acetate. The organic phase was dried over $Na_2SO_4$ and concentrated in vacuo to obtain the target product 14-2 (210 mg, crude product) as a white solid. ESI-MS m/z: 494.06 $[M+H]^+$.

Step 3: Synthesis of compound 14-3

At room temperature, compound 14-2 (170 mg, 0.34 mmol) was added to DMF (5 mL), followed by 2,7-diazaspiro[3.5]nonane-2-carboxylic acid tert-butyl ester (92 mg, 0.408 mmol), BOP (376 mg, 0.85 mmol), DBU (258 mg, 1.7 mmol), react at 40°C for 6 h. The reaction did not proceed until the remaining 30% of the raw material was left. Water was added to the reaction and extracted with ethyl acetate. The organic phase was dried over $Na_2SO_4$ and concentrated in vacuo. The concentrate was passed through pre-TLC (DCM/MeOH=15:1) to obtain a yellow solid product 14-3 (150 mg, 62.2% yield). ESI-MS m/z: 702.28 $[M+H]^+$.

Step 4: Synthesis of compound 14-4

Under the protection of nitrogen, trifluoroethanol (106 mg, 1.06 mmol) was added to compound 14-3 (150 mg, 0.214 mmol), $Cs_2CO_3$ (139 mg, 0.428 mmol) in dioxane (3 mL), and moved to 100°C for reaction 3 h. The mixture was poured into ice water, and extracted with ethyl acetate, the organic layer was washed with brine, dried over $Na_2SO_4$ and

concentrated in vacuo. The concentrate was purified by pre-TLC (developing solvent: DCM/MeOH=15:1) to obtain the desired target product 14-4 (120 mg, 71.9% yield) as a yellow oil. ESI-MS m/z: 782.48 $[M+H]^+$.

Step 5: Synthesis of compound 14-5

Under the protection of nitrogen, $Pd(dppf)Cl_2.CH_2Cl_2$ (12 mg, 14.7 μmol) was added to compound 14-4 (115 mg, 0.147 mmol), $K_2CO_3$ (41 mg, 0.294 mmol), 4,4,5,5-tetramethyl-2-vinyl-1,3,2-dioxaborolane (22.7 mg, 0.147 mmol) in a mixed solution of dioxane (2.5 mL) and $H_2O$ (0.5 mL), stirring at 70°C for 6 h. The reaction mixture was cooled to room temperature, water was added to the reaction mixture, extracted with ethyl acetate, the organic layer was washed with brine, dried over $Na_2SO_4$ and concentrated in vacuo, the residue was purified by flash silica gel column chromatography (PE:EA=1:2), the desired target product 14-5 (65 mg, 65.4% yield) was obtained as a yellow solid. ESI-MS m/z: 682.58$[M+H]^+$.

Step 6: Synthesis of compound 14-6

Under the protection of nitrogen, $Pd(PPh_3)_4$ (22 mg, 19 μmol) was added to compound 14-5 (65 mg, 95 μmol), 5-methyl-1-tetrahydropyran-2-yl-4-(4,4,5,5-tetramethyl-1,3,2-dioxaborolan-2-yl)indazole (65 mg, 0.19 mmol), $K_3PO_4$ (40 mg, 0.19 mmol) of dioxane (2 mL) and $H_2O$ (0.5 mL) solution, the reaction mixture was moved to 85°C and reacted for about 4 hours. The reaction mixture was cooled to room temperature, water was added to the reaction mixture, extracted with ethyl acetate, the organic layer was washed with brine, dried over $Na_2SO_4$ and concentrated in vacuo, the concentrate was purified by pre-TLC (PE:EA=2:1), the desired product 14-6 (70 mg, 89.7% yield) was obtained as a yellow oil. ESI-MS m/z: 818.94 $[M+H]^+$.

Step 7: Synthesis of compound 14-7

At room temperature, compound 14-6 (70 mg, 85.7 μmol) was dissolved in a mixed solvent of DCM (1 mL) and TFA (0.5 mL), and moved to 40°C reacting for about 1 h. After the reaction was complete, it was directly concentrated to remove the solvent to obtain the target product 14-7 (93 mg, crude product) as a yellow solid. ESI-MS m/z: 634.32$[M+H]^+$.

Step 8: Synthesis of compound 14

Under the protection of nitrogen, in an ice water bath, the THF solution of acryloyl chloride (8.4 mg, 94 μmol) was added to compound 14-7 (54 mg, 85 μmol), THF (2 mL), and saturated $Na_2CO_3$ (0.5 mL) solution. The reaction mixture was poured into water, extracted with ethyl acetate: methanol = 10:1, the organic layer was washed with brine, dried over $Na_2SO_4$ and concentrated in vacuo. The concentrate was purified by pre-TLC (DCM/MeOH=10:1) to obtain the desired product 14 (25.7 mg, 43.2% yield, 96.9% purity) as a yellow solid. ESI-MS m/z: 688.49$[M+H]^+$.

**Example 15: (1-(7-(8-methyl-7-(5-methyl-1H-indazol-4-yl)-2-(1-methylpiperidin-4-yl)-6-vinyl quinazolin-4-yl)-2,7-diazaspiro[3.5]nonan-2-yl)prop-2-en-1-one)**

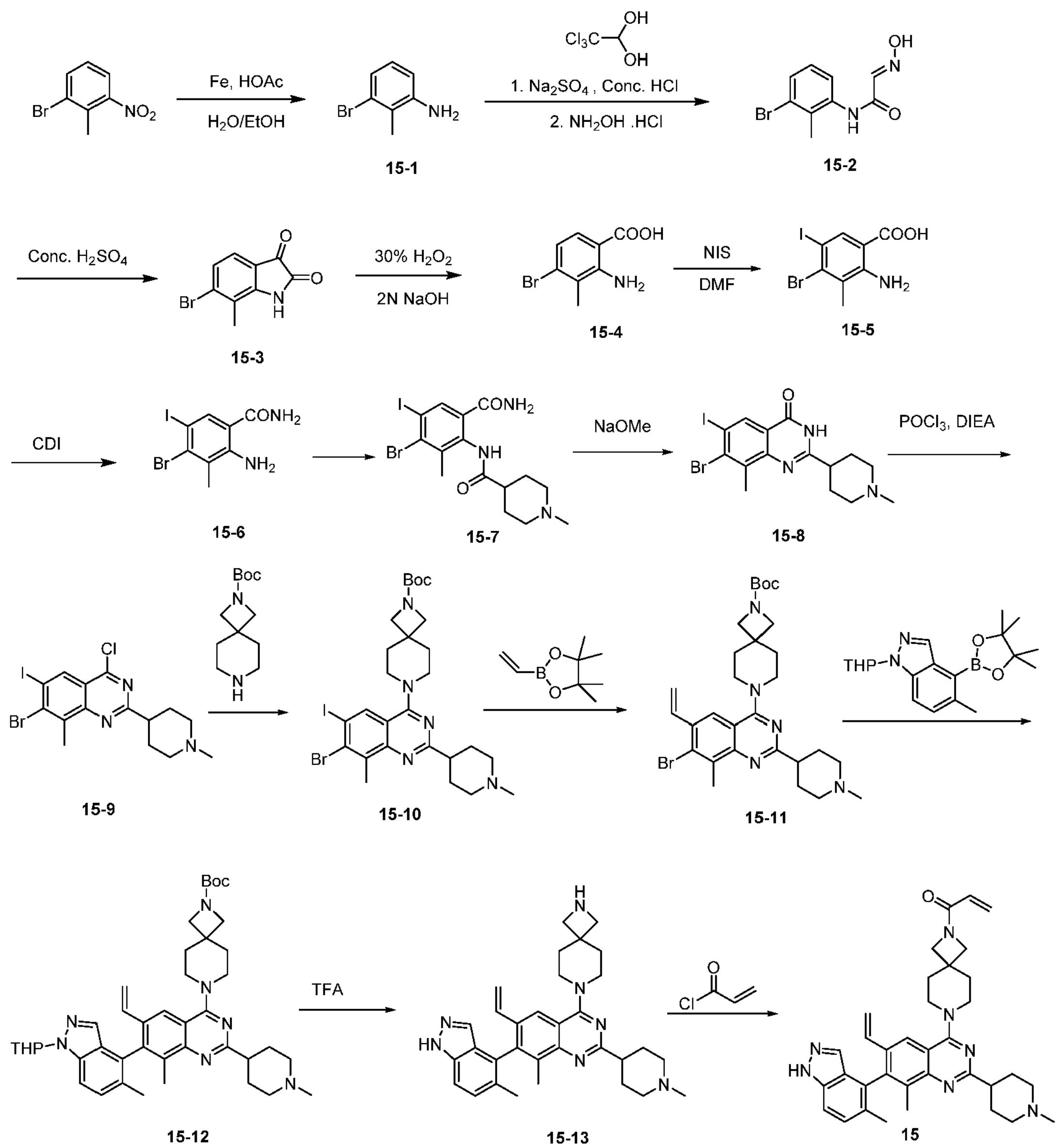


15-1 15-2 15-3 15-4 15-5 15-6 15-7 15-8 15-9 15-10 15-11 15-12 15-13 15

Step 1: Synthesis of compound 15-1

At room temperature, to a mixture of compound 15-1 (40 g, 182.7 mmol), HOAc (76.8 g, 1278.94 mmol), EtOH (400 mL) and $H_2O$ (160 mL) was added iron powder (26.52 g, 475.02 mmol) portionwise. The resulting mixture was stirred at room temperature for 2 hours, and then neutralized with NaOH (5 N) solution. The mixture was then extracted with ethyl acetate, the organic layer was washed with brine, dried over $Na_2SO_4$ and concentrated in vacuo to obtain the desired crude product as a brown oil, namely compound 15-1 (34 g, 98% yield). ESI-MS m/z: 186.15 $[M+H]^+$.

Step 2: Synthesis of compound 15-2

2,2,2-Trichloroethane-1,1-diol (66.4 g, 401.94 mmol) and $Na_2SO_4$ (503.4 g, 3544.77 mmol) were dissolved in water (560 mL), and then heated to 55°C. Water (240 mL) and 35% HCl (72 mL) containing compound 15-1 (34 g, 182.7 mmol) were added, and an aqueous solution (100 mL) of hydroxylamine hydrochloride (81.4 g, 1171.1 mmol) was added. The resulting mixture was stirred at 90°C for 3 hours and a yellow precipitate formed. The mixture was cooled to room temperature. The solid was collected by filtration, washed with water, and air-dried to obtain a yellow-brown solid product, compound 15-2 (47 g, 99% yield). ESI-MS m/z: 257.16$[M+H]^+$.

Step 3: Synthesis of compound 15-3

At 60°C, compound 15-2 (47 g, 180.8 mmol) was added to concentrated sulfuric acid (300 mL), the temperature was increased to 90°C and maintained for 3 hours, the reaction was complete, the reaction mixture was cooled to room temperature and poured into ice water. The yellow precipitate was collected by filtration and dried to obtain a black solid product, namely compound 15-3 (43 g, 99% yield).

Step 4: Synthesis of compound 15-4

To a solution of compound 15-3 (43 g, 180.8 mmol) in NaOH (2 N, 500 mL) at 0°C was added $H_2O_2$ solution (30%, 80 mL) and the resulting mixture was stirred at 0°C for 30 minutes. Then it was moved to room temperature and stirred for 2 hours. The reaction was complete. The mixture was poured into ice water and then acidified with concentrated HCl solution. The precipitate was collected by filtration and air-dried to obtain a white solid product, namely compound 15-4 (20 g, 48.9% yield). ESI-MS m/z: 230.02$[M+H]^+$.

Step 5: Synthesis of compound 15-5

At room temperature, to a solution of compound 15-4 (20 g, 85.86 mmol) in DMF (200 mL) was added NIS (29 g, 128.78 mmol) and the resulting mixture was stirred at 70°C overnight. After the reaction was complete, the mixture was poured into ice water, the mixture was extracted with ethyl acetate, the organic layer was washed with brine, dried over $Na_2SO_4$ and concentrated in vacuo to obtain the desired crude product as a brown solid, namely compound 15-5 (30 g, 98% yield ). ESI-MS m/z: 355.87$[M+H]^+$.

Step 6: Synthesis of compound 15-6

At room temperature, bis(imidazol-1-yl)methanone (2.70 g, 16.67 mmol) was added to the crude compound 15-5 (4.0 g, 11.11 mmol) in THF (20 mL), and the N-ethyl- N-isopropylpropan-2-amine (1.44 g, 11.11 mmol, 1.94 mL) was added to it, and the mixture was moved to 50°C for reaction. After about 2 hours, the raw material was almost completely converted into an intermediate product, and then the mixture was added dropwise to ice in the ammonia water (35 mL), stirring for 5 min to complete the reaction. The mixture was poured into ice water, and extracted with ethyl acetate, the organic layer was washed with brine, dried over $Na_2SO_4$ and concentrated in vacuo. The residue was purified by flash silica gel column chromatography (petroleum ether/ethyl acetate=70:30) to give the desired target product 15-6 (1.64 g) as a brown solid.

Step 7: Synthesis of compound 15-7

At room temperature, 1-methylpiperidine-4-carbonyl chloride (483 mg, 3.0 mmol) was added to compound 15-6 (300 mg, 0.84 mmol) in THF (10 mL), and moved to 50°C stirring for 16 hours. After the completion of the reaction, the THF was directly concentrated to obtain the target product 15-7 (460 mg, crude product) as a yellow solid. ESI-MS m/z: 480.34$[M+H]^+$.

Step 8: Synthesis of compound 15-8

At room temperature, sodium methoxide (200.87 mg, 3.72 mmol) was added to compound 1-2 (600 mg, 1.24 mmol) in toluene (15 mL), and the mixture was moved to 110°C and stirred under reflux for 5 hours. After the reaction was completed, the mixture was poured into ice water, and the mixture was extracted with ethyl acetate. The organic phase was dried over $Na_2SO_4$ and concentrated in vacuo to obtain the target product 15-8 (220 mg, crude product) as an off-white solid. ESI-MS m/z: 461.97$[M+H]^+$.

Step 9: Synthesis of compound 15-9

At room temperature and under nitrogen protection, DIEA (1.0 mL) was added to compound 3-2 (200 mg, 0.46 mmol) in $POCl_3$ (10.0 mL), and the mixture was moved to 110°C and stirred for 12 hours. After the reaction was completed, the $POCl_3$ was directly concentrated to remove the target product 15-9 (240 mg, crude product) as a brown solid. ESI-MS m/z: 479.97$[M+H]^+$.

Step 10: Synthesis of compound 15-10

At room temperature, DIEA (129 mg, 1.0 mmol) was added to compound 15-9 (200 mg, 0.4 mmol), 2,7-diazaspiro[3.5]nonane-2-carboxylic acid tert-butyl ester (90 mg, 0.4 mmol) of dioxane (2 mL), reacting at 60°C for 12 h. The mixture was poured into ice water, and extracted with ethyl acetate, the organic layer was washed with brine, dried over $Na_2SO_4$ and concentrated in vacuo. The residue was purified by Pre-TLC (DCM:MeOH=10:1) to obtain the desired target product 15-10 (200 mg, 71.1% yield) as a yellow solid. ESI-MS m/z: 670.24$[M+H]^+$.

Step 11: Synthesis of compound 15-11

Under the protection of nitrogen, $Pd(dppf)Cl_2.CH_2Cl_2$ (23mg, 28μmol) was added to compound 15-10 (185 mg, 0.276 mmol), $K_2CO_3$ (76 mg, 0.55 mmol), 4,4,5,5-tetramethyl-2-vinyl-1,3,2-dioxaborolane (43 mg, 0.276 mmol) in dioxane (2.5 mL) and $H_2O$ (0.5 mL), the mixture was stirred at 70°C for 2 h. The reaction mixture was cooled to room temperature, water was added to the reaction mixture, extracted with ethyl acetate, the organic layer was washed with brine, dried over $Na_2SO_4$ and concentrated in vacuo, the residue was purified by Pre-TLC (DCM:MeOH=10:1), the desired target product 15-11 (130 mg, 82.8% yield) was obtained as a yellow solid. ESI-MS m/z: 570.21 $[M+H]^+$.

Step 12: Synthesis of compound 15-12

Under the protection of nitrogen, $Pd(PPh_3)_4$ (48 mg, 42 μmol) was added to compound 15-11 (120 mg, 0.21 mmol), 5-methyl-1-tetrahydropyran-2-yl-4-(4,4,5,5-tetramethyl-1,3,2-dioxaborolan-2-yl)indazole (144 mg, 0.42 mmol), $K_3PO_4$ (89 mg, 0.42 mmol) in dioxane (2 mL) , $H_2O$ (0.5 mL) solution, the mixture was moved to 85°C and react for about 4 hours. The reaction mixture was cooled to room temperature, water was added to the reaction mixture, extracted with ethyl acetate, the organic layer was washed with brine, dried over $Na_2SO_4$ and concentrated in vacuo, the concentrate was purified by pre-TLC (DCM:MeOH=10:1), the desired product 15-12 (70 mg, 47.2% yield) was obtained as a yellow solid. ESI-MS m/z: 706.29$[M+H]^+$.

Step 13: Synthesis of compound 15-13

At room temperature, compound 15-12 (70 mg, 99 μmol) was dissolved in a mixed solvent of DCM (1 mL) and TFA (0.5 mL), and moved to 40°C reacting for about 1 h. After the reaction was completed, the solvent was directly concentrated to remove the solvent to obtain the target product 15-13 (80 mg, crude product) as a yellow solid. ESI-MS m/z: 522.32$[M+H]^+$.

Step 14: Synthesis of compound 15

Under the protection of nitrogen, in an ice water bath, the THF solution of acryloyl chloride (8.5 mg, 94 μmol) was added to compound 15-13 (45 mg, 85 μmol), THF (2 mL) and saturated $Na_2CO_3$ (0.5 mL) solution, the reaction was complete. The reaction mixture was poured into water, extracted with ethyl acetate, the organic layer was washed with brine, dried over $Na_2SO_4$ and concentrated in vacuo. The concentrate was purified by pre-TLC (DCM/MeOH=8:1) to give the desired yellow solid, namely product 15 (16.4 mg, 33.2% yield, 91.2% purity). ESI-MS m/z: 576.40 $[M+H]^+$.

| Example | Chemical structure and name |
|---|---|
| 1 | 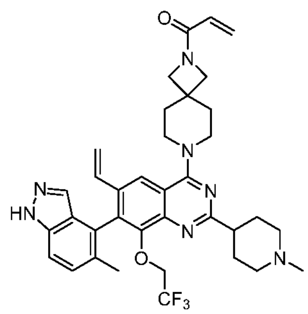 1-(7-(7-(5-methyl-1H-indazole-4-yl)-2-(1-methylpiperidin-4-yl)-8- (2,2,2-trifluoroethoxy)-6-vinylquinazolin-4-yl)-2,7-diazspiro [3.5] nonan-2-yl) propyl-2-en-1-one ; ESI-MS m/z: 660.37 $[M+H]^+$ |

(continued)

| Example | Chemical structure and name |
|---|---|
| 2 | 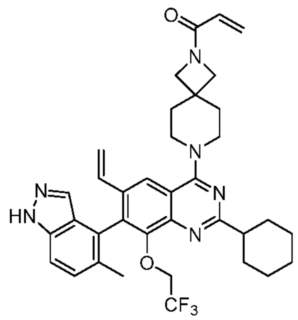<br>1-(7-(2-cyclohexyl)-7-(5-methyl-1h-indazole-4-yl)-8-(2,2,2-trifluoroethoxy)-6-vinylquinazolin-4-yl)-2,7-diazspiro [3.5] nonan-2-yl) propyl-2-en-1-one ; ESI-MS m/z: 645.44 [M+H]+ |
| 3 | 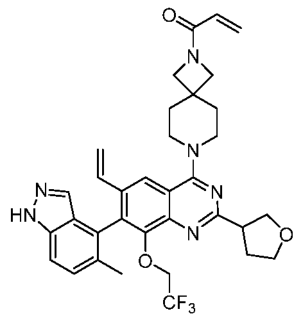<br>1-(7-(7-(5-methyl-1H-indazole-4-yl)-2-(tetrahydrofuran-3-yl)-8- (2,2,2-trifluoroethoxy)-6-vinylquinazolin-4-yl)-2,7-diazspiro [3.5] nonan-2-yl) propyl-2-en-1-one ; ESI-MS m/z: 633.41 [M+H]+ |
| 4 | 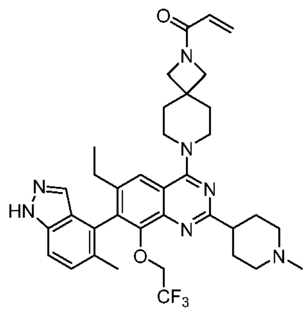<br>1-(7-(6-ethyl-7-(5-methyl-1H-indazol-4-yl)-2-(1-methylpiperidin-4-yl)-8-(2,2,2-trifluoroethoxy) quinazolin-4-yl)-2,7-diazspiro [3.5] nonan-2-yl) propyl-2-en-1-one ; ESI-MS m/z: 662.37 [M+H]+ |
| 5 | 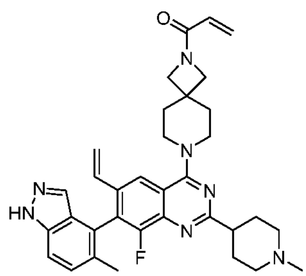<br>1-(7-(8-fluoro-7-(5-methyl-1H-indazole-4-yl)-2-(1-methylpiperidin-4-yl)-6-vinylquinazolin-4-yl)-2,7-diazspiro[3.5]nonan-2-yl)propyl-2-en-1-one ; ESI-MS m/z: 580.34 [M+H]+ |

(continued)

| Example | Chemical structure and name |
|---|---|
| 6 | 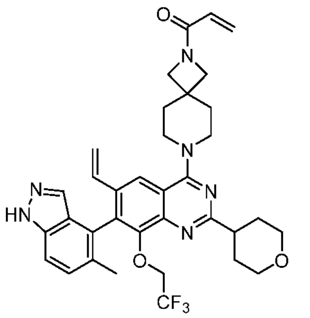 1-(7-(7-(5-methyl-1H-indazole-4-yl)-2-(tetrahydro-2H-pyran-4-yl)- 8-(2,2,2-trifluoroethoxy)-6-vinylquinazolin-4-yl)-2,7-diazspiro [3.5] nonan-2-yl) propyl-2-en-1-one ; ESI-MS m/z: 647.33 $[M+H]^+$ |
| 7 | 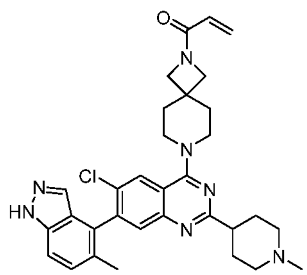 1-(7-(6-chloro-7-(5-methyl-1H-indozol-4-yl)-2-(1-methylpiperidine-4-yl)quinozoline-4-yl-2,7-diazo-spiro [3.5]nonan-2-yl)propyl-2-en-1-one ; ESI-MS m/z: 570.24 $[M+H]^+$ |
| 8 | 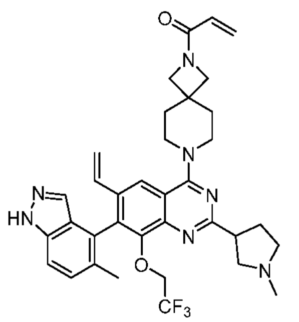 1-(7-(7-(5-methyl-1H-indazole-4-yl)-2-(1-methylpyrrolidine-3-yl)- 8-(2,2,2-trifluoroethoxy)-6-vinylquinazolin-4-yl)-2,7-diazspiro [3.5] nonan-2-yl) propyl-2-en-1-one;ESI-MS m/z: 646.34 $[M+H]^+$ |
| 9 | 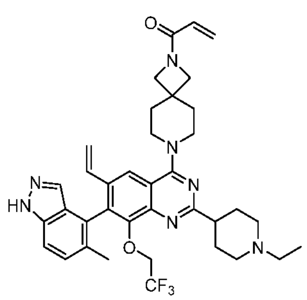 1-(7-(2-(1-ethylpiperidin-4-yl)-7-(5-methyl-1H-indazole-4-yl)-8- (2,2,2-trifluoroethoxy)-6-vinylquinazolin-4-yl)-2,7-diazspiro [3.5] nonan-2-yl) propyl-2-en-1-one; ESI-MS m/z: 674.37 $[M+H]^+$ |

(continued)

| Example | Chemical structure and name |
|---|---|
| 10 | 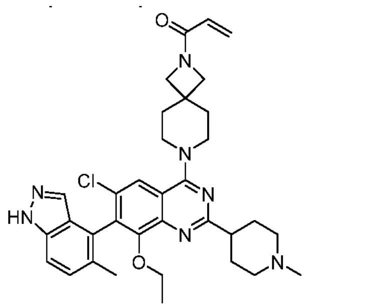<br>1-(7-(6-chloro-8-ethoxy-7-(5-methyl-1H-indazol-4-yl)-2-(1-methylpiperidin-4-yl)-quinazolin-4-yl)-2,7-diazspiro[3.5] nonan-2-yl) propyl-2-en-1-one; ESI-MS m/z: 614.40 $[M+H]^+$ |
| 11 | 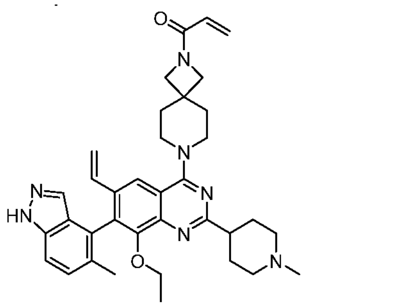<br>1-(7-(8-ethoxy-7-(5-methyl-1H-indazol-4-yl)-2-(1-methylpiperidin-4-yl)-6-vinylquinazolin-4-yl)-2,7-diazspiro [3.5] nonan-2-yl) propyl-2-en-1-one; ESI-MS m/z: 606.45 $[M+H]^+$ |
| 12 | 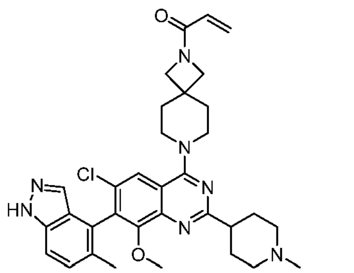<br>1-(7-(6-chloro-8-methoxy-7-(5-methyl-1H-indazol-4-yl)-2-(1-methylpiperidin-4-yl)-quinazolin-4-yl)-2,7-diazspiro [3.5] nonan-2-yl) propyl-2-en-1-one; ESI-MS m/z: 600.35 $[M+H]^+$ |
| 13 | 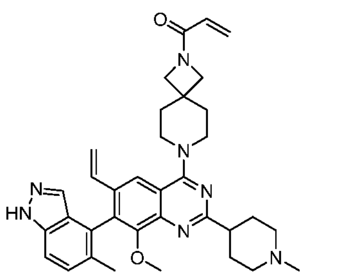<br>1-(7-(8-methoxy-7-(5-methyl-1H-indazol-4-yl)-2-(1-methylpiperidin- |
| | 4-yl)-6-vinylquinazolin-4-yl)-2,7-diazspiro[3.5] nonan-2-yl)propyl-2-en-1-one; ESI-MS m/z: 592.43 $[M+H]^+$ |

(continued)

| Example | Chemical structure and name |
|---|---|
| 14 | 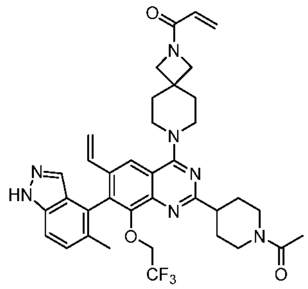 1-(7-(2-(1-acetylpiperidin-4-yl)-7-(5-methyl-1H-indazol-4-yl)-8- (2,2,2-trifluoroethoxy)-6-vinylquinazolin-4-yl)-2,7-diazspiro [3.5] nonan-2-yl) propyl-2-en-1-one; ESI-MS m/z: 688.49 [M+H]$^+$ |
| 15 | 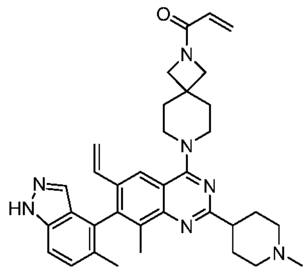 1-(7-(8-methyl-7-(5-methyl-1H-indazol-4-yl)-2-(1-methylpiperidin-4-yl)-6-vinylquinazolin-4-yl)-2,7-diazspiro[3.5] nonan-2-yl)propyl-2-en-1-one; ESI-MS m/z: 576.40 [M+H]$^+$ |

**Example 16: (1-(7-(6-cyclopropyl-8-(2-methoxyethoxy)-7-(5-methyl-1H-indazol-4-yl)-2-(1-methylpiperidin-4-yl)quinazolin-4-yl)-2,7-diazaspiro[3.5]nonan-2-yl)prop-2-en-1-one )**

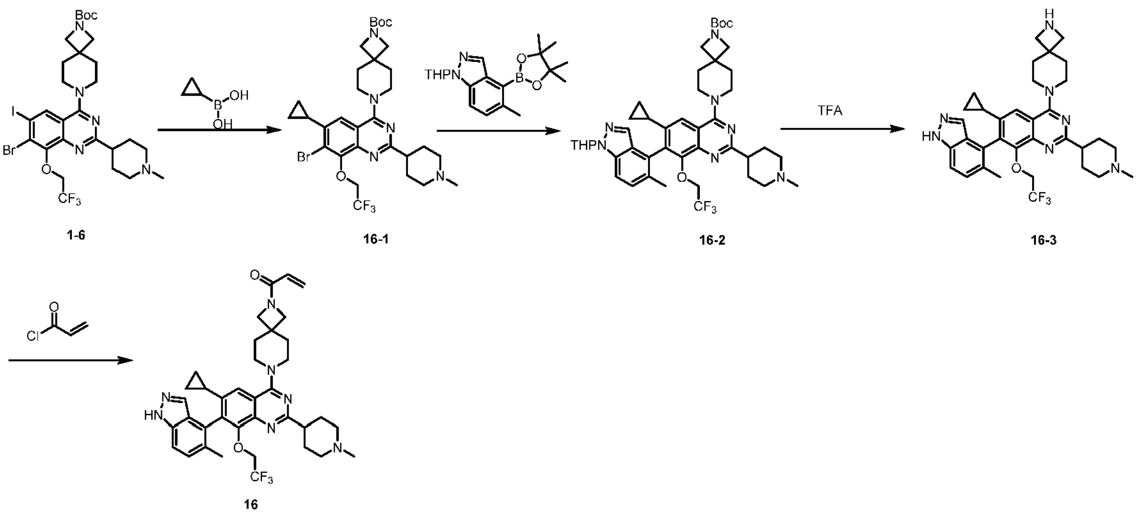


1-6 16-1 16-2 16-3 16

Step 1: Synthesis of compound 16-1

Under nitrogen protection, Pd(dppf)$_2$Cl$_2$.DCM (112 mg, 0.137 mmol, 0.1 eq) was added to potassium carbonate (378 mg, 2.74 mmol, 2 eq), compound 1-6 (1 g, 1.37 mmol, 1 eq) and cyclopropyl boric acid (124 mg, 1.44 mmol, 1.05 eq) in toluene (10 mL) and water (1 mL) solution, stirred at 100°C for 3 hours, TLC and LCMS showed that the reaction was complete, cooled to room temperature, diluted with ethyl acetate and washed with saturated brine two times, the organic layer was dried, filtered, and concentrated to obtain the crude product. The crude product was purified by column

chromatography (DCM/MeOH=15:1) to give the target product (530 mg, 60% yield). ESI-MS m/z: 668.30 [M+H]$^+$.

Step 2: Synthesis of compound 16-2

Under the protection of nitrogen, $Pd_2(dba)_3$ (9.9 mg, 0.011 mmol, 0.1 eq) and SPhos (8.8 mg, 0.022 mmol, 0.2 eq) were added to 16-1 (72 mg, 0.11 mmol, 1 eq), 5-methyl base-1-(tetrahydro-2H-pyran-2-yl)-4-(4,4,5,5-tetramethyl-1,3,2-dioxaborolan-2-yl)- 1H-indazole (37 mg, 0.11 mmol, 1 eq) and potassium phosphate (46 mg, 0.22 mmol, 2 eq) in dioxane (3 mL) and water (0.4 mL) solution, heated to 110°C and reacted for 2 hours. TLC And LCMS showed that the reaction was complete, cooled to room temperature, the mixture was concentrated, and purified by column chromatography (DCM/MeOH=15:1) gave the product (38 mg, 45% yield). ESI-MS m/z: 804.50 [M+H]$^+$.

Step 3: Synthesis of compound 16-3

At room temperature, 16-2 (200 mg, 0.25 mmol) was dissolved in a mixed solvent of DCM (4 mL) and TFA (2 mL), and moved to 40°C reacting for about 1 h. After the reaction was complete, it was directly concentrated to remove the solvent to obtain the crude target product 16-3 (100 mg) as a yellow solid. ESI-MS m/z: 620.38[M+H]$^+$.

Step 4: Synthesis of compound 16

Under the protection of nitrogen, in an ice water bath, add the THF solution of acryloyl chloride (22 mg, 0.22 mmol) to 16-3 (100 mg, 0.16 mmol) in THF (4 mL) and saturated $Na_2CO_3$ (1 mL) solution, add it and the reaction was complete. The reaction mixture was poured into water, and extracted with ethyl acetate: methanol = 10:1, the organic layer was washed with brine, and dried over $Na_2SO_4$ and concentrated in vacuo. The concentrate was purified by pre-TLC (DCM/MeOH=10:1) to obtain the desired product 1 (24.1 mg, 22% yield, 97% purity) as a yellow solid. ESI-MS m/z: 674.40 [M+H]$^+$.

In Examples 17-21, different boronic acid esters were reacted with 16-1 to obtain the corresponding products. According to step 3, the protective group was removed under the action of TFA, and then according to step 4, it was reacted with acryloyl chloride under alkaline conditions to synthesize the final product.

The corresponding borate ester is shown in the figure below.

| Example | Boronic acid ester |
|---|---|
| 16 | 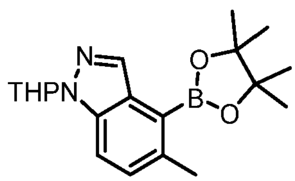 |
| 17 | 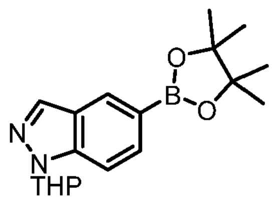 |
| 18 | 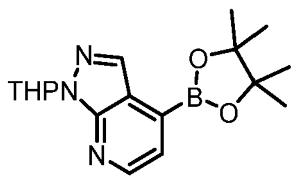 |

(continued)

| Example | Boronic acid ester |
|---|---|
| 19 | 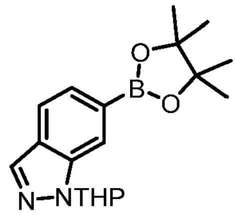 |
| 20 | 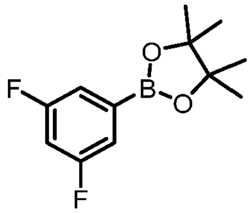 |
| 21 | 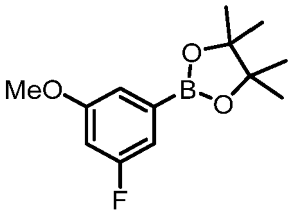 |
| 22 | 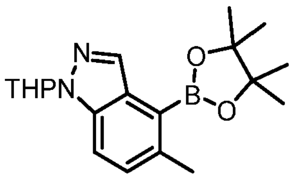 |
| 23 | 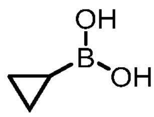 |

**Example 22: (1-(7-(6-cyclopropyl-8-(2-methoxyethoxy)-7-(5-methyl-1H-indazol-4-yl)-2-(1-methylpiperidin-4-yl)quinazolin-4-yl)-2,7-diazaspiro[3.5]nonan-2-yl)prop-2-en-1-one )**

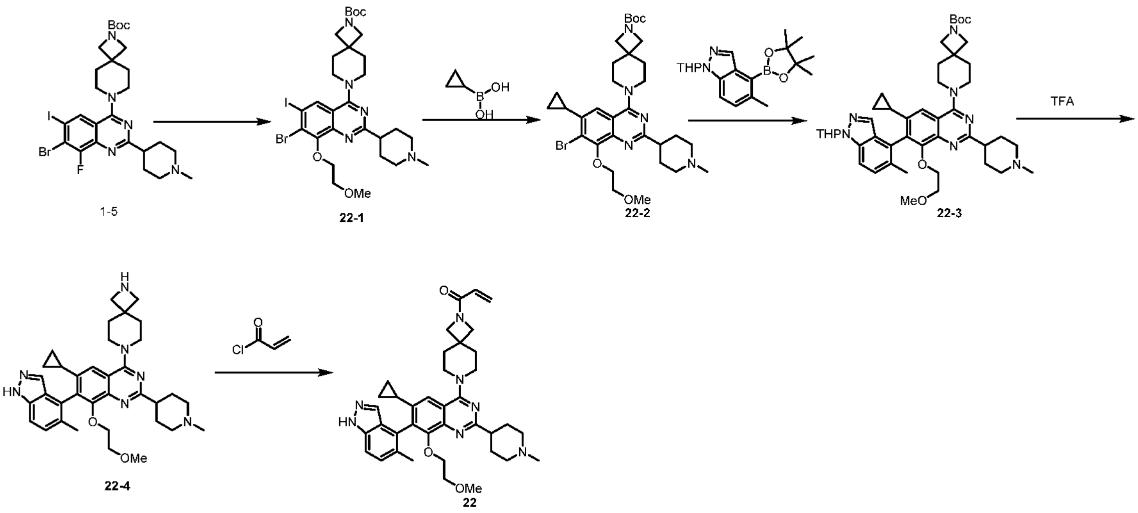


1-5, 22-1, 22-2, 22-3, 22-4, 22

Under the protection of nitrogen, ethylene glycol methyl ether (762 mg, 10 mmol) was added to 1-5 (1.35 g, 2 mmol) and $Cs_2CO_3$ (1.96 g, 6 mmol) of dioxane (15 mL), the mixture was moved to 100°C and reacted for 30 min. The mixture was poured into ice water and extracted with ethyl acetate/methanol=10:1, the organic layer was washed with brine, dried over $Na_2SO_4$ and concentrated in vacuo to obtain the desired target product 22-1 (1.3 g, crude product as a yellow solid). ESI-MS m/z: 730.15[M+H]$^+$.

Then follow the synthetic procedure of compound 16 to synthesize 22-2, 22-3 and 22-4, and finally obtain the target product 22. ESI-MS m/z: 650.4[M+H]$^+$.

**Example 23: (1-(7-(6-cyclopropyl-8-(2-methoxyethoxy)-7-(5-methyl-1H-indazol-4-yl)-2-(1-methylpiperidin-4-yl)quinazolin-4-yl)-2,7-diazaspiro[3.5]nonan-2-yl)prop -2-en-1-one)**

Cyclopropylboronic acid was reacted with compound 22-2 to obtain the corresponding product, and then the protective group was removed under the action of TFA, and then reacted with acryloyl chloride under alkaline conditions to synthesize the final product 23.

**Example 24: (1-(7-(6-cyclopropyl-8-(2,2-difluoroethoxy)-7-(5-methyl-1H-indazol-4-yl) -2-(1-methylpiperidin-4-yl)quinazolin-4-yl)-2,7-diazaspiro [3.5] nonan-2-yl)prop-2-en-1-one)**

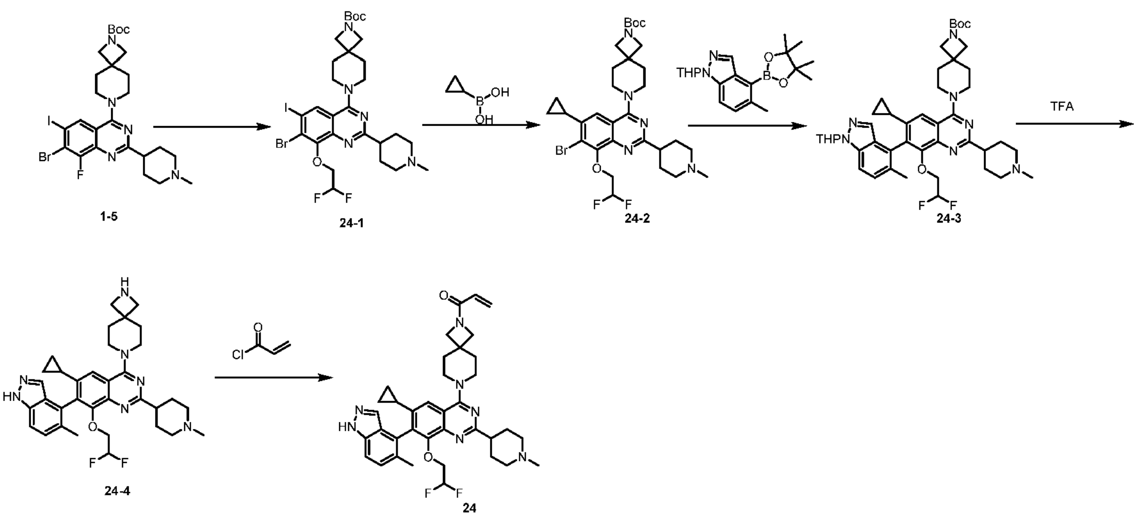


1-5, 24-1, 24-2, 24-3, 24-4, 24

Step 1: Synthesis of compound 24-1

Under the protection of nitrogen, difluoroethanol (201 mg, 2.45 mmol) was added to 1-5 (550 mg, 815.56 umol) and $Cs_2CO_3$ (531.45 mg, 1.63 mmol) of dioxane (5 mL), and the mixture was moved to 100°C for 30 min. The mixture was poured into ice water, the mixture was extracted with ethyl acetate/methanol=10:1, the organic layer was washed with brine, dried over $Na_2SO_4$ and concentrated in vacuo to obtain the desired target product 24-1 (550 mg, crude) as a yellow solid. ESI-MS m/z: 736.30[M+H]$^+$.

Then follow the synthetic procedure of compound 16 to synthesize 24-2, 24-3 and 24-4, and finally obtain the target product 24. ESI-MSm/z: 656.4[M+H]$^+$.

| Example | Chemical structure and name |
|---|---|
| 16 | 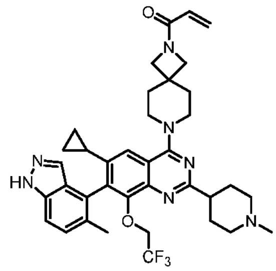 1-(7-(6-cyclopropyl-8-(2-methoxyethoxy)-7-(5-methyl-1H-indazol-4-yl)-2- (1-methylpiperidin-4-yl) quinazolin-4-yl)-2,7-diazspiro[3.5]nonan-2-yl) propyl-2-en-1-one; ESI-MS m/z: 674.40 [M+H]$^+$ |
| 17 | 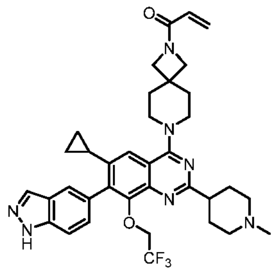 1-(7-(6-cyclopropyl-7-(1H-indazol-5-yl)-2-(1-methylpiperidin-4-yl)-8-(2,2,2-trifluoroethoxy)quinazolin-4-yl)-2,7-diazspiro [3.5] nonan-2-yl) propyl-2-en-1-one; ESI-MS m/z: 660.43 [M+H]$^+$ |
| 18 | 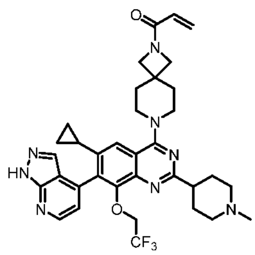 1-(7-(6-cyclopropyl-2-(1-methylpiperidin-4-yl)-7-(1H-pyrazolo[3,4-b]pyridin-4-yl)-8-(2,2,2-trifluoroethoxy)quinazolin-4-yl)-2,7-diazospo [3.5] nonan-2-yl) propyl-2-en-1-one; ESI-MS m/z: 661.45 [M+H]$^+$ |

(continued)

| Example | Chemical structure and name |
|---|---|
| 19 | 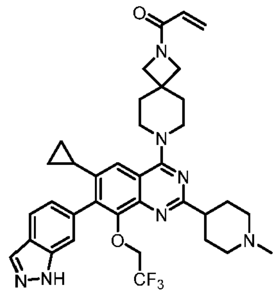<br>1-(7-(6-cyclopropyl-7-(1H-indozol-6-yl)-2-(1-methylpiperidine-4-yl)-8-(2,2,2-trifluoroethyloxy) quinozoline-4-yl-2,7-diazo[3.5]nonan-2-yl)propyl-2-en-1-one; ESI-MS m/z: 660.48 $[M+H]^+$ |
| 20 | 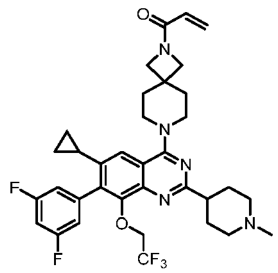<br>1-(7-(6-cyclopropyl-7-(3,5-difluorophenyl)-2-(1-methylpiperidine-4-yl)-8- (2,2,2-trifluoroethyloxy) quinozoline-4-yl)-2,7-diazo [3.5] nonan-2-yl) propyl-2-en-1-one; ESI-MS m/z: 656.47 $[M+H]^+$ |
| 21 | 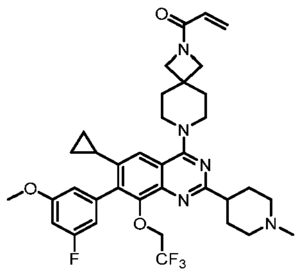<br>1-(7-(6-cyclopropyl-7-(3-fluoro-5-methoxyphenyl)-2-(1-methylpiperidin-4-yl) -8-(2,2,2-trifluoroethoxy) quinazolin-4-yl)-2,7-diazospo [3.5] nonan-2-yl) propyl-2-en-1-one; ESI-MS m/z: 668.45 $[M+H]^+$ |
| 22 | 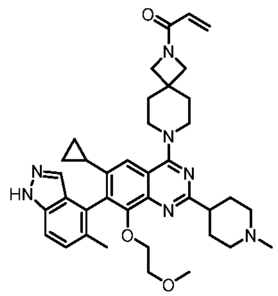<br>1-(7-(6-cyclopropyl-8-(2-methoxyethoxy)-7-(5-methyl-1H-indazol-4-yl)-2- (1-methylpiperidin-4-yl) quinazolin-4-yl)-2,7-diazspiro [3.5] nonan-2-yl) propyl-2-en-1-one; ESI-MS m/z: 650.45 $[M+H]^+$ |

(continued)

| Example | Chemical structure and name |
|---|---|
| 23 | 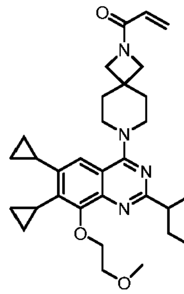<br>1-(7-(6-cyclopropyl-8-(2-methoxyethoxy)-7-(5-methyl-1H-indazol-4-yl)-2- (1-methylpiperidin-4-yl) quinazolin-4-yl)-2,7-diazspiro [3.5] nonan-2-yl) propyl-2-en-1-one; ESI-MS m/z: 560.35 [M+H]$^+$ |
| 24 | 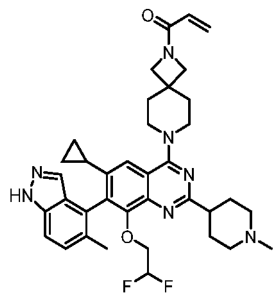<br>1-(7-(6-cyclopropyl-8-(2,2-difluoroethoxy)-7-(5-methyl-1H-indazol-4-yl)-2- (1-methylpiperidin-4-yl) quinazolin-4-yl)-2,7-diazspiro [3.5] nonan-2-yl) propyl-2-en-1-one; ESI-MS m/z: 656.35 [M+H]$^+$ |

**Example 25: (1-(7-(8-ethoxy-6-ethyl-7-(5-methyl-1H-indazol-4-yl)-2-(1-methylpiper (pyridin-4-yl) quinazolin-4-yl)-2,7-dizaspiro[3.5]nonan-2-yl)prop-2-en-1-one)**

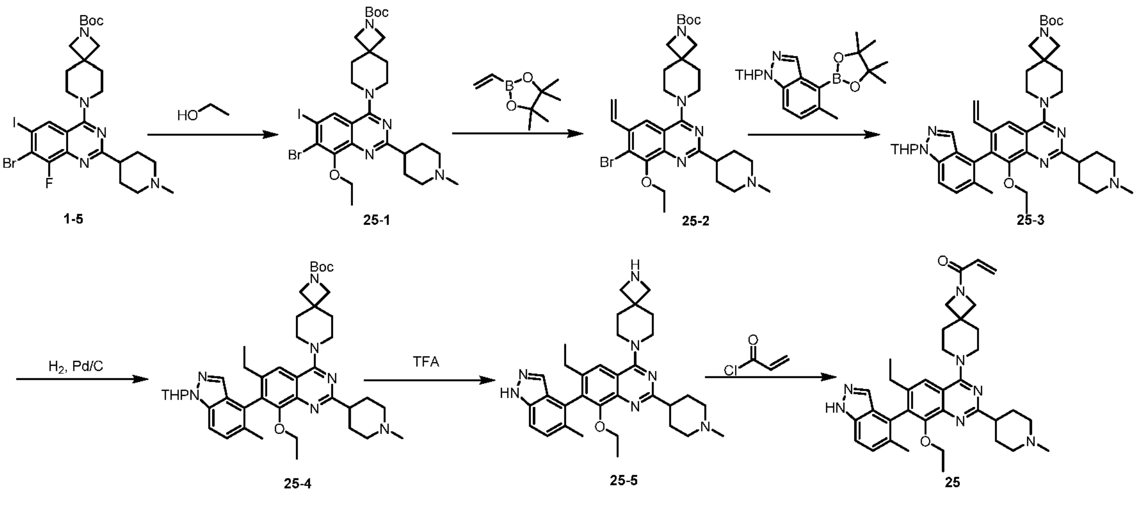


Step 1: Synthesis of compound 25-1

Under the protection of nitrogen, ethanol (112 mg, 2.5 mmol) was added to 1-5 (550 mg, 0.82 mmol) and $Cs_2CO_3$ (531 mg, 1.63 mmol) of dioxane (5 mL), the reaction was moved to 100°C and reacted for 30 min. The mixture was poured into ice water, the mixture was extracted with ethyl acetate/methanol=10:1, the organic layer was washed with brine, dried over $Na_2SO_4$ and concentrated in vacuo to obtain the desired target product 25-1 (590 mg, crude) as a yellow solid. ESI-MS m/z: 700.3 [M+H]$^+$.

Step 2: Synthesis of compound 25-2

Under the protection of nitrogen, Pd(dppf)$Cl_2$.$CH_2Cl_2$ (67 mg, 0.082 mmol) was added to 25-1 (crude product 590 mg, 0.82 mmol), $K_2CO_3$ (226 mg, 1.64 mmol), 4,4,5,5-tetramethyl-2-vinyl-1,3,2-dioxaborolane (126 mg, 0.82 mmol) in a mixed solution of dioxane (5.0 mL) and $H_2O$ (0.5 mL), the reaction was stirred at 70°C for 1 h. The reaction mixture was cooled to room temperature, water was added to the reaction mixture, extracted with ethyl acetate: methanol = 10:1, the organic layer was washed with brine, dried over $Na_2SO_4$ and concentrated in vacuo. The concentrate was passed through pre-TLC (DCM/MeOH= 10:1) to obtain the desired product 25-2 (329 mg, 66% yield in 2 steps) as a yellow solid. ESI-MS m/z: 600.6$[M+H]^+$.

Step 3: Synthesis of compound 25-3

Under the protection of nitrogen, Pd$(PPh_3)_4$ (116.16 mg, 100.53 umol) was added to 25-3 (329 mg, 504 umol), 5-methyl-1-tetrahydropyran-2-yl-4-(4,4,5,5-tetramethyl-1,3,2-dioxaborolan-2-yl)indazole (344.04 mg, 1.01 mmol), $K_3PO_4$ (213.38 mg, 1.01 mmol) in dioxane (3 mL) and $H_2O$ (0.75 mL) solution, the reaction was moved to 85°C and reacted for about 3 hours. The reaction mixture was cooled to room temperature, the reaction mixture was added water, extracted with ethyl acetate: methanol = 10:1, the organic layer was washed with brine, dried over $Na_2SO_4$ and concentrated in vacuo. The concentrate was passed through pre-TLC (DCM/MeOH=10:1) to obtain the desired product 25-3 (244 mg, 65% yield) as a yellow solid. ESI-MS m/z: 736.60 $[M+H]^+$.

Step 4: Synthesis of compound 25-4

25-3 (100 mg, 135 umol) was dissolve in methanol at room temperature, add 300 mg of palladium on carbon, replace hydrogen, and move to 40°C to react overnight. After the reaction was complete, use celite to aid filtration, and the filtrate was concentrated to remove the solvent to obtain the target product 25-4 (150 mg, crude) as a yellow solid. ESI-MS m/z: 738.60$[M+H]^+$.

Step 5: Synthesis of compound 25-5

At room temperature, 25-4 (150 mg, crude) was dissolved in a mixed solvent of DCM (4 mL) and TFA (2 mL), and moved to 40°C to react for about 1 h. After the reaction was complete, it was directly concentrated to remove the solvent to obtain the crude target product 25-5 (100 mg, crude) as a yellow solid. ESI-MS m/z: 554.60$[M+H]^+$.

Step 6: Synthesis of compound 25

Under the protection of nitrogen, in an ice water bath, add the THF solution of acryloyl chloride (17.03 mg, 188.21 umol) to 25-5 (100 mg, 180 umol), THF (4 mL) and saturated $Na_2CO_3$ (1 mL) solution, add it to complete the reaction. The reaction mixture was poured into water, extracted with ethyl acetate: methanol = 10:1, the organic layer was washed with brine, dried over $Na_2SO_4$ and concentrated in vacuo. The concentrate was purified by pre-TLC (DCM/MeOH=10:1) to obtain the desired product 25 (21 mg, 20% yield, 97% purity) as a yellow solid. ESI-MS m/z: 608.40$[M+H]^+$.

Examples 26-31 used cyclobutanol, isopropanol, difluoroethanol, 2-fluoroethanol, ethylene glycol methyl ether and cyclopropanol to react with compound 1-5 to obtain the corresponding products, and then follow the synthesis steps of 25. The corresponding final product compounds 26-31 were synthesized.

| Example | Chemical structure and name |
|---|---|
| 25 | 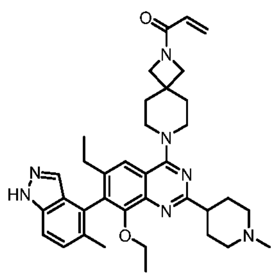 1-(7-(8-ethoxy-6-ethyl-7-(5-methyl-1H-indazol-4-yl)-2-(1-methylpiperidin-4-yl)quinazolin-4-yl)-2,7-diazospo [3.5] nonan-2-yl)propyl-2-en-1-one; ESI-MS m/z: 608.46 $[M+H]^+$ |

(continued)

| Example | Chemical structure and name |
|---|---|
| 26 | 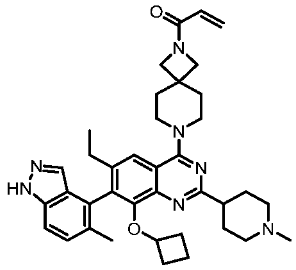 1-(7-(8-cyclobutoxy-6-ethyl-7-(5-methyl-1H-indazol-4-yl)-2-(1-methylpiperidin-4-yl)quinazolin-4-yl)-2,7-diazospo [3.5] nonan-2-yl) propyl-2-en-1-one; ESI-MS m/z: 634.51 $[M+H]^+$ |
| 27 | 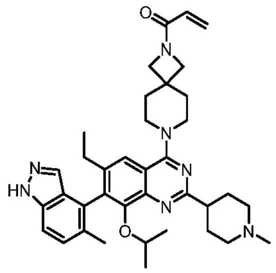 1-(7-(6-ethyl-8-isopropoxy-7-(5-methyl-1H-indazol-4-yl)-2-(1-methylpiperidin-4-yl)quinazolin-4-yl)-2,7-diazospo [3.5] nonan-2-yl) propyl-2-en-1-one; ESI-MS m/z: 622.52 $[M+H]^+$ |
| 28 | 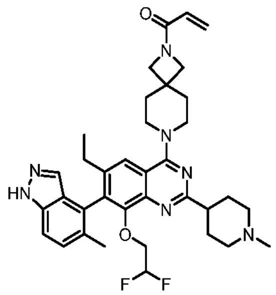 1-(7-(8-(2,2-difluoroethoxy)-6-ethyl-7-(5-methyl-1H-indazol-4-yl)-2-(1-methylpiperidin-4-yl)quinazolin-4-yl)-2,7-diazspiro [3.5] nonan-2-yl) propyl-2-en-1-one; ESI-MS m/z: 644.45 $[M+H]^+$ |
| 29 | 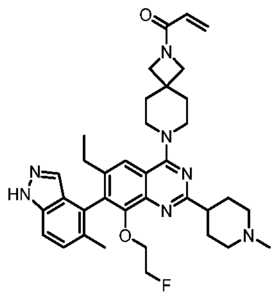 1-(7-(6-ethyl-8-(2-fluoroethoxy)-7-(5-methyl-1H-indazol-4-yl)-2-(1-methylpiperidin-4-yl) quinazolin-4-yl) - 2,7-diazspiro [3.5] nonan-2-yl) propyl-2-en-1-one; ESI-MS m/z: 626.50 $[M+H]^+$ |

(continued)

| Example | Chemical structure and name |
|---|---|
| 30 | 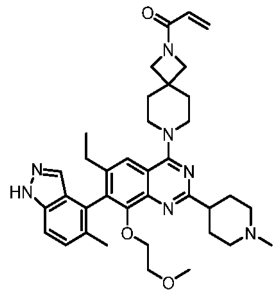<br>1-(7-(6-ethyl-8-(2-methoxyethoxy)-7-(5-methyl-1H-indazol-4-yl)-2-(1-methylpiperidin-4-yl)quinazolin-4-yl)-2,7-diazspiro [3.5] nonan-2-yl) propyl-2-en-1-one; ESI-MS m/z: 638.53 $[M+H]^+$ |
| 31 | 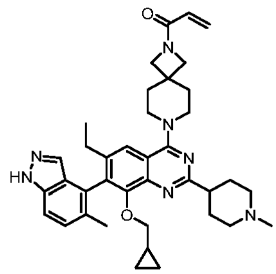<br>1-(7-(8-(cyclopropylmethoxy)-6-ethyl-7-(5-methyl-1H-indazol-4-yl)-2-(1-methylpiperidin-4-yl) quinazolin-4-yl)-2,7-diazspiro [3.5] nonan-2-yl) propyl-2-en-1-one; ESI-MS m/z: 633.38 $[M+H]^+$ |

**Example 32: (1-(7-(6-chloro-7-(5-methyl-1H-indazol-4-yl)-2-(1-methylpiperidin-4-yl)-8-(2,2,2-trifluoroethoxy)quinazoline-4-yl)-2,7-diazaspiro[3.5]nonan-2-yl)prop-2-en-1-one)**

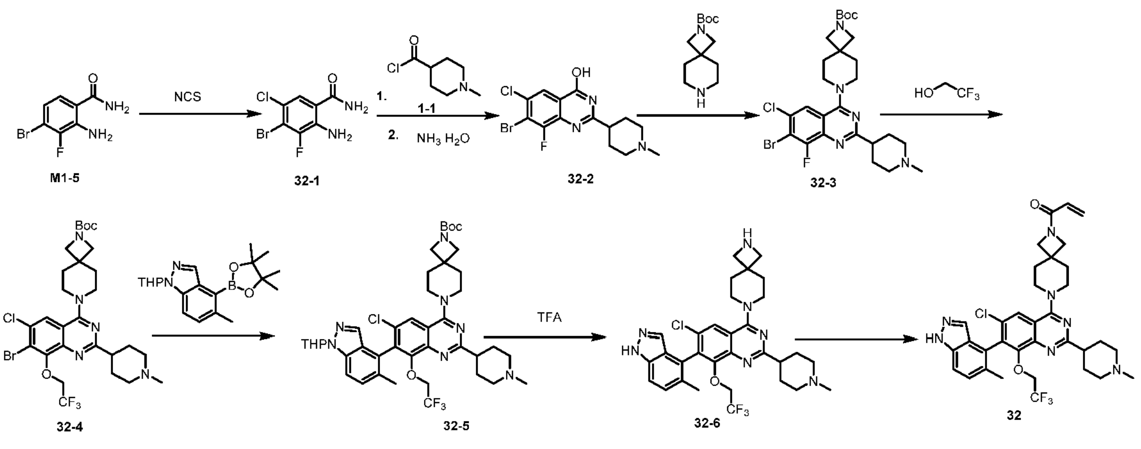


Step 1: Synthesis of compound 32-1

At room temperature, to a solution of 2-amino-4-bromo-3-fluorobenzoic acid (20 g, 85.86 mmol) in DMF (200 mL) was added NCS (13.8 g, 103 mmol) and the resulting mixture was stirred at 70°C overnight. After the reaction was complete, the mixture was poured into ice water, and extracted with ethyl acetate, the organic layer was washed with brine, dried over $Na_2SO_4$ and concentrated in vacuo to obtain the desired crude product (22 g, 98% yield) as a brown solid. ESI-MS m/z: 267.10$[M+H]^+$.

Step 2: Synthesis of compound 32-2

At room temperature, 1-1 (2.42 g, 15 mmol) was added to M1-5 (2.33 g, 10 mmol) in THF (20 mL), the resulting mixture was moved to 40°C and stirred overnight. After the reaction was complete, add 30 mL of ammonia water, adjust the pH of the solution to 11, stir at 35°C, check until the reaction was complete, a large amount of insoluble matter precipitated, which was filtered to obtain insoluble matter, namely crude product 32-2 (3.5 g, crude). ESI-MS m/z: 374.2[M+H]$^+$.

Step 3: Synthesis of compound 32-3

Under nitrogen protection at room temperature, compound 32-1 (1 g, 2.67 mmol) and 2,7-diazaspiro[3.5] nonane-2-carboxylic acid tert-butyl ester (725 mg, 3.2 mmol) were added to the reaction flask of DMF (10 mL) and DBU (2.44 g, 16 mmol), the resulting mixture was cooled in an ice-water bath, added BOP (2.95 g, 6.7 mmol), heated to 45°C and reacted for 2 hours. The reaction was complete, cooled to room temperature, diluted with ethyl acetate, and diluted with water. The aqueous phase was extracted with ethyl acetate once, the organic phases were combined, and washed with 0.5N hydrochloric acid and brine (1:1) for 3 times, washed with $NaHCO_3$ for once, washed with brine for once, dried over sodium sulfate, and concentrated to obtain the target product 32-3 (1.2 g, crude product).

Refer to the synthesis procedure of compound 16, replace the fluorine atom with trifluoroethanol under basic conditions to synthesize 32-4, 32-4 synthesizes 32-5 by suzuki coupling method, which was removed the protective group under the action of trifluoroacetic acid to obtain compound 32-6, 32-6 reacts with acryloyl chloride under basic conditions to synthesize target product 32.

Refer to the synthesis method of compound 32 to complete the synthesis of compounds 33 and 34. For 33 and 34, cyclobutanol and difluoroethanol were used to replace trifluoroethanol to replace fluorine atoms to obtain corresponding products. Then, the target products 33 and 34 were synthesized according to the step 32.

| Example | Chemical structure and name |
|---|---|
| 32 | 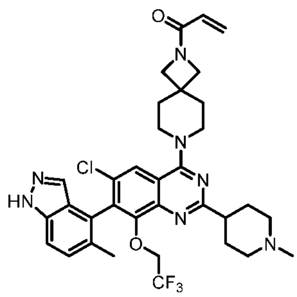 1-(7-(6-chloro-7-(5-methyl-1H-indazol-4-yl)-2-(1-methylpiperidin-4-yl)- 8-(2,2,2-trifluoroethoxy) quinazolin-4-yl)-2,7-diazarospiro[3.5]nonan-2-yl) propyl-2-en-1-one;ESI-MSm/z:668.26[M+H]$^+$ |
| 33 | 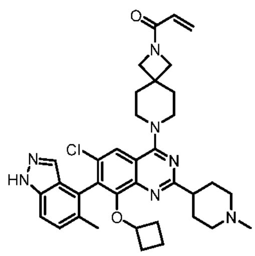 1-(7-(6-chloro-8-cyclobutoxy-7-(5-methyl-1H-indazol-4-yl)-2-(1-methylpiperidin-4-yl)quinazolin-4-yl)-2,7-diazospo[3.5]nonan-2-yl)propyl-2-en-1-one;ESI-MSm/z:321.22[M+2H/2]$^+$ |

(continued)

| Example | Chemical structure and name |
|---|---|
| 34 | 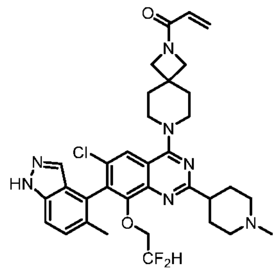 1-(7-(6-chloro-8-(2,2-difluoroethoxy)-7-(5-methyl-1H-indazole-4-yl)-2- (1-methylpiperidin-4-yl) quinazolin-4-yl)-2,7-diazspiro[3.5]nonan-2-yl) propyl-2-en-1-one;ESI-MSm/z:650.67[M+H]$^+$ |

**Example 35: (1-(7-(6-ethyl-2-(1-(2-methoxyethyl)piperidin-4-yl)-7-(5-methyl-1H- indazol-4-yl)-8-(2,2,2-trifluoroethoxy)quinazolin-4-yl)-2,7-diazaspiro [3.5] nonan-2-yl)prop-2-en-1-one)**

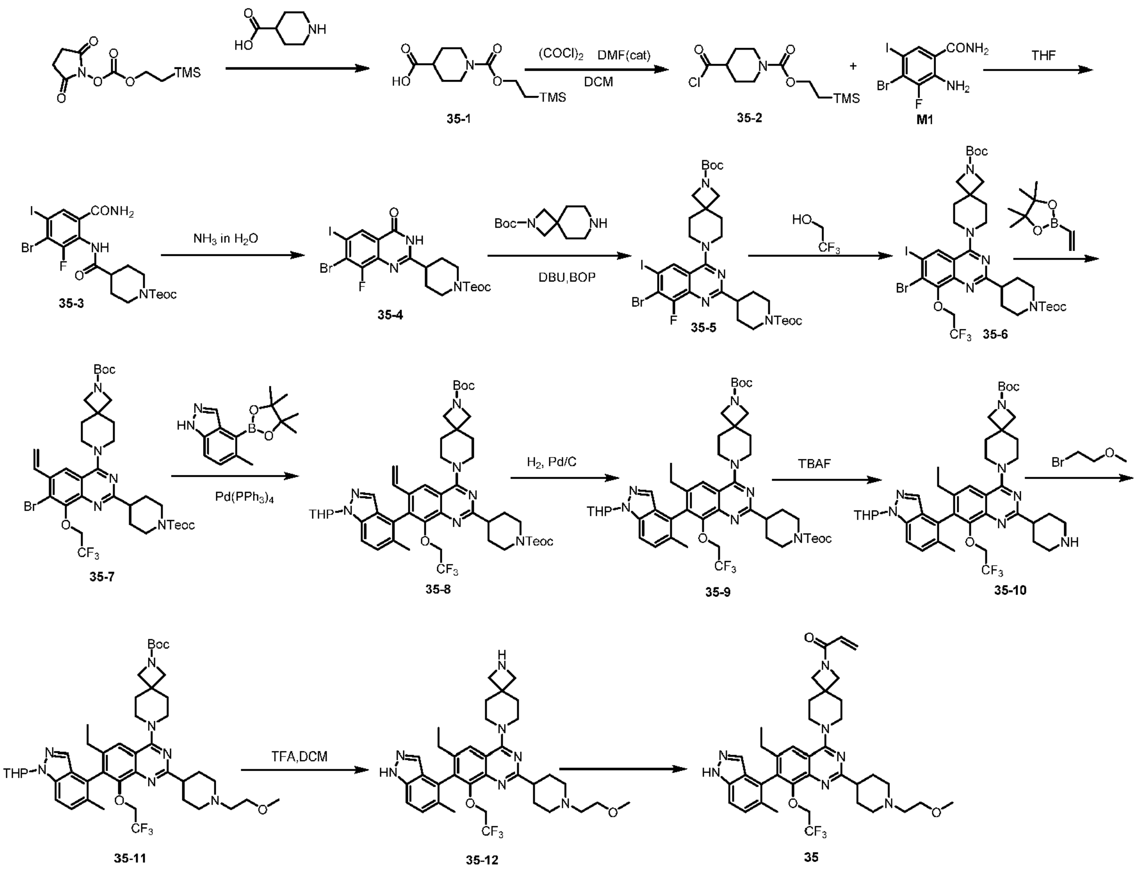


Step 1: Synthesis of compound 35-1

At room temperature, piperidine-4-carboxylic acid (7.000 g, 54.20 mmol) was added to THF (200 mL) and water (40 mL), triethylamine (16.45 g, 162.59 mmol) and (2,5-dioxy heteropyrrolidin-1-yl)2-trimethylsilyl ethyl carbonate (15.46 g, 59.62 mmol) were added, stirring at room temperature until the reaction was complete. The mixture was concentrated to remove THF, cooled down, acidified with 1 N HCl to pH 2-3, extracted with DCM three times, the organic phase was washed twice with brine, dried, and concentrated to obtain the target product 35-1 (16 g, crude product).

Step 2: Synthesis of compound 35-2

At room temperature, add compound 35-1 (2.73 g, 10.00 mmol) to DCM (30 mL), add 2 drops of DMF and add oxalyl chloride (3.81 g, 30.00 mmol) dropwise, bubbles emerged, the reaction was complete. The mixture was concentrated to obtain 35-2 (3 g, crude product) and used directly in the next step.

Step 3: Synthesis of compounds 35-3 and 35-4

At room temperature, 35-2 (3 g, 10 mmol) was added to M1 (1.8 g, 5 mmol) in THF (40 mL), the mixture was moved to 40°C and stirred overnight. After detection until the reaction was complete, 35-3 was obtained. Add 30 mL of ammonia, adjust the pH of the solution to 11, stir at 40°C and check until the reaction was complete, a large amount of insoluble matter precipitated, which was filtered to obtain insoluble matter, namely 35-4 (1.9 g, 63% yield). ESI-MS m/z: 374.2$[M+H]^+$.

Step 4: Synthesis of compound 35-5

Under nitrogen protection at room temperature, compound 35-4 (1.22 g, 2.1 mmol) and 2,7-diazaspiro[3.5]nonane-2-carboxylic acid tert-butyl ester (695 mg, 3.1 mmol) were added to the reaction flask, add DMF (12 mL) and DBU (1.87 g, 12.2 mmol), then the mixture was cooled in an ice-water bath, add BOP (2.2 g, 5.1 mmol), warmed to 45°C and reacted for 2 hours, checking that the reaction was complete and cool to room temperature. The mixture was diluted with ethyl acetate and water, the aqueous phase was extracted with ethyl acetate once, the organic phases was combined, washed with 0.5N hydrochloric acid and brine (1:1) 3 times, aq.NaHCOs once and brine once, then dried over sodium sulfate, and concentrated to obtain the target product 35-5 (1 g, crude product).

Step 5: Synthesis of compound 35-6

Under the protection of nitrogen, trifluoroethanol (2.77 g, 27.7 mmol) was added to 35-5 (2.23 g, 2.77 mmol), $Cs_2CO_3$ (2.7 g, 8.30 mmol) of dioxane (25 mL), the reaction was moved to 100°C and reacted for 30 min. The mixture was poured into ice water, and extracted with ethyl acetate, the organic layer was washed with brine, dried over $Na_2SO_4$ and concentrated in vacuo to obtain the desired target product 35-6 (4.6 g, crude) as a solid. ESI-MS m/z: 784.30$[M+H]^+$.

Step 6: Synthesis of compound 35-7

Under the protection of nitrogen, $Pd(dppf)_2Cl_2DCM$ (1.82 g, 2.23 mmol) was added to potassium carbonate (6.16 g, 44.54 mmol), compound 35-6 (19.7 g, 22.27 mmol) and vinyl borate (3.43 g, 22.27 mmol) in a solution of dioxane (200 mL) and water (20 mL), stirred at 100°C for 3 hours, TLC and LCMS showed that the reaction was complete, cooled to room temperature, diluted with ethyl acetate and washed twice with saturated brine, the organic layer was dried, filtered and concentrated to obtain the crude product, and the target product (9.6 g, 55% yield) was obtained by column chromatography (DCM/EA=20:1). ESI-MS m/z: 895.33$[M+H]^+$.

Step 7: Synthesis of compound 35-8

Under the protection of nitrogen, $Pd(PPh_3)_4$ (2.94 g, 2.55 mmol) was added to D1-7 (10 g, 12.74 mmol), 5-methyl-1-tetrahydropyran-2-yl-4-(4,4,5,5-tetramethyl-1,3,2-dioxaborolan-2-yl)indazole (7.85 g, 22.9 mmol) and potassium phosphate (5.41 g, 25.5 mmol) in dioxane (100 mL) and water (20 mL), the mixture was heated to 85°C and reacted for 4 hours. TLC and LCMS showed that the reaction was complete. Cooled to room temperature, diluted with ethyl acetate, washed with water, the organic layer was washed with saturated brine, dried and concentrated to obtain a crude product. Chromatography gave a yellow foamy solid (10 g, 85% yield). ESI-MSm/z: 920.40$[M+H]^+$.

Step 8: Synthesis of compound 35-9

At room temperature, 35-8 (1 g, 1.09 mmol) was dissolved in methanol, Pa/C (1 g) was added, $H_2$ was replaced, and the reaction was carried out at 40°C overnight. After the reaction was complete, diatomaceous earth was used for filtration, and the filtrate was concentrated to remove the solvent to obtain the crude target product 35-9 (1 g, crude) as a yellow solid. ESI-MS m/z: 922.50$[M+H]^+$.

Step 9: Synthesis of compound 35-10

At room temperature, compound 35-9 (1.6 g, 1.74 mmol) was dissolved in THF (10 mL), the mixture was added TBAF (4 mL, 1M in THF), stirred at 35°C for 4 hours. The reaction mixture was concentrated, diluted with ethyl acetate, washed twice with water and saturated brine twice, dried with sodium sulfate, and concentrated to obtain the product (912 mg, 67% yield).

Step 10: Synthesis of compound 35-11

Compound 35-10 (100 mg, 0.13 mmol) was dissolved in DMF (5 mL) at room temperature, cesium carbonate (126 mg, 0.39 mmol) was added, 2-bromoethyl methyl ether (54 mg, 0.39 mmol) was added and stirred overnight at room temperature, LCMS monitored the completion of the reaction, the mixture was diluted with ethyl acetate, washed once with water, washed twice with saturated brine, the organic layer was dried, and concentrated to obtain the crude product. The pre-TLC (DCM/MeOH=10:1) prepared 35-11 (37 mg, 34% yield). ESI -MS m/z: 836.46$[M+H]^+$.

Step 11: Synthesis of compound 35-12

At room temperature, 35-11 (37 mg, 44 μmol) was dissolved in a mixed solvent of DCM (2 mL) and TFA (1 mL), and moved to 40°C reacting for about 1 h. After the reaction was complete, it was directly concentrated to remove the solvent to obtain the crude product 35-12. ESI-MS m/z: 652.40 $[M+H]^+$.

Step 12: Synthesis of compound 35

Under the protection of nitrogen, in an ice water bath, the THF solution of acryloyl chloride (4 mg, 45 μmol) was added to the 35-12 (29 mg, 44 μmol, crude) of THF (2 mL) and saturated $Na_2CO_3$ (0.5 mL) solution, add it to react complete. The reaction mixture was poured into water, extracted with ethyl acetate, the organic layer was washed with brine, dried over $Na_2SO_4$ and concentrated in vacuo. The concentrate was purified by pre-TLC (DCM/MeOH=10:1) to give the desired product 35 (3.1 mg, 6% yield, 97% purity). ESI-MS m/z: 706.45 $[M+H]^+$.

**Example 36: (1-(7-(6-ethyl-2-(1-(2-hydroxy-2-methylpropyl)piperidin-4-yl)-7-(5-methyl -1H-indazol-4-yl)-8-(2,2,2-trifluoroethoxy)quinazolin-4-yl)-2,7-diazaspiro[3.5]nonan-2-yl) prop-2-en-1-one)**

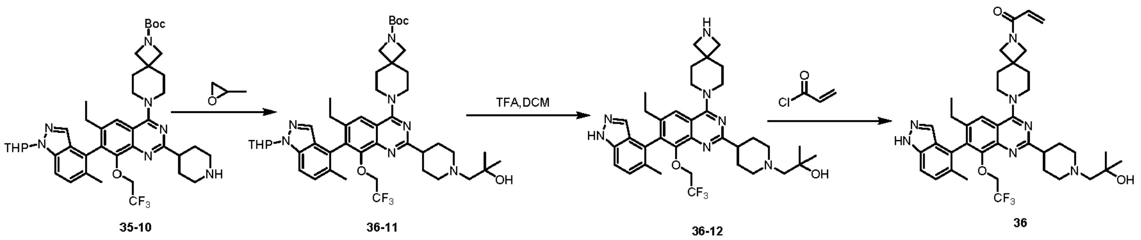

35-10 36-11 36-12 36

Step 1: Synthesis of compound 36-11

Compound 35-10 (100 mg, 0.13 mmol) was dissolved in ethanol (2 mL), and methyl propylene oxide (93 mg, 1.29 mmol) and triethylamine (40 mg, 0.39 mmol) were added. The reaction was carried out in a microwave at 100°C for 0.5 hours, detected by LCMS. After the reaction was completed, it was directly concentrated to obtain crude product 36-11.

Referring to the synthesis method of Example 35, the protective group was removed under the action of trifluoroacetic acid, then the intermediate reacted with acryloyl chloride under alkaline conditions to obtain the target product 36 (8.8 mg). ESI-MS m/z: 720.35$[M+H]^+$.

**Example 37: (1-(7-(6-ethyl-7-(5-methyl-1H-indazol-4-yl)-2-(1-(oxetan-3-yl) )piperidin-4-yl)-8-(2,2,2-trifluoroethoxy)quinazolin-4-yl)-2,7-diazaspiro[3.5]nonan-2-yl) prop-2-en-1-one)**

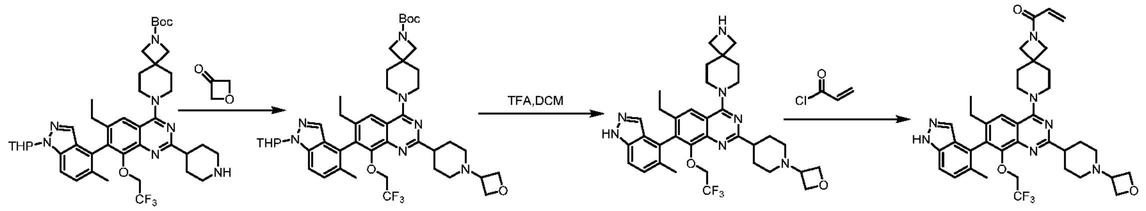


35-10 37-11 37-12 37

Step 1: Synthesis of compound 37-11

Compound 35-10 (165 mg, 0.21 mmol) was dissolved in 1,2-dichloroethane (10 mL), and 3-oxetanone (76.4 mg, 1.1 mmol) and sodium acetate borohydride (225 mg, 1.1 mmol) were added, stirring overnight at 35°C, the reaction was complete, the mixture was diluted with ethyl acetate, washed with water and washed with saturated brine, the organic layer was dried and concentrated to obtain a crude product.

Refer to the synthesis procedure of Example 35 to remove the protective group under the action of trifluoroacetic acid, then the intermediate reacted with acryloyl chloride under alkaline conditions to obtain the target product 37 (13.5 mg). ESI-MS m/z: 704.50$[M+H]^+$.

| Example | Chemical structure and name |
|---|---|
| 35 | 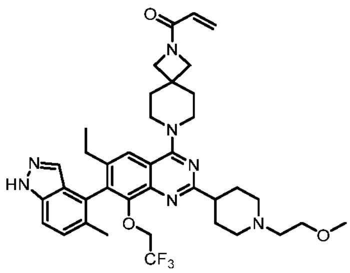 1-(7-(6-ethyl-2-(1-(2-methoxyethyl)piperidin-4-yl)-7-(5-methyl-1H-indazole-4-yl)-8-(2,2,2-trifluoroethoxy)quinazolin-4-yl)-2,7-diazspiro [3.5] nonan-2-yl) propyl-2-en-1-one; ESI-MS m/z: 706.87 $[M+H]^+$ |
| 36 | 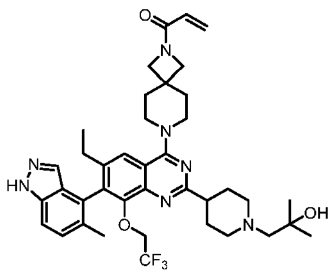 1-(7-(6-ethyl-2-(1-(2-hydroxy-2-methylpropyl)piperidin-4-yl)-7-(5-methyl-1H-indazol-4-yl)-8-(2,2,2-trifluoroethoxy)quinazolin-4-yl)-2,7-diazspiro [3.5] nonan-2-yl) propyl-2-en-1-one; ESI-MS m/z: 720.41 $[M+H]^+$ |

(continued)

| Example | Chemical structure and name |
|---|---|
| 37 | 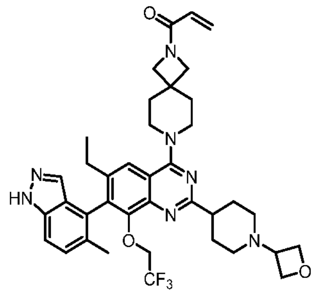 1-(7-(6-ethyl-7-(5-methyl-1H-indazol-4-yl)-2-(1-(oxacyclobutan-3-yl) piperidin-4-yl)-8-(2,2,2-trifluoroethoxy)quinazolin-4-yl)-2,7-diazspiro [3.5] nonan-2-yl) propyl-2-en-1-one; ESI-MS m/z: 704.38 $[M+H]^+$ |

**Example 38: (1-(7-(6-ethyl-2-(1-(2-hydroxy-2-methylpropyl)azetidin-3-yl)-7-(5-methyl-1H-indazol-4-yl)-8-(2,2,2-trifluoroethoxy)quinazolin-4-yl)-2,7-diazaspiro [3.5] nonan 2-yl) prop-2-en-1-one)**

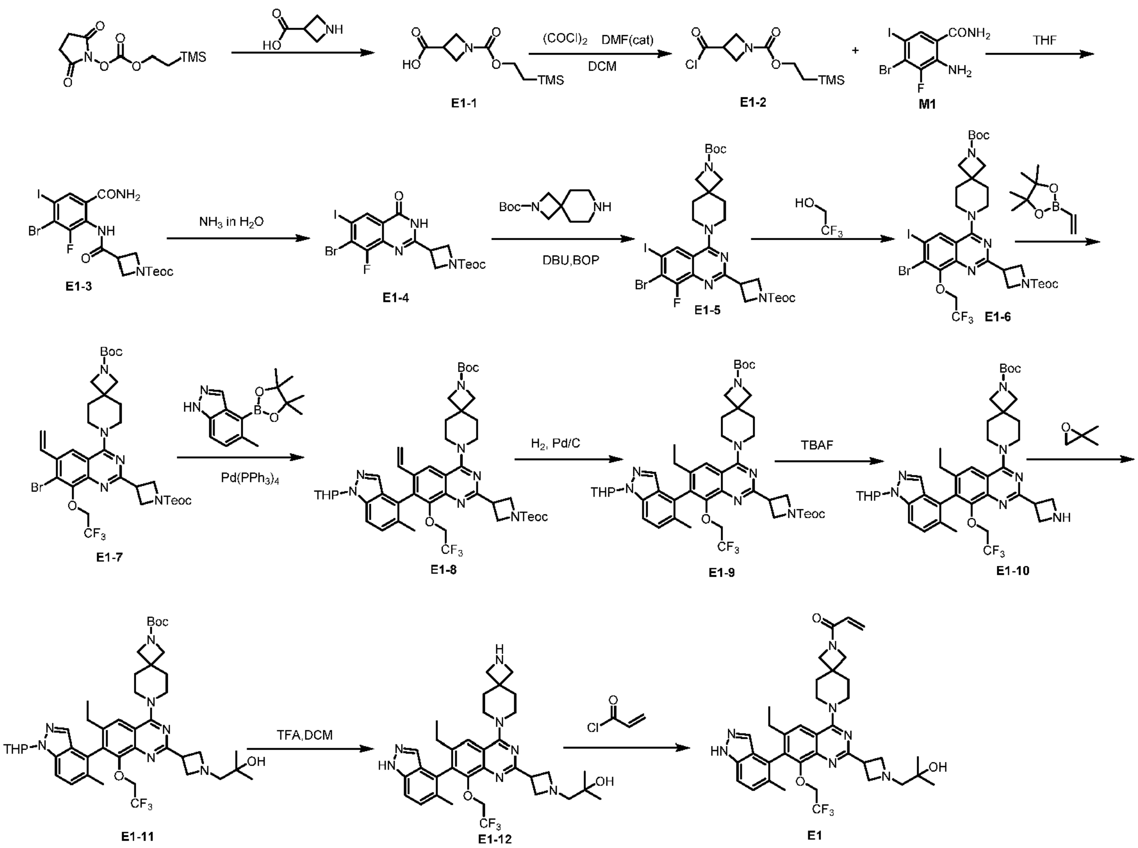


Refer to the synthesis steps of examples 35 and 36.

The operation of compound 38-1 refers to the synthesis operation of compound 35-1, and the nitrogen atom of 3-azetidine carboxylic acid is protected. The operation of compound 38-2 refers to the synthetic operation of compound 35-2. The carboxylic acid of compound 35-2 is converted into acid chloride, and then reacted with compound M1 to obtain compound 38-3. Compound 38-3 is directly added with ammonia water to adjust the pH to 11 ring closure synthesis of compound 38-4. Compound 38-4 is synthesized by introducing a spiro ring under the action of DBU and BOP to synthesize compound 38-5, and then replace its fluorine atom with trifluoroethanol to obtain compound 38-6. Compound

38-6 undergoes two Suzuki coupling reactions to synthesize compound 38-8, after the double bond is reduced, the protective group of the nitrogen atom is removed to obtain the common intermediate 38-10. Compound 38-10 refers to the synthesis steps of 36-11. Microwave reaction with methyl propylene oxide in the system of triethylamine and ethanol can give compound 38-11. Then trifluoroacetic acid deprotection gives compound 38-12. Reacting with acryloyl chloride under neutral conditions can obtain compound 38.

**Example 39: (3-(3-(4-(2-acryloyl-2,7-diazaspiro[3.5]nonan-7-yl)-6-ethyl-7-(5-methyl-1H-indazol-4-yl)-8-(2,2,2-trifluoroethoxy)quinazolin-2-yl)azetidin-1-yl)propionitrile)**

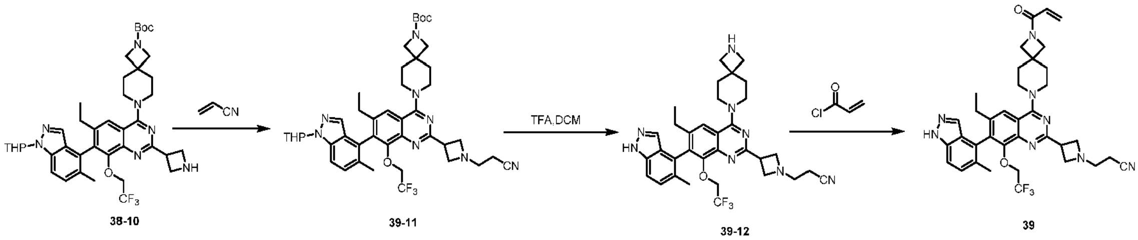

38-10 39-11 39-12 39

Step 1: Synthesis of compound 39-11

Compound 38-10 (50 mg, 67 μmol) was dissolved in ethanol (3 mL), added acrylonitrile (34 mg, 0.64 mmol) and triethylamine (7 mg, 69 μmol), the reaction mixture was reacted at room temperature 35°C for 4 hours. Then the mixture was concentrated, diluted with ethyl acetate, washed with water and saturated brine. The organic layer was dried, concentrated, and purified by a preparation plate (DCM/MeOH=20:1) to obtain the product (26 mg). ESI-MS m/z: 830.20[M+H]$^+$.

Afterwards, referring to the synthesis method of example 35, the protective group was removed under trifluoroacetic acid conditions, and the acryloyl group was introduced under alkaline conditions to obtain compound 39.

Examples 40, 42, 43, 44 and 46 were synthesized by referring to the method of example 37, and the common intermediate 38-10 was used with oxetanone, tetrahydropyrone, cyclobutanone, cyclopentanone and acetaldehyde under the conditions of reductive amination, the corresponding target compounds were prepared by reaction, and then the target compounds 40, 42, 43, 44 and 46 were finally obtained by deprotection and introduction of acryloyl group respectively.

The synthesis operation of example 41 refers to the synthesis operation of example 39, using the 1,4-addition method, methyl vinyl sulfone reacts with the common intermediate 38-10 to obtain the corresponding product, and then deprotection and introduction of acryloyl group are finally used to obtain the target compound of example 41.

The synthesis steps of example 45 are that the common intermediate 38-10 is reacted with cyclopropylformyl chloride in dichloromethane and sodium bicarbonate aqueous solution system to obtain the corresponding compound, and then deprotection and introduction of acryloyl group are finally obtained to obtain the target compound 45.

| Example | Chemical structure and name |
|---|---|
| 38 | 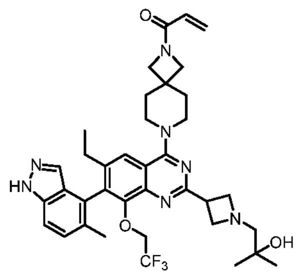 1-(7-(6-ethyl-2-(1-(2-hydroxy-2-methylpropyl)-N-heterocyclobutane-3-yl-7- (5-methyl-1H-indazole-4-yl)-8-(2,2,2-trifluoroexy)quinozoline-4-yl-2,7-diazo [3.5]nonan-2-yl)propyl-2-en-1-one; ESI-MS m/z: 692.37 [M+H]$^+$ |

(continued)

| Example | Chemical structure and name |
|---|---|
| 39 | 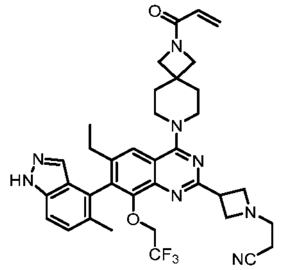<br>3-(3-(4-(2-acryloyl-2,7-diazspiro[3.5]nonan-7-yl)-6-ethyl-7-(5-methyl-1H-indazole-4-yl)-8-(2,2,2-trifluoroethoxy)quinazolin-2-yl) azacyclobutane-1-yl) propionitrile; ESI-MS m/z: 673.35 [M+H]$^+$ |
| 40 | 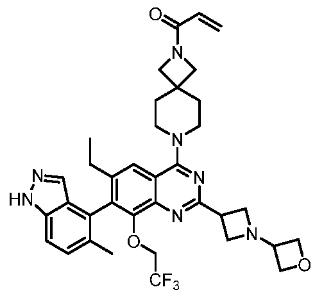<br>1-(7-(6-ethyl-7-(5-methyl-1H-indazole-4-yl)-2-(1-(oxacyclobutan-3-yl) azacyclobutane-3-yl)-8-(2,2,2-trifluoroethoxy)quinazolin-4-yl)-2,7-diazspiro [3.5]nonan-2-yl)propyl-2-en-1-one; ESI-MS m/z: 338.84 [M+2H/2]$^+$ |
| 41 | 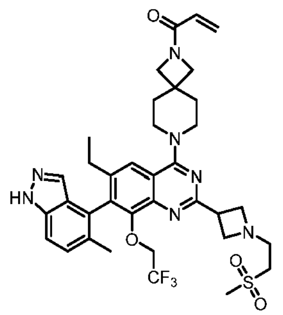<br>1-(7-(6-ethyl-7-(5-methyl-1H-indazol-4-yl)-2-(1-(2-(methylsulfonyl) ethyl)azacyclobutane-3-yl)-8-(2,2,2-trifluoroethoxy)quinazolin-4-yl)-2,7-diazspiro[3.5]nonan-2-yl)propyl-2-en-1-one; ESI-MS m/z: 726.78 [M+H]$^+$ |
| 42 | 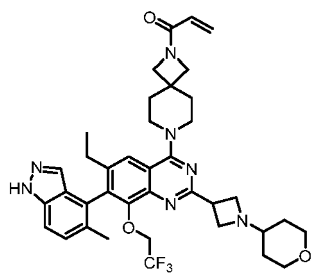<br>1-(7-(6-ethyl-7-(5-methyl-1H-indazole-4-yl)-2-(1-(tetrahydro-2H-pyran-4-yl)azacyclobutane-3-yl)-8-(2,2,2-trifluoroethoxy)quinazolin-4-yl)-2,7-diazspiro[3.5]nonan-2-yl)propyl-2-en-1-one; ESI-MS m/z: 352.89 [M+2H/2]$^+$ |

(continued)

| Example | Chemical structure and name |
|---|---|
| 43 | 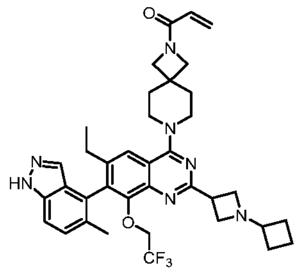<br>1-(7-(2-(1-cyclobutylazocyclobutane-3-yl)-6-ethyl-7-(5-methyl-1H-indazole-4-yl)-8-(2,2,2-trifluoroethoxy)quinazolin-4-yl)-2,7-diazarospiro[3.5]nonan-2-yl)propyl-2-en-1-one; ESI-MS m/z: 337.86 [M+2H/2]+ |
| 44 | 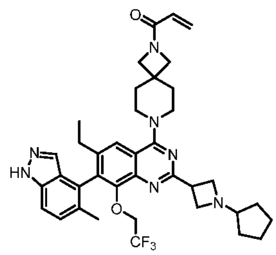<br>1-(7-(2-(1-cyclopentyl-azacyclobutane-3-yl)-6-ethyl-7-(5-methyl-1H-indazole-4-yl)-8-(2,2,2-trifluoroethoxy)quinazolin-4-yl)-2,7-diazarospiro[3.5]nonan-2-yl)propyl-2-en-1-one; ESI-MS m/z: 344.89 [M+2H/2]+ |
| 45 | 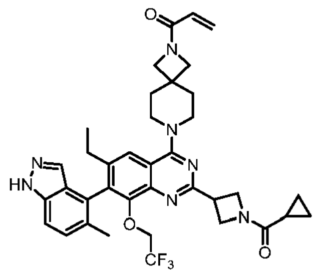<br>1-(7-(2-(1-(Cyclopropanecarbonyl)azacyclobutane-3-yl)-6-ethyl-7-(5-methyl-1H-indazol-4-yl)-8-(2,2,2-trifluoroethoxy)quinazolin-4-yl)-2,7-diazaspo[3.5]nonan-2-yl)propyl-2-en-1-one; ESI-MS m/z: 688.39 [M+H]+ |
| 46 | 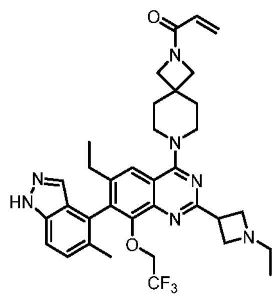<br>1-(7-(6-ethyl-2-(1-ethyl-azacyclobutane-3-yl)-7-(5-methyl-1H-indazole-4-yl)-8-(2,2,2-trifluoroethoxy) quinazolin-4-yl)-2,7-diazarospiro[3.5]nonan-2-yl)propyl-2-en-1-one; ESI-MS m/z: 325.09 [M+2H/2]+ |

**Example 47: (1-(7-(8-(2-hydroxy-2-methylpropoxy)-7-(5-methyl-1H-indazol-4-yl)-2-(1-methyl piperidin-4-yl)-6-vinylquinazoline-4-yl)-2,7-diazaspiro[3.5]nonan-2-yl)prop-2-en-1 -one)**

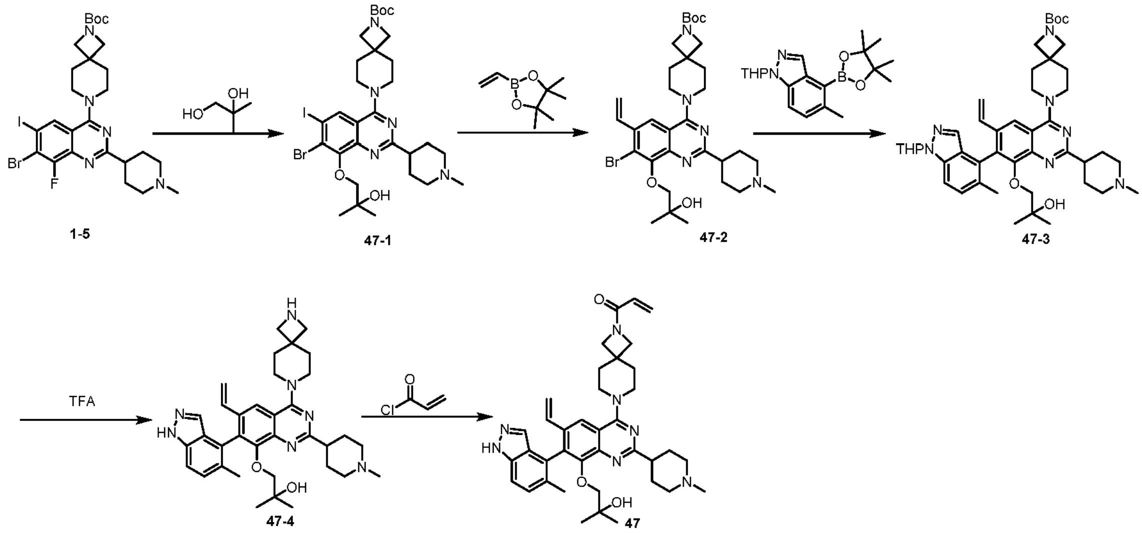

1-5 47-1 47-2 47-3 47-4 47

The synthesis operation of example 47 refers to the operation of example 25, using 2-methyl-1,2-propanediol to react with the common intermediate 1-5 to obtain compound 47-1, and then performing two coupling reactions according to example 25. The steps of deprotection and introduction of acryloyl group complete the synthesis of 47.

In examples 48 and 49, difluoroethanol and 1-(hydroxymethyl)cyclopropanecarbonitrile were used to react with the common intermediate 1-5 to obtain the corresponding target compounds, and then the final products 48 and 49 were prepared according to the steps in example 25.

| Example | Chemical structure and name |
|---|---|
| 47 | 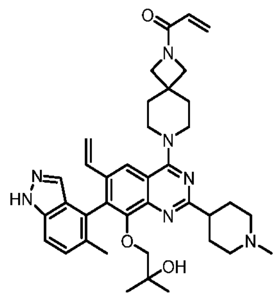 1-(7-(8-(2-hydroxy-2-methylpropoxy)-7-(5-methyl-1H-indazole-4-yl)-2-(1-methylpiperidin-4-yl)-6-vinylquinazoline-4-yl)-2,7-diazspiro[3.5]nonan-2-yl) propyl-2-en-1-one; ESI-MS m/z: 650.50 $[M+H]^+$ |
| 48 | 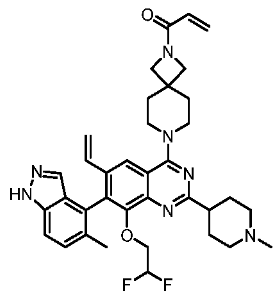 1-(7-(8-(2,2-difluoroethoxy)-7-(5-methyl-1H-indazol-4-yl)-2-(1-methylpiperidin-4-yl)-6-vinylquinazolin-4-yl)-2,7-diazspiro[3.5]nonan-2-yl) propyl-2-en-1-one; ESI-MS m/z: 642.36 $[M+H]^+$ |

(continued)

| Example | Chemical structure and name |
|---|---|
| 49 | 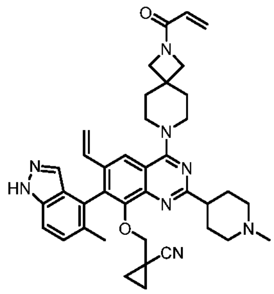<br>1-((4-(2-acryloyl-2,7-diazazospiro[3.5]nonan-7-yl]-7-(5-methyl-1H-indazole-4-yl)-2-(1-methylpiperidine-4-yl)-6-vinylquinazolin-8-yl)oxy)methyl) cyclopropane-1-methylnitrile; ESI-MS m/z: 657.50 [M+H]+ |

**Example 50: (1-(6-(7-(5-methyl-1H-indazol-4-yl)-2-(1-methylpiperidin-4-yl)-8-(2,2,2-trifluoro ethoxy)-6-vinylquinazolin-4-yl)-2,6-diazaspiro [3.3] heptane-2-yl)prop-2-en-1-one)**

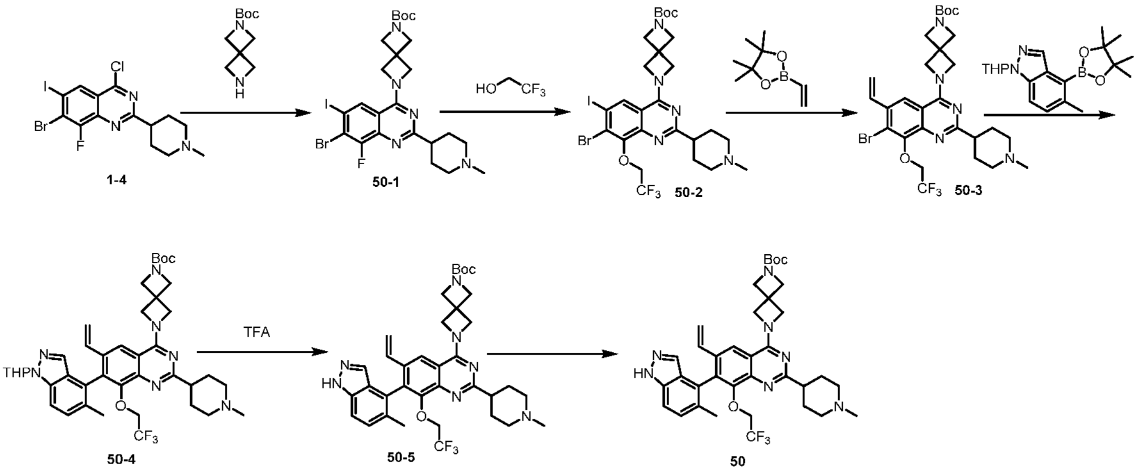


1-4 50-1 50-2 50-3 50-4 50-5 50

Step 1: Synthesis of compound 50-1

At room temperature, DIEA (480 mg, 3.71 mmol) was added to 1-4 (600 mg, 1.24 mmol), 2,6-diazaspiro[3.3]heptane-2-carboxylic acid tert-butyl ester (245 mg, 1.24 mmol) of dioxane solution (10 mL), stirring at room temperature at 40°C. The mixture was poured into ice water, and extracted with ethyl acetate, the organic layer was washed with brine, dried over $Na_2SO_4$ and concentrated in vacuo. The crude product was purified by flash silica gel column chromatography (DCM:MeOH=10:1) to obtain a yellow solid of the desired target product 50-1 (680 mg, 85% yield).

Afterwards, the synthesis of the target product 50 was completed with reference to the synthesis procedure of example 25.

For the synthesis of examples 51 and 52 refer to the synthesis of 50-1 using 2,7-diazaspiro[3.5]nonane-7-tert-butyl carboxylate and (S)-4-N-tert-butoxycarbonyl-2-methylpiperazine reacts with the common intermediate 1-4 to obtain the corresponding compound, and then referring to the synthesis step of 25 to complete the synthesis of the target products 51 and 52.

| Example | Chemical structure and name |
|---|---|
| 50 | 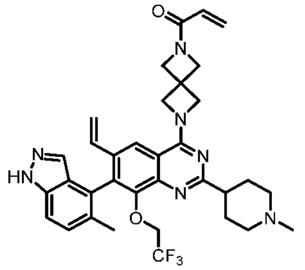 1-(6-(7-(5-methyl-1H-indazol-4-yl)-2-(1-methylpiperidin-4-yl)-8-(2,2,2-trifluoroethoxy)-6-vinylquinazolin-4-yl)-2,6-diazspiro [3.3] heptane-2-yl) propyl-2-en-1-one; ESI-MS m/z: 632.36 $[M+H]^+$ |
| 51 | 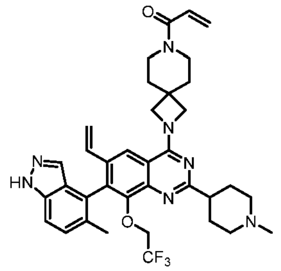 1-(2-(7-(5-methyl-1H-indazol-4-yl)-2-(1-methylpiperidin-4-yl)-8-(2,2,2-trifluoroethoxy)-6-vinylquinazolin-4-yl)-2,7-diazspiro [3.5] nonan-2-yl) propyl-2-en-1-one; ESI-MS m/z: 660.50 $[M+H]^+$ |
| 52 | 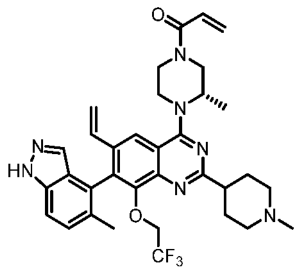 1-((3S)-3-methyl-4-(7-(5-methyl-1H-indazole-4-yl)-2-(1-methylpiperidin-4-yl)-8-(2,2,2-trifluoroethoxy)-6-vinylquinazolin-4-yl) piperazin-1-yl) propyl-2-en-1-one; ESI-MS m/z: 634.45 $[M+H]^+$ |

**Example 53: (1-(7-(7-(5-methyl-1H-pyrazolo[3,4-b]pyridin-4-yl)-2-(1-methylpiperidine-4-yl)-8-(2,2,2-trifluoroethoxy)-6-vinylquinazolin-4-yl)-2,7-diazaspiro [3.5] nonan-2-yl)prop-2-en-1-one)**

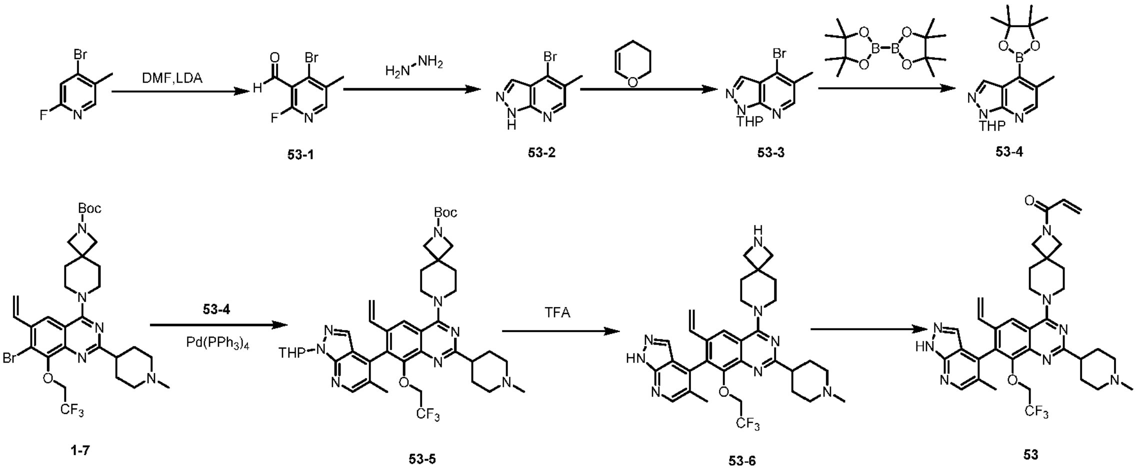

Step 1: Synthesis of compound 53-1

LDA (2.7 mL, 2M in THF) was diluted into 5 mL tetrahydrofuran, the mixture was cooled to - 78°C, and added 4-bromo-2-fluoro-5-methylpyridine (1 g, 5.26 mmol) in tetrahydrofuran solution (5mL), after stirring for 1 hour at low temperature, DMF (385 mg, 5.26 mmol) was added. After the addition, the mixture was stirred at low temperature for 1 hour. The reaction was monitored by TLC and LCMS. After the reaction was completed, saturated ammonium chloride was added for quenching, and extracted with ethyl acetate, the organic layer was dried and spin-dried to obtain a crude product (0.8 g, 70% yield).

Step 2: Synthesis of compound 53-2

Compound 53-1 (0.8 g, 3.67 mmol) was dissolved in dioxane, hydrazine hydrate (118 mg, 3.67 mmol) was added, then heating to 90°C and reacting for 2 hours. After the reaction was completed and cooled to room temperature, the mixture was dried and added 3 mL EA/PE(1/2) mixed solvent, the solid was collected by filtration to obtain the product (0.4 g, 51% yield).

Step 3: Synthesis of compound 53-3

Compound 53-2 (0.18 g, 0.85 mmol) was dissolved in dry THF (10 mL), 3,4-dihydro-2H-pyran (143 mg, 1.7 mmol) and p-toluenesulfonic acid (15mg, 85μmol) were added, stirring at room temperature overnight. Concentrating under reduced pressure can obtain the crude product, which was prepared (PE/EA=15:1) to obtain the target product (0.19 g, 75% yield).

Step 4: Synthesis of compound 53-4

The compound 53-3 (0.19 g, 0.64 mmol), pinacol diborate (196 mg, 0.77 mmol), potassium acetate (126 mg, 1.28 mmol) and Pd(dppf)$Cl_2$.$CH_2Cl_2$ (104 mg, 0.13 mmol) ) were dissolved in dioxane, which was protected by nitrogen exchange, heated to 90°C and reacted for 2 hours. Then the mixture was cooled to room temperature, diluted with water, and extracted with ethyl acetate, the organic layer was dried, concentrated, and prepared by Pre-TLC (PE/EA=15:1). The target product (127 mg, 58% yield) was obtained.

Afterwards, the synthesis of the target product 53 was completed by referring to the steps of example 25.

**Example 54: (1-(7-(8-(((5-methyl-1H-indazol-4-yl)oxy)-2-(1-methylpiperidin-4-yl) quinazo lin-4-yl)-2,7-diazaspiro [3.5] nonan-2-yl)prop-2-en-1-one)**

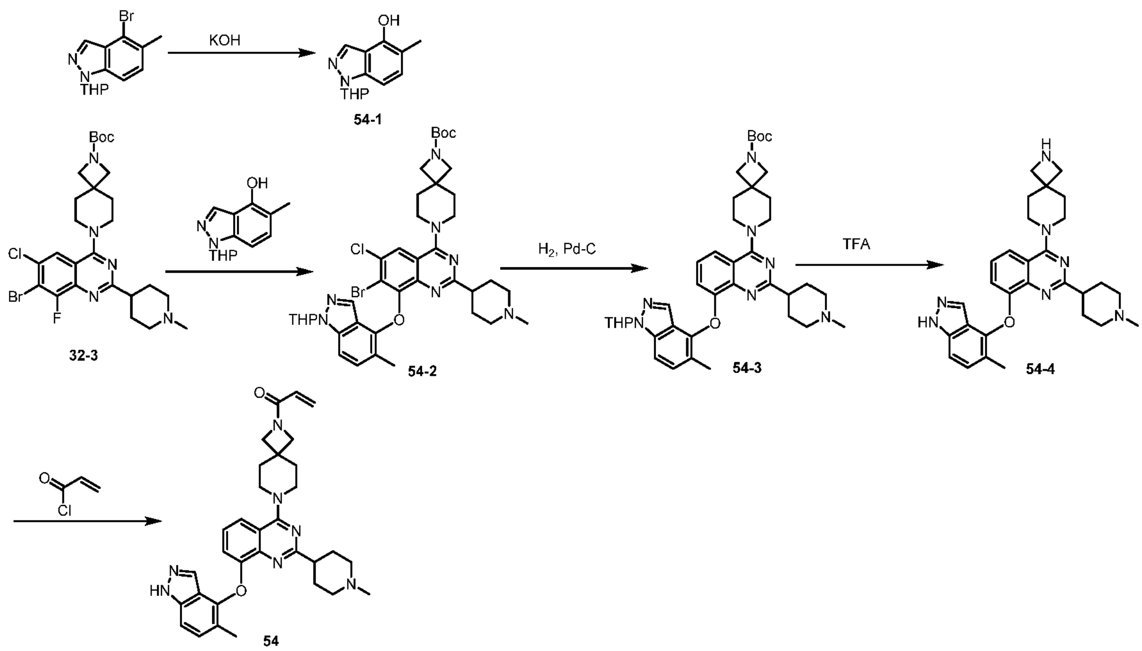

54-1
32-3
54-2
54-3
54-4
54

Step 1: Synthesis of compound 54-1

An aqueous solution (2 mL) of potassium hydroxide (861 mg, 15.35 mmol) was added to dioxane (18 mL), and the compound 4-bromo-5-methyl-1-(tetrahydro-2H-pyran-2-base)-1H-indazole (1.5 g, 5.12 mmol), $Pd_2(dba)_3$ (234 mg, 0.26 mmol) and t-Bu-Xphos(217 mg, 0.51 mmol) were added in turn, stirring overnight at 95°C. The reaction mixture was cooled to room temperature, added water to dilute, adjusted the pH to 2-3 with 2 N hydrochloric acid, extracted with ethyl acetate, and washed with saturated brine once. The organic layer was dried, concentrated, and purified by column chromatography (PE/EA=100:10) to obtain the target product (1.06 g, 89% yield).

Step 2: Synthesis of compound 54-2

Compound 32-3 (324 mg, 0.56 mmol) was dissolved in dioxane (8 mL), cesium carbonate (365 mg, 1.11 mmol) and 54-1 (129 mg, 0.56 mmol) were added. The reaction mixture was heated to 100°C and stirred overnight, there was a small amount target product. The mixture was added with saturated brine, extracted with ethyl acetate, dried, concentrated and purified by Pre-TLC (DCM/MeOH=100:7) to obtain the target product (50 mg, 11% yield). ESI-MS m/z: 794.23 $[M+H]^+$.

Step 3: Synthesis of compound 54-3

Compound 54-2 (50 mg, 63 μmol) was dissolved in methanol (10 mL), Pd/C (100 mg) was added, hydrogen was replaced three times, and the reaction was carried out at 35°C overnight. The reaction mixture was filtered, concentrated, and purified by Pre-TLC (DCM/MeOH=10/1) to obtain the target product (12 mg, 27% yield). ESI-MS m/z: 682.31$[M+H]^+$.

Then the target product 54 was synthesized by referring to the operation of 25.

**Example 55: (1-(7-(7-(1,7-dihydropyrazolo[4,3-fJindazol-4-yl)-2-(1-methylpiperidine- 4-yl)-8-(2,2,2-trifluoroethoxy)-6-vinylquinazolin-4-yl)-2,7-diazaspiro [3.5] nonan-2-yl)prop-2-en-1-one)**

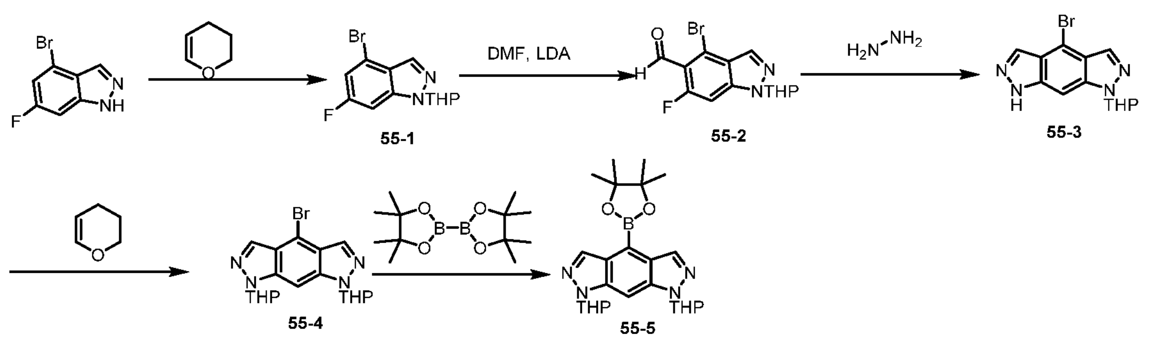

55-1
55-2
55-3
55-4
55-5

Step 1: Synthesis of compound 55-1

The reactant 4-bromo-6-fluoroindazole (1 g, 4.65 mmol) was dissolved in dichloromethane (10 mL), 3,4-dihydro-2H-pyran (782 mg, 9.3 mmol) and p-toluenesulfonic acid were added (74 mg, 0.45 mmol), stirring at room temperature for 4 hours. The reaction mixture was dried, diluted with ethyl acetate, washed with aqueous sodium bicarbonate solution and saturated brine, the organic layer was dried over sodium sulfate, and purified by column chromatography (PE/EA=10:1) to obtain the target product (440 mg, 31% yield).

Step 2: Synthesis of compound 55-2

Compound 55-1 (200 mg, 0.67 mmol) was dissolved in anhydrous tetrahydrofuran at room temperature, which was cooled to -78°C, added LDA (0.5 mL, 2 M in THF), stirred at low temperature for 30 minutes, added DMF (0.7 mL), and stirred at low temperature for 30 minutes. The reaction mixture was quenched by adding saturated ammonium chloride, diluted with ethyl acetate, washed with saturated brine, the organic layer was dried with sodium sulfate, and concentrated to obtain the crude product (270 mg), which was used directly in the next step.

Step 3: Synthesis of compound 55-3

Compound 55-2 (270 mg, crude product, 0.67 mmol) was dissolved in dioxane (2 mL), hydrazine hydrate (65 mg, 1.3 mmol) was added, and the temperature was raised to 90°C to react. After the reaction was completed, it was diluted with water and extracted with dichloromethane. The organic layer was dried and concentrated to obtain the target product (300 mg, crude product).

Step 4: Synthesis of compound 55-4

Compound 55-3 (300 mg, crude product, 0.67 mmol) was dissolved in dichloromethane (5 mL), p-toluenesulfonic acid (15 mg, 94 μmol) and 3,4-dihydro-2*H*-pyran (63 mg, 0.75 mmol) were added, stirring overnight at room temperature. The reaction mixture was concentrated, diluted with ethyl acetate, washed with aqueous sodium bicarbonate solution and saturated brine, the organic layer was dried over sodium sulfate, filtered and concentrated to obtain a crude product. The crude product was prepared by Pre-TLC (PE/EA=8:1) to obtain the target product (48 mg, three-step yield 18%).

Step 5: Synthesis of compound 55-5

Compound 55-4 (48 mg, 0.12 mmol), pinacol biborate (45 mg, 0.18 mmol), potassium acetate (35 mg, 0.36 mmol) and $Pd(dppf)Cl_2.CH_2Cl_2$ (29 mg, 35 μmol) were dissolved in dioxane, nitrogen exchange protection, which was heating to 90°C and reacting for 2 hours. The reaction mixture was cooled to room temperature, added water to dilute, extracted with ethyl acetate, the organic layer was dried, concentrated, and prepared by Pre-TLC (PE/EA = 15:1) to obtain the target product (38 mg, 70% yield).

After that, the synthesis of the target product 55 was completed according to the steps of example 25.

**Example 56: (1-(7-(7-(5-methyl-1H-pyrazolo [3,4-c]pyridin-4-yl)-2-(1-methylpiperidine-4-yl)-8-(2,2,2-trifluoroethoxy)-6-vinylquinazolin-4-yl)-2,7-diazaspiro[3.5]nonan-2-yl) prop-2-en-1-one)**

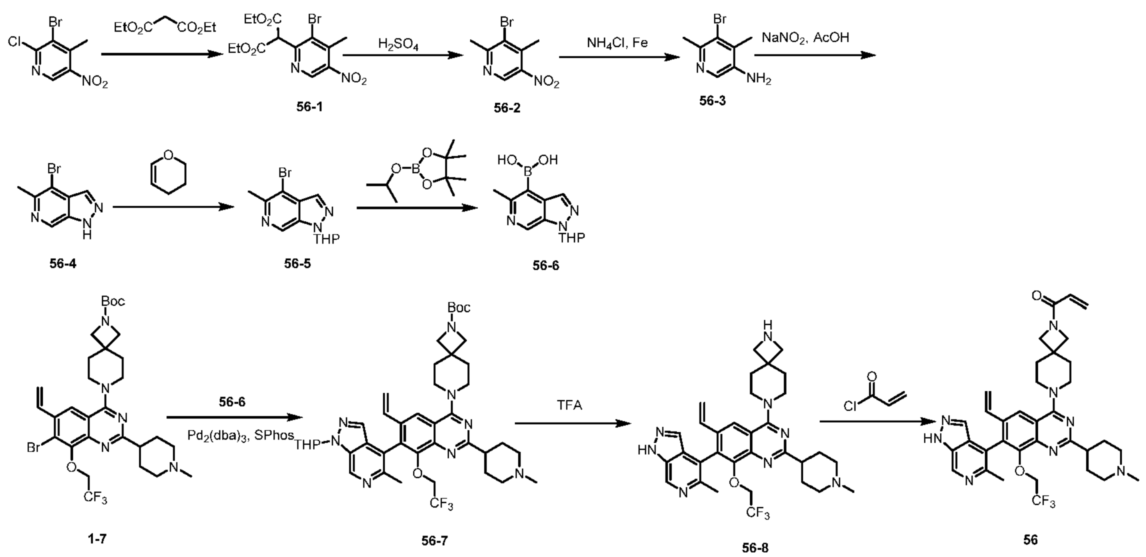


Step 1: Synthesis of compound 56-1

NaH (344 mg, 14.3 mmol) was added to diethyl malonate (2.3 g, 14.3 mmol) in tetrahydrofuran (20 mL) and stirred for 5 minutes, the reactant 3-bromo-2-chloro-4-methyl-5-nitropyridine (3 g, 11.9 mmol) was added, continuing to stir for 2 hours. The reaction was complete, added saturated ammonium chloride to quench, and ethyl acetate to extract, the organic layer was washed with saturated brine, dried, concentrated, and purified by column chromatography to obtain the target product (2.5 g, 55% yield).

Step 2: Synthesis of compound 56-2

Compound 56-1 (2.5 g, 6.7 mmol) was added to 6M $H_2SO_4$ (15 mL), which was heated to 110°C and stirred overnight. The reaction was complete, cooled to room temperature, sodium hydroxide neutralized the pH of the reaction solution to about 10. The organic layer was washed with brine, dried and concentrated to obtain the product (1.2 g, 78% yield).

Step 3: Synthesis of compound 56-3

Put compound 56-2 (1.2 g, 5.2 mmol), ammonium chloride (336 mg, 6.23 mmol), iron powder (1.5 g, 26 mmol) in the reaction flask, the reaction was added ethanol (12 mL) and water (3 mL), stirred at 80°C. After 1 hour, the reaction was completed, cooled to room temperature, spin-dried, and purified by column chromatography (DCM/MeOH=10:1) to obtain the target product (0.87 g, 83% yield).

Step 4: Synthesis of compound 56-4

Sodium nitrite (328 mg, 4.76 mmol) was added to the acetic acid solution of 56-3, which was stirred at 60°C for 2 hours, and the solvent was spin-dried. The crude was purified by TLC (DCM/MeOH=20:1) to obtain the target product (400 mg, 44 % yield).

Step 5: Synthesis of compound 56-5

Compound 56-4 (1.1 g, 5.2 mmol) was dissolved in dichloromethane (10 mL), *p*-toluenesulfonic acid (89 mg, 0.52 mmol) was added, 3,4-dihydro-2*H*-pyran (873 mg, 10.4 mmol) was added, the reaction mixture was stirred overnight at room temperature, concentrated, diluted with ethyl acetate, washed with aqueous sodium bicarbonate solution and saturated brine, the organic layer was dried over sodium sulfate, filtered and concentrated to obtain a crude product. The crude product was prepared by TLC (PE/EA=8:1) to obtain the target product (1.2 g, 78% yield).

Step 6: Synthesis of compound 56-6

Dissolve compound 56-5 (200 mg, 0.68 mmol) in tetrahydrofuran (5 mL), which was cooled to -78°C, added butyllithium (0.3 mL, 2.5 M), stirred at low temperature for 1 hour, and added isopropanol pinacol boric acid ester (188 mg, 1.0 mmol), the mixture was stirred at low temperature for 1 hour. After the reaction was complete, which was added saturated ammonium chloride to quench, extracted with ethyl acetate, the organic layer was dried and concentrated to obtain the product (100 mg, 57% yield).

Step 7: Synthesis of compound 56-7

Compound 56-6 (100 mg, 0.38 mmol), compound 1-7 (84 mg, 0.13 mmol), potassium phosphate (81 mg, 0.38 mmol), S-Phos (10 mg, 25 $\mu$mol), and $Pd_2(dba)_3$ (13 mg, 13 $\mu$mol) were placed in a reaction flask, which was added dioxane (2 mL) and water (0.5 mL), replaced with nitrogen three times, warmed to 90°C and stirred overnight. After the reaction was complete, the reaction mixture was cooled down, extracted with ethyl acetate, the organic layer was washed with saturated brine, dried and concentrated. The target product (20 mg, 20% yield) was prepared from the crude product by TLC. ESI-MS m/z: 791.41 $[M+H]^+$.

Afterwards, the synthesis of the target product 56 was completed according to the steps of Example 25.

| Example | Chemical structure and name |
|---|---|
| 53 | 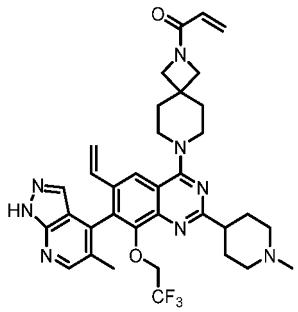<br>1-(7-(7-(5-methyl-1H-pyrazolo[3,4-b]pyridin-4-yl)-2-(1-methylpiperidin-4-yl)-8-(2,2,2-trifluoroethoxy)-6-vinylquinazolin-4-yl)-2,7-diazspiro [3.5] nonan-2-yl) propyl-2-en-1-one; ESI-MS m/z: 631.70 [M+H]$^+$ |
| 54 | 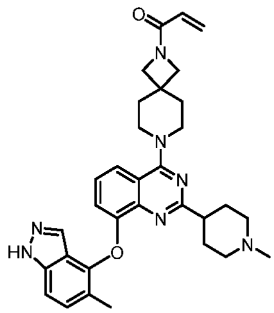<br>1-(7-(8-((5-methyl-1H-indazol-4-yl)oxy)-2-(1-methylpiperidin-4-yl) quinazolin-4-yl)-2,7-diazazospiro [3.5] nonan-2-yl) propyl-2-en-1-one; ESI-MS m/z: 552.72 [M+H]$^+$ |
| 55 | 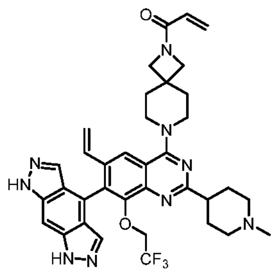<br>1-(7-(7-(1,7-dihydropyrazolo[4,3-f]indazol-4-yl)-2-(1-methylpiperidin-4-yl)-8-(2,2,2-trifluoroethoxy)-6-vinylquinazolin-4-yl)-2,7-diazspiro [3.5] nonan-2-yl) propyl-2-en-1-one; ESI-MS m/z: 686.72 [M+H]$^+$ |
| 56 | 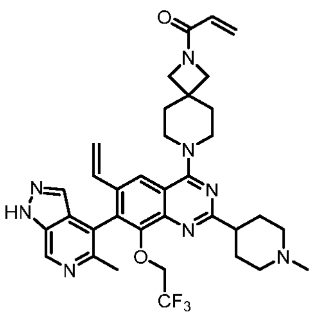<br>1-(7-(7-(5-methyl-1H-pyrazo-3,4-c)pyridine-4-yl-2-(1-methylpiperidine-4-yl) -8-(2,2,2-trifluoroethoxy)-6-vinylquinozoline-4-yl-2,7-diazo[3.5] nonan-2-yl) propyl-2-en-1-one; ESI-MS m/z: 631.70 [M+H]$^+$ |

**Example 57: (1-(7-(7-(5-methyl-1H-indazol-4-yl)-2-(1-(2-(methylsulfonyl)ethyl)piperidine -4-yl)-8-(2,2,2-trifluoroethoxy)-6-vinylquinazolin-4-yl)-2,7-diazaspiro[3.5]nonan-2-yl) prop-2-en-1-one)**

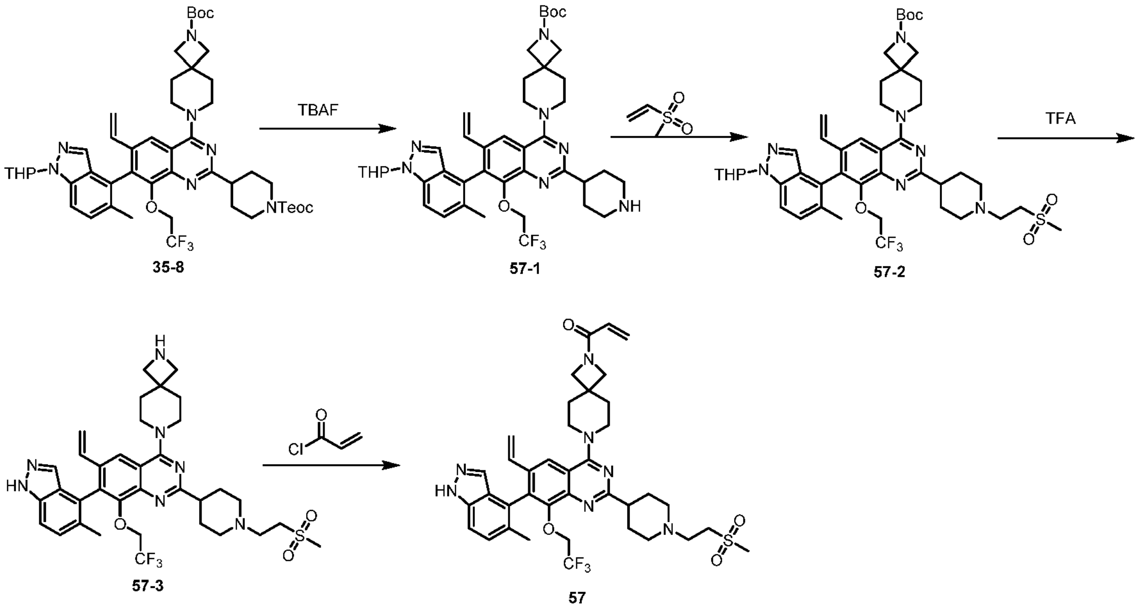


35-8 57-1 57-2 57-3 57

Step 1: Synthesis of compound 57-1

At room temperature, dissolve compound 35-8 (340 mg, 0.37 mmol) in THF (5 mL), which was added TBAF (2 mL, 1 M in THF), stirred overnight at 35°C. The reaction mixture was concentrated, and diluted with ethyl acetate, the organic layer was washed twice with water, twice with saturated brine, dried with sodium sulfate, and concentrated to obtain the product (311 mg, crude product).

Step 2: Synthesis of compound 57-2

Compound 57-1 (50 mg, 64 μmol) was dissolved in ethanol (3 mL) at room temperature, methyl vinyl sulfone (68 mg, 0.64 mmol) was added, and the mixture was stirred overnight at room temperature. The reaction was monitored by LCMS, concentrated, and diluted with ethyl acetate, the organic layer was washed once with water, washed twice with saturated brine, dried, and concentrated to obtain a crude product. TLC (DCM/MeOH=20:1) was prepared to obtain 35-11 (32 mg, 36% yield). ESI-MS m/z: 882.46 $[M+H]^+$.

Step 3: Synthesis of compound 57-3

At room temperature, 57-2 (32 mg, 36 μmol) was dissolved in a mixed solvent of DCM (2 mL) and TFA (1 mL), and moved to 40°C to react for about 1 h. After the reaction was complete, it was directly concentrated to remove the solvent to obtain the crude target product 57-3. ESI-MS m/z: 698.40 $[M+H]^+$.

Step 4: Synthesis of compound 57

Under the protection of nitrogen, in an ice-water bath, the THF solution of acryloyl chloride (4 mg, 45 μmol) was added to 57-3 (25 mg, 36 μmol, crude) in THF (2 mL) and saturated $Na_2CO_3$ (0.5 mL) solution, adding it to react completely. The reaction mixture was poured into water, extracted with ethyl acetate, the organic layer was washed with brine, dried over $Na_2SO_4$ and concentrated in vacuo. The concentrate was purified by pre-TLC (DCM/MeOH=10:1) to give the desired product 57 (6.6 mg, 24% yield). ESI-MS m/z: 752.45 $[M+H]^+$.

**Example 63: (1-(7-(7-(5-methyl-1H-indazol-4-yl)-2-(piperidin-4-yl)-8-(2,2,2-trifluoroethoxy) - 6-vinylquinazoline-4-yl)-2,7-diazaspiro[3.5]nonan-2-yl)prop-2-en-1-one)**

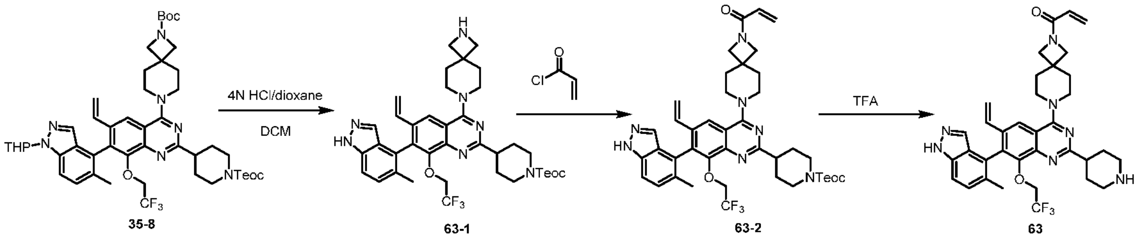


35-8 63-1 63-2 63

Step 1: Synthesis of compound 63-1

Compound 35-8 (100 mg, 0.11 mmol) was dissolved in dichloromethane (2 mL), and dioxane hydrochloride solution (0.5 mL, 4 N in dioxane) was added. The reaction solution was concentrated to obtain the crude product (50 mg), which was used directly in the next step.

Step 2: Synthesis of compound 63-2

Under the protection of nitrogen, in an ice water bath, the THF solution of acryloyl chloride (6 mg, 68 $\mu$mol) was added to 63-1 (50 mg, 68 $\mu$mol, crude) in THF (2 mL) and saturated $Na_2CO_3$ (1 mL) solution, add it and the reaction was complete. The reaction mixture was poured into water, extracted with ethyl acetate, the organic layer was washed with brine, dried over $Na_2SO_4$ and concentrated in vacuo, and concentrated to obtain a crude product (30 mg).

Step 3: Synthesis of compound 63

Compound 63-2 (30 mg) was dissolved in dichloromethane (1 mL), TFA (1 mL) was added, and the mixture was stirred at room temperature for 2 hours. After the reaction was completed, concentrated, the preparation plate was prepared (DCM/MeOH=10:1) to obtain the target product (2.6 mg, 11% yield).

Example 59 was synthesized by referring to the steps in Example 1. Acrylonitrile was reacted with the common intermediate 57-1 to obtain the corresponding target product, and then deprotection and introduction of acryloyl group were performed to finally obtain the target compound 59.

Examples 58, 65, 66, 67, 68, 69, 71, 72, 73, 74, 77, 78, 79, 80, 82, and 83 reference Example 37 adopting the method of reductive amination, using different ketones or aldehydes reacting with the common intermediate 57-1 to obtain the corresponding product, and then deprotecting and introducting the acetyl group to synthesize the final target product. Example 60 refers to Example 36, and the common intermediate 57-1 is reacted with methyl propylene oxide to form the corresponding product. Then deprotection and introduction of acryloyl group finally obtain the target compound 60; Examples 61, 70, 75, 76, 81 adopt the strategy of alkylation to react with the common intermediate 57-1 to produce the corresponding product, and then deprotection and introduction of acryloyl group finally obtain the final target product.

The corresponding synthetic intermediates are as follows:

| Example | Intermediate |
|---|---|
| 57 | 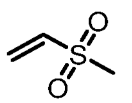 |
| 58 | 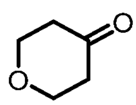 |
| 59 | 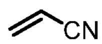 |
| 60 | 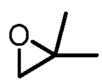 |

(continued)

| Example | Intermediate |
|---|---|
| 61 | 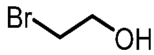 |
| 62 | 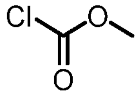 |
| 63 | / |
| 64 | 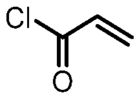 |
| 65 | 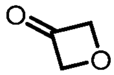 |
| 66 | 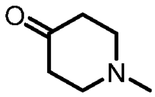 |
| 67 | 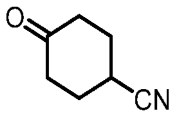 |
| 68 | 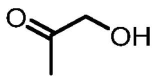 |
| 69 | 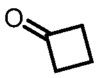 |
| 70 | 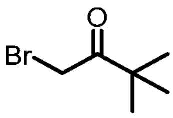 |
| 71 | 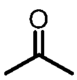 |
| 72 | 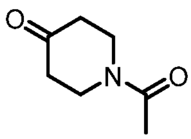 |
| 73 | 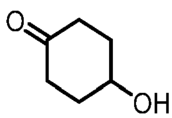 |
| 74 | 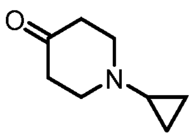 |

(continued)

| Example | Intermediate |
|---|---|
| 75 | 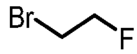 |
| 76 | 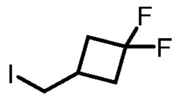 |
| 77 | 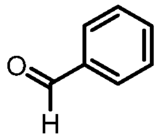 |
| 78 | 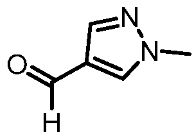 |
| 79 | 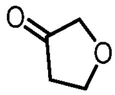 |
| 80 | 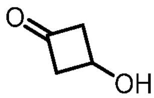 |
| 81 | 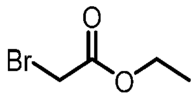 |
| 82 | 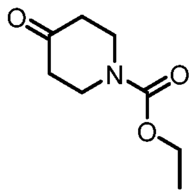 |
| 83 | 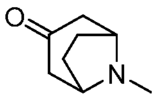 |

| Example | Chemical structure and name |
|---|---|
| 57 | 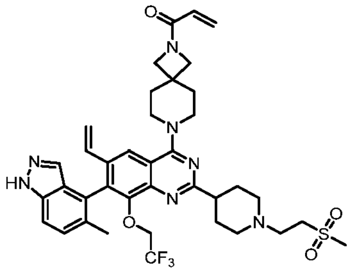<br>1-(7-(7-(5-methyl-1H-indazol-4-yl)-2-(1-(2-(methylsulfonyl)ethyl) piperidin-4-yl)-8-(2,2,2-trifluoroethoxy)-6-vinylquinazolin-4-yl)-2,7-diazspiro[3.5]nonan-2-yl)propyl-2-en-1-one; ESI-MS m/z: 752.79 $[M+H]^+$ |
| 58 | 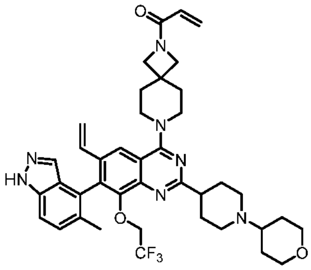<br>1-(7-(7-(5-methyl-1H-indazole-4-yl)-2-(1-(tetrahydro-2H-pyran-4-yl) piperidin-4-yl)-8-(2,2,2-trifluoroethoxy)-6-vinylquinazolin-4-yl)-2,7-diazspiro[3.5]nonan-2-yl)propyl-2-en-1-one; ESI-MS m/z: 730.82 $[M+H]^+$ |
| 59 | 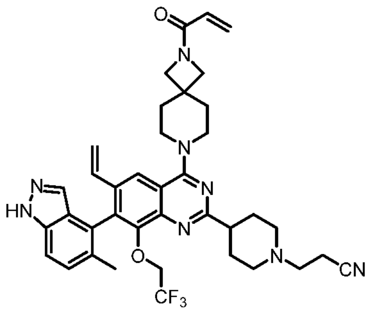<br>3-(4-(4-(2-acryloyl-2,7-diazazospiro[3.5]nonan-7-yl)-7-(5-methyl-1H-indazole-4-yl)-8-(2,2,2-trifluoroethoxy)-6-vinylquinazolin-2-yl)piperidin-1-yl) propionitrile; ESI-MS m/z: 699.77 $[M+H]^+$. $^1H$ NMR (500 MHz, DMSO) δ 13.01 (s, 1H), 8.00 (s, 1H), 7.49 (m, 1H), 7.38 (m, 1H), 7.31 (m, 1H), 6.35 (m, 1H), 6.16-6.02 (m, 2H), 5.72 (m, 2H), 5.11 (m, 1H), 4.98 (m, 1H), 4.69 (m, 1H), 4.05 (s, 2H), 3.77 (s, 6H), 3.01 (m, 2H), 2.76-2.64 (m, 3H), 2.60 (m, 2H), 2.14 (m, 2H), 2.07-1.93 (m, 9H), 1.84 (m, 2H). |
| 60 | 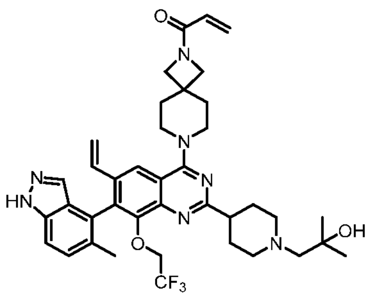<br>1-(7-(2-(1-(2-(2-hydroxy-2-methylpropyl))piperidine-4-yl-7-(5-methyl-1H-indozol-4-yl)-8-(2,2,2-trifluoroethoxy)-6-vinylquinozoline-4-yl-2,7-diazo [3.5]nonan-2-yl)propyl-2-en-1-one; ESI-MS m/z: 718.40 $[M+H]^+$ |

(continued)

| Example | Chemical structure and name |
|---|---|
| 61 | 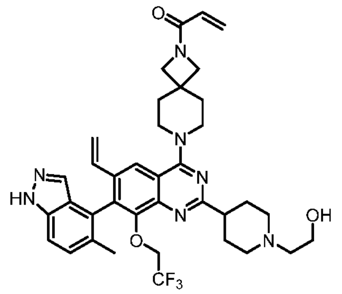 1-(7-(2-(1-(2-(hydroxyethyl))piperidin-4-yl)-7-(5-methyl-1H-indazole-4-yl)-8-(2,2,2-trifluoroethoxy)-6-vinylquinazolin-4-yl)-2,7-diazarospiro[3.5] nonan-2-yl)propyl-2-en-1-one; ESI-MS m/z: 690.36 $[M+H]^+$ |
| 62 | 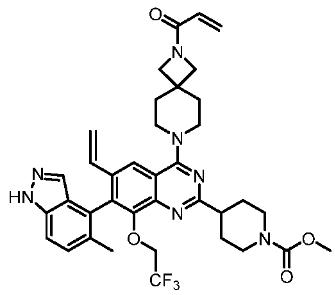 Methyl 4-(4-(2-acryloyl-2,7-diazspiro[3.5]nonan-7-yl)-7-(5-methyl-1H-indazole-4-yl)-8-(2,2,2-trifluoroethoxy)-6-vinylquinazolin-2-yl)piperidine-1-carboxylic acid; ESI-MS m/z: 704.73 $[M+H]^+$ |
| 63 | 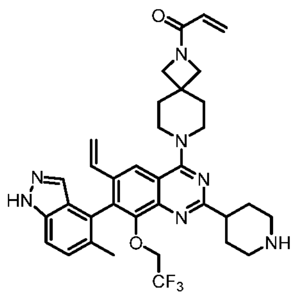 1-(7-(7-(5-methyl-1H-indozol-4-yl)-2-(piperidine-4-yl)-8-(2,2,2-trifluoroethyloxy)-6-vinylquinoline-4-yl-2,7-diazo[3.5]nonan-2-yl)propyl-2-en-1-one; ESI-MS m/z: 646.36 $[M+H]^+$ |
| 64 | 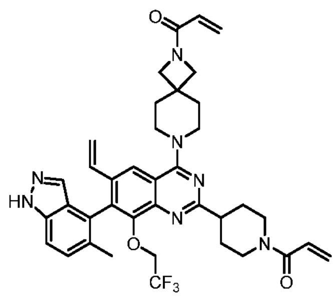 1-(4-(4-(2-acryloyl-2,7-diazazospiro[3.5]nonan-7-yl)-7-(5-methyl-1H-indazole-4-yl)-8-(2,2,2-trifluoroethoxy)-6-vinylquinazolin-2-yl)piperidin-1-yl)propyl-2-en-1-one; ESI-MS m/z: 700.80 $[M+H]^+$ |

(continued)

| Example | Chemical structure and name |
|---|---|
| 65 | 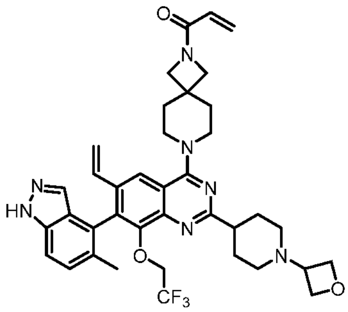 1-(7-(7-(5-methyl-1H-indazol-4-yl)-2-(1-(oxacyclobutan-3-yl)piperidin-4-yl)-8-(2,2,2-trifluoroethoxy)-6-vinylquinazolin-4-yl)-2,7-diazspiro[3.5] nonan-2-yl)propyl-2-en-1-one; ESI-MS m/z: 702.47 [M+H]$^+$ |
| 66 | 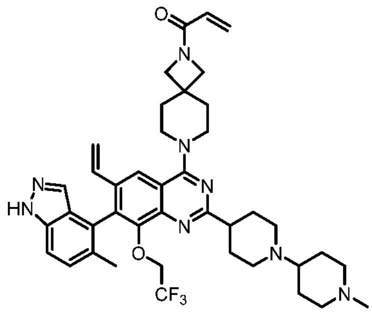 1-(7-(7-(5-methyl-1H-indazol-4-yl)-2-(1'-methyl-[1,4'-bipiperidine]-4-yl)-8-(2,2,2-trifluoroethoxy)-6-vinylquinazolin-4-yl)-2,7-diazspiro[3.5] nonan-2-yl)propyl-2-en-1-one; ESI-MS m/z: 743.89 [M+H]$^+$ |
| 67 | 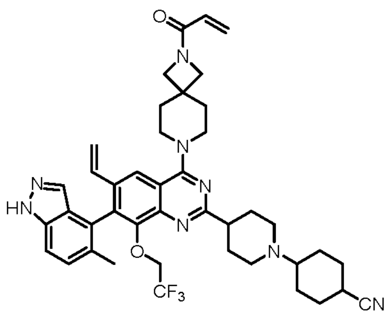 4-(4-(4-(2-acryloyl-2,7-diazazospiro[3.5]nonan-7-yl)-7-(5-methyl-1H-indazole-4-yl)-8-(2,2,2-trifluoroethoxy)-6-vinylquinazolin-2-yl)piperidin-1-yl)cyclohexane-1-methylnitrile; ESI-MS m/z: 753.90 [M+H]$^+$ |

(continued)

| Example | Chemical structure and name |
|---|---|
| 68 | 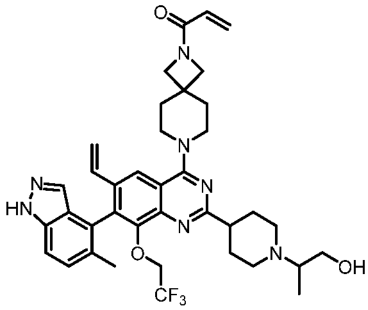 1-(7-(2-(1-(1-hydroxypropane-2-yl)piperidin-4-yl)-7-(5-methyl-1H-indazol-4-yl)-8-(2,2,2-trifluoroethoxy)-6-vinylquinazolin-4-yl)-2,7-diazspiro [3.5]nonan-2-yl)propyl-2-en-1-one; ESI-MS m/z: 704.34 $[M+H]^+$. $^1H$ NMR (500 MHz, DMSO) δ 13.02 (s, 1H), 8.01 (s, 1H), 7.50 (m, 1H), 7.39 (s, 1H), 7.33 (m, 1H), 6.35 (m, 1H), 6.17-6.03 (m, 2H), 5.73 (m, 2H), 5.12 (m, 1H), 4.96 (s, 1H), 4.67 (s, 1H), 4.05 (s, 2H), 3.77 (s, 8H), 2.89 (m, 2H), 2.26-1.81 (m, 15H), 1.07 (m, 3H). |
| 69 | 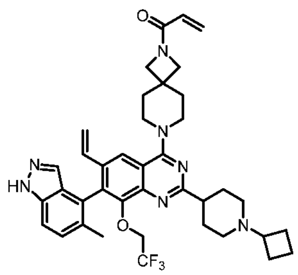 1-(7-(2-(1-cyclobutylpiperidin-4-yl)-7-(5-methyl-1H-indazol-4-yl)-8- (2,2,2-trifluoroethoxy)-6-vinylquinazolin-4-yl)-2,7-diazspiro[3.5]nonan-2-yl) propyl-2-en-1-one; ESI-MS m/z: 700.36 $[M+H]^+$. $^1H$ NMR(500 MHz, DMSO) δ 13.03 (s, 1H), 8.01 (s, 1H), 7.50 (m, 1H), 7.39 (s, 1H), 7.33 (m, 1H), 6.35 (m, 1H), 6.17-6.07 (m, 2H), 5.73 (m, 2H), 5.12 (m, 1H), 4.96 (s, 1H), 4.7-4.54 (m, 1H), 4.01 (m, 2H), 3.77 (s, 6H), 2.81 (m, 3H), 2.41-1.82 (m, 17H), 1.64 (m, 3H). |
| 70 | 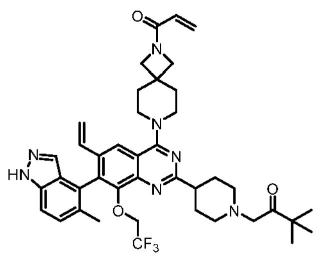 1-(4-(4-(2-acryloyl-2,7-diazspiro[3.5]nonan-7-yl)-7-(5-methyl-1H-indazole-4-yl)-8-(2,2,2-trifluoroethoxy)-6-vinylquinazolin-2-yl)piperidin-1-yl)-3,3-dimethylbutane-2-one; ESI-MS m/z: 744.43 $[M+H]^+$. $^1H$ NMR (500 MHz, DMSO) δ 13.01 (s, 1H), 8.00 (s, 1H), 7.55-7.48 (m, 1H), 7.39 (m, 1H), 7.32 (s, 1H), 6.35 (m, 1H), 6.17-6.05 (m, 2H), 5.81-5.65 (m, 2H), 5.11 (m, 1H), 4.98 (m, 1H), 4.68 (m, 1H), 4.02 (m, 2H), 3.77 (m, 6H), 2.89 (m, 2H), 2.73 (s, 1H), 2.08-1.78 (m, 13H), 1.16-1.04 (m, 11H). |

(continued)

| Example | Chemical structure and name |
|---|---|
| 71 | 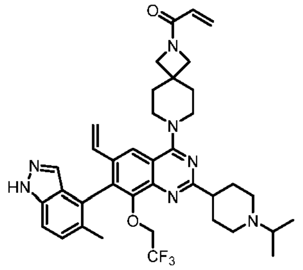<br>1-(7-(2-(1-isopropylpiperidine-4-yl)-7-(5-methyl-1H-indozol-4-yl)-8- (2,2,2-trifluoroethoxy)-6-vinyl quinoline-4-yl-2,7-diazo[3.5]nonan-2-yl) propyl-2-en-1-one; ESI-MS m/z: 688.50 $[M+H]^+$ |
| 72 | 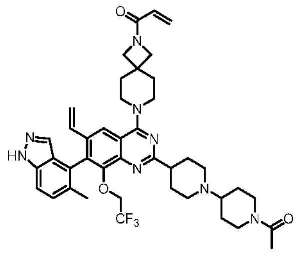<br>1-(7-(2-(1'-acetyl-[1,4'-bipiperidine]-4-yl)-7-(5-methyl-1H-indazole-4-yl)-8-(2,2,2-trifluoroethoxy)-6-vinylquinazolin-4-yl)-2,7-diazspiro[3.5] nonan-2-yl)propyl-2-en-1-one; ESI-MS m/z: 801.59 $[M+H]^+$ |
| 73 | 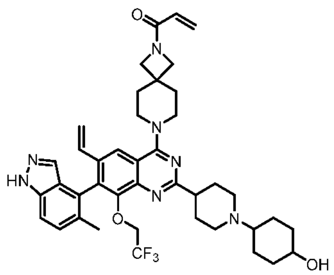<br>1-(7-(2-(1-(4-(hydroxyhexyl))piperidine-4-yl-7-(5-methyl-1H-indozol-4-yl) -8-(2,2,2-trifluoroethoxy)-6-vinyl quinoline-4-yl-2,7-diazo[3.5]nonan-2-yl) propyl-2-en-1-one; ESI-MS m/z: 744.54 $[M+H]^+$ |
| 74 | 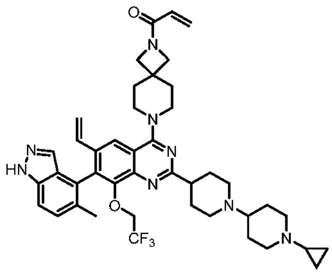<br>1-(7-(2-(1'-cyclopropyl-[1,4'-bipiperidine]-4-yl)-7-(5-methyl-1H-indazole-4-yl)-8-(2,2,2-trifluoroethoxy)-6-vinylquinazolin-4-yl)-2,7-diazspiro[3.5]nonan-2-yl)propyl-2-en-1-one; ESI-MS m/z: 769.62 $[M+H]^+$ |

(continued)

| Example | Chemical structure and name |
|---|---|
| 75 | 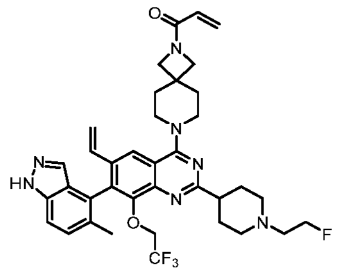<br>1-(7-(2-(1-(2-(fluoroethyl))piperidin-4-yl)-7-(5-methyl-1H-indazole-4-yl)-8-(2,2,2-trifluoroethoxy)-6-vinylquinazolin-4-yl)-2,7-diazarospiro[3.5] nonan-2-yl)propyl-2-en-1-one; ESI-MS m/z: 692.48 $[M+H]^+$ |
| 76 | 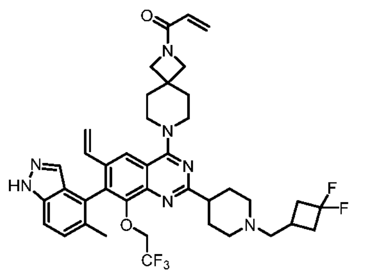<br>1-(7-(2-(1-((3,3-difluorocyclobutyl)methyl)piperidin-4-yl)-7-(5-methyl-1H-indazole-4-yl)-8-(2,2,2-trifluoroethoxy)-6-vinylquinazolin-4-yl)-2,7-diazspiro[3.5]nonan-2-yl)propyl-2-en-1-one; ESI-MS m/z: 750.77$[M+H]^+$ |
| 77 | 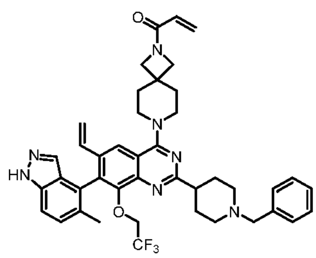<br>1-(7-(2-(1-benzylpiperidin-4-yl)-7-(5-methyl-1H-indazol-4-yl)-8-(2,2,2-trifluoroethoxy)-6-vinylquinazolin-4-yl)-2,7-diazarospiro[3.5]nonan-2-yl) propyl-2-en-1-one; ESI-MS m/z: 736.48 $[M+H]^+$ |
| 78 | 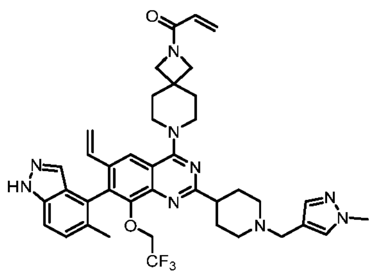<br>1-(7-(7-(5-methyl-1H-indazol-4-yl)-2-(1-((1-methyl-1H-pyrazol-4-yl) methyl)piperidin-4-yl]-8-(2,2,2-trifluoroethoxy)-6-vinylquinazolin-4-yl)- 2,7-diazarospiro[3.5]nonan-2-yl)propyl-2-en-1-one; ESI-MS m/z: 740.40 $[M+H]^+$ |

(continued)

| Example | Chemical structure and name |
|---|---|
| 79 | 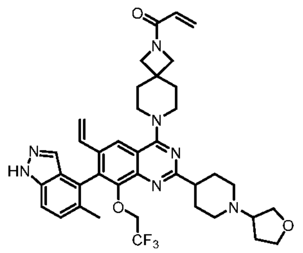 1-(7-(7-(5-methyl-1H-indazol-4-yl)-2-(1-(tetrahydrofuran-3-yl)piperidin-4-yl)-8-(2,2,2-trifluoroethoxy)-6-vinylquinazolin-4-yl)-2,7-diazspiro[3.5] nonan-2-yl)propyl-2-en-1-one; ESI-MS m/z: 715.41 $[M+H]^+$. $^1H$ NMR (500 MHz, DMSO) δ 13.01 (s, 1H), 8.00 (s, 1H), 7.49 (m, 1H), 7.49 (m, 1H), 7.38 (m, 1H), 7.32 (m, 1H), 6.35 (m, 1H), 6.19-6.02 (m, 2H), 5.87-5.61 (m, 2H), 5.11 (m, 1H), 4.96 (m, 1H), 4.74 -4.53 (m, 1H), 4.05 (s, 2H), 3.85-3.73 (m, 8H), 3.65 (m, 1H), 3.15-2.70 (m, 4H) 2.20 -1.72 (m, 16H). |
| 80 | 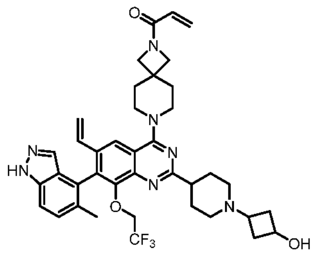 1-(7-(2-(1-(3-(hydroxycyclobutyl))piperidine-4-yl-7-(5-methyl-1H-indozol-4-yl)-8-(2,2,2-trifluoroethoxy)-6-vinyl quinoline-4-yl-2,7-diazo[3.5]nonan-2-yl)propyl-2-en-1-one; ESI-MS m/z: 716.77 $[M+H]^+$ |
| 81 | 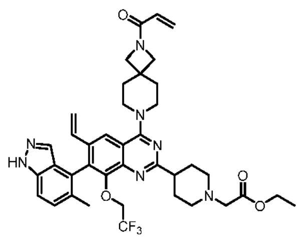 Ethyl 2-(4-(4-(2-acryloyl-2,7-diazazospiro[3.5]nonan-7-yl)-7-(5-methyl-1H-indazole-4-yl)-8-(2,2,2-trifluoroethoxy)-6-vinyl quinazolin-2-yl piperidine-1-ylacetate; ESI-MS m/z: 732.81 $[M+H]^+$. $^1H$ NMR (500 MHz, DMSO) δ 13.03 (s, 1H), 8.00 (s, 1H), 7.49 (m, 1H), 7.40 (m, 1H), 7.32 (m, 1H), 6.35 (m, 1H), 6.19-6.05 (m, 2H), 5.82-5.64 (m, 2H), 5.11 (m, 1H), 4.98 (m, 1H), 4.69 (m, 1H), 4.19-3.99 (m, 4H), 3.74 (m, 6H), 3.34 (s, 2H), 2.94 (m 2H), 2.71 (m, 1H), 2.32 (m, 2H), 2.08-1.75 (m, 11H), 1.21 (m, 3H). |
| 82 | 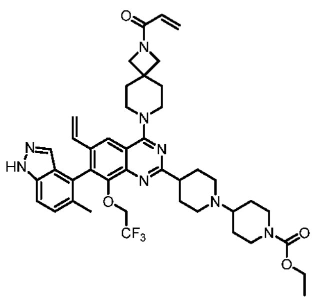 Ethyl 4-(4-(2-acryloyl-2,7-diazspiro[3.5]nonan-7-yl)-7-(5-methyl-1H-indazole-4-yl)-8-(2,2,2-trifluoroethoxy)-6-vinylquinazolin-2-yl)-[1,4'-bipiperidine]-1'-carboxylate; ESI-MS m/z: 801.59 $[M+H]^+$ |

(continued)

| Example | Chemical structure and name |
|---|---|
| 83 | 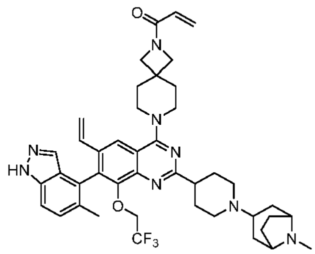 1-(7-(7-(5-methyl-1H-indazole-4-yl)-2-(1-(8-methyl-8-azabicyclo[3.2.1] cindan-3-yl]piperidin-4-yl)-8-(2,2,2-trifluoroethoxy)-6-vinylquinazolin-4-yl) -2,7-diazspiro[3.5]nonan-2-yl)propyl-2-en-1-one; ESI-MS m/z: 769.41 [M+H]$^+$ |

**Example 84: (2-Fluoro-1-(7-(7-(5-methyl-1H-indazol-4-yl)-2-(1-methylpiperidin-4-yl)-8-(2,2,2-trifluoroethoxy)-6-vinylquinazolin-4-yl)-2,7-diazaspiro [3.5] nonan-2-yl)propan-2-en-1-one)**

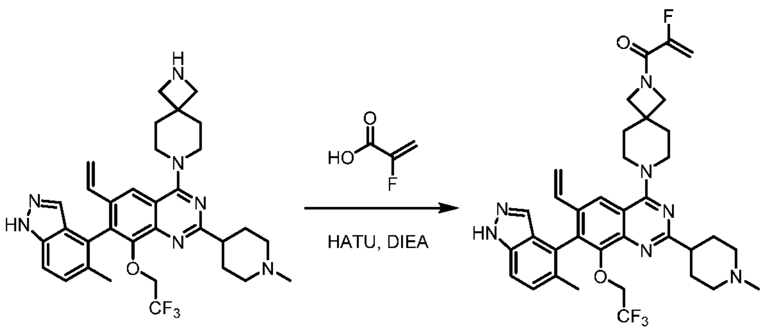


2-8 84

Compound 2-8 (120 mg, 0.20 mmol) was dissolved in DMF (5 mL), 2-fluoroacrylic acid (54 mg, 0.60 mmol), HATU (226 mmol, 0.60 mmol) and DIEA (77 mg, 0.60 mmol) were added and stirred for 2 hours. After the reaction was completed, the mixture was diluted with ethyl acetate, washed with saturated brine for three times, dried and concentrated to obtain a crude product. The preparation plate was prepared (DCM/MeOH=10/1) to obtain the target product (3 mg).

**Example 87: ((E)-1-(7-(6-chloro-7-(5-methyl-1H-indazol-4-yl)-2-(1-methylpiperidine-4-yl)-8-(2,2,2-trifluoroethoxy)quinazolin-4-yl)-2,7-diazaspiro[3.5]nonan-2-yl)-4-(dimethylamino) but-2-en-1-one)**

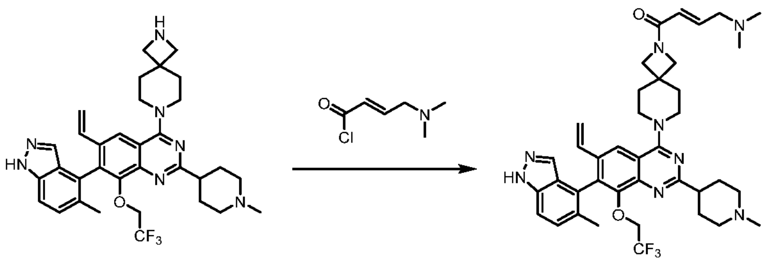

32-6 87

Under the protection of nitrogen, in an ice water bath, a freshly prepared THF solution of trans-4-dimethylamino crotonyl chloride hydrochloride (12 mg, 83 mmol) was added to 32-6 (100 mg, 163 μmol, crude) of THF (2 mL) and

saturated $Na_2CO_3$ (1 mL) solution, adding it to complete the reaction. The reaction mixture was poured into water, extracted with ethyl acetate, the organic layer was washed with brine, dried over $Na_2SO_4$ and concentrated in vacuo, and concentrated to obtain a crude product. The crude product was prepared by a plate preparation (DCM/MeOH=10:1)(6 mg, 5% yield).

The synthesis of Example 85 refers to the synthesis procedure of reference 62, excepting that difluoroethanol is used instead of trifluoroethanol.

Synthesis of Example 86 reference 84 synthesis procedure.

| Example | Chemical structure and name |
|---|---|
| 84 | 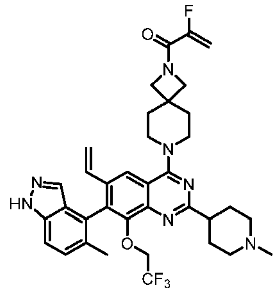<br>2-Fluoro-1-(7-(7-(5-methyl-1H-indazole-4-yl)-2-(1-methylpiperidin-4-yl)- 8-(2,2,2-trifluoroethoxy)-6-vinylquinazolin-4-yl)-2,7-diazarospiro[3.5] nonan-2-yl)propyl-2-en-1-one; ESI-MS m/z: 678.43 $[M+H]^+$ |
| 85 | 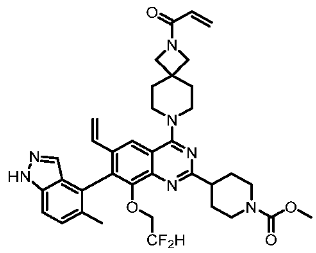<br>Methyl 4 -(4-(2-acryloyl-2,7-diazazospiro[3.5]nonan-7-yl)-8-(2,2-difluoroethoxy)-7-(5-methyl-1H-indazole-4-yl)-6-vinylquinazolin-2-yl) piperidine-1-carboxylic acid; ESI-MS m/z: 686.50 $[M+H]^+$ |
| 86 | 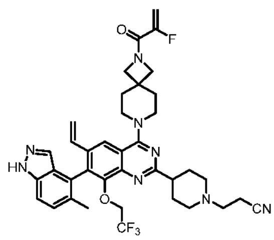<br>3-(4-(4-(2-(2-Fluoroacryloyl)-2,7-diazspiro[3.5]nonan-7-yl)-7-(5-methyl-1H-indazole-4-yl)-8-(2,2,2-trifluoroethoxy)-6-vinylquinazolin-2-yl) piperidin-1-yl)propionitrile; ESI-MS m/z: 717.75 $[M+H]^+$ |
| 87 | 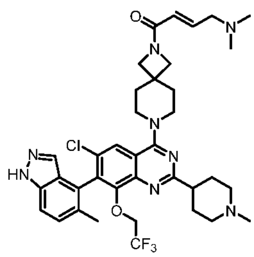<br>(E)-1-(7-(6-chloro-7-(5-methyl-1H-indazol-4-yl)-2-(1-methylpiperidin-4-yl)-8-(2,2,2-trifluoroethoxy) quinazolin-4-yl)-2,7-diaza[3.5]nonan-2-yl)- 4-(dimethylamino)butan-2-en-1-one; ESI-MS m/z: 725.32 $[M+H]^+$ |

**Comparative Example: Compound D1 (1-(7-(7-(5-methyl-1H-indazol-4-yl)-2-(1-(tetrahydro-2H-pyran-4-yl) piperidin-4-yl)oxy)-8-(2,2,2-trifluoroethoxy)-6-vinylquinazolin-4-yl)-2,7-diaza spiro[3.5 ]nonan-2-yl)prop-2-en-1-one)**

The following comparative compound D1 was prepared as described in Example 39 in WO2018143315A1

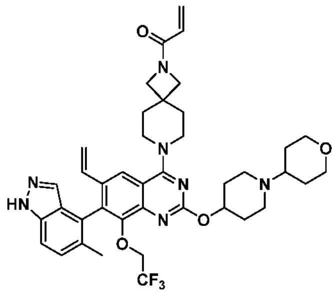

D1

**Biological Assay**

**Example A: Cell Proliferation Assay**

The CALU-1 cells were spread on a 96-well ultra-low adsorption plate with 1000 cells and 190 μL/well. After incubating overnight, compound solutions of gradient concentration was prepared, and added 10 μL of DMSO solution of the test compound of each concentration to the cells of each well. The final concentration of the compound is 30000, 10000, 3333.3, 1111.1, 370.4, 123.5, 41.2, 13.7, 4.6, 0 nM (the final concentration of DMSO is 0.25%). Incubated for 120 h at 37°C and 5% $CO_2$. 60 μL of Cell-titer Glo working solution was added to each well, shaked and mixed, and incubated at room temperature for 10 minutes. The multi-functional microplate reader read the luminescence value, and converted the luminescence value reading into inhibition percentage.

$$\text{Inhibition percentage} = (\text{maximum value-reading})/(\text{maximum value-minimum value})*100.$$

The "maximum value" was the DMSO control, the "minimum value" indicates the cell-free control group.

Graphpad Prism software was used to perform curve fitting and obtain $IC_{50}$ value.

The compounds of the examples have good inhibition on CALU-1 cells, and their activity is better than that of ARS-1620. Refer to Table 2 for the $IC_{50}$ data of some compounds in the examples inhibiting CALU-1 cells.

Table 2

| Example | $IC_{50}$ ( nM ) |
|---|---|
| ARS-1620 | 245 |
| Compound 1 | 2.7 |
| Compound 4 | 2.9 |
| Compound 5 | 102.9 |
| Compound 6 | 45.7 |
| Compound 8 | 4.6 |

**Example B: Cell Proliferation Assay**

Pave the MIA-PACA-2 cells into a 96-well ultra-low adsorption plate at 600 cells, 160 μL/well. After incubating overnight, compound solutions of gradient concentration was prepared, and added 40 μL of DMSO solution of the test compound of each concentration to the cells of each well. The final concentration of the compound is 10000, 2000, 400, 80, 16, 3.2, 0.64, 0.12, 0.025, 0 nM (the final concentration of DMSO is 0.25%). Incubated for 96 h at 37°C and 5% $CO_2$. 50 μL of Cell-titer Glo working solution was added to each well, shaked and mixed, and incubated at room temperature for 10 minutes. The multi-functional microplate reader read the luminescence value, and converted the luminescence

value reading into inhibition percentage.

Inhibition percentage = (maximum value-reading)/(maximum value-minimum value)* 100.

The "maximum value" was the DMSO control, the "minimum value" indicates the cell-free control group.

Graphpad Prism software was used to perform curve fitting and obtain $IC_{50}$ value.

Refer to Table 3 for the $IC_{50}$ data of some compounds in the examples inhibiting MIA-PACA-2 cells.

**Example C: Cell Proliferation Assay**

Pave the H358 cells on a 96-well ultra-low adsorption plate with 2000 cells and 190 μL/well. After incubating overnight, compound solutions of gradient concentration was prepared, and added 10 μL of DMSO solution of the test compound of each concentration to the cells of each well. The final concentration of the compound is 10000, 3333.3, 1111.1, 370.4, 123.5, 41.2, 13.7, 4.6, 1.5, 0 nM (the final concentration of DMSO is 0.25%). Incubated for 96 h at 37°C and 5% $CO_2$. 50 μL of Cell-titer Glo working solution was added to each well, shaked and mixed, and incubated at room temperature for 10 minutes. The multi-functional microplate reader read the luminescence value, and converted the luminescence value reading into inhibition percentage.

Inhibition percentage = (maximum value-reading)/(maximum value-minimum value)* 100.

The "maximum value" was the DMSO control, the "minimum value" indicates the cell-free control group.

Graphpad Prism software was used to perform curve fitting and obtain $IC_{50}$ value.

Refer to table 3 for the $IC_{50}$ data of some compounds in the Examples for inhibiting H358 cells.

Table 3

| Example | $IC_{50}$ ( nM ) | |
|---|---|---|
| | MIA-PACA-2 | H358 |
| ARS-1620 | 284.5 | / |
| 9 | 2.1 | 3.7 |
| 10 | 25 | / |
| 11 | 4.8 | 4.7 |
| 12 | 107.3 | 76.2 |
| 13 | 22 | 26.3 |
| 14 | 40.6 | 6.4 |
| 15 | 50.5 | / |
| 16 | 4.8 | 3.2 |
| 17 | 1543 | 8439 |
| 18 | 177 | 715 |
| 19 | 2288 | >10uM |
| 20 | 1320 | 4201 |
| 21 | >2uM | 2717 |
| 22 | 21.0 | 49.6 |
| 23 | 647 | 1705 |
| 24 | 14.5 | 17.7 |
| 25 | 32.6 | 13.0 |
| 26 | 68.2 | 24.9 |
| 27 | 388 | 392 |
| 28 | 61.4 | 44.7 |
| 29 | 455 | 1083 |
| 30 | 170.9 | 60.5 |

(continued)

| Example | $IC_{50}$ ( nM ) | |
|---|---|---|
| | MIA-PACA-2 | H358 |
| 31 | 91.9 | 20.4 |
| 32 | 16.7 | 17.9 |
| 33 | 42.9 | 69.7 |
| 34 | 36.9 | 39 |
| 35 | 10.9 | 9.6 |
| 36 | 10.1 | 10.6 |
| 37 | 23.4 | 18.4 |
| 38 | 74.2 | 59.3 |
| 39 | 78.0 | 97.8 |
| 40 | 47.7 | 39.0 |
| 41 | 988 | 881 |
| 42 | 29.6 | 19.6 |
| 43 | 378 | 315 |
| 44 | 169 | 155 |
| 45 | 778 | 466 |
| 46 | 34 | 37 |
| 47 | 47.7 | 39.0 |
| 48 | 9.8 | 11.5 |
| 49 | 9.8 | 11.5 |
| 50 | 278 | 243 |
| 51 | 188 | 462 |
| 52 | 114 | 104 |
| 53 | 105 | 163 |
| 54 | 9595 | >10uM |
| 55 | >10uM | >10uM |
| 56 | 2584 | 2518 |
| 57 | 6.8 | 7.3 |
| 58 | 2.6 | 4.4 |
| 59 | 6.9 | 6.4 |
| 60 | 4.0 | 4.1 |
| 61 | 8.0 | 4.9 |
| 62 | 43.5 | 29.3 |
| 63 | 406 | 187 |
| 64 | 57.1 | 16.6 |
| 65 | 8.9 | 4.4 |
| 66 | 8.5 | 11.5 |
| 67 | 6.9 | 4.6 |

(continued)

| Example | $IC_{50}$ ( nM ) | |
|---|---|---|
| | MIA-PACA-2 | H358 |
| 68 | 2.4 | 1.6 |
| 69 | 3.7 | 3.4 |
| 70 | 13.7 | 15.5 |
| 71 | 8.3 | 7.4 |
| 72 | 5.9 | 5.2 |
| 73 | 24.1 | 24.3 |
| 74 | 10.3 | 6.7 |
| 75 | 3.6 | 2.6 |
| 76 | 21.4 | 19.3 |
| 77 | 33.7 | 37.6 |
| 78 | 62.8 | 27.2 |
| 79 | 3.5 | 2.7 |
| 80 | 46.1 | 14.9 |
| 81 | 88.0 | 89.7 |
| 82 | 12.5 | 7.6 |
| 83 | 136 | 159 |
| 84 | 60.3 | 16.5 |
| 85 | 91.7 | 124 |
| 86 | 208 | 85.8 |
| 87 | 2188 | 1939 |

**Example D: Permeability Assay**

The Caco-2 monolayer cell model was used to determine the bidirectional permeability and efflux rate of the example compounds and the comparative compounds. In the experiment, 50 μL of $6.86\times10^5$ cells/mL Caco-2 cells (passage 45) were seeded into 96-well cell culture plates, and cultured continuously for 14-18 days for transport experiments. The corresponding compound was administered bidirectionally with or without verapamil (P glycoprotein (P-gp) inhibitor), at a concentration of 5 μM. At 37°C, after incubating with shaking at 150 rpm for 120 minutes, the top and basal samples were collected, and the content of each compound in the samples was determined by liquid chromatography tandem mass spectrometry (LC/MS/MS).

Refer to the Table 4 for permeability data of some compounds in the examples. Compared with the comparative compound D1, the permeability of the example compound was significantly enhanced.

Table 4

| Example | Verapamil (μM) | Papp(A-B) ($10^6$, cm/s) | Papp(B-A) ($10^6$, cm/s) | RE (Efflux Ratio) |
|---|---|---|---|---|
| Compound 1 | 0 | 0.51 | 7.18 | 14.19 |
| Compound 1 | 100 | 2.07 | 4.41 | 2.13 |
| Compound 4 | 0 | 0.21 | 9.64 | 46.46 |
| Compound 4 | 100 | 1.62 | 4.47 | 2.76 |
| Compound 14 | 0 | 1.33 | 5.82 | 4.36 |
| Compound 14 | 100 | 1.72 | 2.5 | 1.45 |

(continued)

| Example | Verapamil ($\mu$M) | Papp(A-B) ($10^6$, cm/s) | Papp(B-A) ($10^6$, cm/s) | RE (Efflux Ratio) |
|---|---|---|---|---|
| Compound 58 | 0 | 0.34 | 10.26 | 30.58 |
| Compound 58 | 100 | 3.40 | 5.29 | 1.56 |
| Compound D1 | 0 | 0.06 | 6.75 | 112.67 |
| Compound D1 | 100 | 1.27 | 2.36 | 1.85 |

## Claims

**1.** A compound of the Formula (I) or a tautomer, pharmaceutically-acceptable salt, solvate, chelate or non-covalent complex thereof,

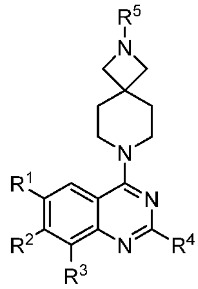

(I)

wherein,

$R^1$ is selected from the group consisting of $C_{1-6}$ alkyl, substituted $C_{1-6}$ alkyl, halogen, $C_{2-6}$ alkenyl, substituted $C_{2-6}$ alkenyl, $C_{3-6}$ cycloalkyl and substituted $C_{3-6}$ cycloalkyl
$R^2$ is 6-10 membered heteroaryl or substituted 6-10 membered heteroaryl, wherein the 6-10 membered heteroaryl independently contains 1, 2 or 3 heteroatom selected from the group consisting of N, O and S;
$R^3$ is selected from the group consisting of H, amino, cyano, halogen, hydroxy, $C_{1-6}$ alkyl, substituted $C_{1-6}$ alkyl, $C_{3-8}$ cycloalkyl, substituted $C_{3-8}$ cycloalkyl, $C_{1-6}$ alkoxy, substituted $C_{1-6}$ alkoxy, $C_{3-8}$ cycloalkyloxy, substituted $C_{3-8}$ cycloalkyloxy and cyano substituted cyclopropyl $C_{1-6}$ alkyleneoxy;
$R^4$ is

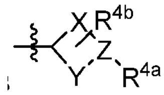
;

wherein X and Y are independently $C_{1-2}$ alkylene;
Z is selected from the group consisting of CH, N and O;
$R^{4a}$ and $R^{4b}$ are independently absent or are selected from the group consisting of H, $C_{1-6}$ alkyl, substituted $C_{1-6}$ alkyl, acryloyl, $C_{3-6}$ cycloalkyl, substituted $C_{3-6}$ cycloalkyl, 3-8 membered heterocyclyl, substituted 3-8 membered heterocyclyl and -(C=O) $C_{1-6}$ alkyl, wherein the 3-8 membered heterocyclyl independently contains 1, 2 and 3 heteroatoms independently selected from the group consisting of N, O and S;
wherein the substituted $C_{1-6}$ alkyl, the substituted $C_{2-6}$ alkenyl, the substituted $C_{3-6}$ cycloalkyl, the substituted 6-10 membered heteroaryl, the substituted $C_{1-6}$ alkoxy, the substituted $C_{3-8}$ cycloalkyloxy and the substituted 3-8 membered heterocyclyl are substituted by halogen, $C_{1-8}$ alkyl, $C_{3-12}$ cycloalkyl, -ORi', $-SR_1'$, =O, =S, $-C(O)R_1'$, $-C(S)R_1'$, $=NR_1'$, $-C(O)OR_1'$, $-C(S)OR_1'$, $-NR_1'R_2'$, $-C(O)NR_1'R_2'$, cyano, nitro, $-S(O)_2R_1'$, $-OS(O_2)OR_1'$, $-OS(O)_2R_1'$ or $-OP(O)(OR_1')(OR_2')$, wherein $R_1'$ and $R_2'$ are independently selected from -H, $C_{1-6}$ alkyl, and $C_{1-6}$ haloalkyl;
$R^5$ is unsubstituted or halo substituted acryloyl;
or a compound selected from the group consisting of:

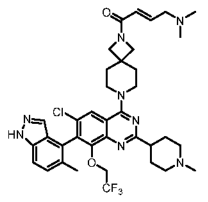, 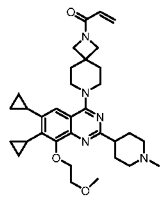, 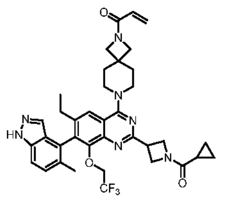, 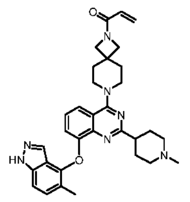, 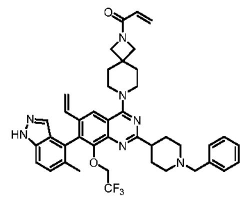

and

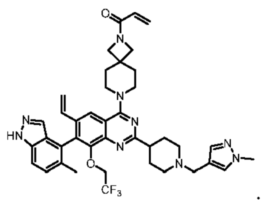.

2. The compound as claimed in claim 1, wherein $R^1$ is selected from the group consisting of $C_{1-3}$ alkyl, $C_{3-6}$ cycloalkyl, halogen and $C_{2-4}$ alkenyl; preferably, $R^1$ is selected from the group consisting of ethyl, cyclopropyl, Cl and vinyl.

3. The compound as claimed in any of claims 1 or 2, wherein $R^2$ is indazolyl or $C_{1-3}$ alkyl substituted indazolyl; preferably, $R^2$ is methyl substituted indazolyl; more preferably, $R^2$ is

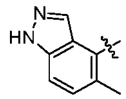.

4. The compound as claimed in any of claims 1 to 3, wherein $R^3$ is selected from the group consisting of H, $C_{1-3}$ alkyl, halogen, cyano substituted cyclopropyl $C_{1-3}$ alkyleneoxy and $-OR^{3a}$, wherein $R3^a$ is $C_{1-6}$ alkyl or $C_{3-8}$ cycloalkyl, $R^{3a}$ is independently unsubstituted or further substituted with one or more substituents selected from the group consisting of hydroxyl, halogen, $C_{1-3}$ alkyl, $C_{1-3}$ alkoxy and cyclopropyl; preferably, $R^3$ is selected from the group consisting of H, F, methyl,

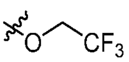, 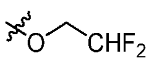, 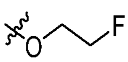, 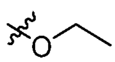, 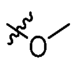, 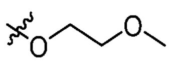, 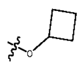, 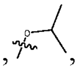, 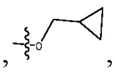, 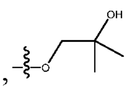

and

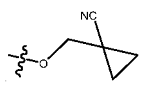;

more preferably, $R^3$ is selected from the group consisting of H, F, methyl,

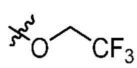,

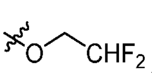, 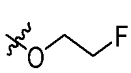, 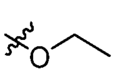, 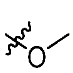, 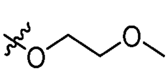, 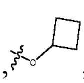, 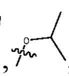, 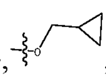, 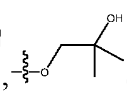 and 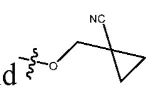.

5. The compound as claimed in any of claims 1 to 4, wherin $R^{4a}$ is absent or is selected from the group consisting of H, acryloyl, $C_{1-3}$ alkyl, $C_{4-6}$ cycloalkyl,

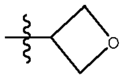, 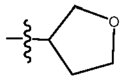, 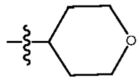,

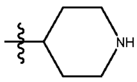 and 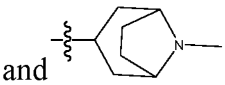

, wherein any of the $C_{1-3}$ alkyl, $C_{4-6}$ cycloalkyl ,

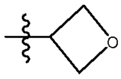, 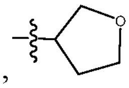,

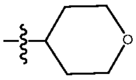, 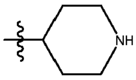 or 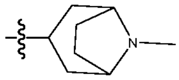

is unsubstituted or substituted with one or more substituents selected from the group consisting of halogen, hydroxyl, cyano, =O, $C_{1-3}$ alkyl, $-S(O_2)C_{1-3}$ alkyl, $C_{1-3}$ alkoxy, $C_{1-3}$ haloalkyl, cyclopropyl, cyclobutyl, halocyclopropyl, halocyclobutyl, phenyl, $C_{1-3}$ alkyl substituted pyrazolyl, $C_{1-6}$ acyl and

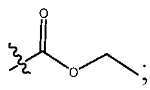;

preferably, $R^{4a}$ is independently absent or is selected from the group consisting of H, methyl, ethyl, propyl, acetyl,

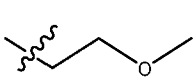, 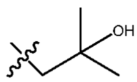, 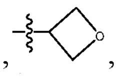,

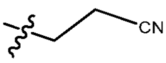, 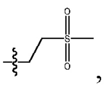, 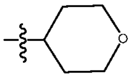, 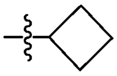, 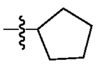, 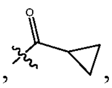, 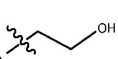, 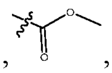, 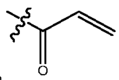, 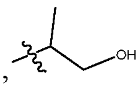,

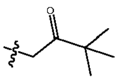, 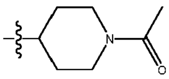, 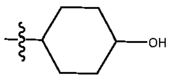, 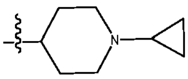, 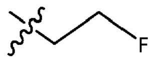, 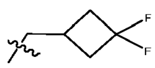, 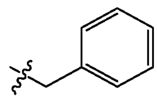,

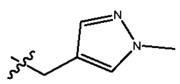, 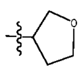, 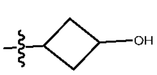, 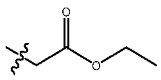, 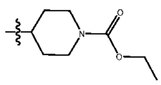, 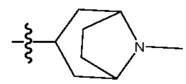, 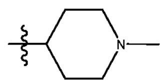

and

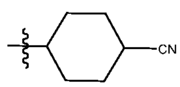.

**6.** The compound as claimed in any of claims 1 to 5, wherein $R^{4b}$ is independently absent, H or $C_{1-3}$ alkyl.

**7.** The compound as claimed in any of claims 1 to 6, wherein $R^4$ is

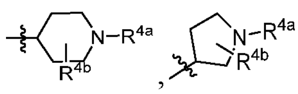,

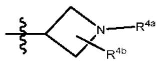, 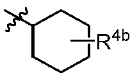, 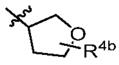 or 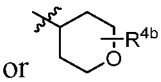;

preferably, $R^4$ is

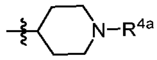, 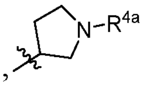, 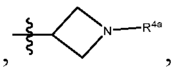,

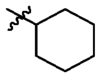, 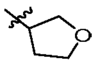 or 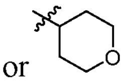.

**8.** The compound as claimed in any of claims 1 to 7, wherein $R^5$ is independently

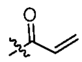

or

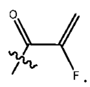.

**9.** The compound as claimed in any of claims 1 to 8, wherein the compound is selected from the group consisting of a compound of formula (IA) to formula (ID):

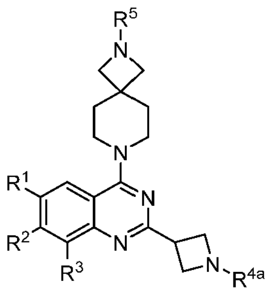

(IA)

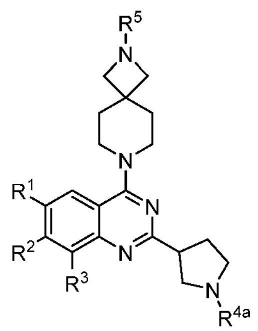

(IB)

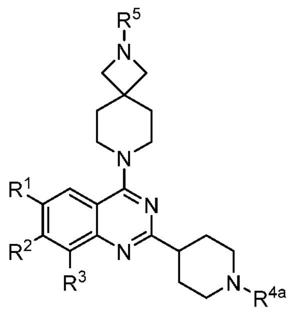

(IC)

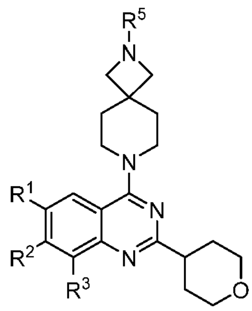

(ID)

wherein the substituents are as defined in formula (I).

**10.** The compound as claimed in claim 1, which is selected from the group consisting of:

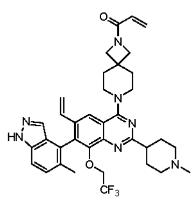

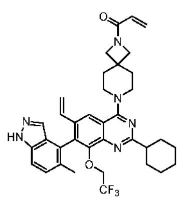

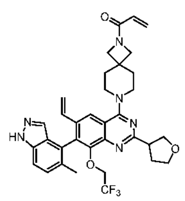

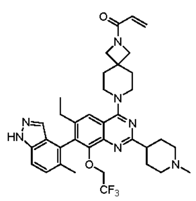

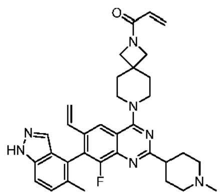
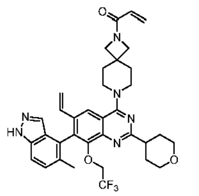
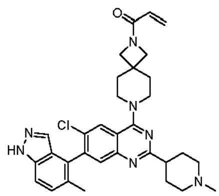
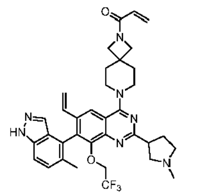

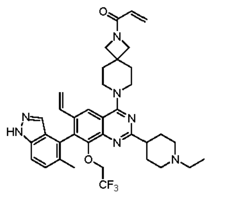
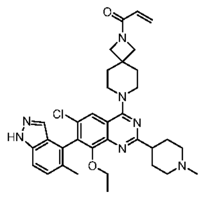
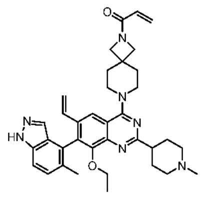
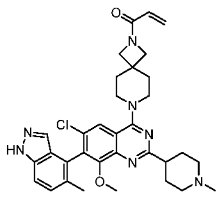

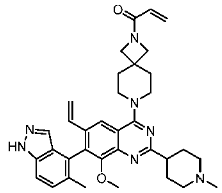
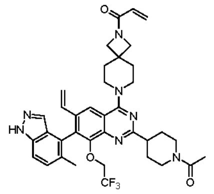
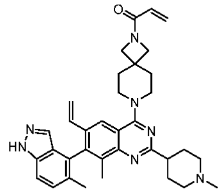
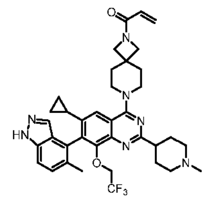

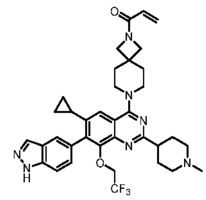
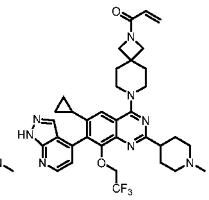
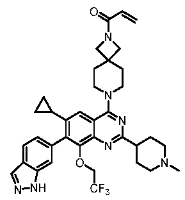
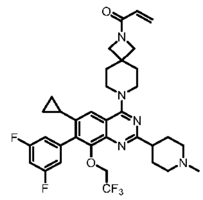

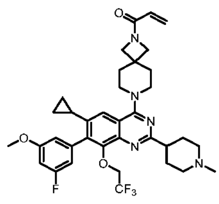
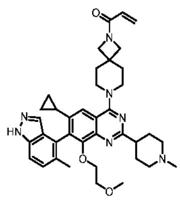
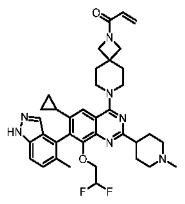

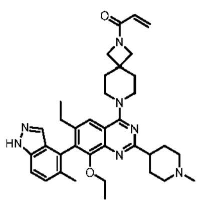
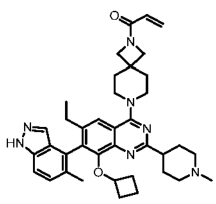
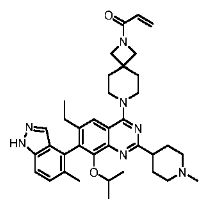
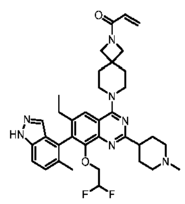

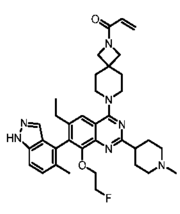
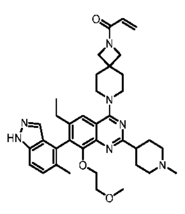
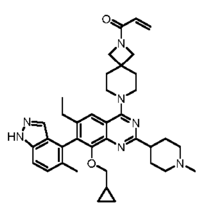
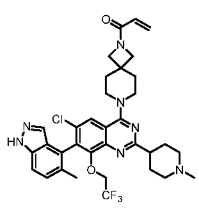

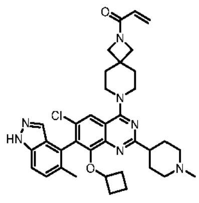 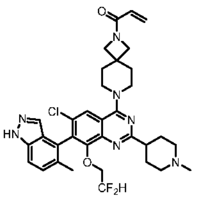 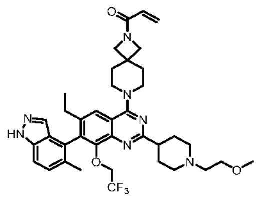 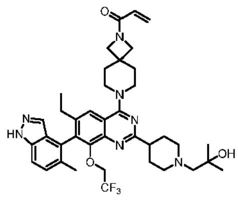

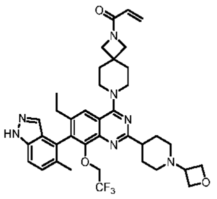 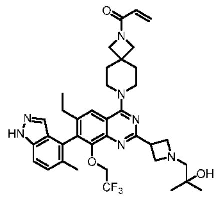 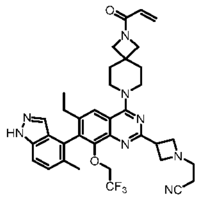 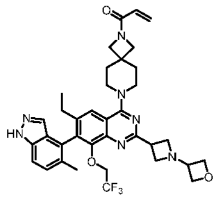

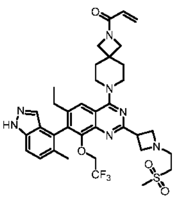 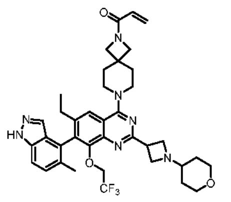 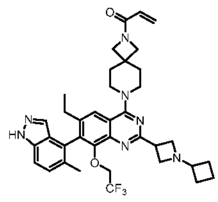 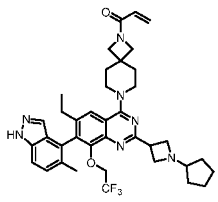

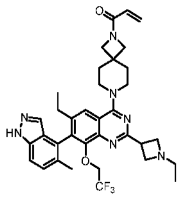 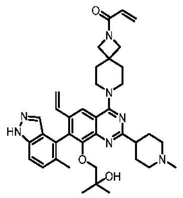 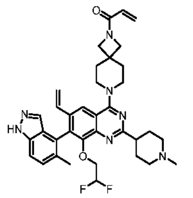

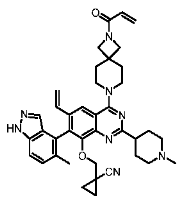 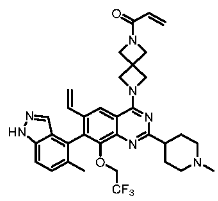 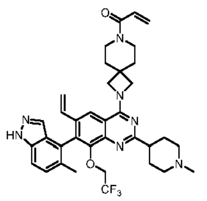 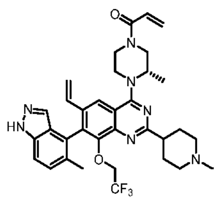

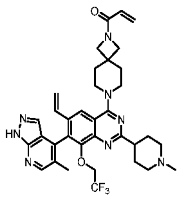 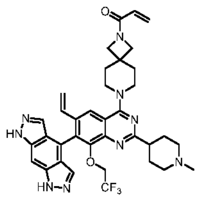 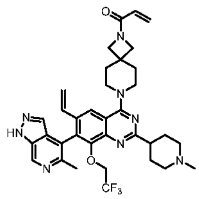

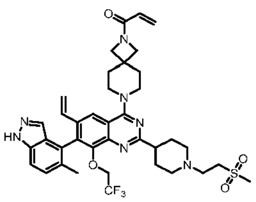 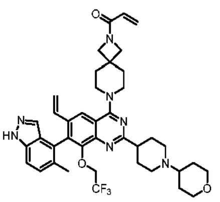 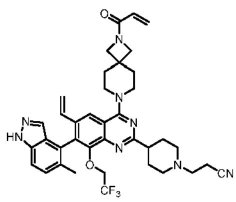 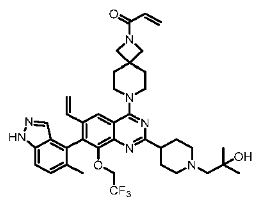

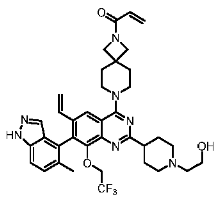
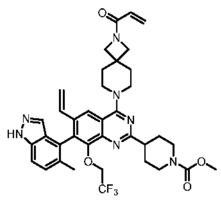
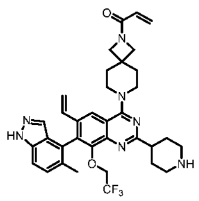
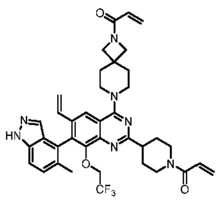

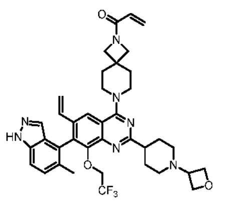
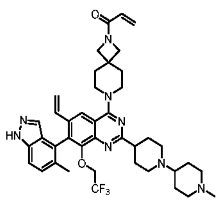
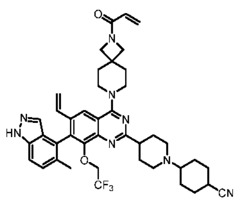
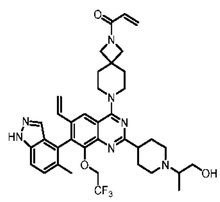

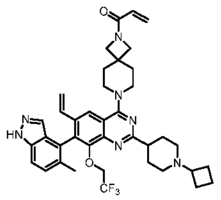
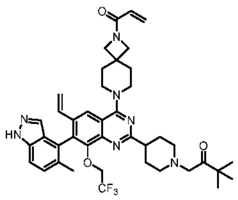
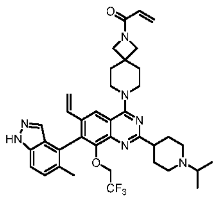
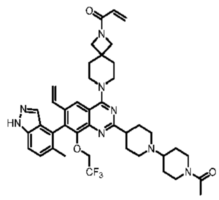

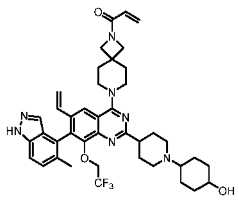
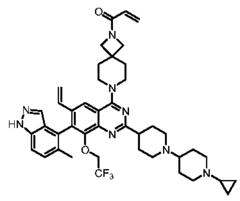
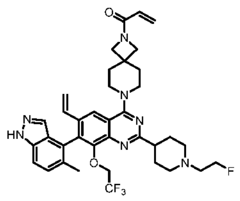
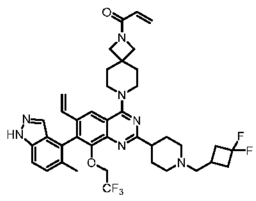

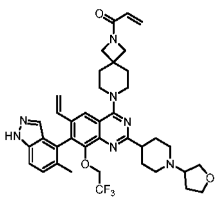
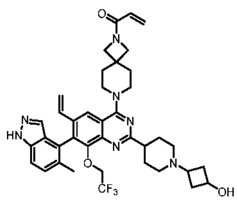

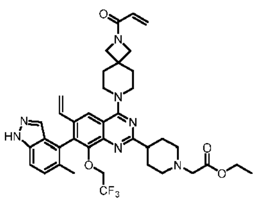
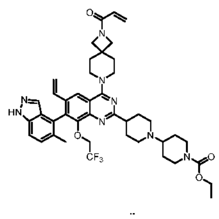
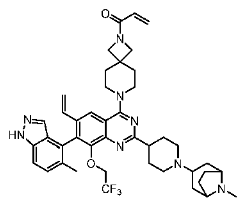
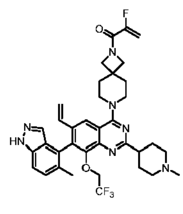

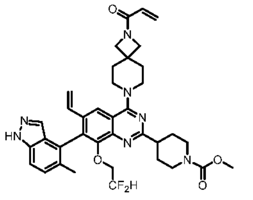 and 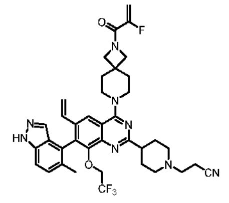 .

**11.** A pharmaceutical composition comprising a therapeutically effective amount of at least one compound of any of claims 1 to 10 and at least one pharmaceutically acceptable carrier.

**12.** The pharmaceutical composition as claimed in claim 11, wherein the weight ratio of the compound to the pharmaceutically acceptable carrier is 0.0001:1-10.

**13.** The compound as claimed in any of claims 1 to 10, or the pharmaceutical composition as claimed in claim 11 or 12 for use as a medicament.

**14.** The compound as claimed in any of claims 1 to 10, or the pharmaceutical composition as claimed in claim 11 or 12 for use in treating and/or preventing cancer.

**15.** The compound for use, or the pharmaceutical composition for use as claimed in claim 14, wherein the cancer is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophagus cancer, melanoma, colorectal cancer, hepatocellular carcinoma, head and neck cancer, hepatobiliary cell carcinoma, myelodysplastic syndrome, malignant glioma, prostate cancer, thyroid cancer, Schwann cell tumor, lung squamous cell carcinoma, lichenoid keratosis, synovial sarcoma, skin cancer, pancreatic cancer, testicular cancer and liposarcoma.

**Patentansprüche**

**1.** Verbindung der Formel (I) oder ein Tautomer, pharmazeutisch akzeptables Salz, Solvat, Chelat oder nicht-kovalenter Komplex davon,

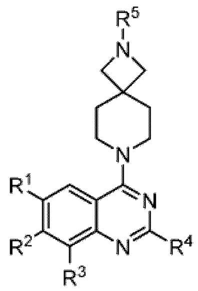

(I)

wobei

$R^1$ ausgewählt ist aus der Gruppe bestehend aus $C_{1-6}$-Alkyl, substituiertem $C_{1-6}$-Alkyl, Halogen, $C_{2-6}$-Alkenyl, substituiertem $C_{2-6}$-Alkenyl, $C_{3-6}$-Cycloalkyl und substituiertem $C_{3-6}$-Cycloalkyl,
$R^2$ ein 6-10-gliedriges Heteroaryl oder ein substituiertes 6-10-gliedriges Heteroaryl ist, wobei das 6-10-gliedrige Heteroaryl unabhängig 1, 2 oder 3 Heteroatome ausgewählt aus der Gruppe bestehend aus N, O und S, enthält;
$R^3$ ausgewählt ist aus der Gruppe bestehend aus H, Amino, Cyano, Halogen, Hydroxy, $C_{1-6}$-Alkyl, substituiertem $C_{1-6}$-Alkyl, $C_{3-8}$-Cycloalkyl, substituiertem $C_{3-8}$-Cycloalkyl, $C_{1-6}$-Alkoxy, substituiertem $C_{1-6}$-Alkoxy, $C_{3-8}$-Cycloalkyloxy, substituiertem $C_{3-8}$-Cycloalkyloxy und cyanosubstituiertem Cyclopropyl-$C_{1-6}$-Alkylenoxy;
$R^4$

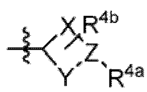

ist wobei X und Y unabhängig $C_{1-2}$-Alkylen sind;
Z ausgewählt ist aus der Gruppe bestehend aus CH, N und O;
$R^{4a}$ und $R^{4b}$ unabhängig abwesend sind oder ausgewählt sind aus der Gruppe bestehend aus H, $C_{1-6}$-Alkyl, substituiertem $C_{1-6}$-Alkyl, Acryloyl, $C_{3-6}$-Cycloalkyl, substituiertem $C_{3-6}$-Cycloalkyl, 3-8-gliedrigem Heterocyclyl, substituiertem 3-8-gliedrigem Heterocyclyl und -(C=O)-$C_{1-6}$-Alkyl, wobei das 3-8-gliedrige Heterocyclyl unabhängig 1, 2 und 3 Heteroatome enthält, die unabhängig ausgewählt sind aus der Gruppe bestehend aus N, O und S;
wobei das substituierte $C_{1-6}$-Alkyl, das substituierte $C_{2-6}$-Alkenyl, das substituierte $C_{3-6}$-Cycloalkyl, das substi-

tuierte 6-10-gliedrige Heteroaryl, das substituierte $C_{1-6}$-Alkoxy, das substituierte $C_{3-8}$-Cycloalkyloxy und das substituierte 3-8-gliedrige Heterocyclyl durch Halogen $C_{1-8}$-Alkyl, $C_{3-12}$-Cycloalkyl, $-OR_1'$, $-SR_1'$, =O, =S, $-C(O)R_1'$, - $C(S)R_1'$, $=NR_1'$, - $C(0)OR_1'$, $-C(S)OR_1'$, $-NR_1'R_2'$, $-C(O)NR_1'R_2'$, Cyano, Nitro, $-S(O)_2R_1'$, - $OS(O_2)OR_1'$, $-OS(O)_2R_1'$ oder $-OP(O)(OR_1')(OR_2')$ substituiert sind, wobei $R_1'$ und $R_2'$ unabhängig ausgewählt sind aus -H, $C_{1-6}$-Alkyl, und $C_{1-6}$-Halogenalkyl;

$R^5$ unsubstituiertes oder halogensubstituiertes Acryloyl ist; oder eine Verbindung ausgewählt aus der Gruppe bestehend aus:

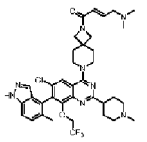, 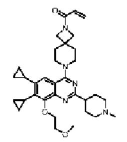, 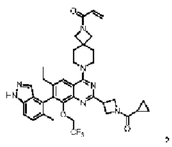, 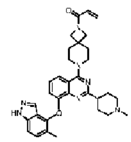, 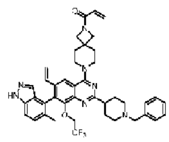

und

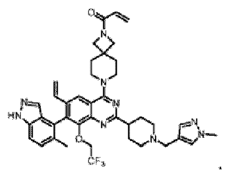.

2. Verbindung nach Anspruch 1, wobei $R^1$ ausgewählt ist aus der Gruppe bestehend aus $C_{1-3}$-Alkyl, $C_{3-6}$ -Cycloalkyl, Halogen und $C_{2-4}$-Alkenyl; vorzugsweise $R^1$ ausgewählt aus der Gruppe bestehend aus Ethyl, Cyclopropyl, Cl und Vinyl.

3. Verbindung nach einem der Ansprüche 1 oder 2, wobei $R^2$ Indazolyl oder $C_{1-3}$-alkylsubstituiertes Indazolyl ist; $R^2$ vorzugsweise methylsubstituiertes Indazolyl ist; $R^2$ noch bevorzugter

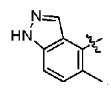

ist.

4. Verbindung nach einem der Ansprüche 1 bis 3, wobei $R^3$ ausgewählt ist aus der Gruppe bestehend aus H, $C_{1-3}$-Alkyl, Halogen, cyanosubstituiertem Cyclopropyl, $C_{1-3}$-Alkylenoxy und $-OR^{3a}$, wobei $R^{3a}$ $C_{1-6}$-Alkyl oder $C_{3-8}$-Cycloalkyl ist, $R^{3a}$ unabhängig unsubstituiert oder weiter substituiert ist mit einem oder mehreren Substituenten, ausgewählt aus der Gruppe bestehend aus Hydroxyl, Halogen, $C_{1-3}$-Alkyl, $C_{1-3}$-Alkoxy und Cyclopropyl; $R^3$vorzugsweise ausgewählt aus der Gruppe bestehend aus H, F, Methyl,

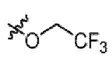, 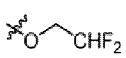, 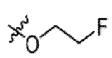, 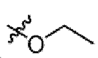, 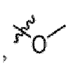, 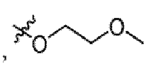, 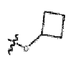, 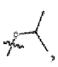, 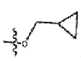, 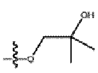

und

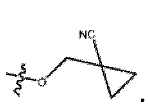;

weiter bevorzugt $R^3$ ausgewählt ist aus der Gruppe bestehend aus H, F, Methyl

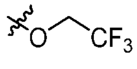,

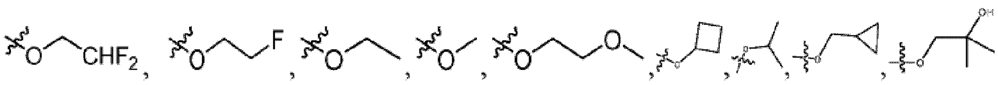, , , , , , , ,

und

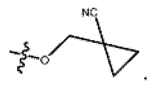.

**5.** Verbindung nach einem der Ansprüche 1 bis 4, wobei $R^{4a}$ abwesend ist oder ausgewählt ist aus der Gruppe bestehend aus H, Acryloyl, $C_{1-3}$-Alkyl, $C_{4-6}$-Cycloalkyl

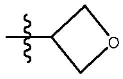, 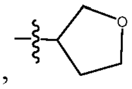, 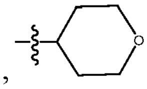, 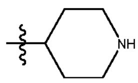

und

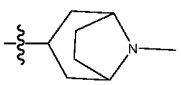

, wobei ein beliebiges von $C_{1-3}$-Alkyl, $C_{4-6}$-Cycloalkyl

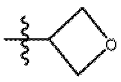, 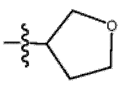, 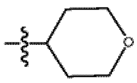, 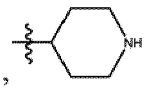 oder 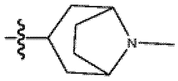

unsubstituiert oder mit einem oder mehreren Substituenten substituiert ist, ausgewählt aus der Gruppe bestehend aus Halogen, Hydroxyl, Cyano, =O, $C_{1-3}$-Alkyl, -$S(O_2)C_{1-3}$-Alkyl, $C_{1-3}$-Alkoxy, $C_{1-3}$-Halogenalkyl, Cyclopropyl, Cyclobutyl, Halocyclopropyl, Halocyclobutyl, Phenyl, $C_{1-3}$-Alkyl substituiertem Pyrazolyl, $C_{1-6}$-Acyl und

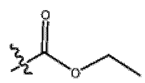;

$R^{4a}$ vorzugsweise unabhängig abwesend ist oder ausgewählt ist aus der Gruppe bestehend aus H, Methyl, Ethyl, Propyl, Acetyl,

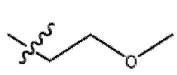, 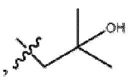, 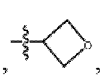,

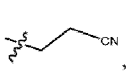, 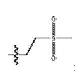, 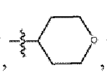, 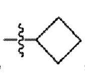, 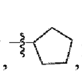, 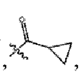, 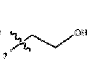, 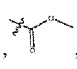, 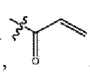, 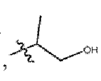,

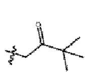, 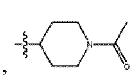, 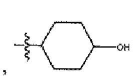, 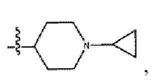, 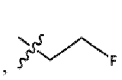, 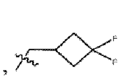, 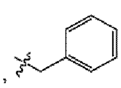,

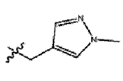, 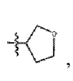, 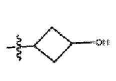, 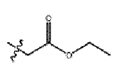, 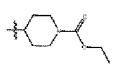, 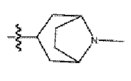, 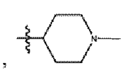

und

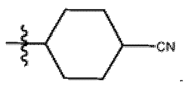

6. Verbindung nach einem der Ansprüche 1 bis 5, wobei $R^{4b}$ unabhängig abwesend ist, H oder $C_{1-3}$-Alkyl ist.

7. Verbindung nach einem der Ansprüche 1 bis 6, wobei $R^4$

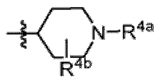, 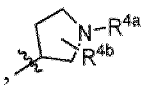, 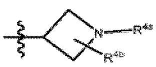, 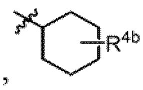, 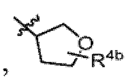

oder

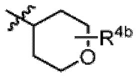

ist, $R^4$ vorzugsweise

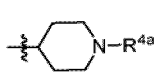, 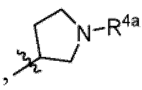, 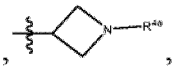,

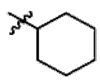, 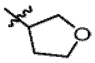 oder 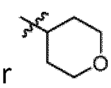

ist

8. Verbindung nach einem der Ansprüche 1 bis 7, wobei $R^5$ unabhängig

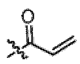 oder 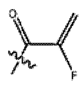

ist.

9. Verbindung nach einem der Ansprüche 1 bis 8, wobei die Verbindung ausgewählt ist aus der Gruppe bestehend aus einer Verbindung der Formel (IA) bis Formel (ID):

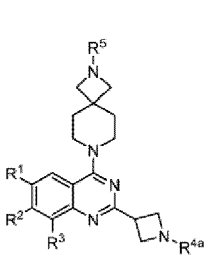
(IA)

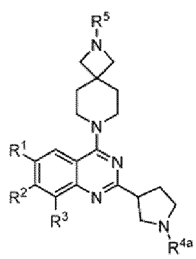
(IB)

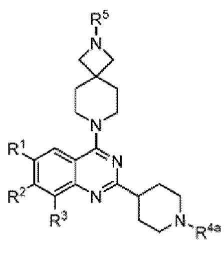
(IC)

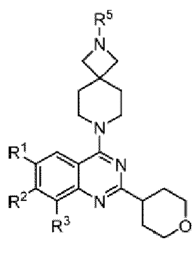
(ID)

wobei die Substituenten wie in Formel (I) definiert sind.

10. Verbindung nach Anspruch 1, welche ausgewählt ist aus der Gruppe bestehend aus:

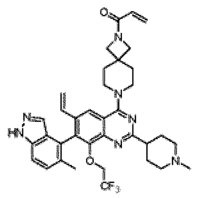
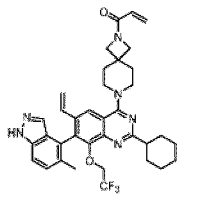
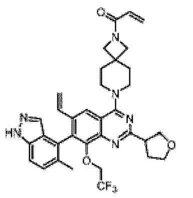
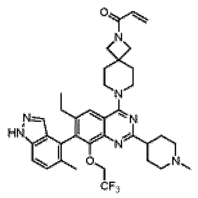

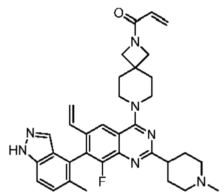
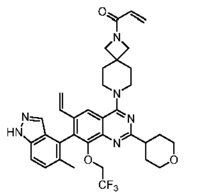
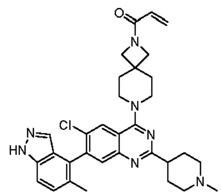
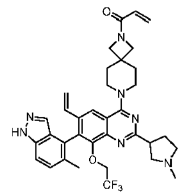

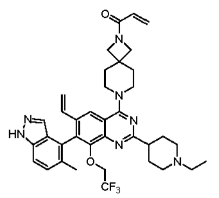
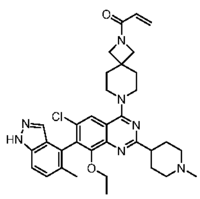
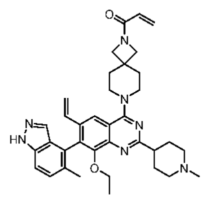
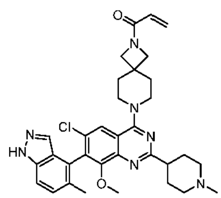

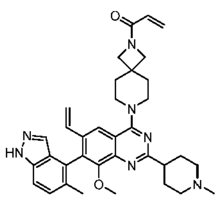
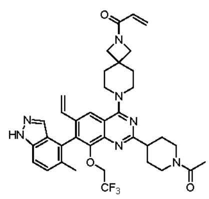
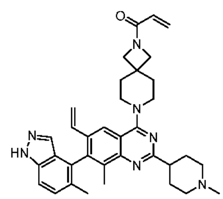
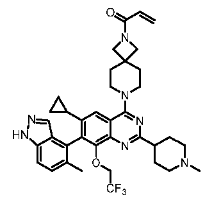

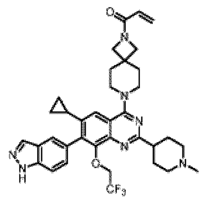
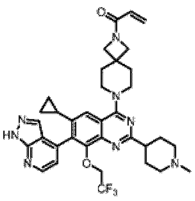
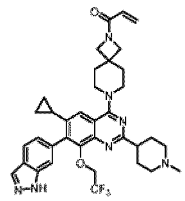
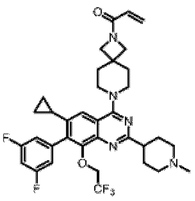

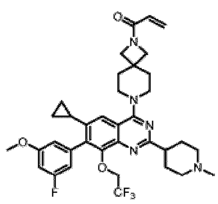
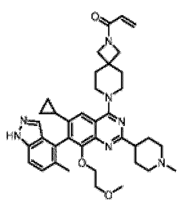
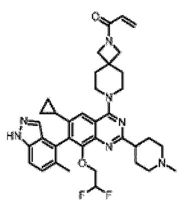

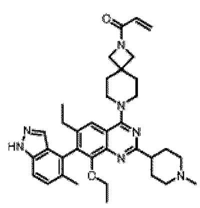
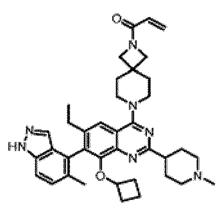
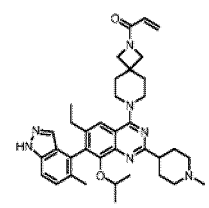
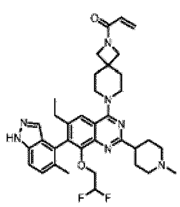

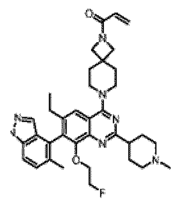
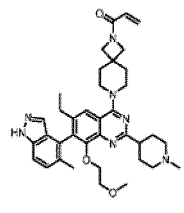
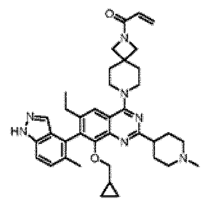
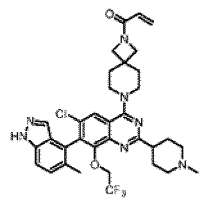

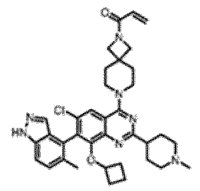
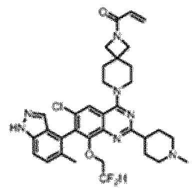
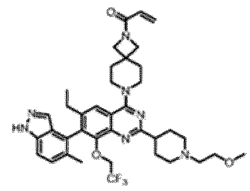
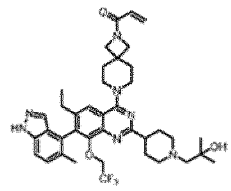

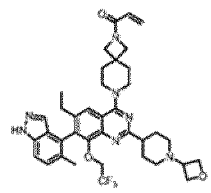
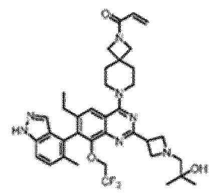
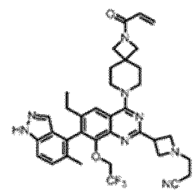
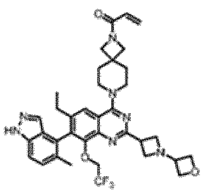

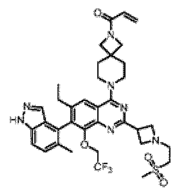
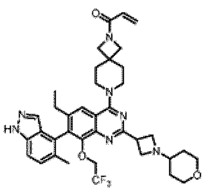
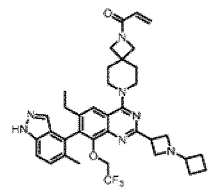
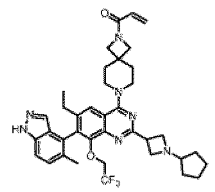

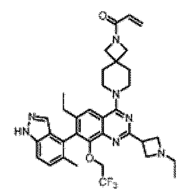
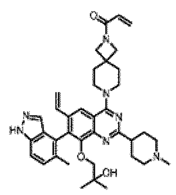
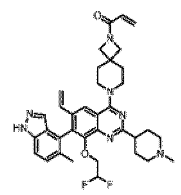

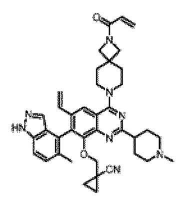
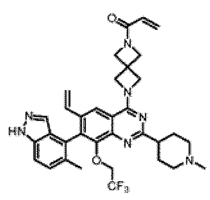
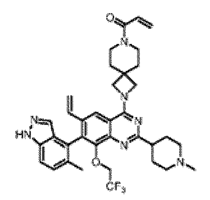
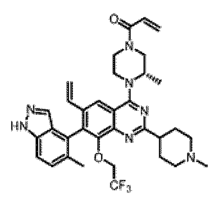

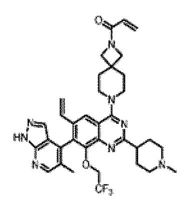
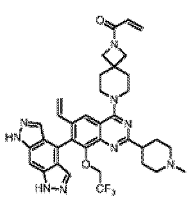
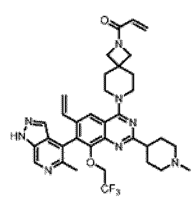

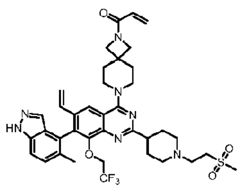 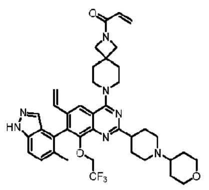 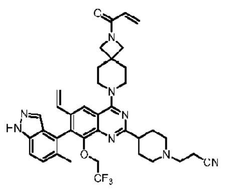 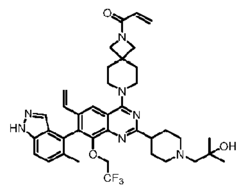

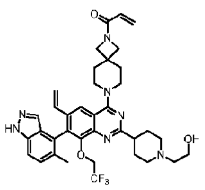 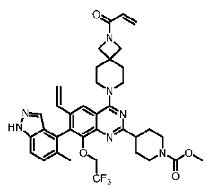 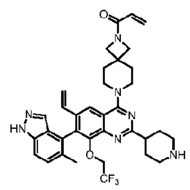 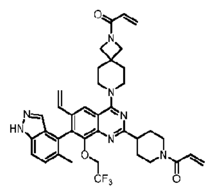

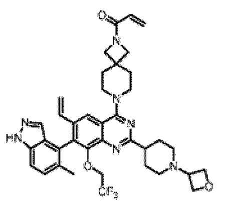 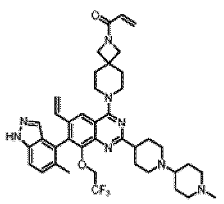 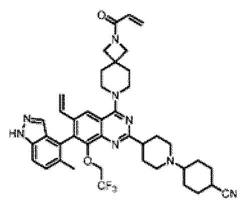 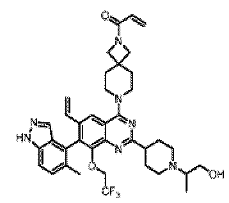

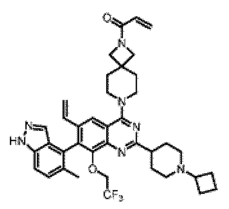 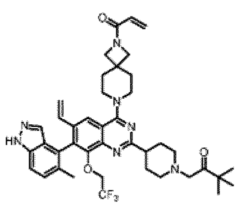 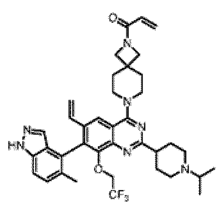 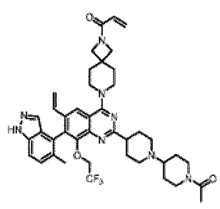

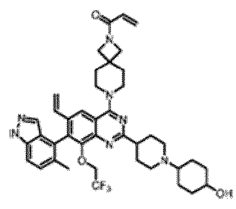 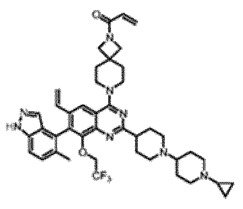 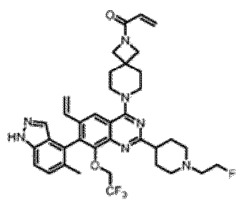 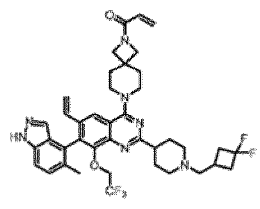

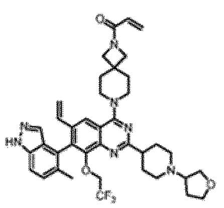 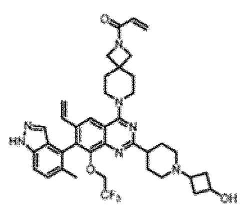

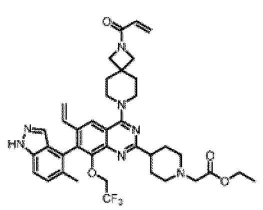 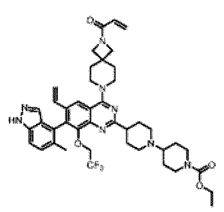 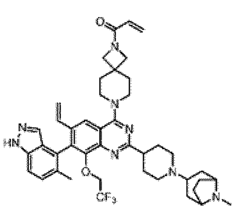 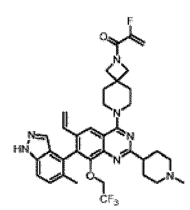

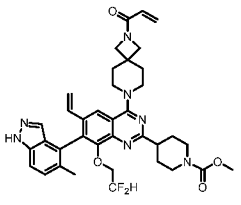 und 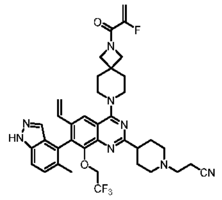 .

11. Pharmazeutische Zusammensetzung, umfassend eine therapeutisch wirksame Menge mindestens einer Verbindung nach einem der Ansprüche 1 bis 10 und mindestens einen pharmazeutisch akzeptablen Träger.

12. Pharmazeutische Zusammensetzung nach Anspruch 11, wobei das Gewichtsverhältnis der Verbindung zu dem pharmazeutisch akzeptablen Träger 0,0001:1-10 beträgt.

13. Verbindung nach einem der Ansprüche 1 bis 10, oder pharmazeutische Zusammensetzung nach Anspruch 11 oder 12 zur Verwendung als ein Medikament.

14. Verbindung nach einem der Ansprüche 1 bis 10, oder pharmazeutische Zusammensetzung nach Anspruch 11 oder 12 zur Verwendung bei der Behandlung und/oder Prävention von Krebs.

15. Verbindung zur Verwendung oder pharmazeutische Zusammensetzung zur Verwendung gemäß Anspruch 14, wobei der Krebs ausgewählt ist aus der Gruppe bestehend aus Brustkrebs, multiplem Myelom, Blasenkrebs, Endometriumkrebs, Magenkrebs, Gebärmutterhalskrebs, Rhabdomyosarkom, nicht-kleinzelligem Lungenkrebs, kleinzelligem Lungenkrebs, pleomorphem Lungenkrebs, Eierstockkrebs, Speiseröhrenkrebs, Melanom, kolorektales Karzinom, hepatozelluläres Karzinom, Kopf- und Halskrebs, hepatobiliäres Zellkarzinom, myelodysplastisches Syndrom, malignes Gliom, Prostatakrebs, Schilddrüsenkrebs, Schwann-Zelltumor, Plattenepithelkarzinom der Lunge, lichenoide Keratose, synoviales Sarkom, Hautkrebs, Bauchspeicheldrüsenkrebs, Hodenkrebs und Liposarkom.

## Revendications

1. Composé de formule (I) ou un tautomère, sel pharmaceutiquement acceptable, solvate, chélate ou complexe non-covalent de celui-ci,

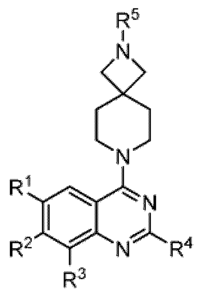

(I)

dans lequel

$R^1$ est choisi dans le groupe constitué par un alkyle en $C_1$ à $C_6$, un alkyle en $C_1$ à $C_6$ substitué, un halogène, un alcényle en $C_2$ à $C_6$, un alcényle en $C_2$ à $C_6$ substitué, un cycloalkyle en $C_3$ à $C_6$ et un cycloalkyle en $C_3$ à $C_6$ substitué ;

$R^2$ est un hétéroaryle à 6 à 10 chaînons ou un hétéroaryle à 6 à 10 chaînons substitué, dans lequel l'hétéroaryle à 6 à 10 chaînons contient indépendamment 1, 2 ou 3 hétéroatomes choisis dans le groupe constitué par N, O et S ;

$R^3$ est choisi dans le groupe constitué par H, amino, cyano, un halogène, hydroxy, un alkyle en $C_1$ à $C_6$, un alkyle en $C_1$ à $C_6$ substitué, un cycloalkyle en $C_3$ à $C_8$, un cycloalkyle en $C_3$ à $C_8$ substitué, un alcoxy en $C_1$ à $C_6$, un alcoxy en $C_1$ à $C_6$ substitué, un cycloalkyloxy en $C_3$ à $C_8$, un cycloalkyloxy en $C_3$ à $C_8$ substitué et un cyclopropyl(alkylène en $C_1$ à $C_6$)oxy substitué par cyano ;

$R^4$ est

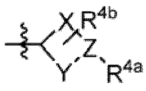;

où X et Y sont indépendamment un alkylène en $C_1$ à $C_2$ ;
Z est choisi dans le groupe constitué par CH, N et O ;
$R^{4a}$ et $R^{4b}$ sont indépendamment absents ou sont choisis dans le groupe constitué par H, un alkyle en $C_1$ à $C_6$, un alkyle en $C_1$ à $C_6$ substitué, un acryloyle, un cycloalkyle en $C_3$ à $C_6$, un cycloalkyle en $C_3$ à $C_6$ substitué, un hétérocyclyle à 3 à 8 chaînons, un hétérocyclyle à 3 à 8 chaînons substitué et un -(C=O) -alkyle en $C_1$ à Ce, dans lequel l'hétérocyclyle à 3 à 8 chaînons contient indépendamment 1, 2 et 3 hétéroatomes indépendamment choisis dans le groupe constitué par N, O et S;
dans lequel l'alkyle en $C_1$ à $C_6$ substitué, l'alcényle en $C_2$ à $C_6$ substitué, le cycloalkyle en $C_3$ à $C_6$ substitué, l'hétéroaryle à 6 à 10 chaînons substitué, l'alcoxy en $C_1$ à $C_6$ substitué, le cycloalkyloxy en $C_3$ à $C_8$ substitué et l'hétérocyclyle à 3 à 8 chaînons substitué sont substitués par un halogène, un alkyle en $C_1$ à $C_8$, un cycloalkyle en $C_3$ à $C_{12}$, - $OR_1$', -$SR_1$', =O, =S, -C(O)$R_1$', -C(S)$R_1$', =$NR_1$',- C(O)ORi', -C(S)$OR_1$', - $NR_1$'$R_2$', -C(O)$NR_1$'$R_2$', cyano, nitro, -S(O)$_2R_1$', -OS($O_2$)$OR_1$', -OS(O)$_2R_1$' ou -OP(O)($OR_1$')($OR_2$'), dans lequel $R_1$' et $R_2$' sont indépendamment choisis parmi -H, un alkyle en $C_1$ à $C_6$ et un halogénoalkyle en $C_1$ à $C_6$ ;
$R^5$ est un acryloyle non substitué ou substitué par halogéno ; ou un composé choisi dans le groupe constitué par :

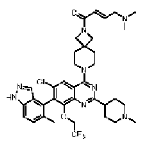, 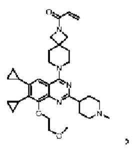, 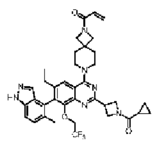, 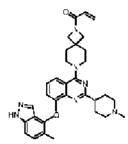, 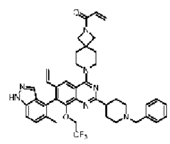

et

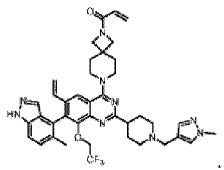.

2. Composé selon la revendication 1, dans lequel $R^1$ est choisi dans le groupe constitué par un alkyle en $C_1$ à $C_3$, un cycloalkyle en $C_3$ à $C_6$, un halogène et un alcényle en $C_2$ à $C_4$; de préférence $R^1$ est choisi dans le groupe constitué par éthyle, cyclopropyle, Cl et vinyle.

3. Composé selon l'une quelconque des revendications 1 et 2, dans lequel $R^2$ est un indazolyle ou un indazolyle substitué par alkyle en $C_1$ à $C_3$ ; de préférence $R^2$ est un indazolyle substitué par méthyle ; mieux encore $R^2$ est

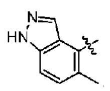.

4. Composé selon l'une quelconque des revendications 1 à 3, dans lequel $R^3$ est choisi dans le groupe constitué par H, un alkyle en $C_1$ à $C_3$, un halogène, un cyclopropyl(alkylène en $C_1$ à $C_3$)oxy substitué par cyano et - $OR^{3a}$, dans lequel $R^{3a}$ est un alkyle en $C_1$ à $C_6$ ou un cycloalkyle en $C_3$ à $C_8$, $R^{3a}$ est indépendamment non substitué ou encore substitué par un ou plusieurs substituants choisis dans le groupe constitué par hydroxyle, un halogène, un alkyle en $C_1$ à $C_3$, un alcoxy en $C_1$ à $C_3$ et cyclopropyle ; de préférence $R^3$ est choisi dans le groupe constitué par H, F, méthyle,

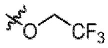, 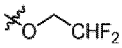, 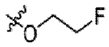, 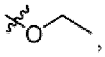, 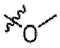, 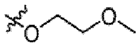, 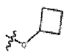, 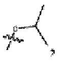, 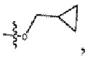, 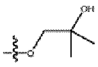

et

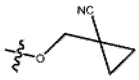;

mieux encore $R^3$ est choisi dans le groupe constitué par H, F, méthyle,

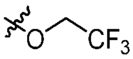

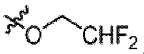, 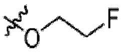, 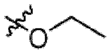, 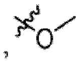, 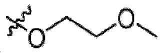, 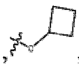, 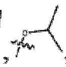, 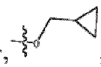, 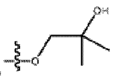

et

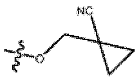.

**5.** Composé selon l'une quelconque des revendications 1 à 4, dans lequel $R^{4a}$ est absent ou est choisi dans le groupe constitué par H, acryloyle, un alkyle en $C_1$ à $C_3$, un cycloalkyle en $C_4$ à $C_6$,

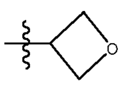, 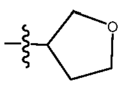, 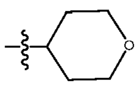, 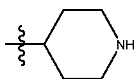 et 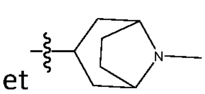

dans lequel l'un quelconque parmi l'alkyle en $C_1$ à $C_3$, un cycloalkyle en $C_4$ à $C_6$,

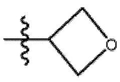, 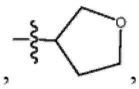, 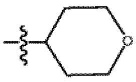, 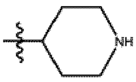 ou 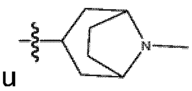

est non substitué ou substitué par un ou plusieurs substituants choisis dans le groupe constitué par un halogène, hydroxyle, cyano, =O, un alkyle en $C_1$ à $C_3$, un $-S(O)_2$-alkyle en $C_1$ à $C_3$, un alcoxy en $C_1$ à $C_3$, un halogénoalkyle en $C_1$ à $C_3$, cyclopropyle, cyclobutyle, un halogénocyclopropyle, un halogénocyclobutyle, phényle, un pyrazolyle substitué par alkyle en $C_1$ à $C_3$, un acyle en $C_1$ à $C_6$ et

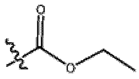;

de préférence $R^{4a}$ est indépendamment absent ou est choisi dans le groupe constitué par H, méthyle, éthyle, propyle, acétyle,

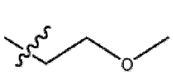, 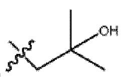, 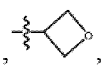,

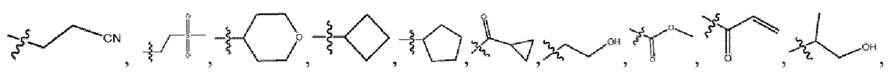

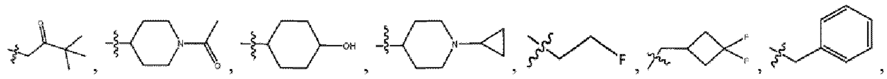

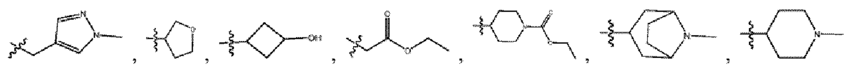

et

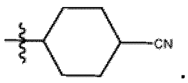

6. Composé selon l'une quelconque des revendications 1 à 5, dans lequel $R^{4b}$ est indépendamment absent, H ou un alkyle en $C_1$ à $C_3$.

7. Composé selon l'une quelconque des revendications 1 à 6, dans lequel $R^4$ est

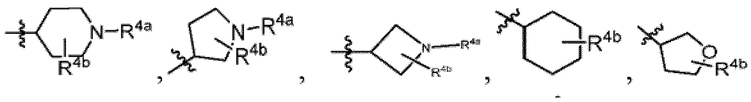

ou

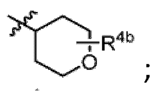

de préférence $R^4$ est

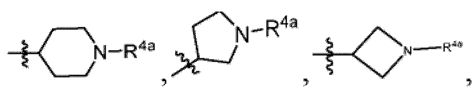

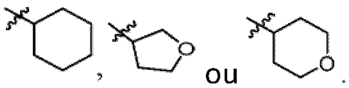

8. Composé selon l'une quelconque des revendications 1 à 7, dans lequel $R^5$ est indépendamment

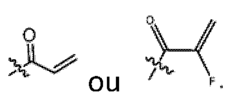

9. Composé selon l'une quelconque des revendications 1 à 8, lequel composé est choisi dans le groupe constitué par les composés de formules (IA) à (ID) :

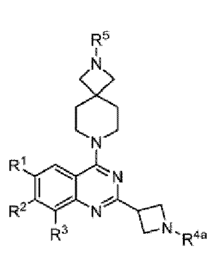
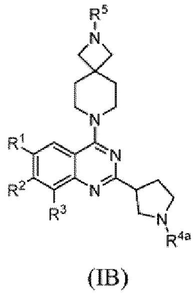
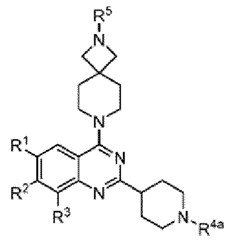
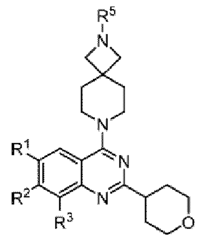

(IA) (IB) (IC) (ID)

dans lequel les substituants sont tels que définis à propos de la formule (I).

**10.** Composé selon la revendication 1, qui est choisi dans le groupe constitué par :

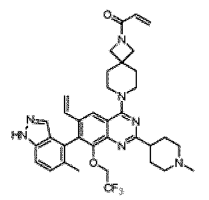
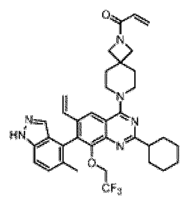
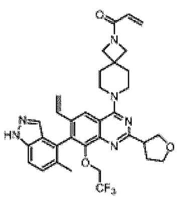
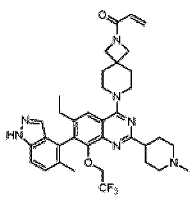
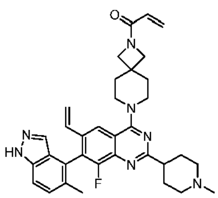
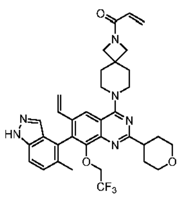
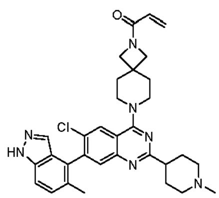
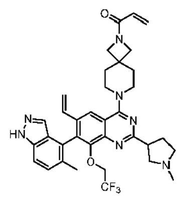
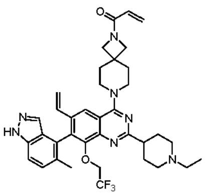
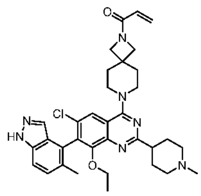
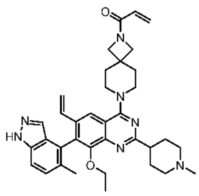
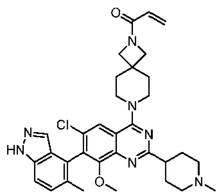
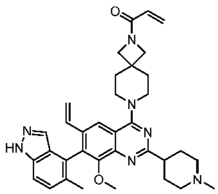
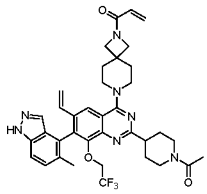
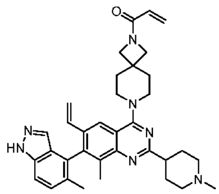
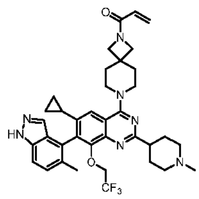
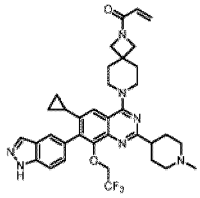
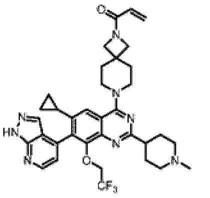
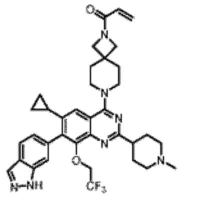
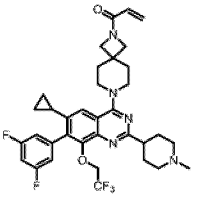

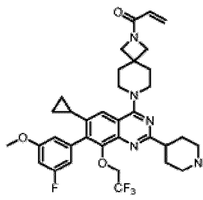
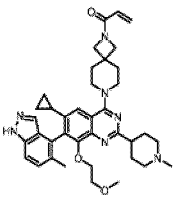
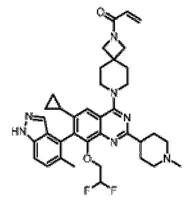

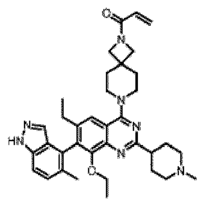
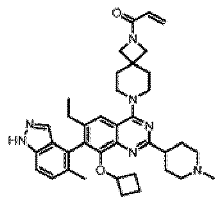
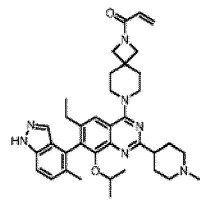
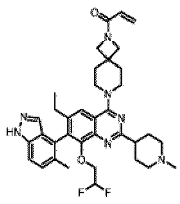

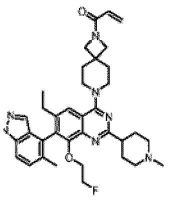
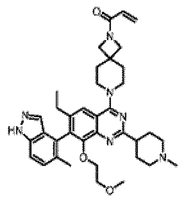
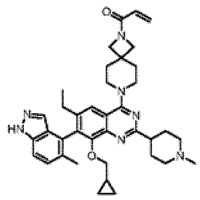
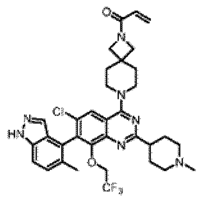

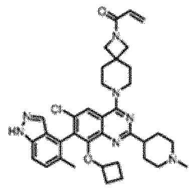
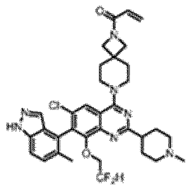
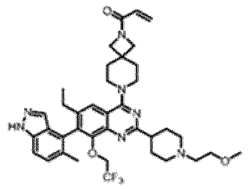
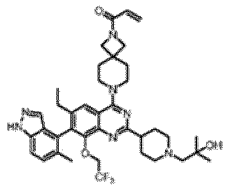

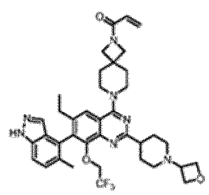
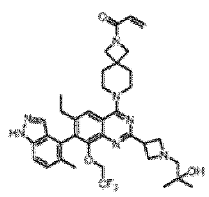
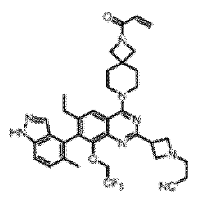
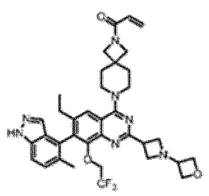

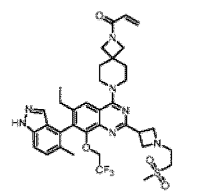
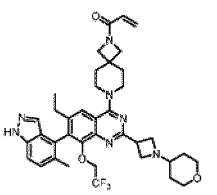
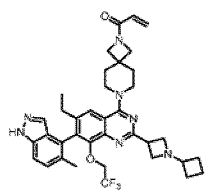
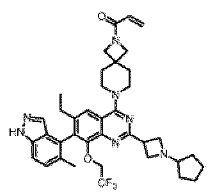

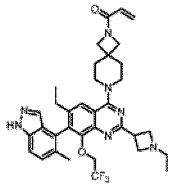
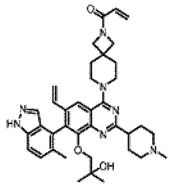
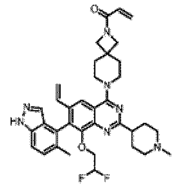

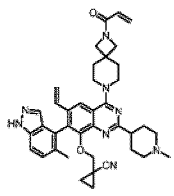
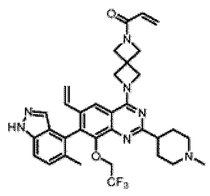
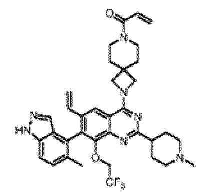
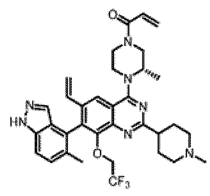

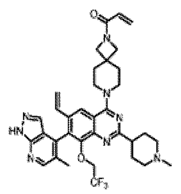
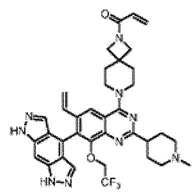
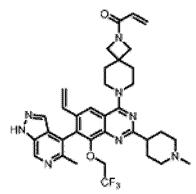

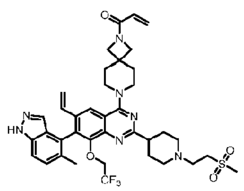
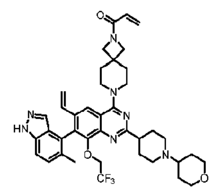
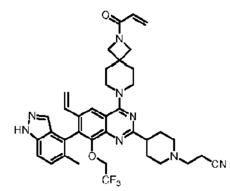
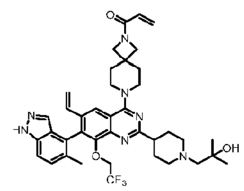

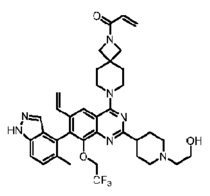
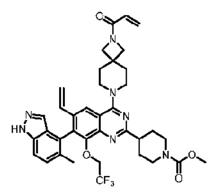
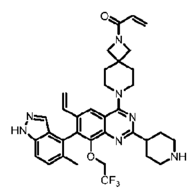
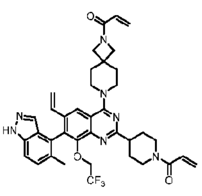

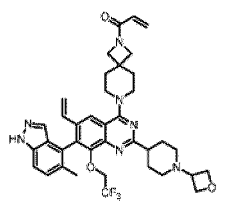
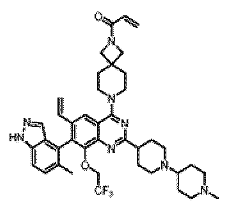
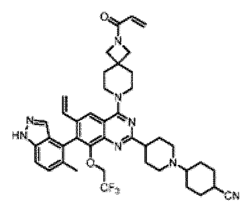
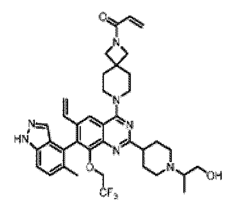

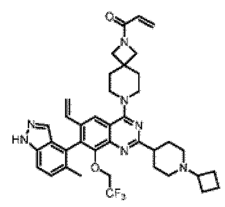
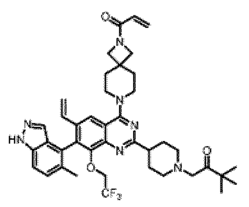
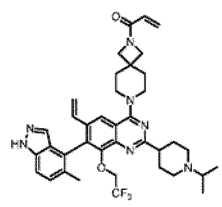
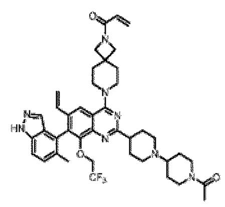

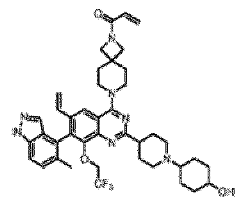
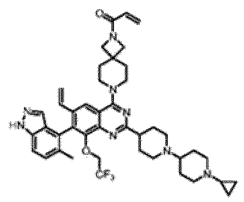
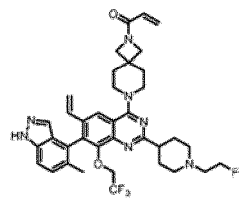
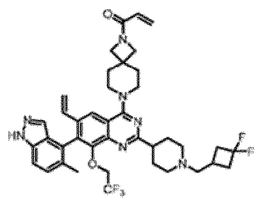

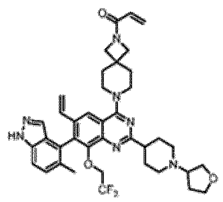

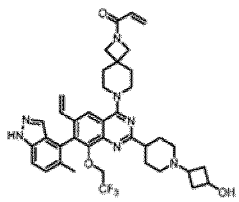

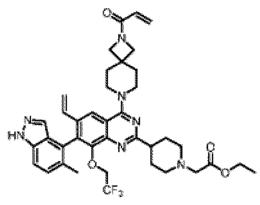

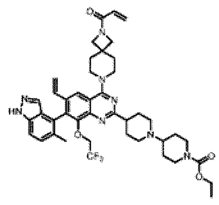

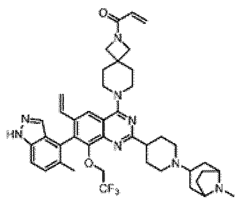

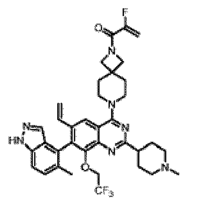

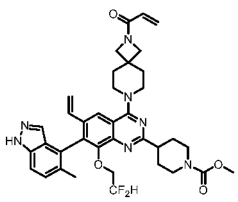

et

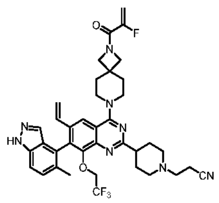

.

**11.** Composition pharmaceutique comprenant une quantité thérapeutiquement efficace d'au moins un composé selon l'une quelconque des revendications 1 à 10 et au moins un véhicule pharmaceutiquement acceptable.

**12.** Composition thérapeutique selon la revendication 11, dans laquelle le rapport en poids du composé au véhicule pharmaceutiquement acceptable est de 0,0001/1 à 10.

**13.** Composé selon l'une quelconque des revendications 1 à 10, ou composition pharmaceutique selon la revendication 11 ou 12, pour une utilisation en tant que médicament.

**14.** Composé selon l'une quelconque des revendications 1 à 10, ou composition pharmaceutique selon la revendication 11 ou 12, pour une utilisation dans le traitement et/ou la prévention d'un cancer.

**15.** Composé pour une utilisation ou composition pharmaceutique pour une utilisation selon la revendication 14, dans lequel le cancer est choisi dans le groupe constitué par un cancer du sein, un myélome multiple, un cancer de la vessie, un cancer de l'endomètre, un cancer gastrique, un cancer du col, un rhabdomyosarcome, un cancer du poumon non à petites cellules, un cancer du poumon à petites cellules, un cancer du poumon pléomorphe, un cancer ovarien, un cancer de l'oesophage, un mélanome, un cancer colorectal, un carcinome hépatocellulaire, un cancer de la tête et du cou, un carcinome à cellules hépatobiliaires, un syndrome myélodysplasique, un gliome malin, un cancer de la prostate, un cancer de la thyroïde, une tumeur à cellules de Schwann, un carcinome pulmonaire à cellules squameuses, une kératose lichénoïde, un sarcome synovial, un cancer de la peau, un cancer du pancréas, un cancer des testicules et un liposarcome.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018143315 A1
- CN 108779097 A
- CN 107849022 A
- CN 106488910 A